# EUROPÄISCHE PATENTANMELDUNG

(11) **EP 0 716 077 A1**
(43) Veröffentlichungstag der Anmeldung: **12.06.1996**
(21) Anmeldenummer: 95810743.5
(22) Anmeldetag: 29.11.1995
(51) Int. Cl.: C07C 237/22, C07D 209/42, C07D 401/04, C07D 213/82, C07C 237/42, C07D 295/13, C07D 295/088, C07D 295/155, C07C 237/36, C07D 295/185, C07D 211/46, C07C 237/08, C07D 257/04, C07D 295/18, C07D 211/60, A61K 31/165, A61K 31/40

(54) **Aromatisch substituierte Omega-Aminoalkansäureamide und Alkansäurediamide und ihre Verwendung als Renininhibitoren**

(30) Priorität: 08.12.1994 CH 3724/94
(71) Anmelder: CIBA-GEIGY AG, CH-4002 Basel (CH)
(72) Erfinder: Maibaum, Jürgen K., Dr., D-79576 Weil-Haltingen (DE); Rigollier, Pascal, Dr., F-68100 Mulhouse (FR); Herold, Peter, Dr., CH-4144 Arlesheim (CH); Cohen, Nissim C., Dr., F-68300 Village-Neuf (FR); Göschke, Richard, Dr., CH-4103 Bottmingen (FR); Stutz, Stefan, CH-4053 Basel (CH)

(57) **Zusammenfassung**

Verbindungen der Formel I 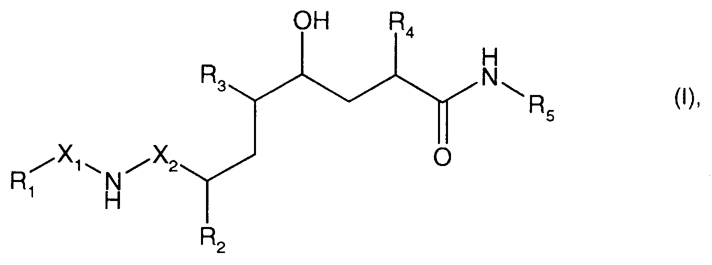
worin R₁ einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-lndol-3-ylrest bedeutet, wobei
einer der Reste R_{A} und R_{B} einen aliphatischen oder hetero cycloaliphatisch-aliphatischen Rest oder gegebenenfalls aliphatisch, araliphatisch oder heteroaraliphatisch verethertes Hydroxy und der andere Wasserstoff, einen aliphatischen Rest oder gegebenenfalls verestertes oder amidiertes Carboxy darstellt, R_{C} Wasserstoff, einen aliphatischen Rest, gegebenenfalls aliphatisch, araliphatisch, heteroarylaliphatisch oder heterocycloaliphatischaliphatisch verethertes Hydroxy oder eine gegebenenfalls heteroaliphatisch substituierte Aminogruppe ist und R_{D} für einen aliphatischen, araliphatischen oder heteroaliphatischen Rest steht, einer der Reste X₁ und X₂ Carbonyl und der andere Methylen darstellt, R₂ ein aliphatischer Rest ist, R₃ gegebenenfalls aliphatisch substituiertes Amino darstellt, R₄ einen aliphatischen oder araliphatischen Rest bedeutet und R₅ einen aliphatischen oder cycloaliphatisch-aliphatischen Rest oder einen gegebenenfalls hydrierten und/oder oxosubstituierten Heteroarylrest oder einen durch einen über ein C-Atom gebundenen und gegebenenfalls hydrierten und/oder oxosubstituierten Heteroaryl- oder Heteroaliphatylrest substituierten Niederalkylrest darstellt,und ihre Salze haben reninhemmende Eigenschaften und können als antihypertensive Arzneimittelwirkstoffe verwendet werden.

## Beschreibung

Die Erfindung betrifft Verbindungen der Formel I 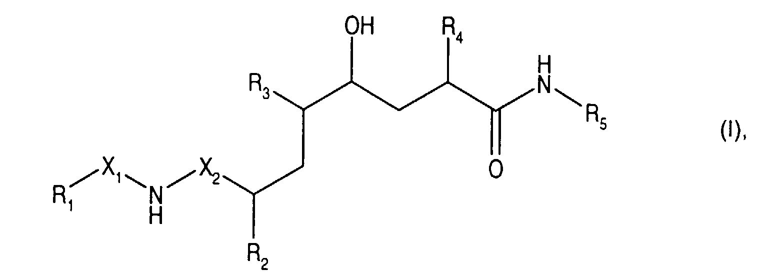 worin

R₁ einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-Indol-3-ylrest bedeutet, wobei
einer der Reste R_{A} und R_{B} einen aliphatischen oder hetero cycloaliphatisch-aliphatischen Rest oder gegebenenfalls aliphatisch, araliphatisch oder heteroaraliphatisch verethertes Hydroxy und der andere Wasserstoff, einen aliphatischen Rest oder gegebenenfalls verestertes oder amidiertes Carboxy darstellt,
R_{C} Wasserstoff, einen aliphatischen Rest, gegebenenfalls aliphatisch, araliphatisch, heteroarylaliphatisch oder heterocycloaliphatisch-aliphatisch verethertes Hydroxy oder eine gegebenenfalls heteroaliphatisch substituierte Aminogruppe ist und
R_{D} für einen aliphatischen, araliphatischen oder heteroaliphatischen Rest steht, einer der Reste X₁ und X₂ Carbonyl und der andere Methylen darstellt,
R₂ ein aliphatischer Rest ist,
R₃ gegebenenfalls aliphatisch substituiertes Amino darstellt,
R₄ einen aliphatischen oder araliphatischen Rest bedeutet und
R₅ einen aliphatischen oder cycloaliphatisch-aliphatischen Rest oder einen gegebenenfalls hydrierten und/oder oxosubstituierten Heteroarylrest oder einen durch einen über ein C-Atom gebundenen und gegebenenfalls hydrierten und/oder oxosubstituierten Heteroaryl- oder Heteroaliphatylrest substituierten Niederalkylrest darstellt,
und ihre Salze, Verfahren zur Herstellung der erfindungsgemäßen Verbindungen, diese enthaltende pharmazeutische Präparate und ihre Verwendung als Arzneimittelwirkstoffe. Vorstehend haben die Allgemeinbegriffe vorzugsweise die folgenden Bedeutungen:

Aliphatischen Reste sind beispielsweise Niederalkyl, Hydroxyniederalkyl, Niederalkanoyloxyniederalkyl, Niederalkoxyniederalkyl, Niederalkoxyniederalkoxyniederalkyl, Niederalkoxyniederalkoxyniederalkyl, eine gegebenenfalls amidierte Carboxy- oder Carboxyniederalkylgruppe, gegebenenfalls verestertes oder amidiertes Dicarboxynieder alkyl, gegebenenfalls verestertes oder amidiertes Carboxy(hydroxy)niederalkyl, Niederalkansulfonylniederalkyl oder gegebenenfalls N-mono- oder N,N-diniederalkyliertes Sulfamoylniederalkyl.

Gegebenenfalls aliphatisch, araliphatisch, heterocycloaliphatisch-aliphatisch oder heteroarylaliphatisch verethertes Hydroxy ist beispielsweise Hydroxy, Niederalkoxy, Hydroxyniederalkoxy, Niederalkanoyloxyniederalkoxy, Niederalkoxyniederalkoxy, Niederalkoxyniederalkoxyniederalkoxy, Polyhalogenniederalkoxy, Cyanniederalkoxy, ein gegebenenfalls N-niederalkanoylierter, N-mono- oder N,N-diniederalkylierter oder durch Niederalkylen, Hydroxy-, Niederalkoxy- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituierter Aminoniederalkoxyrest, gegebenenfalls substituiertes Phenyl- oder Pyridylniederalkoxy, gegebenenfalls amidiertes Carboxy- oder Carboxyniederalkoxy oder Tetrazolylniederalkoxy.

Heteroaliphatische Reste sind beispielsweise gegebenenfalls N-niederalkanoylierte, N-mono- oder N,N-diniederalkylierte oder durch Niederalkylen, Hydroxy-, Niederalkoxy- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituierte Aminoniederalkylreste oder N-mono- oder N,N-diniederalkylierte Thiocarbamoylniederalkylreste.

Araliphatische oder heteroaliphatischen Reste sind beispielsweise gegebenenfalls substituierte Phenyl- oder Pyddylniederalkylgruppen.

Cycloaliphatisch-aliphatische Reste sind beispielsweise Cycloalkyl niederalkyl oder gegebenenfalls verestertes oder amidiertes Carboxycyloalkylniederalkyl.

Gegebenenfalls aliphatisch substituiertes Amino ist beispielsweise gegebenenfalls N-niederalkanoyliertes oder N-mono- oder N,N-diniederalkyliertes Amino.

Gegebenenfalls heteroaliphatisch substituiertes Amino ist beispielsweise gegebenenfalls N-niederalkanoyliertes, N-mono- oder N,N-diniederalkyliertes oder durch Niederalkylen, Hydroxy-, Niederalkoxy-, Niederalkoxycarbonyl- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Amino.

Gegebenenfalls verestertes oder amidiertes Carboxy ist beispielsweise gegebenenfalls aliphatisch oder araliphatisch verethertes Carboxy oder aliphatisch substituiertes Carbamoyl.

Als Substituenten von Phenyl, Phenylniederalkoxy, Pyridylniederalkyl, Pyridylniederalkoxy und gegebenenfalls hydriertem und/oder oxosubstituierten Heteroaryl oder kommen beispielsweise Niederalkyl, Niederalkoxy, Hydroxy, Nitro, Amino, Niederalkylamino, Diniederalkylamino, Halogen und Trifluormethyl in Betracht, wobei bis zu 3, insbesondere 1 oder 2 gleiche oder verschiedene der genannten Substituenten vorhanden sein können.

Gegebenenfalls N-niederalkanoyliertes, N-mono- oder N,N-diniederalkyliertes oder durch Niederalkylen, Hydroxy-, Niederalkoxy-, Niederalkoxycarbonyl- oder Niederalkoxyniederalkoxyniederalkylen, gegebenenfalls N-niederalkyliertes, N-niederalkanoyliertes oder durch Niederalkoxycarbonyl oder Niederalkoxyniederalkyl N'-substituiertes Azaniederalkylen, Oxaniederalkylen oder gegebenenfalls S-oxidiertes Thianiederalkylen N,N-disubstituiertes Amino ist beispielsweise Amino, Niederalkanoylamino, Niederalkylamino, Diniederalkylamino, eine gegebenenfalls durch Hydroxy, Niederalkoxy oder Niederalkoxyniederalkyl substituierte Piperidino- oder Pyrrolidinogruppe, wie Piperidino, Hydroxypiperidino, Niederalkoxypiperidino, Niederalkoxyniederalkoxyperidino, Niederalkoxycarbonylpiperidino, Pyrrolidino, Hydroxypyrrolidino, Niederalkoxypyrrolidino, Niederalkoxyniederalkoxypyrrolidino, gegebenenfalls N'-niederalkyliertes, N'-niederalkanoyliertes oder durch Niederalkoxycarbonyl oder Niederalkoxyniederalkyl N'-substituiertes Piperazino, wie Piperazino, N'-Niederalkylpiperazino, N'-Niederalkanoylpiperazino, N'-Niederalkoxycarbonylpiperazino oder N'-Niederalkoxyniederalkylpiperazino, gegebenenfalls niederalkyliertes Morpholino, wie Morpholino oder Niederalkylmorpholino, oder gegebenenfalls S-oxidiertes Thiomorpholino, wie Thiomorpholino, S-Oxythiomorpholino oder S,S-Dioxythiomorpholino.

Gegebenenfalls N-niederalkanoyliertes, N-mono- oder N,N-diniederalkyliertes oder durch Niederalkylen, Hydroxy-, Niederalkoxy-, Niederalkoxycarbonyl- oder Niederalkoxyniederalkoxyniederalkylen, gegebenenfalls N-niederalkyliertes, N-niederalkanoyliertes oder durch Niederalkoxycarbonyl oder Niederalkoxyniederalkyl N'-substituiertes Azaniederalkylen, Oxaniederalkylen oder gegebenenfalls S-oxidiertes Thianiederalkylen N,N-disubstituiertes Aminoniederalkyl ist beispielsweise Aminoniederalkyl, Niederalkanoylaminoniederalkyl, Niederalkylaminoniederalkyl, Diniederalkylaminoniederalkyl, gegebenenfalls durch Hydroxy, Niederalkoxy oder Niederalkoxyniederalkyl substituiertes Piperidino- oder Pyrrolidinoniederalkyl, wie Piperidinoniederalkyl, Hydroxypiperidinoniederalkyl, Niederalkoxypiperidinoniederalkyl, Niederalkoxyniederalkoxyperidinoniederalkyl, Niederalkoxycarbonylpiperidinoniederalkyl, Pyrrolidinoniederalkyl, Hydroxypyrrolidinoniederalkyl, Niederalkoxypyrrolidinoniederalkyl, Niederalkoxyniederalkoxypyrrolidinoniederalkyl, gegebenenfalls N'-niederalkyliertes, N'-niederalkanoyliertes oder durch Niederalkoxycarbonyl oder Niederalkoxyniederalkyl N'-substituiertes Piperazinoniederalkyl, wie Piperazinoniederalkyl, N'-Niederalkylpiperazinoniederalkyl, N'-Niederalkanoylpiperazinoniederalkyl, N'-Niederalkoxycarbonylpiperazinoniederalkyl oder N'-Niederalkoxyniederalkylpiperazinoniederalkyl, gegebenenfalls niederalkyliertes Morpholinoniederalkyl, wie Morpholinoniederalkyl oder Niederalkylmorpholinoniederalkyl, oder gegebenenfalls S-oxidiertes Thiomorpholinoniederalkyl, wie Thiomorpholinoniederalkyl, S-Oxythiomorpholinoniederalkyl oder S,S-Dioxythiomorpholinoniederalkyl.

Gegebenenfalls N-niederalkanoyliertes, N-mono- oder N,N-diniederalkyliertes oder durch Niederalkylen, Hydroxy-, Niederalkoxy- oder Niederalkoxyniederalkoxyniederalkylen, gegebenenfalls N-niederalkyliertes, N-niederalkanoyliertes oder durch Niederalkoxycarbonyl oder Niederalkoxyniederalkyl N'-substituiertes Azaniederalkylen, Oxaniederalkylen oder gegebenenfalls S-oxidiertes Thianiederalkylen N,N-disubstituiertes Aminoniederalkoxy ist beispielsweise Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Niederalkoxyniederalkyl substituiertes Piperidino- oder Pyrrolidinoniederalkoxy, wie Piperidinoniederalkoxy, Hydroxypiperidinoniederalkoxy, Nieder alkoxypiperidinoniederalkoxy, Niederalkoxyniederalkoxyperidinoniederalkoxy, Pyrrolidinoniederalkoxy, Hydroxypyrrolidinoniederalkoxy, Niederalkoxypyrrolidinoniederalkoxy, Niederalkoxyniederalkoxypyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, wie Piperazinoniederalkoxy, N'-Niederalkylpiperazinoniederalkoxy, N'-Niederalkanoylpiperazinoniederalkoxy, N'-Niederalkoxycarbonylpiperazinoniederalkoxy oder N'-Niederalkoxyniederalkylpiperazinoniederalkoxy, gegebenenfalls niederalkyliertes Morpholinoniederalkoxy, wie Morpholinoniederalkoxy oder Niederalkylmorpholinoniederalkoxy, oder gegebenenfalls S-oxidiertes Thiomorpholinoniederalkoxy, wie Thiomorpholinoniederalkoxy, S-Oxythiomorpholinoniederalkoxy oder S,S-Dioxythiomorpholinoniederalkoxy.

Gegebenenfalls S-oxidiertes Niederalkylthioniederalkoxy ist beispielsweise Niederalkylthioniederalkoxy, Niederalkansulfinylniederalkoxy oder Niederalkansulfonylniederalkoxy.

Gegebenenfalls amidiertes Carboxy ist beispielsweise Carboxy, Carbamoyl, Niederalkylcarbamoyl, Diniederalkylcarbamoyl, gegebenenfalls durch Hydroxy, Niederalkoxy oder Niederalkoxyniederalkyl substituiertes Piperidino- oder Pyrrolidi nocarbonyl, wie Piperidinocarbonyl, Hydroxypiperidinocarbonyl, Niederalkoxypiperidinocarbonyl, Niederalkoxyniederalkoxyperidinocarbonyl, Pyrrolidinocarbonyl, Hydroxypyrrolidinocarbonyl, Niederalkoxypyrrolidinocarbonyl, Niederalkoxyniederalkoxypyrrolidinocarbonyl, gegebenenfalls N'-niederalkyliertes, N'-niederalkanoyliertes oder durch Niederalkoxycarbonyl oder Niederalkoxyniederalkyl N'-substituiertes Piperazinocarbonyl, wie Piperazinocarbonyl, N'-Niederalkylpiperazinocarbonyl, N'-Niederalkanoylpiperazinocarbonyl, N'-Niederalkoxycarbonylpiperazinocarbonyl oder N'-Niederalkoxyniederalkylpiperazinocarbonyl, gegebenenfalls niederalkyliertes Morpholinocarbonyl, wie Morpholinocarbonyl oder Niederalkylmorpholinocarbonyl, oder gegebenenfalls S-oxidiertes Thiomorpholinocarbonyl, wie Thiomorpholinocarbonyl, S-Oxythiomorpholinocarbonyl oder S,S-Dioxy thiomorpholinocarbonyl.

Gegebenenfalls verestertes Carboxy ist beispielsweise Carboxy oder Niederalkoxycarbonyl.

Gegebenenfalls amidiertes Carboxyniederalkoxy ist beispielsweise Carboxyniederalkoxy, Carbamoylniederalkoxy, Niederalkylcarbamoylniederalkoxy, Diniederalkyl carbamoylniederalkoxy, gegebenenfalls durch Hydroxy, Niederalkoxy oder Niederalkoxyniederalkyl substituiertes Piperidino- oder Pyrrolidinocarbonylniederalkoxy, wie Piperidinocarbonylniederalkoxy, Hydroxypiperidinocarbonylniederalkoxy, Niederalkoxypiperidinocarbonylniederalkoxy, Niederalkoxyniederalkoxyperidinocarbonyiniederalkoxy, Pyrrolidinocarbonylniederalkoxy, Hydroxypyrrolidinocarbonylniederalkoxy, Niederalkoxypyrrolidinocarbonylniederalkoxy, Niederalkoxyniederalkoxypyrrolidinocarbonylniederalkoxy, gegebenenfalls N'-niederalkyliertes, N'-niederalkanoyliertes oder durch Niederalkoxycarbonyl oder Niederalkoxyniederalkyl N'-substituiertes Piperazinocarbonylniederalkoxy, wie Piperazinocarbonylniederalkoxy, N'-Niederalkylpiperazinocarbonylniederalkoxy, N'-Niederalkanoyl piperazinocarbonylniederalkoxy, N'-Niederalkoxycarbonylpiperazinocarbonyl oder N'-Niederalkoxyniederalkylpiperazinocarbonylni ederalkoxy, gegebenenfalls niederalkyliertes Morpholinocarbonylniederalkoxy, wie Morpholinocarbonylniederalkoxy oder Niederalkylmorpholinocarbonylniederalkoxy, oder gegebenenfalls S-oxidiertes ThiomorpholinocarbonyIniederalkoxy, wie Thiomorpholinocarbonylniederalkoxy, S-Oxythiomorpholinocarbonyl oder S,S-Dioxythiomorpholinocarbonylniederalkoxy.

Gegebenenfalls amidiertes Carboxyniederalkyl ist beispielsweise Carboxyniederalkyl, Carbamoylniederalkyl, Niederalkylcarbamoylniederalkyl, Diniederalkylcarbamoylniederalkyl, gegebenenfalls durch Hydroxy, Niederalkoxy oder Niederalkoxyniederalkyl substituiertes Piperidino- oder Pyrrolidinocarbonylniederalkyl, wie Piperidinocarbonylniederalkyl, Hydroxypiperidinocarbonylniederalkyl, Niederalkoxypiperidinocarbonyiniederalkyl, Niederalkoxyniederalkoxyperidinocarbonylniederalkyl, Pyrrolidinocarbonylniederalkyl, Hydroxypyrrolidinocarbonylniederalkyl, Niederalkoxypyrrolidinocarbonylniederalkyl, Nieder alkoxyniederalkoxypyrrolidinocarbonylniederalkyl, gegebenenfalls N'-niederalkyliertes, N'-niederalkanoyliertes oder durch Niederalkoxycarbonyl oder Niederalkoxyniederalkyl N'-substituiertes Piperazinocarbonylniederalkyl, wie Piperazinocarbonylniederalkyl, N'-Niederalkylpiperazinocarbonylniederalkyl, N'-Niederalkanoylpiperazinocarbonylniederalkyl, N'-Niederalkoxycarbonylpiperazinocarbonyl oder N'-Niederalkoxyniederalkylpiperazinocarbonylniederalkyl, gegebenenfalls niederalkyliertes Morpholinocarbonyl niederalkyl, wie Morpholinocarbonylniederalkyl oder Niederalkylmorpholinocarbonylniederalkyl, oder gegebenenfalls S-oxidiertes Thiomorpholinocarbonylniederalkyl, wie Thiomorpholinocarbonylniederalkyl, S-Oxythiomorpholinocarbonylniederalkyl oder S,S-Dioxythiomorpholinocarbonylniederalkyl.

Gegebenenfalls verestertes Carboxyniederalkyl ist beispielsweise Carboxyniederalkyl oder Niederalkoxycarbonylniederalkyl.

Gegebenenfalls N-niederalkanoyliertes oder N-mono- oder N,N-diniederalkyliertes Amino ist beispielsweise Amino, Niederalkanoylamino, Niederalkylamino oder Diniederalkylamino.

Gegebenenfalls verestertes oder amidiertes Dicarboxyniederalkyl ist beispielsweise Dicarboxyniederalkyl, Niederalkoxycarbonyl(carboxy)niederalkyl, Diniederalkoxycarbonylniederalkyl, Dicarbamoylniederalkyl, Carbamoyl(carboxy)niederalkyl, Di(niederalkylcarbamoyl)niederalkyl oder Di(diniederalkylcarbamoyl)niederalkyl.

Gegebenenfalls verestertes oder amidiertes Carboxy(hydroxy)niederalkyl ist beispielsweise Carboxy(hydroxy)niederalkyl, Niederalkoxycarbonyl(hydroxy)niederalkyl, Carbamoyl(hydroxy)niederalkyl, Niederalkylcarbamoyl(hydroxy)niederalkyl oder Diniederalkylcarbamoyl(hydroxy)niederalkyl.

Gegebenenfalls verestertes oder amidiertes Carboxycyloalkylniederalkyl ist beispielsweise Carboxycydoalkylniederalkyl, Niederalkoxycarbonylcydoalkylniederalkyl, Carbamoylcycloalkylniederalkyl, Niederalkylcarbamoylcycloalkylniederalkyl oder Diniederalkylcarbamoylcydoalkylniederalkyl.

Gegebenenfalls N-mono- oder N,N-diniederalkyliertes Thiocarbamoylniederalkyl ist beispielsweise Thiocarbamoylniederalkyl, N-Niederalkylthiocarbamoylniederalkyl oder N,N-Diniederalkylthiocarbamoylniederalkyl.

Gegebenenfalls N-mono- oder N,N-diniederalkyliertes Sulfamoylniederalkyl ist beispielsweise Sulfamoylniederalkyl, Niederalkylsulfamoylniederalkyl oder Diniederalkylsulfamoylniederalkyl.

Gegebenenfalls hydrierte und/oder oxosubstituierte Heteroarylreste sind beispielsweise gegebenenfalls partiell hydrierte und/oder benzokondensierte 5-gliedrigen Aza-, Diaza-, Triaza-, Oxadiaza- oder Tetraaza-aryl- oder 6-gliedriges Aza- oder Diaza-arylreste, wie gegebenenfalls oxosubstituiertes Pyrrolidinyl, z.B. Pyrrolidinyl oder Oxopyrrolidinyl, Imidazolyl, z.B. Imidazol-4-yl, Benzimidazolyl, z.B. Benzimidazol-2-yl, Oxadiazolyl, z.B. 1,2,4-Oxadiazol-5-yl, Pyridyl, z.B. Pyridin-2-yl, Oxopiperidinyl, Dioxopiperidinyl, Oxothiazolyl, Oxo-oxazolinyl oder Chinolinyl, z.B. Chinolin-2-yl, oder gegebenenfalls N-niederalkanoyliertes Piperidyl, wie 1-Niederalkanoylpiperidinyl.

Durch einen über ein C-Atom gebundenen und gegebenenfalls hydrierten und/oder oxosubstituierten Heteroarylrest substituiertes Niederalkyl weist als gegebenenfalls hydrierten Heteroarylrest beispielsweise einen gegebenenfalls partiell hydrierten und/oder benzokondensierten 5-gliedrigen Aza-, Diaza-, Triaza-, Oxadiaza- oder Tetraaza-arylrest oder 6-gliedrigen Aza- oder Diaza-arylrest auf und ist beispielsweise gegebenenfalls oxosubstituiertes Pyrrolidinylniederalkyl, z.B. Pyrrolidinylniederalkyl oder Oxopyrrolidinylniederalkyl, Imidazolylniederalkyl, Benzimidazolylniederalkyl, Oxadiazolylniederalkyl, Pyridylniederalkyl, Oxopiperidinylniederalkyl, Dioxopiperidinylniederalkyl, Oxothiazolylniederalkyl, Oxo-oxazolinylniederalkyl oder Chinolinylniederalkyl, ebenso Morpholinocarbonylniederalkyl oder gegebenenfalls N-niederalkanoyliertes Piperidyl niederalkyl, wie 1-Niederalkanoylpiperidinylniederalkyl.

Aminoniederalkoxy ist beispielsweise Amino-C₁-C₇-alkoxy, wie 2-Aminoethoxy, 3-Aminopropyloxy, 4-Aminobutyloxy oder 5-Aminopentyloxy.

Aminoniederalkyl ist beispielsweise Amino-C₁-C₄-alkyl, wie 2-Aminoethyl, 3-Aminopropyl oder 4-Aminobutyl.

Benzimidazolylniederalkyl ist beispielsweise Benzimidazolyl-C₁-C₄-alkyl, wie Benzimidazolylmethyl, 2-Benzimidazolylethyl, 3-Benzimidazolylpropyl oder 4-Benzimidazolylbutyl.

Carbamoyl(carboxy)niederalkyl ist beispielsweise Carbamoyl(carboxy)-C₁-C₇-alkyl, vor allem Carbamoyl(carboxy)-C₂-C₇-alkyl, wie 2-Carbamoyl-1-Carboxyethyl, 1-Carbamoyl-2-carboxyethyl, 3-Carbamoyl-2-carboxy-propyl oder 2-Carbamoyl-3-carboxy-propyl.

Carbamoyl(hydroxy)niederalkyl ist beispielsweise Carbamoyl-C₁-C₄-(hydroxy)alkyl, wie 1-Carbamoyl-2-hydroxyethyl.

Carbamoylcydoalkylniederalkyl weist beispielsweise 3 bis 8, insbesondere 5 bis 7 Ringglieder auf und ist beispielsweise Carbamoylcyclopentyl-, Carbamoylcyclohexyl- oder Carbamoylcycloheptylmethyl.

Carbamoylniederalkoxy ist beispielsweise Carbamoyl-C₁-C₇-alkoxy, wie Carbamoylmethoxy, 2-Carbamoylethoxy, 3-Carbamoylpropyloxy, 2-(3-Carbamoyl)propyloxy, 2-Carbamoyl propyloxy, 3-(1-Carbamoyl)propyloxy, 2-(2-Carbamoyl)propyloxy, 2-(Carbamoyl-2-methyl)propyloxy, 4-Carbamoylbutyloxy, 1-Carbamoylbutyloxy, 1-(1-Carbamoyl-2-methyl)butyloxy, 3-(4-Carbamoyl-2-methyl)butyloxy, insbesondere 3-Carbamoylpropyloxy oder 2-Carbamoyl-2-methyl-ethoxy.

Carbamoylniederalkyl ist beispielsweise Carbamoyl-C₁-C₇-alkyl, wie Carbamoylmethyl, 2-Carbamoylethyl, 3-Carbamoylpropyl, 2-(3-Carbamoyl)propyl, 2-Carbamoylpropyl, 3-(1-Carbamoyl)propyl, 2-(2-Carbamoyl)propyl, 2-(Carbamoyl-2-methyl)propyl, 4-Carbamoyl butyl, 1-Carbamoylbutyl, 1-(1-Carbamoyl-2-methyl)butyl, 3-(4-Carbamoyl-2-methyl)butyl, insbesondere 3-Carbamoylpropyl oder 2-Carbamoyl-2-methyl-ethyl.

Carboxycyloalkylniederalkyl weist beispielsweise 3 bis 8, insbesondere 5 bis 7 Ringglieder auf und ist beispielsweise Carboxycyclopentyl-, Carboxycyclohexyl- oder Carboxycycloheptylmethyl.

Carboxy(hydroxy)niederalkyl ist beispielsweise Carboxy-C₁-C₇-(hydroxy)alkyl, wie 1-Carboxy-2-hydroxy-ethyl.

Carboxyniederalkoxy ist beispielsweise Carboxy-C₁-C₄-alkoxy, wie Carboxymethoxy, 2-Carboxyethoxy, 2- oder 3-Carboxypropyloxy, 2-Carboxy-2-methyl-propyloxy, 2-Carboxy-2-ethyl-butyloxy oder 4-Carboxybutyloxy, insbesondere Carboxymethoxy.

Carboxyniederalkyl ist beispielsweise Carboxy-C₁-C₄-alkyl, wie Carboxymethyl, 2-Carboxyethyl, 2- oder 3-Carboxypropyl, 2-Carboxy-2-methyl-propyl, 2-Carboxy-2-ethyl-butyl oder 4-Carboxybutyl, insbesondere Carboxymethyl.

Chinolinylniederalkyl ist beispielsweise Chinolinyl-C₁-C₄-alkyl, wie Chinolinylmethyl, 2-Chinolinylethyl, oder 3-Chinolinylpropyl, insbesondere Chinolinylmethyl.

Cyanniederalkoxy ist beispielsweise Cyano-C₁-C₄-alkoxy, wie Cyanomethoxy, 2-Cyanoethoxy, 2- oder 3-Cyanopropyloxy, 2-Cyano-2-methyl-propyloxy, 2-Cyano-2-ethylbutyloxy oder 4-Cyanobutyloxy, insbesondere Cyanomethoxy.

Cyanoniederalkyl ist beispielsweise Cyano-C₁-C₄-alkyl, wie Cyanomethyl, 2-Cyanoethyl, 2-oder 3-Cyanopropyl, 2-Cyano-2-methyl-propyl, 2-Cyano-2-ethyl-butyl oder 4-Cyanobutyl, insbesondere Cyanomethyl.

Cycloalkylniederalkyl weist beispielsweise 3 bis 8, insbesondere 5 bis 7 Ringglieder auf und ist beispielsweise Cyclopentyl, Cyclohexyl oder Cycloheptyl, ferner Cyclopropyl, Cyclobutyl oder Cyclooctyl.

Di(diniederalkylcarbamoyl)niederalkyl ist beispielsweise Di-(Di-C₁-C₄-alkylcarbamoyl)-C₁-C₄-alkyl, wie 1 ,2-Di-(Di-C₁-C₄-alkylcarbamoyl)ethyl oder 1,3-Di-(Di-C₁-C₄-alkyl)carbamoyl)propyl, worin C₁-C₄-Alkyl beispielsweise Methyl, Ethyl oder Propyl ist.

Di(niederalkylcarbamoyl)niederalkyl ist beispielsweise Di-(C₁-C₄-alkylcarbamoyl)-C₁-C₄-alkyl, wie 1,2-Di-(C₁-C₄-alkylcarbamoyl)ethyl oder 1,3-Di-(C₁-C₄-alkylcarbamoyl)propyl, worin C₁-C₄-Alkyl beispielsweise Methyl, Ethyl oder Propyl ist.

Dicarbamoylniederalkyl ist beispielsweise Dicarbamoyl-C₁-C₄-alkyl, wie 1,2-Dicarbamoylethyl oder 1,3-Dicarbamoylpropyl.

Dicarboxyniederalkyl ist beispielsweise Dicarboxy-C₁-C₄-alkyl, wie 1,2-Dicarboxyethyl oder 1,3-Dicarboxypropyl.

Diniederalkylaminoniederalkoxy ist beispielsweise N,N-Di-C₁-C₄-alkylamino-C₁-C₄-alkoxy wie 2-Dimethylaminoethoxy, 3-Dimethylaminopropyloxy, 4-Dimethylaminobutyloxy, 2-Diethylaminoethoxy, 2-(N-Methyl-N-ethyl-amino)ethoxy oder 2-(N-Butyl-N-methylamino)ethoxy, insbesondere 3-Dimethylaminopropyloxy.

Diniederalkylaminoniederalkyl ist beispielsweise N,N-Di-C₁-C₄-alkylamino-C₁-C₄-alkyl, wie 2-Dimethylaminoethyl, 3-Dimethylaminopropyl, 4-Dimethylaminobutyl, 2-Diethylaminoethyl, 2-(N-Methyl-N-ethyl-amino)ethyl oder 2-(N-Butyl-N-methyl-amino)ethyl, insbesondere Dimethylaminomethyl.

Diniederalkoxycarbonylniederalkyl ist beispielsweise Diniederalkoxycarbonyl-C₁-C₄-alkyl, wie 1,2-Dimethoxycarbonylethyl, 1,3-Dimethoxycarbonylpropyl, 1,2-Dimethoxycarbonylethyl oder 1 ,3-Diethoxycarbonylpropyl.

Diniederalkylamino ist beispielsweise Di-C₁-C₄-alkylamino, wie Dimethylamino, N-Methyl-N-ethylamino, Diethylamino, N-Methyl-N-propylamino oder N-Butyl-N-methyl-amino.

Diniederalkylaminoniederalkoxy ist beispielsweise N,N-Di-C₁-C₄-alkylaminc-C₁-C₄-alkoxy, wie 2-Dimethylaminoethoxy, 3-Dimethylaminopropyloxy, 2-Dimethylaminopropyloxy, 2-(Dimethylamino-2-methyl)propyloxy oder 2-(1-Dimethylamino-3-methyl)butyloxy, insbesondere 3-Dimethylaminopropyloxy.

Diniederalkylcarbamoyl ist beispielsweise Di-C₁-C₄-alkylcarbamoyl, wie Dimethylcarbamoyl, N-Methyl-N-ethylcarbamoyl, Diethylcarbamoyl, N-Methyl-N-propylcarbamoyl oder N-Butyl-N-methyl-carbamoyl.

Diniederalkylcarbamoyl(hydroxy)niederalkyl ist beispielsweise Di-C₁-C₄-alkylcarbamoyl-C₁-C₇-(hydroxy)alkyl, wie 1-Dimethylcarbamoyl- oder 1-Diethylcarbamoyl-2-hydroxy-ethyl.

Diniederalkylcarbamoylcycloalkylniederalkyl weist beispielsweise 3 bis 8, insbesondere 5 bis 7 Ringglieder auf und ist beispielsweise Di-C₁-C₄-alkylcarbamoyl-C₅-C₇-cycloalkyl-C₁-C₄-alkyl, wie Dimethylcarbamoylcyclopentyl-, Dimethylcarbamoylcyclohexyl- oder Dimethylcarbamoylcycloheptylmethyl.

Diniederalkylcarbamoylniederalkoxy ist beispielsweise N,N-Di-C₁-C₄-alkylcarbamoyl-C₁-C₄-alkoxy, wie 2-Dimethylcarbamoylethoxy, 3-Dimethylcarbamoylpropyloxy, 2-Dimethylcarbamoylpropyloxy, 2-(Dimethylcarbamoyl-2-methyl)propyloxy oder 2-(1-Dimethylcarbamoyl-3-methyl)butyloxy, insbesondere 2-Dimethylcarbamoylethoxy.

Diniederalkylcarbamoylniederalkyl ist beispielsweise N,N-Di-C₁-C₄-alkylcarbamoyl-C₁-C₄-alkyl, wie 2-Dimethylcarbamoylethyl, 3-Dimethylcarbamoylpropyl, 2-Dimethyl carbamoylpropyl, 2-(Dimethylcarbamoyl-2-methyl)propyl oder 2-(1-Dimethylcarbamoyl-3-methyl)butyl, insbesondere 2-Dimethylcarbamoylethyl.

Diniederalkylsulfamoylniederalkyl ist beispielsweise N,N-Di-C₁-C₄-alkylsulfamoyl-C₁-C₄-alkyl, N,N-Dimethylsulfamoyl-C₁-C₄-alkyl, wie N,N-Dimethylsulfamoylmethyl, (N,N-Dimethylcarbamoyl)ethyl 3-(N,N-Dimethylcarbamoyl)propyl oder 4-(N,N-Dimethylcarbamoyl)butyl, insbesondere N,N-Dimethylcarbamoylmethyl.

Dioxopiperidinylniederalkyl ist beispielsweise Dioxopiperidino-C₁-C₄-alkyl, wie 2,6-Dioxopiperidin-1-ylmethyl, wie 2-(2,6-Dioxopiperidin-1-yl)ethyl oder 2,6-Dioxopiperidin-4-ylmethyl.

S,S-Dioxothiomorpholinocarbonylniederalkoxy ist beispielsweise S,S-Dioxothiomorpholinocarbonyl-C₁-C₄-alkoxy, wie S,S-Dioxothiomorpholinocarbonylmethoxy, 2-(S,S-Dioxo)thiomorpholinocarbonylethoxy, 3-(S,S-Dioxo)thiomorpholinocarbonylpropyloxy oder 1- oder 2-[4-(S,S-Dioxo)thiomorpholinocarbonyl]butyloxy.

S,S-Dioxothiomorpholinocarbonylniederalkyl ist beispielsweise S,S-Dioxothiomorpholinocarbonyl-C₁-C₄-alkyl, wie S,S-Dioxothiomorpholinocarbonylmethyl, 2-(S,S-Dioxo)thiomorpholinocarbonylethyl, 3-(S,S-Dioxo)thiomorpholinocarbonylpropyl oder 1-oder 2-[4-(S,S-Dioxo)thiomorpholinocarbonyl]butyl.

S,S-Dioxothiomorpholinoniederalkoxy ist beispielsweise S,S-Dioxothiomorpholino-C₁-C₄-alkoxy, wie S,S-Dioxothiomorpholinomethoxy, 2-(S,S-Dioxo)thiomorpholinoethoxy, 3-(S,S-Dioxo)thiomorpholinopropyloxy oder 1- oder 2-[4-(S,S-Dioxo)thiomorpholino]butyloxy.

S,S-Dioxothiomorpholinoniederalkyl ist beispielsweise S,S-Dioxothiomorpholino-C₁-C₄-alkyl, wie S,S-Dioxothiomorpholinomethyl, 2-(S,S-Dioxo)thiomorpholinoethyl, 3-(S,S-Dioxo)thiomorpholinopropyl oder 1- oder 2-[4-(S,S-Dioxo)thiomorpholino]butyl.

Hydroxyniederalkoxy ist beispielsweise Hydroxy-C₂-C₇-alkoxy, insbesondere Hydroxy-C₂-C₄-alkoxy, wie 2-Hydroxyethoxy, 3-Hydroxypropyloxy oder 4-Hydroxybutyloxy.

Hydroxyniederalkoxyniederalkyl ist beispielsweise Hydroxy-C₁-C₄-alkoxy-C₁-C₄-alkyl, wie 2-Hydroxyethoxymethyl, 2-(2-Hydroxyethoxy)ethyl, 3-(3-Hydroxypropyloxy)propyl oder 4-(2-Hydroxybutyloxy)butyl, insbesondere 2-(3-Hydroxypropyloxy)ethyl oder 2-(4-Hydroxybutyloxy)ethyl.

Hydroxyniederalkyl ist beispielsweise Hydroxy-C₂-C₇-alkyl, insbesondere Hydroxy-C₂-C₄-alkyl, wie 2-Hydroxyethyl, 3-Hydroxypropyl oder 4-Hydroxybutyl.

Hydroxyniederalkylen ist gemeinsam mit dem seine freien Valenzen verbindenden N-Atom beispielsweise 3-Hydroxypyrrolidino oder 3- oder 4-Hydroxypiperidino.

Hydroxypiperidinocarbonyl ist beispielsweise 3- oder 4-Hydroxypiperidinocarbonyl.

Hydroxypiperidinocarbonylniederalkoxy ist beispielsweise Hydroxypiperidinocarbonyl-C₁-C₄-alkoxy, wie 3- oder 4-Hydroxypiperidinocarbonylmethoxy.

Hydroxypiperidinocarbonylniederalkyl ist beispielsweise Hydroxypiperidinocarbonyl-C₁-C₄-alkyl, wie 3- oder 4-Hydroxypiperidinocarbonylmethyl.

Hydroxypiperidinoniederalkoxy ist beispielsweise 3- oder 4-Hydroxypiperidino-C₁-C₄-alkoxy, wie 3- oder 4-Hydroxypiperidinomethoxy, 2-(3- oder 4-Hydroxypiperidino)ethoxy, 3-(3- oder 4-Hydroxypiperidino)propyloxy oder 4-(3- oder 4-Hydroxypiperidino)butyloxy.

Hydroxypiperidinoniederalkyl ist beispielsweise 3- oder 4-Hydroxypiperidino-C₁-C₄-alkyl, wie 3- oder 4-Hydroxypiperidinomethyl, 2-(3- oder 4-Hydroxypiperidino)ethyl, 3-(3- oder 4-Hydroxypiperidino)propyl oder 4-(3- oder 4-Hydroxypiperidino)butyl.

Hydroxypyrrolidinocarbonylniederalkoxy ist beispielsweise Hydroxypyrrolidinocarbonyl-C₁-C₄-alkoxy, wie 3-Hydroxypyrrolidinocarbonylmethoxy.

Hydroxypyrrolidinocarbonylniederalkyl ist beispielsweise Hydroxypyrrolidinocarbonyl-C₁-C₄-alkyl, wie 3-Hydroxypyrrolidinocarbonylmethyl.

Hydroxypyrrolidinoniederalkoxy ist beispielsweise 3-Hydroxypyrrolidino-C₁-C₄-alkoxy, wie 3-Hyd roxypyrrolidinomethoxy.

Hydroxypyrrolidinoniederalkyl ist beispielsweise 3- oder 4-Hydroxypyrrolidino-C₁-C₄-alkyl, wie 3-Hydroxypyrrolidinomethyl.

Imidazolylniederalkyl ist beispielsweise Imidazolyl-C₁-C₄-alkyl, wie Imidazolylmethyl, 2-Imidazolylethyl, 3-lmidazolylpropyl oder 4-lmidazolylbutyl.

Morpholinocarbonylniederalkoxy ist beispielsweise Morpholinocarbonyl-C₁-C₄-alkoxy, wie Morpholinocarbonylmethoxy, 2-Morpholinocarbonylethoxy, 3-Morpholinocarbonylpropyloxy oder 4-Morpholinocarbonylbutyloxy.

Morpholinocarbonylniederalkyl ist beispielsweise Morpholinocarbonyl-C₁-C₄-alkyl, wie Morpholinocarbonylmethyl, 2-Morpholinocarbonylethyl, 3-Morpholinocarbonylpropyl oder 4-Morpholinocarbonylbutyl, insbesondere 2-Morpholinocarbonylethyl.

Morpholinoniederalkoxy ist beispielsweise Morpholino-C₁-C₄-alkoxy, wie Morpholinomethoxy, 2-Morpholinoethoxy, 3-Morpholinopropyloxy oder 4-Morpholinobutyloxy, insbesondere 2-Morpholinoethoxy oder 3-Morpholinopropyloxy.

Morpholinoniederalkyl ist beispielsweise Morpholino-C₁-C₄-alkyl, wie Morpholinomethyl, 2-Morpholinoethyl, 3-Morpholinopropyl oder 4-Morpholinobutyl, insbesondere Morpholinomethyl, 2-Morpholinoethyl oder 3-Morpholinopropyl.

Morpholinoniederalkylcarbamoylniederalkoxy ist beispielsweise N-(Morpholino-C₁-C₄-alkylcarbamoyl)-C₁-C₄-alkoxy, wie insbesondere 2-Morpholinoethylcarbamoylmethoxy.

Niederalkanoylaminoniederalkyl ist beispielsweise N-C₁-C₄-Alkanoylamino-C₁-C₄-alkyl, wie 2-Acetoxyaminoethyl.

Niederalkanoylamino ist beispielsweise N-C₁-C₇-Alkanoylamino, wie Formylamino, Acetylamino oder Pivaloylamino.

Niederalkanoylaminoniederalkoxy trägt die Niederalkanoylaminogruppe vorzugsweise in höherer als der α-Stellung und ist beispielsweise N-C₁-C₇-Alkanoylamino-C₁-C₄-alkoxy, wie 2-Formylaminoethoxy, 2-Acetylaminoethoxy oder 2-Pivaloylaminoethoxy, insbesondere 2-Acetylaminoethoxy.

Niederalkanoyloxyniederalkoxy trägt die Niederalkanoyloxygruppe vorzugsweise in höherer als der α-Stellung und ist beispielsweise C₁-C₇-Alkanoyloxy-C₁-C₄-alkoxy, wie 4-Acetoxybutyloxy.

Niederalkanoyloxyniederalkyl trägt die Niederalkanoyloxygruppe vorzugsweise in höherer als der α-Stellung und ist beispielsweise C₁-C₇-Alkanoyloxy-C₁-C₄-alkyl, wie 4-Acetoxybutyl.

Niederalkanoylpiperazinocarbonyl ist beispielsweise N-C₂-C₇-Alkanoylpiperazinocarbonyl, wie 4-Acetylpiperazinocarbonyl.

Niederalkanoylpiperazinocarbonylniederalkoxy ist beispielsweise N'-C₂-C₇-Alkanoylpiperazinocarbonyl-C₁-C₄-alkoxy, wie 4-Acetylpiperazinocarbonylmethoxy.

Niederalkanoylpiperazinocarbonylniederalkyl ist beispielsweise N'-C₂-C₇-Alkanoylpiperazinocarbonyl-C₁-C₄-alkyl, wie insbesondere N'-Acetylpiperazinomethyl.

Niederalkanoylpiperazinoniederalkoxy ist beispielsweise N'-C₂-C₇-Alkanoylpiperazino-C₁-C₄-alkoxy, wie 4-Acetylpiperazinomethoxy.

Niederalkanoylpiperazinoniederalkyl ist beispielsweise N'-C₂-C₇-Niederalkanoylpiperazino-C₁-C₄-alkyl, wie 4-Acetylpiperazinomethyl.

Niederalkanoylpiperidinyl ist beispielsweise N'-C₂-C₇-Niederalkanoylpiperidin-4-yl, wie 1-Acetylpipereridin-4-ylmethyl.

Niederalkanoylpiperidinylniederalkyl ist beispielsweise N'-C₂-C₇-Niederalkanoylpiperidin-4-yl-C₁-C₄-alkyl, wie insbesondere 2-(1-Acetylpiperidin-4-yl)ethyl.

Niederalkansulfinylniederalkoxy ist beispielsweise C₁-C₇-Alkansulfinyl-C₁-C₄-alkoxy, wie Methansulfinylmethoxy oder 3-Methansulfinyl-2-hydroxy-propyloxy.

Niederalkansulfonylniederalkoxy ist beispielsweise C₁-C₇-Alkansulfonyl-C₁-C₄-alkoxy, wie Methansulfonylmethoxy oder 3-Methansulfonyl-2-hydroxy-propyloxy.

Niederalkansulfonylniederalkyl ist beispielsweise C₁-C₇-Alkansulfonyl-C₁-C₄-alkyl, wie Ethansulfonylmethyl, 2-Ethansulfonylethyl, 3-Ethansulfonylpropyl oder 3-(1,1-Dimethylethansulfonyl)propyl.

Niederalkoxy ist beispielsweise C₁-C₇-Alkoxy, vorzugsweise C₁-C₄-Alkoxy, wie Methoxy, Ethoxy, Propyloxy, Isopropyloxy, Butyloxy, Isobutyloxy, Sekundärbutyloxy, Tertiärbutyloxy, Pentyloxy oder eine Hexyloxy- oder Heptyloxygruppe.

Niederalkoxycarbonyl ist beispielsweise C₁-C₄-Alkoxycarbonyl, wie Methoxycarbonyl- oder Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl, Tertiärbutyloxycarbonyl, Pentyloxycarbonyl oder eine Hexyloxycarbonyl- oder Heptyloxygruppe.

Niederalkoxycarbonyl(carboxy)niederalkyl ist beispielsweise C₁-C₄-Alkoxycarbonyl(carboxy)-C₁-C₇-alkyl, vor allem C₁-C₄-Alkoxycarbonyl(carboxy)-C₂-C₇-alkyl, wie 2-Methoxycarbonyl-1-carboxyethyl, 1-Methoxycarbony-2-carboxyethyl, 3-Methoxycarbony-2-carboxy-propyl oder 2-Methoxycarbony-3-carboxy-propyl

Niederalkoxycarbonyl(hydroxy)niederalkyl ist beispielsweise C₁-C₄-Alkoxycarbonyl-C₁-C₇-(hydroxy)alkyl, wie 1-Methoxycarbonyl- oder 1-Ethoxycarbonyl-2-hydroxy-ethyl.

Niederalkoxycarbonylcydoalkylniederalkyl weist beispielsweise 3 bis 8, insbesondere 5 bis 7 Ringglieder auf und ist beispielsweise C₁-C₄-Alkoxycarbonylcyclopentyl-, C₁-C₄-Alkoxycarbonylcyclohexyl- oder C₁-C₄-Alkoxycarbonylcycloheptylmethyl.

Niederalkoxycarbonylniederalkyl ist beispielsweise C₁-C₄-Alkoxycarbonyl-C₁-C₄-alkyl, wie Methoxycarbonyl- oder Ethoxycarbonylmethoxy, 2-Methoxycarbonyl- oder 2-Ethoxycarbonylethoxy, 3-Methoxycarbonyl- oder 3-Ethoxycarbonylpropyloxy oder 4-Ethoxycarbonyl butyloxy.

Niederalkoxycarbonylpiperazinocarbonyl ist beispielsweise N'-C₁-C₄-Alkoxycarbonylpiperazinocarbonyl, wie 4-Methoxycarbonylpiperazinocarbonyl.

Niederalkoxycarbonylpiperazinoniederalkoxy ist beispielsweise N'-C₁-C₄-Alkoxycarbonyl piperazinocarbonyl-C₁-C₄-alkoxy, wie 2-(4-Methoxycarbonylpiperazinocarbonyl)ethoxy.

Niederalkoxycarbonylpiperazinoniederalkyl ist beispielsweise N'-C₁-C₄-Alkoxycarbonyl piperazinocarbonyl-C₁-C₄-alkyl, wie 2-(4-Methoxycarbonylpiperazinocarbonyl)ethyl.

Niederalkoxycarbonylpiperidinylniederalkyl ist beispielsweise N'-C₁-C₄-Alkoxycarbonylpiperidinylcarbonyl-C₁-C₄-alkyl, wie 2-(1-Methoxycarbonylpiperidin-4-yl)ethyl.

Niederalkoxyniederalkenyloxy ist beispielsweise C₁-C₄-Alkoxy-C₂-C₄-alkenyloxy, wie 4-Methoxybut-2-enyloxy.

Niederalkoxyniederalkoxy ist beispielsweise C₁-C₄-Alkoxy-C₂-C₄-alkoxy, wie 2-Methoxy-, 2-Ethoxy- oder 2-Propyloxyethoxy, 3-Methoxy- oder 3-Ethoxypropyloxy oder 4-Methoxybutyloxy, insbesondere 2-Methoxyethoxy, 3-Methoxypropyloxy, 4-Methoxybutyloxy, 5-Methoxypentyloxy.

Niederalkoxyniederalkoxyniederalkoxy ist beispielsweise C₁-C₄-Alkoxy-C₁-C₄-alkoxy-C₁-C₄-alkoxy, wie 2-Methoxy-, 2-Ethoxy- oder 2-Propyloxyethoxymethoxy, 2-(2-Methoxy-, 2-Ethoxy- oder 2-Propyloxyethoxy)ethoxy, 3-(3-Methoxy- oder 3-Ethoxypropyloxy)propyloxy oder 4-(2-Methoxybutyloxy)butyloxy, insbesondere 2-(Methoxymethoxy)ethoxy oder 2-(2-Methoxyethoxy)ethoxy.

Niederalkoxyniederalkoxyniederalkyl ist beispielsweise C₁-C₄-Alkoxy-C₁-C₄-alkoxy-C₁-C₄-alkyl, wie 2-Methoxy-, 2-Ethoxy- oder 2-Propyloxyethoxymethyl, 2-(2-Methoxy-, 2-Ethoxy- oder 2-Propyloxyethoxy)ethyl, 3-(3-Methoxy- oder 3-Ethoxypropyloxy)propyl oder 4-(2-Methoxybutyloxy)butyl, insbesondere 2-(3-Methoxypropyloxy)ethyl oder 2-(4-Methoxybutyloxy)ethyl.

Niederalkoxyniederalkoxyniederalkylen ist gemeinsam mit dem seine freien Valenzen verbindenden N-Atom beispielsweise C₁-C₄-Alkoxy-C₁-C₄-alkoxy-C₁-C₄-alkylen, wie 3-(3-Methoxypropyloxy)pyrrolidino, 3-(3-Methoxypropyloxy)piperidino oder 4-(3-Methoxypropyloxy)piperidino.

Niederalkoxyniederalkoxypiperidinocarbonyl ist beispielsweise C₁-C₄-Alkoxy-C₁-C₄-alkoxypiperidinocarbonyl, wie 3-(3-Methoxypropyloxy)- oder 4-(3-Methoxypropyloxy)piperidinocarbonyl.

Niederalkoxyniederalkoxypiperidinocarbonylniederalkoxy ist beispielsweise C₁-C₄-Alkoxy-C₁-C₄-alkoxy-piperidinocarbonyl-C₁-C₄-alkoxy, wie 3-(3-Methoxypropyloxy)- oder 4-(3-Methoxypropyloxy)piperidinocarbonylmethoxy.

Niederalkoxyniederalkoxypiperidinocarbonylniederalkyl ist beispielsweise C₁-C₄-Alkoxy-C₁-C₄-alkoxy-piperidinocarbonyl-C₁-C₄-alkyl, wie 3-(3-Methoxypropyloxy)- oder 4-(3-Methoxypropyloxy)piperidinocarbonylmethyl.

Niederalkoxyniederalkoxypiperidinoniederalkoxy ist beispielsweise 3- oder 4-C₁-C₄-Alkoxy-C₁-C₄-alkoxy-piperidino-C₁-C₄-alkoxy, wie 3-(3-Methoxypropyloxy)- oder 4-(3-Methoxypropyloxy)pipeddinomethoxy, 2-[3-(3-Methoxypropyloxy) oder 2-[4-(3-Methoxypropyl oxy)piperidino]ethoxy, 3-(3- oder 4-Hydroxypiperidino)propyloxy oder 4-(3- oder 4-Hydroxypiperidino)butyloxy.

Niederalkoxyniederalkoxypiperidinoniederalkyl ist beispielsweise 3- oder 4-C₁-C₄-Alkoxy-C₁-C₄-alkoxy-piperidino-C₁-C₄-alkyl, wie 3-(3-Methoxypropyloxy)- oder 4-(3-Methoxypropyloxy)piperidinomethyl, 2-(3- oder 4-Hydroxypiperidino)ethyl, 3-(3- oder 4-Hydroxypiperidino)propyl oder 4-(3- oder 4-Hydroxypiperidino)butyl.

Niederalkoxyniederalkoxypyrrolidinocarbonylniederalkoxy ist beispielsweise C₁-C₄-Alkoxy-C₁-C₄-alkoxy-pyrrolidinocarbonyl- Hydroxypyrrolidinocarbonyl-C₁-C₄-alkoxy, wie 3-(3-Methoxypropyloxy)pyrrolidinocarbonylmethoxy.

Niederalkoxyniederalkoxypyrrolidinocarbonylniederalkyl ist beispielsweise C₁-C₄-Alkoxy-C₁-C₄-alkoxy-pyrrolidinocarbonyl-C₁-C₄-alkyl, wie 3-(3-Methoxypropyloxy)pyrrolidinocarbonylmethyl.

Niederalkoxyniederalkoxypyrrolidinoniederalkoxy ist beispielsweise 3- oder 4-C₁-C₄-Alkoxy-C₁-C₄-alkoxy-pyrrolidino-C₁-C₄-alkoxy, wie 3-(3-Methoxypropyloxy)pyrrolidin-1-ylmethoxy.

Niederalkoxyniederalkoxypyrrolidinoniederalkyl ist beispielsweise 3- oder 4-C₁-C₄-Alkoxy-C₁-C4-alkoxy-pyrrolidino-C₁-C₄-alkyl, wie 3-(3-Methoxypropyloxy)pyrrolidin-1-ylmethyl.

Niederalkoxyniederalkyl beispielsweise C₁-C₄-Alkoxy-C₁-C₄-alkyl, wie Ethoxymethyl, Propyloxymethyl, Butyloxymethyl, 2-Methoxy-, 2-Ethoxy- oder 2-Propyloxyethyl, 3-Methoxy- oder 3-Ethoxypropyl oder 4-Methoxybutyl, insbesondere 3-Methoxypropyl oder 4-Methoxybutyl, insbesondere Propyloxymethoxy.

Niederalkoxyniederalkylen ist gemeinsam mit dem seine freien Valenzen verbindenden N-Atom beispielsweise C₁-C₄-Alkoxy-C₁-C₄-alkylen, wie 3-Methoxypyrrolidino, 3-Methoxypiperidino oder 4-Methoxypiperidino.

Niederalkoxyniederalkylpiperazinocarbonyl ist beispielsweise N'-C₁-C₄-Alkoxy-C₁-C₄-alkylpiperazinocarbonyl, wie N'-(3-Methoxypropyl)piperazinocarbonyl, N'-(4-Methoxybutyl) piperazinocarbonyl oder N'-(3-Ethoxypropyl)piperazinocarbonyl.

Niederalkoxyniederalkylpiperazinocarbonylniederalkoxy ist beispielsweise N'-C₁-C₄-Alkoxy-C₁-C₄-alkylpiperazinocarbonyl-C₁-C₄-alkoxy, wie N'-(3-Methoxypropyl)piperazinocarbonylmethoxy, 2-[N'-(3-Methoxypropyl)piperazinocarbonyl]ethoxy, 3-[N'-(3-Methoxypropyl)piperazinocarbonyl]propyloxy oder 4-[N'-(3-Methoxypropyl)piperazinocarbonyl]butyloxy.

Niederalkoxyniederalkylpiperazinocarbonylniederalkyl ist beispielsweise N'-C₁-C₄-Alkoxy-C₁-C₄-alkylpiperazinocarbonyl-C₁-C₄-alkyl, wie N'-(3-Methoxypropyl)piperazinocarbonylmethyl, 2-[N'-(3-Methoxypropyl)piperazinocarbonyl]ethyl, 3-[N'-(3-Methoxypropyl)piperazinocarbonyl]propyl oder 4-[N'-(3-Methoxypropyl)piperazinocarbonyl]butyl.

Niederalkylpiperazinoniederalkoxy ist beispielsweise N'-C₁-C₄-Alkylpiperazino-C₁-C₄-alkoxy, wie N'-(3-Methoxypropyl)piperazinomethoxy, 2-[N'-(3-Methoxypropyl)piperazino]ethoxy, 3-(N'-(3-Methoxypropyl)piperazino]propyloxy oder 4-[N'-(3-Methoxypropyl)piperazino]butyloxy.

Niederalkoxyniederalkylpiperazinoniederalkyl ist beispielsweise N'-C₁-C₄-Alkoxy-C₁-C₄-alkylpiperazino-C₁-C₄-alkyl, wie N'-(3-Methoxypropyl)piperazinomethyl, 2-[N'-(3-Methoxypropyl)piperazino]ethyl, 3-[N'-(3-Methoxypropyl)piperazino]propyl oder 4-(N'-(3-Methoxypropyl)piperazino]butyl.

Niederalkoxypipeddinocarbonyl ist beispielsweise C₁-C₄-Alkoxypiperidinocarbonyl, wie 3-oder 4-Methoxypiperidinocarbonyl, 3- oder 4-Ethoxypiperidinocarbonyl, 3- oder 4-Propyloxypiperidinocarbonyl oder 3- oder 4-Butyloxypiperidinocarbonyl.

Niederalkoxypiperidinocarbonylniederalkoxy ist beispielsweise C₁-C₄-Alkoxypiperidinocarbonyl-C₁-C₄-alkoxy, wie 3- oder 4-Methoxypiperidinocarbonylmethoxy, 2- (3- oder 4-Methoxypiperidinocarbonyl)ethoxy, 3-(3- oder 4-Methoxypiperidinocarbonyl)propyloxy oder 4-(3- oder 4-Methoxypiperidinocarbonyl)butyloxy.

Niederalkoxypiperidinocarbonylniederalkyl ist beispielsweise C₁-C₄-Alkoxypiperidinocarbonyl-C₁-C₄-alkyl, wie 3- oder 4-Methoxypiperidinocarbonylmethyl, 2-(3- oder 4-Methoxypiperidinocarbonyl)ethyl, 3-(3- oder 4-Methoxypiperidinocarbonyl)propyl oder 4-(3- oder 4-Methoxypiperidinocarbonyl)butyl.

Niederalkoxypiperidinoniederalkoxy ist beispielsweise C₁-C₄-Alkoxypiperidino-C₁-C₄-alkoxy, wie 2-(3- oder 4-Methoxypiperidino)ethoxy, 3-(3- oder 4-Methoxypiperidino)propyloxy oder 4-(3- oder 4-Methoxypiperidino)butyloxy.

Niederalkoxypiperidinoniederalkyl ist beispielsweise C₁-C₄-Alkoxypiperidino-C₁-C₄-alkyl, wie 3- oder 4-Methoxypiperidinomethyl, 2-(3- oder 4-Methoxypiperidino) ethyl, 3-(3- oder 4-Methoxypiperidino)propyl oder 4-(3- oder 4-Methoxypiperidino)butyl.

Niederalkoxypyrrolidinocarbonylniederalkoxy ist beispielsweise C₁-C₄-Alkoxy-pyrrolidinocarbonyl-C₁-C₄-alkoxy, wie 3-Methoxypyrrolidinocarbonylmethoxy, 2-(3-Methoxy pyrrolidinocarbonyl)ethoxy, 3-(3-Methoxypyrrolidinocarbonyl)propyloxy oder 4-(3-Methoxy pyrrolidinocarbonyl)butyloxy.
Niederalkoxypyrrolidinocarbonylniederalkyl ist beispielsweise C₁-C₄-Alkoxy-pyrrolidinocarbonyl-C₁-C₄-alkyl, wie 3-Methoxypyrrolidinocarbonylmethyl, 2-(3-Methoxypyrrolidinocarbonyl)ethyl, 3-(3-Methoxypyrrolidinocarbonyl)propyl oder 4-(3-Methoxypyrrolidinocarbonyl)butyl.

Niederalkoxypyrrolidinoniederalkoxy ist beispielsweise C₁-C₄-Alkoxy-pyrrolidino-C₁-C₄-alkoxy, wie 3-Methoxypyrrolidinomethoxy, 2-(3-Methoxypyrrolidino)ethoxy, 3-(3-Methoxypyrrolidino)propyloxy oder 4-(3-Methoxypyrrolidino)butyloxy.

Niederalkoxypyrrolidinoniederalkyl ist beispielsweise C₁-C₄-Alkoxy-pyrrolidino-C₁-C₄-alkyl, wie 3-Methoxypyrrolidinomethyl, 2-(3-Methoxypyrrolidino)ethyl, 3-(3-Methoxypyrrolidino)-propyl oder 4-(3-Methoxypyrrolidino)butyl..

Niederalkanoylaminoniederalkoxy ist beispielsweise N-C₁-C₄-Alkanoylamino-C₁-C₄-alkoxy, wie 2-Acetoxyaminoethoxy.

Niederalkyl kann geradkettig oder verzweigt und/oder überbrückt sein und ist beispielsweise entsprechendes C₁-C₇-Alkyl, wie Methyl, Ethyl, Propyl, Isopropyl, Butyl, Isobutyl, Sekundärbutyl, Tertiärbutyl, oder eine Pentyl-, Hexyl- oder Heptylgruppe.

Niederalkylamino ist beispielsweise C₁-C₄-Alkylamino, wie Methylamino, Ethylamino, Propylamino, Butylamino, Isobutylamino, Sekundärbutylamino oder Tertiärbutylamino.

Niederalkylaminoniederalkoxy ist beispielsweise C₁-C₄-Alkylamino-C₁-C₄-alkoxy, wie Propylaminomethoxy, 2-Methylamino-, 2-Ethylamino-, 2-Propylamino- oder 2-Butylaminoethoxy, 3-Ethylamino- oder 3-Propylaminopropyloxy oder 4-Methylaminobutoxy.

Niederalkylaminoniederalkoxy ist beispielsweise C₁-C₄-Alkylamino-C₁-C₄-alkoxy, wie Propylaminomethoxy, 2-Methylamino-, 2-Ethylamino-, 2-Propylamino- oder 2-Butylaminoethoxy, 3-Ethylamino- oder 3-Propylaminopropyloxy oder 4-Methylaminobutoxy.

Niederalkylcarbamoyl ist beispielsweise C₁-C₄-Alkylcarbamoyl, wie Methylcarbamoyl, Ethylcarbamoyl, Propylcarbamoyl, Butylcarbamoyl, Isobutylcarbamoyl, Sekundärbutylcarbamoyl oder Tertiärbutylcarbamoyl, insbesondere Methylcarbamoyl.

Niederalkylcarbamoyl(hydroxy)niederalkyl ist beispielsweise C₁-C₄-Alkylcarbamoyl-C₁-C₇-(hydroxy)alkyl, wie 1-Methylcarbamoyl- oder 1-Ethylcarbamoyl-2-hydroxy-ethyl.

Niederalkylcarbamoylcydoalkylniederalkyl weist beispielsweise 3 bis 8, insbesondere 5 bis 7 Ringglieder auf und ist beispielsweise C₁-C₄-Alkylcarbamoyl-C₅-C₇-cycloalkyl-C₁-C₄-alkyl, wie Methylcarbamoylcyclopentyl-, Methylcarbamoylcydohexyl- oder Methylcarbamoylcycloheptylmethyl.

Niederalkylcarbamoylniederalkoxy ist beispielsweise N-C₁-C₄-Alkylcarbamoyl-C₁-C₄-alkoxy, wie 2-Propylcarbamoylethoxy, 3-Ethylcarbamoylpropyloxy, 2-Ethylcarbamoylpropyloxy, 2-(Methylcarbamoyl-2-methyl)propyloxy 2-(1-Methylcarbamoyl-3-methyl)butyloxy oder insbesondere Butylcarbamoylmethoxy.

Niederalkylcarbamoylniederalkyl ist beispielsweise N- C₁-C₄-Alkylcarbamoyl-C₁-C₄-alkyl, wie 2-Methylcarbamoylethyl, 3-Methylcarbamoylpropyl, 2-Methylcarbamoylpropyl, 2-(Methylcarbamoyl-2-methyl)propyl oder 2-(1-Methylcarbamoyl-3-methyl)butyl,insbesondere 2-Methylcarbamoylethyl.

Niederalkylcarbamoylniederalkyl ist beispielsweise N-C₁-C₇-Alkylcarbamoyl-C₁-C₄-alkyl, wie Methyl- oder Dimethylcarbamoyl-C₁-C₄-alkyl, z.B. Methylcarbamoylmethyl, 2-Methylcarbamoylethyl, 3-Methylcarbamoylpropyl oder insbesondere 2-Methylcarbamoyl-2-methylpropyl.

Niederalkylen ist gemeinsam mit dem seine freien Valenzen verbindenden N-Atom beispielsweise Pyrrolidino oder Piperidino.

Niederalkylmorpholinocarbonyl ist beispielsweise 4-(C₁-C₄-Alkyl)morpholinocarbonyl, wie 4-Methylmorpholinocarbonyl, 4-Ethylmorpholinocarbonyl, 4-Propylmorpholinocarbonyl oder 4-Butylmorpholinocarbonyl.

Niederalkylmorpholinocarbonylniederalkoxy ist beispielsweise C₁-C₄-Alkylmorpholinocarbonyl-C₁-C₄-alkoxy, wie Methylmorpholinocarbonylmethoxy, 2-Methylmorpholinocarbonylethoxy, 3-Methylmorpholinocarbonylpropyloxy oder 4-Methyl morpholinocarbonylbutyloxy.

Niederalkylmorpholinocarbonylniederalkyl ist beispielsweise C₁-C₄-Alkylmorpholinocarbonyl-C₁-C₄-alkyl, wie Methylmorpholinocarbonylmethyl, 2-Methylmorpholinocarbonylethyl, 3-Methylmorpholinocarbonylpropyl oder 4-Methylmorpholinocarbonylbutyl, insbesondere 2-Methylmorpholinocarbonylethyl.

Niederalkylmorpholinoniederalkoxy ist beispielsweise C₁-C₄-Alkylmorpholino-C₁-C₄-alkoxy, wie Methylmorpholinomethoxy, 2-Methylmorpholinoethoxy, 3-Methylmorpholinopropyloxy oder 4-Methylmorpholinobutyloxy, insbesondere 2-Methylmorpholinoethoxy oder 3-Methylmorpholinopropyloxy.

Niederalkylmorpholinoniederalkyl ist beispielsweise C₁-C₄-Alkylmorpholino-C₁-C₄-alkyl, wie Methylmorpholinomethyl, 2-Methylmorpholinoethyl, 3-Methylmorpholinopropyl oder 4-Methylmorpholinobutyl.

Niederalkylpiperazinocarbonyl ist beispielsweise N'-C₁-C₄-Alkylpiperazinocarbonyl, wie N'-Methylpiperazinocarbonyl, N'-Ethylpiperazinocarbonyl, N'-Propylpiperazinocarbonyl oder N'-Butylpiperazinocarbonyl.

Niederalkylpiperazinocarbonylniederalkoxy ist beispielsweise N'-C₁-C₄-Alkylpiperazinocarbonyl-C₁-C₄-alkoxy, wie N'-Methylpiperazinocarbonylmethoxy, 2-(N'-Methylpiperazinocarbonyl)ethoxy, 3-(N'-Methylpiperazi nocarbonyl)propyloxy oder 4-(N'-Methylpiperazinocarbonyl)butyloxy.

NiederalkylpiperazinocarbonyIniederalkyl ist beispielsweise N'-C₁-C₄-Alkylpiperazinocarbonyl-C₁-C₄-alkyl, wie N'-Methylpiperazinocarbonylmethyl, 2-(N'-Methylpiperazinocarbonyl)ethyl, 3-(N'-Methylpiperazinocarbonyl)propyl oder 4-(N'-Methylpiperazinocarbonyl)-butyl, insbesondere N'-Methylpiperazinocarbonylmethyl.

Niederalkylpiperazinoniederalkoxy ist beispielsweise N'-C₁-C₄-Alkylpiperazino-C₁-C₄-alkoxy, wie N'-Methylpiperazinomethoxy, 2-(N'-Methylpiperazino)ethoxy, 3-(N'-Methylpiperazino)-propyloxy oder 4-(N'-Methylpiperazino)butyloxy.

Niederalkylpiperazinoniederalkyl ist beispielsweise N'-C₁-C₄-Alkylpiperazino-C₁-C₄-alkyl, wie N'-Methylpiperazinomethyl, 2-(N'-Methylpiperazino)ethyl, 3-(N'-Methylpiperazino)propyl oder 4-(N'-Methylpiperazino)butyl, insbesondere N'-Methylpiperazinomethyl.

Niederalkylsulfamoylniederalkyl ist beispielsweise N-C₁-C₇-Alkylsulfamoyl-C₁-C₄-alkyl, wie N-Methyl-, N-Ethyl- N-Propyl oder N-Butylsulfamoyl-C₁-C₄-alkyl, wie N-Methyl-, N-Ethyl- N-Propyl oder N-Butylsulfamoylmethyl, 2-(N-Methylsulfamoyl)ethyl, 2-(N-Butylsulfamoyl)ethyl, 3-(N-Methylsulfamoyl)propyl, 3-(N-Butylsulfamoyl)propyl, oder 4-(N-Methylsulfamoyl)butyl, 4-(N-Butylsulfamoyl)butyl oder 4-(N,N-Dimethylsulfamoyl)butyl, insbesondere N-Methyl-, N-Butyl oder N,N-Dimethylsulfamoylmethyl.

Niederalkylthioniederalkoxy ist beispielsweise N-C₁-C₄-Alkylthio-C₁-C₄-alkoxy, wie Methylthio-C₁-C₄-alkoxy, z.B. Methylthiomethoxy, 2-Methylthioethoxy oder 3-Methylthiopropyloxy.

Oxadiazolylniederalkyl ist beispielsweise 1,2,4-Oxadiazol-5-yl-C₁-C₄-alkyl, wie 1,2,4-Oxadiazol-5-ylmethyl.

Oxo-oxazolinylniederalkyl ist beispielsweise Oxo-oxazolinyl-C₁-C₄-alkyl, wie 5-Oxo-oxazolin-3-ylmethyl.

Oxopiperidinylniederalkyl ist beispielsweise Oxopiperidinyl-C₁-C₄-alkyl, wie 2-Oxopiperidin-1-ylmethyl oder 2-Oxopiperidin-4-ylmethyl.

Oxopyrrolidinylniederalkyl ist beispielsweise Oxopyrrolidinyl-C₁-C₄-alkyl, wie 2-Oxpyrrolidin-1-ylmethyl, 2-Oxo-pyorrolidin-4-ylmethyl oder 2-Oxo-pyorrolidin-5-ylmethyl Oxothiazolylniederalkyl ist beispielsweise Oxothiazolyl-C₁-C₄-alkyl, wie 2-Oxothialzol-4-ylmethyl oder 2-Oxothialzol-5-ylmethyl.

S-Oxothiomorpholinocarbonylniederalkoxy ist beispielsweise S-Oxothiomorpholinocarbonyl-C₁-C₄-alkoxy, wie S-Oxothiomorpholinocarbonylmethoxy, 2-(S-Oxo)thiomorpholinocarbonylethoxy, 3-(S-Oxo)thiomorpholinocarbonylpropyloxy oder 1- oder 2-[4-(S-Oxo)-thiomorpholinocarbonyl]butyloxy.

S-Oxothiomorpholinoniederalkyl ist beispielsweise S-Oxothiomorpholino-C₁-C₄-alkyl, wie S-Oxothiomorpholinomethyl, 2-(S-Oxo)thiomorpholinoethyl, 3-(S-Oxo)thiomorpholinopropyl oder 1- oder 2-[4-(S-Oxo)thiomorpholino]butyl.

Phenylniederalkoxy ist beispielsweise Phenyl-C₁-C₄-alkoxy, wie Benzyloxy, 2-Phenylethoxy, 3-Phenylpropyloxy oder 4-Phenylbutyloxy.
Phenylniederalkyl ist beispielsweise C₁-C₄-Alkylmorpholino-C₁-C₄-alkyl, wie Methylmorpholinomethyl, 2-Methylmorpholinocarbonylethyl, 3-Methylmorpholinopropyl oder 4-Methylmorpholinobutyl.

Piperazinocarbonylniederalkoxy ist beispielsweise Piperazinocarbonyl-C₁-C₄-alkoxy, wie Piperazinocarbonylmethoxy, 2-Piperazinocarbonylethoxy, 3-Piperazinocarbonylpropyloxy oder 4-Piperazinocarbonylbutyloxy.

Piperazinocarbonylniederalkyl ist beispielsweise Piperazinocarbonyl-C₁-C₄-alkyl, wie Piperazinocarbonylmethyl, 2-Piperazinocarbonylethyl, 3-Piperazinocarbonylpropyl oder 4-Piperazinocarbonylbutyl, insbesondere Piperazinocarbonylmethyl.

Pipeddinocarbonylniederalkoxy ist beispielsweise Piperidinocarbonyl-C₁-C₄-alkoxy, wie Piperidinocarbonylmethoxy, 2-Piperidinocarbonylethoxy, 3-Piperidinocarbonylpropyloxy oder 4-Piperidinocarbonylbutyloxy.

Piperidinocarbonylniederalkyl ist beispielsweise Piperidinocarbonyl-C₁-C₄-alkyl, wie Pipeddinocarbonylmethyl, 2-Piperidinocarbonylethyl, 3-Piperidinocarbonylpropyl oder 4-Piperidinocarbonylbutyl.

Piperidinoniederalkoxy ist beispielsweise Piperidino-C₁-C₄-alkoxy, wie 2-Piperidinoethoxy, 3-Piperidinopropyloxy oder 4-Piperidinobutyloxy, insbesondere 2-Piperidinoethoxy.

Piperidinoniederalkyl ist beispielsweise Piperidino-C₁-C₄-alkyl, wie Piperidinomethyl, 2-Piperidinoethyl, 3-Piperidinopropyl oder 4-Piperidinobutyl, insbesondere Piperidinomethyl.

Polyhalogenniederalkoxy ist beispielsweise Di-, Tri- oder Tetrahalogen-C₁-C₄-alkoxy, wie Trifluormethoxy.

Pyridylniederalkoxy ist beispielsweise Pyridyl-C₁-C₄-alkoxy, wie Pyridylmethoxy, 2-Pyridylethoxy, 3-Pyridylpropyloxy oder 4-Pyridylbutyloxy.

Pyridylniederalkyl ist beispielsweise Pyridyl-C₁-C₄-alkyl, wie Pyridylmethyl, 2-Pyridylethyl, 3-Pyridylpropyl oder 4-Pyridylbutyl, insbesondere Pyridylmethyl.

Pyrrolidinocarbonylniederalkoxy ist beispielsweise Pyrrolidinocarbonyl-C₁-C₄-alkoxy, wie Pyrrolidinocarbonylmethoxy, 2-Pyrrolidinocarbonylethoxy, 3-Pyrrolidinocarbonylpropyloxy oder 4-Pyrrolidi nocarbonylbutyloxy.

Pyrrolidinocarbonylniederalkyl ist beispielsweise Pyrrolidinocarbonyl-C₁-C₄-alkyl, wie Pyrrolidinocarbonylmethyl, 2-Pyrrolidinocarbonylethyl, 3-Pyrrolidinocarbonylpropyl oder 4-Pyrrolidinocarbonylbutyl.

Pyrrolidinoniederalkyl ist beispielsweise Pyrrolidino-C₁-C₄-alkyl, wie Pyrrolidinomethyl, 2-Pyrrolidinoethyl, 3-Pyrrolidinopropyl oder 4-Pyrrolidinobutyl, insbesondere Pyrrolidinomethyl.

Pyrrolidinylniederalkyl ist beispielsweise Pyrrolidinylo-C₁-C₄-alkyl, wie Pyrrolidin-2-ylmethyl, Pyrrolidin-3-ylmethyl, 2-Pyrrolidin-2-ylethyl, 2-Pyrrolidin-3-ylethyl, 3-Pyrrolidin-2-ylpropyl oder 4-Pyrrolidin-2-ylbutyl.

Sulfamoylniederalkyl ist beispielsweise Sulfamoyl-C₁-C₄-alkyl, wie Sulfamoyl-C₁-C₄-alkyl, wie Sulfamoylmethyl, 2-Sulfamoylethyl, 3-Sulfamoylpropyl oder 4-Sulfamoylbutyl.

Tetrazolylniederalkoxy ist beispielsweise Tetrazolyl-C₁-C₄-alkoxy, wie Tetrazol-5-ylmethoxy, 2-(Tetrazol-5-yl)ethoxy, 3-(Tetrazol-5-yl)propyloxy oder 4-(Tetrazol-4-yl)butyloxy, insbesondere Tetrazol-5-ylmethoxy.

Thiocarbamoylniederalkyl ist beispielsweise Thiocarbamoyl-C₁-C₄-alkyl, wie Thiocarbamoylmethyl, 2-Thiocarbamoylethyl, 3-Thiocarbamoylpropyl oder 4-Thiocarbamoylbutyl.

Thiomorpholinocarbonylniederalkyl ist beispielsweise Thiomorpholinocarbonyl-C₁-C₄-alkyl, wie Thiomorpholinocarbonylmethyl, 2-Thiomorpholinocarbonylethyl, 3-Thiomorpholinocarbonylpropyl oder 1- oder 2-(4-Thiomorpholinocarbonyl)butyl.

Thiomorpholinoniederalkoxy ist beispielsweise Thiomorpholino-C₁-C₄-alkoxy, wie Thiomorpholinomethoxy, 2-Thiomorpholinoethoxy, 3-Thiomorpholinopropyloxy oder 1- oder 2-(4-Thiomorpholino)butyloxy.

Thiomorpholinoniederalkyl ist beispielsweise Thiomorpholino-C₁-C₄-alkyl, wie Thiomorpholinomethyl, 2-Thiomorpholinoethyl, 3-Thiomorpholinopropyl oder 1- oder 2-(4-Thiomorpholino)butyl, insbesondere 2-Thiomorpholinoethyl.
Abhängig vom Vorhandensein asymmetrischer Kohlenstoffatome können die erfindungsgemäßen Verbindungen in Form von Isomerengemischen, speziell als Racemate, oder in Form reiner Isomere, speziell von optischen Antipoden vorliegen.

Salze von Verbindungen mit salzbildenden Gruppen sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren oder nicht-toxischen Salze von Verbindungen der Formel I.

Solche Salze werden beispielsweise von Verbindungen der Formel I mit einer sauren Gruppe, z.B. einer Carboxy- oder Sulfogruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, z.B. Alkalimetall-, insbesondere Lithium-, Natrium- oder Kaliumsalze, Erdalkalimetallsalze, beispielsweise Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quatemären Ammoniumbasen gebildet werden, z.B. Methyl-, Ethyl-, Diethyl- oder Triethylamin, Mono-, Bis- oder Tris-(2-hydroxyniederalkyl)-aminen, wie Ethanol-, Diethanol- oder Triethanolamin, Tris-(hydroxymethyl)-methylamin oder 2-Hydroxytertiärbutylamin, N,N-Diniederalkyl-N-(hydroxyniederalkyl)-amin, wie N,N-Di-N-Dimethyl-N-(2-hydroxyethyl)-amin, oder N-Methyl-D-glucamin, oder quaternären Ammoniumhydroxiden, wie Tetrabutylammoniumhydroxid. Die Verbindungen der Formel I mit einer basischen Gruppe, z.B. einer Aminogruppe, können Säureadditionssalze bilden, z.B. mit geeigneten anorganischen Säuren, z.B. Halogenwasserstoffsäure, wie Salzsäure, Bromwasserstoffsäure, Schwefelsäure unter Ersatz eines oder beider Protonen, Phosphorsäure unter Ersatz eines oder mehrerer Protonen, z.B. Orthophosphorsäure oder Metaphosphorsäure, oder Pyrophosphorsäure unter Ersatz eines oder mehrerer Protonen, oder mit organischen Carbon-, Sulfon-, Sulfo- oder Phosphonsäuren oder N-substituierter Sulfaminsäuren, z.B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure, Isonicotinsäure, femer Aminosäuren, wie z.B. den weiter vom genannten α-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2-oder 3- Phosphoglycerat, Glucose-6-Phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung und Reinigung können auch pharmazeutisch ungeeignete Salze Verwendung finden.

Die Verbindungen der vorliegenden Erfindung weisen Enzym-hemmende Eigenschaften auf. Insbesondere hemmen sie die Wirkung des natürlichen Enzyms Renin. Letzteres gelangt aus den Nieren ins Blut und bewirkt dort die Spaltung von Angiotensinogen unter Freisetzung des Dekapeptides Angiotensin I, das dann in der Lunge, den Nieren und anderen Organen zum Octapeptid Angiotensinogen II gespalten wird. Das Octapeptid erhöht den Blutdruck sowohl direkt durch arterielle Vasokonstriktion als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmem.

Die Wirkung von Reninhemmem wird unter anderem experimentell mittels *in vitro*-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende *in vitro*-Test verwendet: Ein Extrakt von menschlichem Renin aus der Niere (0,5 mGU [Milli-Goldblatt-Einheiten]/ml) wird eine Stunde lang bei 37 °C und pH 7,2 in 1-molarer wäßriger 2-N-(Trishydroxymethylmethyl)amino-ethansulfonsäure-Pufferlösung mit 23 µg/ml synthetischem Reninsubstrat, dem Tetradecapeptid H-Asp-Arg-Val-Tyr-lle-His-ProPhe-His-Leu-Leu-Val-Tyr-Ser-OH, inkubiert. Die Menge an gebildetem Angiotensin I wird in einem Radioimmunoassay ermittelt. Die erfindungsgemäßen Hemmstoffe werden dem Inkubationgemisch jeweils in verschiedenen Konzentrationen zugefügt. Als IC₅₀ wird diejenige Konzentration des jeweiligen Hemmstoffes bezeichnet, welche die Bildung von Angiotensin I um 50 % reduziert. Die Verbindungen der vorliegenden Erfindung zeigen in den *in vitro*-Systemen Hemmwirkungen bei minimalen Konzentrationen von etwa 10 ⁻⁶ bis etwa 10⁻¹⁰ Mol/l.

An salzverarmten Tieren bewirken Reninhemmer einen Blutdruckabfall. Das menschliche Renin unterscheidet sich von Renin anderer Spezies. Zur Prüfung von Hemmstoffen des humanen Renins werden Primaten (Marmosets, Callithrix jacchus) verwendet, weil humanes Renin und Primaten-Renin im enzymatisch aktiven Bereich weitgehende homolog sind. Unter anderem wird der folgende *in vivo*-Test eingesetzt: Die Testverbindungen werden an normotensiven Marmosets beider Geschlechter mit einem Körpergewicht von etwa 350 g, die bei Bewußtsein, freibeweglich und in ihren Normalkäfigen sind, geprüft. Blutdruck und Herzfrequenz werden mit einem Katheter in der Aortha descendens gemessen und radiometrisch erfaßt. Die endogene Freisetzung von Renin wird durch die Kombination einer 1-wöchigen salzarmen Diät mit einer einmaligen intramuskulären Injektion von Furosemid (5-(Aminosulfonyl)-4-chloro-2-[(2-furanyl methyl)-amino]benzoesäure) (5 mg/kg) angeregt. 16 Stunden nach der Injektion von Furosemid werden die Testsubstanzen entweder direkt in die Oberschenkelarterie mittels einer Injektionskanüle oder als Suspension oder Lösung über eine Schlundsonde in den Magen verabreicht und ihre Wirkung auf Blutdruck und Herzfrequenz ausgewertet. Die Verbindungen der vorliegenden Erfindung sind in dem beschriebenen *in vivo*-Test bei Dosen von etwa 0.003 bis etwa 0.3 mg/kg i.v. und bei Dosen von etwa 0.31 bis etwa 10 mg/kg p.o. blutdrucksenkend wirksam.

Die Verbindungen der vorliegenden Erfindung besitzen auch die Eigenschaft, den Augeninnendruck regulieren, insbesondere senken zu können.

Die Messung der Verminderung des Augeninnendruckes nach Applikation eines pharmazeutischen Wirkstoffes der Formel I gemäß der vorliegenden Erfindung kann beispielsweise am Tier, insbesondere am Kaninchen oder am Affen bestimmt werden. Nachfolgend sind zwei typische Versuchsanordnungen beschrieben, welche die vorliegende Erfindung verdeutlichen, aber in keiner Weise einschränken sollen.

Die in vivo Testierung am Kaninchen des Typs "Fauve de Bourgogne", zur Bestimmung der Augeninnendruck senkenden Aktivität von topisch applizierten Präparaten, kann beispielsweise wie folgt konzipiert sein. Der Augeninnendruck (AID) wird mit Hilfe eines Applanation-Tonometers jeweils vor dem Experiment und in regelmäßigen zeitlichen Abständen gemessen. Die geeignet formulierte Testsubstanz wird nach einer lokalen Anästhesie topisch in einer präzise definierten Konzentration (z.B. 0.000001 - 5 Gew. %) an einem Auge des jeweiligen Tiers appliziert. Das kontralaterale Auge wird beispielsweise mit physiologischer Kochsalzlösung behandelt. Die so ermittelten Meßwerte werden statistisch ausgewertet.

Die in vivo Tests am Affen der Spezies *Macaca fascicularis,* zur Bestimmung der Augeninnendruck senkenden Aktivität von topisch applizierten Präparaten, können beispielsweise folgendermaßen durchgeführt werden. Die geeignet formulierte Testsubstanz wird in präzise definierter Konzentration (z.B. 0.000001 -5 Gew.-%) jeweils an einem Auge der Affen appliziert. Das zweite Auge der Affen wird entsprechend beispielsweise mit physiologischer Kochsalzlösung behandelt. Vor Beginn der Tests werden die Tiere mit intramuskulären Injektionen von z.B. Ketamin anästhesiert. In regelmäßigen zeitlichen Abständen wird der Augeninnendruck (AID) gemessen. Die Durchführung und Auswertung erfolgt nach den Regeln der "good laboratory practice" (GLP).

Die Verbindungen der vorliegenden Erfindungen können zur Behandlung von Bluthochdruck (Hypertension), Herzinsuffizienz (Congestive Heart Failure), Herzerweiterung (Cardiac Hypertrophy), Herzfibrose (Cardiac Fibrosis), infarktbedingter Herzmuskelschwäche (Cardiomyopathy postinfarction), Komplikationen infolge Diabetes, wie Nephropathie, Vaskulopathie und Neuropathie, Erkrankungen der Herzkranzgefäße, Restenosis nach Angioplastie, erhöhten Augeninnendruck, Glaukom, abnormalem Gefäßwachstum, Hyperaldosteroismus, Angstzuständen und kognitiven Störungen Verwendung finden.

Die im folgenden genannten Verbindungsgruppen sind nicht als geschlossen zu betrachten, sondem es können in sinnvoller Weise, z.B. zur Ersetzung allgemeiner durch speziellere Definitionen, Teile dieser Verbindungsgruppen untereinander oder durch die oben gegebenen Definitionen ausgetauscht oder weggelassen werden.

Die Erfindung betrifft in erster Linie Verbindungen der Formel I, worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyndin-2-ylrest oder 1-R_{D}-Indol-3-ylrest bedeutet, wobei
einer der Reste R_{A} und R_{B} Niederalkyl, Hydroxyniederalkyl, Niederalkanoyloxyniederalkyl, Niederalkoxyniederalkyl, Niederalkoxyniederalkoxyniederalkyl, gegebenenfalls N-niederalkanoyliertes, N-mono- oder N,N-diniederalkyliertes oder durch Niederalkylen, Hydroxy-, Niederalkoxy- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkyl, Hydroxy, Niederalkoxy, Hydroxyniederalkoxy, Niederalkanoyloxyniederalkoxy, Niederalkoxyniederalkoxy, Niederalkoxyniederalkoxyniederalkoxy, Polyhalogenniederalkoxy, Cyanniederalkoxy, gegebenenfalls substituiertes Phenyl- oder Pyridylniederalkoxy, Niederalkoxyniederalkenyloxy, gegebenenfalls S-oxidiertes Niederalkylthioniederalkoxy oder gegebenenfalls N-niederalkanoyliertes, N-mono- oder N,N-diniederalkyliertes oder durch Niederalkylen, Hydroxy-, Niederalkoxy- oder Niederalkoxyniederalkoxyniederalkylen oder gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkoxy und der andere Wasserstoff, Niederalkyl, Carbamoyl, Hydroxy, Niederalkoxy oder Polyhalogenniederalkoxy darstellt,
R_{C} Wasserstoff, Niederalkyl, Hydroxy, Niederalkoxy, Hydroxyniederalkoxy, Niederalkoxyniederalkoxy, Morpholinoniederalkycarbamoylniederalkoxy, Niederalkoxyniederalkoxyniederalkyl, eine gegebenenfalls N-niederalkanoylierte, N-mono- oder N,N-diniederalkylierte oder durch Niederalkylen, Hydroxy-, Niederalkoxy-, Niederalkoxycarbonyl- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Amino-, Aminoniederalkyl- oder Aminonieder alkoxygruppe, eine gegebenenfalls amidierte Carboxy- oder Carboxyniederalkoxygruppe oder Tetrazolylniederalkoxy ist und
R_{D} für Niederalkyl, Hydroxyniederalkyl, Niederalkoxyniederalkyl, Niederalkoxyniederalkoxyniederalkyl, Hydroxyniederalkoxyniederalkyl, eine gegebenenfalls amidierte Carboxy- oder Carboxyniederalkylgruppe, Cyanoniederalkyl, eine gegebenenfalls substituierte Phenyl- oder Pyddylniederalkylgruppe steht,
einer der Reste X₁ und X₂ Carbonyl und der andere Methylen darstellt,
R₂ Niederalkyl ist,
R₃ gegebenenfalls N-niederalkanoyliertes oder N-mono- oder N,N-diniederalkyliertes Amino darstellt,
R₄ Niederalkyl oder Phenylniederalkyl bedeutet und
R₅ Niederalkyl, Cydoalkylniederalkyl, Hydroxyniederalkyl, Niederalkoxyniederalkyl, Niederalkanoyloxyniederalkyl, gegebenenfalls N-niederalkanoyliertes, N-mono- oder N,N-diniederalkyliertes oder durch Niederalkylen, Hydroxy-, Niederalkoxy-, Niederalkoxyniederalkyl- oder Niederalkanoyloxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkyl, gegebenenfalls verestertes oder amidiertes Carboxyniederalkyl, Cyanoniederalkyl, gegebenenfalls verestertes oder amidiertes Dicarboxyniederalkyl, gegebenenfalls verestertes oder amidiertes Carboxy(hydroxy)niederalkyl, gegebenenfalls verestertes oder amidiertes Carboxycyl oalkylniederalkyl, Niederalkansulfonylniederalkyl, gegebenenfalls N-mono- oder N,N-diniederalkyliertes Thiocarbamoylniederalkyl, gegebenenfalls N-mono- oder N,N-diniederalkyliertes Sulfamoylniederalkyl oder einen gegebenenfalls hydrierten und/oder oxosubstituierten Heteroarylrest oder einen durch einen über ein C-Atom gebundenen und gegebenenfalls hydrierten und/oder oxosubstituierten Heteroarylrest substituiertes Niederalkyl darstellt, und ihre Salze.

Die Erfindung betrifft insbesondere Verbindungen der Formel I, worin R₁einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-lndol-3-ylrest bedeutet, wobei einer der Reste R_{A} und R_{B} Niederalkyl, Hydroxyniederalkyl, Niederalkanoyloxyniederalkyl, Niederalkoxyniederalkyl, Nieder alkoxyniederalkoxyniederalkyl, Aminoniederalkyl, Niederalkanoylaminoniederalkyl, Niederalkylaminoniederalkyl, Diniederalkylaminoniederalkyl, gegebenenfalls durch Hydroxy, Niederalkoxy oder Niederalkoxyniederalkyl substituiertes Piperidino- oder Pyrrolidinoniederalkyl, gegebenenfalls N'-niederalkyliertes, N'-niederalkanoyliertes oder durch Niederalkoxycarbonyl oder Niederalkoxyniederalkyl N'-substituiertes Piperazinoniederalkyl, gegebenenfalls niederalkyliertes Morpholinoniederalkyl, gegebenenfalls S-oxidiertes Thiomorpholinoniederalkyl, Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Niederalkoxyniederalkyl substituiertes Piperidino- oder Pyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, gegebenenfalls niederalkyliertes Morpholinoniederalkoxy, gegebenenfalls S-oxidiertes Thiomorpholinoniederalkoxy, Hydroxy, Niederalkoxy, Hydroxyniederalkoxy, Niederalkanoyloxyniederalkoxy, Niederalkoxyniederalkoxy, Niederalkoxyniederalkoxyniederalkoxy, Polyhalogenniederalkoxy, Cyanniederalkoxy,
gegebenenfalls durch Niederalkyl, Niederalkoxy, Hydroxy, Nitro, Amino, Niederalkylamino, Diniederalkylamino, Halogen und/oder Trifluormethyl substituiertes Phenyl- oder Pyridylniederalkoxy, Niederalkoxyniederalkenyloxy, Niederalkylthioniederalkoxy, Niederalkansulfinylniederalkoxy, Niederalkansulfonylniederalkoxy, Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Niederalkoxyniederalkyl substituiertes Piperidino- oder Pyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, gegebenenfalls niederalkyliertes Morpholinoniederalkoxy oder gegebenenfalls S-oxidiertes Thiomorpholinoniederalkoxy und der andere Wasserstoff, Carbamoyl, Hydroxy, Niederalkoxy oder Polyhalogenniederalkoxy darstellt, Rc Wasserstoff, Niederalkyl, Niederalkoxyniederalkoxyniederalkyl, Aminoniederalkyl, Niederalkanoylaminoniederalkyl, Niederalkylaminoniederalkyl, Diniederalkyl aminoniederalkyl, gegebenenfalls durch Hydroxy, Niederalkoxy oder Niederalkoxyniederalkyl substituiertes Piperidino- oder Pyrrolidinoniederalkyl, gegebenenfalls N'-niederalkyliertes, N'-niederalkanoyliertes oder durch Niederalkoxycarbonyl oder Niederalkoxyniederalkyl N'-substituiertes Piperazinoniederalkyl, gegebenenfalls niederalkyliertes Morpholinoniederalkyl, gegebenenfalls S-oxidiertes Thiomorpholinoniederalkyl, Diniederalkylamino, eine gegebenenfalls durch Hydroxy, Niederalkoxy oder Niederalkoxyniederalkyl substituiertes Piperidino- oder Pyrrolidinogruppe, gegebenenfalls N'-niederalkyliertes, N'-niederalkanoyliertes oder durch Niederalkoxycarbonyl oder Niederalkoxyniederalkyl N'-substituiertes Piperazino, gegebenenfalls niederalkyliertes Morpholino, gegebenenfalls S-oxidiertes Thiomorpholino, Hydroxy, Niederalkoxy, Hydroxyniederalkoxy, Niederalkoxyniederalkoxy, Morpholinoniederalkycarbamoylniederalkoxy, Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Nieder alkoxyniederalkyl substituiertes Piperidino- oder Pyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, gegebenenfalls niederalkyliertes Morpholinoniederalkoxy, gegebenenfalls S-oxidiertes Thiomorpholinoniederalkoxy, Carboxyniederalkoxy, Carbamoylniederalkoxy, Niederalkylcarbamoylniederalkoxy, Diniederalkylcarbamoylniederalkoxy, gegebenenfalls durch Hydroxy, Niederalkoxy oder Niederalkoxyniederalkyl substituiertes Piperidino- oder Pyrrolidinocarbonylniederalkoxy, gegebenenfalls N'-niederalkyliertes, N'-niederalkanoyliertes oder durch Nieder alkoxycarbonyl oder Niederalkoxyniederalkyl N'-substituiertes Piperazinocarbonylniederalkoxy, gegebenenfalls niederalkyliertes Morpholinocarbonylniederalkoxy, gegebenenfalls S-oxidiertes Thiomorpholinocarbonylniederalkoxy, Tetrazolylniederalkoxy, Carboxy, Carbamoyl, Niederalkylcarbamoyl oder Diniederalkylcarbamoyl ist und
R_{D} für Niederalkyl, Hydroxyniederalkyl, Niederalkoxyniederalkyl, Niederalkoxyniederalkoxyniederalkyl, Hydroxyniederalkoxyniederalkyl, Carboxyniederalkyl, Nieder alkoxycarbonylniederalkyl, Carbamoylniederalkyl, NiederalkylcarbamoyIniederalkyl, Diniederalkylcarbamoylniederalkyl, gegebenenfalls durch Hydroxy, Niederalkoxy oder Niederalkoxyniederalkyl substituiertes Piperidino- oder Pyrrolidinocarbonylniederalkyl, gegebenenfalls N'-niederalkyliertes, N'-niederalkanoyliertes oder durch Nieder alkoxycarbonyl oder Niederalkoxyniederalkyl N'-substituiertes Piperazinocarbonylniederalkyl, gegebenenfalls niederalkyliertes Morpholinocarbonylniederalkyl, gegebenenfalls S-oxidiertes Thiomorpholinocarbonylniederalkyl,
Carboxyniederalkyl, Niederalkoxycarbonylniederalkyl oder eine gegebenenfalls durch Niederalkyl, Niederalkoxy, Hydroxy, Nitro, Amino, Niederalkylamino, Diniederalkylamino, Halogen und/oder Trifluormethyl substituierte Phenyl- oder Pyridylniederalkylgruppe steht, einer der Reste X₁ und X₂ Carbonyl und der andere Methylen darstellt, R₂ Niederalkyl ist, R₃ Amino, Niederalkanoylamino, Niederalkylamino oder Diniederalkylamino darstellt, R₄ Niederalkyl oder Phenylniederalkyl bedeutet und R₅ Niederalkyl, Cycloalkylniederalkyl, Hydroxyniederalkyl, Niederalkoxyniederalkyl, Niederalkanoyloxyniederalkyl, gegebenenfalls durch Hydroxy, Niederalkoxy oder Niederalkoxyniederalkyl substituiertes Piperidino- oder Pyrrolidinocarbonyiniederalkyl, gegebenenfalls N'-niederalkyliertes, N'-niederalkanoyliertes oder durch Niederalkoxycarbonyl oder Niederalkoxyniederalkyl N'-substituiertes Piperazinocarbonylniederalkyl, gegebenenfalls niederalkyliertes Morpholinocarbonylniederalkyl, gegebenenfalls S-oxidiertes Thiomorpholinocarbonylniederalkyl, Carboxyniederalkyl, Niederalkoxycarbonylniederalkyl, Carbamoylniederalkyl, Niederalkylcarbamoylniederalkyl, Diniederalkylcarbamoylniederalkyl, gegebenenfalls durch Hydroxy, Niederalkoxy oder Niederalkoxyniederalkyl substituiertes Piperidino- oder Pyrrolidinocarbonylniederalkyl, gegebenenfalls N'-niederalkyliertes, N'-niederalkanoyliertes oder durch Nieder alkoxycarbonyl oder Niederalkoxyniederalkyl N'-substituiertes Piperazinocarbonylniederalkyl, gegebenenfalls niederalkyliertes Morpholinocarbonylniederalkyl, gegebenenfalls S-oxidiertes Thiomorpholinocarbonylniederalkyl,
Cyanoniederalkyl, Dicarboxyniederalkyl, Niederalkoxycarbonyl(carboxy)niederalkyl, Diniederalkoxycarbonylniederalkyl, Dicarbamoylniederalkyl, Carbamoyl(carboxy)niederalkyl, Di(niederalkylcarbamoyl)niederalkyl, Di(diniederalkylcarbamoyl)niederalkyl, Carboxy(hydroxy)niederalkyl, Niederalkoxycarbonyl(hydroxy)niederalkyl, Carbamoyl (hydroxy)niederalkyl, Niederalkylcarbamoyl(hydroxy)niederalkyl oder Diniederalkylcarbamoyl(hydroxy)niederalkyl, Carboxycycloalkylniederalkyl, Niederalkoxycarbonylcydoalkylniederalkyl, Carbamoylcydoalkylniederalkyl, Niederalkyl carbamoylcycloalkylniederalkyl, Diniederalkylcarbamoylcydoalkylniederalkyl Niederalkansulfonylniederalkyl, Thiocarbamoylniederalkyl, N-Niederalkylthiocarbamoylniederalkyl oder N,N-Diniederalkylthiocarbamoylniederalkyl, Sulfamoylniederalkyl, Niederalkylsulfamoylniederalkyl oder Diniederalkylsulfamoylniederalkyl, gegebenenfalls oxosubstituiertes Pyrrolidinyl, Imidazolyl, Benzimidazolyl, Oxadiazolyl, Pyridyl, Oxopiperidinyl, Dioxopiperidinyl, Oxothiazolyl, Oxo-oxazolinyl oder Chinolinyl, gegebenenfalls oxosubstituiertes Pyrrolidinylniederalkyl, Imidazolylniederalkyl, Benzimidazolylniederalkyl, Oxadiazolylniederalkyl, Pyridylniederalkyl, Oxopiperidinylniederalkyl, Dioxopiperidinylniederalkyl, Oxothiazolylniederalkyl, Oxooxazolinylniederalkyl oder Chinolinylniederalkyl, Morpholinocarbonylniederalkyl oder gegebenenfalls N-niederalkanoyliertes Piperidylniederalkyl oder gegebenenfalls N-niederalkanoyliertes Piperidyl, darstellt, und ihre Salze.

Die Erfindung betrifft vor allem Verbindungen der Formel I, worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-Indol-3-ylrest bedeutet, wobei
einer der Reste R_{A} und R_{B} C₁-C₄-Alkyl, Hydroxy-C₁-C₄-alkyl, C₁-C₄-Alkanoyloxy-C₁-C₄-alkyl, C₁-C₄-Alkoxy-C₁-C₄-alkyl, C₁-C₄-Alkoxy-C₁-C₄-alkoxy-C₁-C₄-alkyl, Amino-C₁-C₄-alkyl, C₁-C₄-Alkanoylamino-C₁-C₄-alkyl, C₁-C₄-Alkylamino-C₁-C₄-alkyl, Di-C₁-C₄-alkylamino-C₁-C₄-alkyl, Piperidino-C₁-C₄-alkyl, Hydroxypiperidino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄- Piperazino-C₁-C₄-alkyl, N'-C₁-C₄-ASkypiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, C₁-C₇-Alkoxy, wie Propyloxy, Amino-C₁-C₇-alkoxy, C₁-C₄-Alkanoylamino-C₁-C₄-alkoxy, C₁-C₄-Alkylamino-C₁-C₄-alkoxy, Di-C₁-C₄-alkylamino-C₁-C₄-alkoxy, Piperidino-C₁-C₄-alkoxy, Hydroxypiperidino-C₁-C₄-alkoxy, C₁-C₄-Alkoxypiperidino-C₁-C₄-alkoxy, C₁-C₄-Alkoxy-C₁-C₄-alkoxyperidino-C₁-C₄-alkoxy, Pyrrolidino-C₁-C₄-alkoxy, Hydroxypyrrolidino-C₁-C₄-alkoxy, C₁-C₄-Alkoxypyrrolidino-C₁-C₄-alkoxy, C₁-C₄-Alkoxy-C₁-C₄-alkoxypyrrolidino-C₁-C₄-alkoxy, Piperazino-C₁-C₄-alkoxy, N'-C₁-C₄-Alkylpiperazino-C₁-C₄-alkoxy, N'-C₁-C₄-Alkanoylpiperazino-C₁-C₄-alkoxy, N'-C₁-C₄-Alkoxycarbonylpiperazino-C₁-C₄-alkoxy, N'-C₁-C₄-Alkoxy-C₁-C₄-alkylpiperazino-C₁-C₄-alkoxy, Morpholino-C₁-C₄-alkoxy oder C₁-C₄-Alkylmorpholino-C₁-C₄-alkoxy, Thiomorpholino-C₁-C₄-alkoxy, S-Oxythiomorpholino-C₁-C₄-alkoxy, S,S-Dioxythiomorpholino-C₁-C₄-alkoxy, Hydroxy, Hydroxy-C₁-C₄-alkoxy, C₁-C₄-Alkanoyloxy-C₁-C₄-alkoxy, C₁-C₄-Alkoxy-C₁-C₄-alkoxy, C₁-C₄-Alkoxy-C₁-C₄-alkoxy-C₁-C₄-alkoxy, Polyhalogen-C₁-C₄-alkoxy, Cyan-C₁-C₄-alkoxy, Carbamoyl C₁-C₄-alkoxy, gegebenenfalls durch C₁-C₄-Alkyl, C₁-C₄-Alkoxy, Hydroxy, Nitro, Amino, C₁-C₄-Alkylamino, Di-C₁-C₄-alkylamino, Halogen und/oder Trifluormethyl substituiertes Phenyl- oder Pyridyl-C₁-C₄-alkoxy, C₁-C₄-Alkoxy-C₁-C₄-alkenyloxy, C₁-C₄-Alkylthio-C₁-C₄-alkoxy, C₁-C₄-Alkansulfinyl-C₁-C₄-alkoxy, C₁-C₄-Alkansulfonyl-C₁-C₄-alkoxy, Amino-C₁-C₇-alkoxy, C₁-C₄-Alkanoylamino-C₁-C₄-alkoxy, C₁-C₄-Alkylamino-C₁-C₄-alkoxy, Di-C₁-C₄-alkylamino-C₁-C₄-alkoxy, Piperidino-C₁-C₄-alkoxy, Hydroxypiperidino-C₁-C₄-alkoxy, C₁-C₄-Alkoxypiperidino-C₁-C₄-alkoxy, C₁-C₄-Alkoxy-C₁-C₄-alkoxyperidino-C₁-C₄-alkoxy, Pyrrolidino-C₁-C₄-alkoxy, Hydroxypyrrolidino-C₁-C₄-alkoxy, C₁-C₄-Alkoxypyrrolidino-C₁-C₄-alkoxy, C₁-C₄-Alkoxy-C₁-C₄-alkoxypyrrolidino-C₁-C₄-alkoxy, Piperazino-C₁-C₄-alkoxy, N'-C₁-C₄-Alkylpiperazino-C₁-C₄-alkoxy, N'-C₁-C₄-Alkanoylpiperazino-C₁-C₄-alkoxy, N'-C₁-C₄-Alkoxycarbonylpiperazino-C₁-C₄-alkoxy, N'-C₁-C₄-Alkoxy-C₁-C₄-alkylpiperazino-C₁-C₄-alkoxy, Morpholino-C₁-C₄-alkoxy oder C₁-C₄-Alkylmorpholino-C₁-C₄-alkoxy oder Thiomorpholino-C₁-C₄-alkoxy und der andere Wasserstoff, Carbamoyl, C₁-C₄-Alkyl, Hydroxy, C₁-C₄-Alkoxy oder Trihalogen-C₁-C₄-alkoxy darstellt,
R_{C} Wasserstoff, Hydroxy, Di-C₁-C₄-Alkylamino, Piperidino, Pyrrolidino, Morpholino, Thiomorpholino, S-Oxythiomorpholino, S,S-Dioxythiomorpholino, C₁-C₄-Alkoxy, Hydroxy-C₁C₄-alkoxy, C₁-C₄-Alkoxy-C₁-C₄-alkoxy, Morpholino-C₁-C₄-alkylcarbamoyl-_{C}1-_{C}4-alkoxy, C₁-C₄-Alkoxy-C₁-C₄-alkoxy-C₁-C₄-alkyl, Amino-C₁-C₄-alkyl, C₁-C₄-Alkanoylamino-C₁-C₄-alkyl, C₁-C₄-Alkylamino-C₁-C₄-alkyl, Di-C₁-C₄-alkylamino-C₁-C₄-alkyl, gegebenenfalls durch Hydroxy, C₁-C₄-ASkoxy oder C₁-C₄-Alkoxy-C₁-C₄-alkyl substituiertes Piperidino- oder Pyrrolidino-C₁-C₄-alkyl, Amino-C₁-C₄-alkyl, C₁-C₄-Alkanoylamino-C₁-C₄-alkyl, C₁-C₄-Alkylamino-C₁-C₄-alkyl, Di-C₁-C₄-Alkylamino-C₁-C₄-alkyl, Piperidino-C₁-C₄-alkyl, Hydroxypiperidino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S'S-Dioxythiomorpholino-C₁-C₄-alkyl, Amino-C₁-C₇-alkoxy, C₁-C₄-Alkanoylamino-C₁-C₄-alkoxy, C₁-C₄-Alkylamino-C₁-C₄-alkoxy, Di-C₁-C₄-alkylamino-C₁-C₄-alkoxy, Piperidino-C₁-C₄-alkoxy, Hydroxypiperidino-C₁-C₄-alkoxy, C₁-C₄-Alkoxypiperidino-C₁-C₄-alkoxy, C₁-C₄-Alkoxy-C₁-C₄-alkoxyperidino-C₁-C₄-alkoxy, Pyrrolidino-C₁-C₄-alkoxy, Hydroxypyrrolidino-C₁-C₄-alkoxy, C₁-C₄-Alkoxypyrrolidino-C₁-C₄-alkoxy, C₁-C₄-Alkoxy-C₁-C₄-alkoxypyrrolidino-C₁-C₄-alkoxy, Piperazino-C₁-C₄-alkoxy, N'-C₁-C₄-Alkylpiperazino-C₁-C₄-alkoxy, N'-C₁-C₄-Alkanoylpiperazino-C₁-C₄-alkoxy, N'-C₁-C₄-Alkoxycarbonylpiperazino-C₁-C₄-alkoxy, N'-C₁-C₄-Alkoxy-C₁-C₄-alkylpiperazino-C₁-C₄-alkoxy, Morpholino-C₁-C₄-alkoxy oder C₁-C₄-Alkylmorpholino-C₁-C₄-alkoxy, Thiomorpholino-C₁-C₄-alkoxy, S-Oxythiomorpholino-C₁-C₄-alkoxy, S,S-Dioxythiomorpholino-C₁-C₄-alkoxy, Carboxy-C₁-C₄-alkoxyl Carbamoyl-C₁-C₄-alkoxy, C₁-C₄-Alkylcarbamoyl-C₁-C₄-alkoxy, Di-C₁-C₄-alkylcarbamoyl-C₁-C₄-alkoxy, Di-C₁-C₄-alkylamino-C₁-C₄-alkoxy, wie 3-Dimethylaminopropyloxy,
Piperidinocarbonyl-C₁-C₄-alkoxy, Hydroxypiperidinocarbonyl-C₁-C₄-alkoxy, C₁-C₄-Alkoxypiperidinocarbonyl-C₁-C₄-alkoxy, C₁-C₄-Alkoxy-C₁-C₄-alkoxyperidinocarbonyl-C₁-C₄-alkoxy, Pyrrolidinocarbonyl-C₁-C₄-alkoxy, Hydroxypyrrolidinocarbonyl-C₁-C₄-alkoxy, C₁-C₄-Alkoxypyrrolidinocarbonyl-C₁-C₄-alkoxy, C₁-C₄-Alkoxy-C₁-C₄-alkoxypyrrolidinocarbonyl-C₁-C₄-alkoxy, Piperazinocarbonyl-C₁-C₄-alkoxy, N'-C₁-C₄-Alkylpiperazinocarbonyl-C₁-C₄-alkoxy, N'-C₁-C₄-Alkanoylpiperazinocarbonyl-C₁-C₄-alkoxy, N'-C₁-C₄-Alkoxycarbonylpiperazinocarbonyl oder N'-C₁-C₄-Alkoxy-C₁-C₄-alkylpiperazinocarbonyl-C₁-C₄-alkoxy, Morpholinocarbonyl-C₁-C₄-alkoxy, C₁-C₄-Alkylmorpholinocarbonyl-C₁-C₄-alkoxy, Thiomorpholinocarbonyl-C₁-C₄-alkoxy, S-Oxythiomorpholinocarbonyl, S,S-Dioxythiomorpholinocarbonyl-C₁-C₄-alkoxy, Tetrazolyl-C₁-C₄-alkoxy, Carboxy, Carbamoyl, C₁-C₄-Alkylcarbamoyl, wie Methylcarbamoyl,
oder ist und
R_{D} für C₁-C₄-Alkyl, Hydroxy-C₁-C₄-alkyl, C₁-C₄-Alkoxy-C₁-C₄-alkyl, C₁-C₄-Alkoxy-C₁-C₄-alkoxy-C₁-C₄-alkyl, Hydroxy-C₁-C₄-alkoxy-C₁-C₄-alkyl, Carboxy-C₁-C₄-alkyl, C₁-C₄-Alkoxycarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Piperidino-C₁-C₄-alkyl, Hydroxypiperidino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C,-C4-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, Carboxy-C₁-C₄-aSky-a C₁-C₄-Alkoxycarbonyl-C₁-C₄-alkyl, unsubstituiertes oder durch C₁-C₄-Alkyl, C₁-C₄-Alkoxy, Hydroxy, Nitro, Amino, C₁-C₄-Alkylamino, Di-C₁-C₄-alkylamino, Halogen und/oder Trifluormethyl substituiertes Phenyl-C₁-C₄-alkyl oder Pyridyl-C₁-C₄-alkyl steht,
einer der Reste X₁ und X₂ Carbonyl und der andere Methylen darstellt,
R₂ C₁-C₄-Alkyl ist,
R₃ Amino, C₁-C₄-ASkanoykaminoa C₁-C₄-Alkylamino oder Di-C₁-C₄-alkylamino darstellt,
R₄ C₁-C₄-Alkyl oder Phenyl-C₁-C₄-alkyl bedeutet und
R₅ C₁-C₄-Alkyl, Cycloalkyl-C₁-C₄-alkyl, Hydroxy-C₁-C₄-alkyl, C₁-C₄-Alkoxy-C₁-C₄-alkyl, C₁-C₄-Alkanoyloxy-C₁-C₄-alkyl, Piperidino-C₁-C₄-alkyl, Hydroxypiperidino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, Carboxy-C₁-C₄-alkyl, C₁-C₄-Alkoxycarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Piperidinocarbonyl-C₁-C₄-alkyl, Hydroxypiperidinocarbonyl-C₁-C₄-alkyl, C₁-C₄-Alkoxypiperidinocarbonyl-C₁-C₄-alkyl, C₁-C₄-Alkoxy-C₁-C₄-alkoxyperidinocarbonyl-C₁-C₄-alkyl, Pyrrolidinocarbonyl-C₁-C₄-alkyl, Hydroxypyrrolidinocarbonyl-C₁-C₄-alkyl, C₁-C₄-Alkoxypyrrolidinocarbonyl-C₁-C₄-alkyl, C₁-C₄-Alkoxy-C₁-C₄-alkoxypyrrolidinocarbonyl-C₁-C₄-alkyl, Piperazinocarbonyl-C₁-C₄-alkyl, N'-C₁-C₄-Alkylpiperazinocarbonyl-C₁-C₄-alkyl, N'-C₁-C₄-Alkanoylpiperazinocarbonyl-C₁-C₄-alkyl, N'-C₁-C₄-Alkoxycarbonylpiperazinocarbonyl, N'-C₁-C₄-Alkoxy-C₁-C₄-alkylpiperazinocarbonyl-C₁-C₄-alkyl, Morpholinocarbonyl-C₁-C₄-alkyl, C₁-C₄-Alkylmorpholinocarbonyl-C₁-C₄-alkyl, Thiomorpholinocarbonyl-C₁-C₄-alkyl, S-Oxythiomorpholinocarbonyl-C₁-C₄-alkyl, S,S-Dioxythiomorpholinocarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Cyano-C₁-C₄-alkyl, Dicarboxy-C₁-C₄-alkyl, C₁-C₄-Alkoxycarbonyl(carboxy)-C₁-C₄-alkyl, Di-C₁-C₄-alkoxycarbonyl-C₁-C₄-alkyl, Dicarbamoyl-C₁-C₄-alkyl, Carbamoyl(carboxy)-C₁-C₄-alkyl, Di(-C₁-C₄-alkylcarbamoyl)-C₁-C₄-alkyl, Di(di-C₁-C₄-alkylcarbamoyl)-C₁-C₄-alkyl, Carboxy(hydroxy)-C₁-C₄-alkyl, C₁-C₄-Alkoxycarbonyl(hydroxy)-C₁-C₄-alkyl, Carbamoyl(hydroxy)-C₁-C₄-alkyl, C₁-C₄-Alkylcarbamoyl(hydroxy)-C₁-C₄-alkyl oder Di-C₁-C₄-alkylcarbamoyl(hydroxy)-C₁-C₄-alkyl, Carboxycycloalkyl-C₁-C₄-alkyl, C₁-C₄-Alkoxycarbonylcycloalkyl-C₁-C₄-alkyl, Carbamoylcycloalkyl-C₁-C₄-alkyl, C₁-C₄-Alkylcarbamoylcycloalkyl-C₁-C₄-alkyl, Di-C₁-C₄-alkylcarbamoylcycloalkyl-C₁-C₄-alkyl, C₁-C₄-Alkansulfonyl-C₁-C₄-alkyl, Thiocarbamoyl-C₁-C₄-alkyl, N-C₁-C₄-Alkylthiocarbamoyl-C₁-C₄-alkyl oder N,N-Di-C₁-C₄-alkylthiocarbamoyl-C₁-C₄-alkyl, Sulfamoyl-C₁-C₄-alkyl, C₁-C₄-Alkylsulfamoyl-C₁-C₄-alkyl oder Di-C₁-C₄-alkylsulfamoyl-C₁-C₄-alkyl, gegebenenfalls oxosubstituiertes Pyrrolidinyl, Imidazolyl, Benzimidazolyl, Oxadiazolyl, Pyridyl, Oxopiperidinyl, Dioxopiperidinyl, Oxothiazolyl, Oxo-oxazolinyl oder Chinolinyl, gegebenenfalls oxosubstituiertes Pyrrolidinyl-C₁-C₄-alkyl, Imidazolyl-C₁-C₄-alkyl, Benzimidazolyl-C₁-C₄-alkyl, Oxadiazolyl-C₁-C₄-alkyl, Pyridyl-C₁-C₄-alkyl, Oxopiperidinyl-C₁-C₄-alkyl, Dioxopiperidinyl-C₁-C₄-alkyl, Oxothiazolyl-C₁-C₄-alkyl, Oxo-oxazolinyl-C₁-C₄-alkyl oder Chinolinyl-C₁-C₄-alkyl, Morpholinocarbonyl-C₁-C₄-alkyl oder gegebenenfalls N-C₁-C₄-alkanoyliertes Piperidyl-C₁-C₄-alkyl oder gegebenenfalls N-C₁-C₄-alkanoyliertes Piperidyl darstellt, und ihre Salze.

Die Erfindung betrifft insbesondere Verbindungen der Formel I, worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-Indol-3-ylrest bedeutet, wobei einer der Reste R_{A} und R_{B} C₁-C₄-Alkyl, C₁-C₄-Alkoxy-C₁-C₄-alkyl, wie Propyloxymethyl, Di-C₁-C₄-alkylamino-C₁-C₄-alkyl, wie Dimethylaminomethyl, Piperidino-C₁-C₄-alkyl, wie Piperidinomethyl, C₁-C₄-Alkanoylpiperidinyl-C₁-C₄-alkyl, wie 2-(1-Acetylpiperidin-4-yl)ethyl, C₁-C₄-Alkoxycarbonylpiperidino-C₁-C₄-alkyl, wie 2-(1-Methoxycarbonylpiperidin-4-yl)ethyl, Pyrrolidino-C₁-C₄-alkyl, wie Pyrrolidinomethyl, Piperazino-C₁-C₄-alkyl, N'-C₁-C₄-Alkylpiperazino-C₁-C₄-alkyl, wie N'-Methylpiperazinomethyl, N'-C₁-C₄-Alkanoylpiperazino-C₁-C₄-alkyl, wie N'-Acetylpiperazinomethyl, Morpholino-C₁-C₄-alkyl, wie Morpholinomethyl, 2-Morpholinoethyl oder 3-Morpholinopropyl, C₁-C₄-Alkylmorpholino-C₁-C₄-alkyl, Thiomorpholino-C₁-C₄-alkyl, wie 2-Thiomorpholinoethyl, Amino-C₁-C₇-alkoxy, wie 2-Aminoethoxy, 3-Aminopropyloxy, C₁-C₄-Alkanoylamino-C₁-C₄-alkoxy, wie 2-Acetylaminoethoxy, Di-C₁-C₄-alkylamino-C₁-C₄-alkoxy, wie 3-Dimethylaminopropyloxy, Piperidino-C₁-C₄-alkoxy, wie 2-Piperidinoethoxy, Morpholino-C₁-C₄-alkoxy, wie 2-Morpholinoethoxy oder 3-Morpholinopropyloxy, Hydroxy, C₁-C₇-Alkoxy, wie Propyloxy, C₁-C₄-Alkoxy-C₁-C₄-alkoxy, wie 2-Methoxyethoxy, 3-Methoxypropyloxy, 4-Methoxybutyloxy oder 5-Methoxypentyloxy, C₁-C₄-Alkoxy-C₁-C₄-alkoxy-C₁-C₄-alkoxy, wie 2-(Methoxymethoxy)ethoxy oder 2-(2-Methoxyethoxy)ethoxy, C₁-C₄-Alkoxy-C₁-C₄-alkenyloxy, wie 4-Methoxy-but-2-enyloxy, Amino-C₁-C₇-alkoxy, wie 2-Aminoethoxy oder 3-Aminopropyloxy, C₁-C₄-Alkanoylamino-C₁-C₄-alkoxy, wie 2-Acetylaminoethoxy, Di-C₁-C₄-alkylamino-C₁-C₄-alkoxy, wie 3-Dimethylaminopropyloxy, Piperidino-C₁-C₄-alkoxy, wie 2-Piperidinoethoxy, Morpholino-C₁-C₄-alkoxy, wie 2-Morpholinoethoxy oder 3-Morpholinopropyloxy, Carbamoyl, Carbamoyl-C₁-C₄-alkoxy, wie 2-Carbamoylethoxy, und der andere Wasserstoff, C₁-C₄-Alkyl, wie Methyl, Hydroxy, oder C₁-C₄-Alkoxy, wie Methoxy, darstellt,
R_{C} Wasserstoff, Hydroxy, C₁-C₄-Alkoxy, wie Methoxy, C₁-C₄-Alkoxy-C₁-C₄-alkoxy, wie 2-Methoxyethoxy, 3-Methoxypropyloxy, 4-Methoxybutyloxy oder 5-Methoxypentyloxy, Morpholino-C₁-C₄-alkylcarbamoyl-C₁-C₄-alkoxy, wie 2-Morpholinoethylcarbamoylmethoxy, Di-C₁-C₄-alkylamino-C₁-C₄-alkyl, wie Dimethylaminomethyl, Piperidino-C₁-C₄-alkyl, wie Piperidinomethyl, C₁-C₄-Alkoxycarbonylpiperidino-C₁-C₄-alkyl, wie 2-(1-Methoxycarbonylpiperidin-4-yl)ethyl, Pyrrolidino-C₁-C₄-alkyl, wie Pyrrolidinomethyl, Piperazinocarbonyl-C₁-C₄-alkyl, N'-C₁-C₄-Alkylpiperazinocarbonyl-C₁-C₄-alkyl, N'-C₁-C₄-Alkanoylpiperazinocarbonyl-C₁-C₄-alkyl, wie N'-Acetylpiperazinocarbonylmethyl, Morpholino, Morpholino-C₁-C₄-alkyl, wie Morpholinomethyl, 2-Morpholinoethyl oder 3-Morpholinopropyl, Thiomorpholino-C₁-C₄-alkyl, wie 2-Thiomorpholinoethyl, C₁-C₄-Alkoxy, wie Methoxy, Amino-C₁-C₇-alkoxy, wie 2-Aminoethoxy oder 3-Aminopropyloxy, C₁-C₄-Alkanoylamino-C₁-C₄-alkoxy, wie 2-Acetylaminoethoxy, Di-C₁-C₄-alkylamino-C₁-C₄-alkoxy, wie 3-Dimethylaminopropyloxy, Piperidino-C₁-C₄-alkoxy, wie 2-Piperidinoethoxy, Morpholino-C₁-C₄-alkoxy, wie 2-Morpholinoethoxy oder 3-Morpholinopropyloxy, Morpholino-C₁-C₄-alkylcarbamoyl-C₁-C₄-alkoxy, wie 2-Morpholinoethylcarbamoylmethoxy, Carboxy, Carbamoyl, C₁-C₄-Alkylcarbamoyl, wie Methylcarbamoyl, Carboxy-C₁-C₄-alkoxy, wie Carboxymethoxy, Carbamoyl-C₁-C₄-alkoxy, wie 2-Carbamoylethoxy, C₁-C₄-Alkylcarbamoyl-C₁-C₄-alkoxy, wie Butylcarbamoylmethoxy, Di-C₁-C₄-alkylamino-C₁-C₄-alkoxy, wie 3-Dimethylaminopropyloxy, oder Tetrazolyl-C₁-C₄-alkoxy, wie Tetrazol-5-ylmethoxy, ist und
R_{D} für C₁-C₄-Alkyl, wie Methyl, C₁-C₄-Alkoxy-C₁-C₄-alkyl, wie Propyloxymethyl, Carbamoyl-C₁-C₄-alkyl, wie 3-Carbamoylpropyl oder 2-Carbamoyl-2-methyl-ethyl, C₁-C₄-Alkylcarbamoyl-C₁-C₄-alkyl, wie 2-Methylcarbamoyl-2-methyl-propyl, Di-C₁-C₄-alkylcarbamoyl-C₁-C₄-alkyl, wie 2-Dimethylcarbamoylethyl, Piperidino-C₁-C₄-alkyl, wie Pyrrolidinomethyl, oder C₁-C₄-Alkoxycarbonylpiperidino-C₁-C₄-alkyl, wie 2-(1-Methoxycarbonylpiperidin-4-yl)ethyl, steht, einer der Reste X₁ und X₂ Carbonyl und der andere Methylen darstellt,
R₂ C₁-C₄-Alkyl, wie Methyl oder Isopropyl, ist,
R₃ Amino oder C₁-C₄-Alkanoylamino, wie Acetylamino, darstellt,
R₄ C₁-C₄-Alkyl, wie Methyl oder Isopropyl, bedeutet und
R₅ C₁-C₄-Alkyl, wie Butyl, C₁-C₄-Alkoxy-C₁-C₄-alkyl, wie Propyloxymethyl, C₁-C₄-Alkoxycarbonylpiperidino-C₁-C₄-alkyl, wie 2-(1-Methoxycarbonylpiperidin-4-yl)ethyl, Pyrrolidino-C₁-C₄-alkyl, wie Pyrrolidinomethyl, N'-C₁-C₄-Alkylpiperazino-C₁-C₄-alkyl, wie N'-Methylpiperazinomethyl, N'-C₁-C₄-Alkoxycarbonylpiperazino-C₁-C₄-alkyl, wie N'-Methoxycarbonylpiperazinomethyl, oder N'-C₁-C₇-Alkanoylpiperazino-C₁-C₄-alkyl, wie N'-Acetylpiperazinomethyl, Morpholino-C₁-C₄-alkyl, wie 2-Morpholinoethyl oder 3-Morpholinopropyl, Thiomorpholino-C₁-C₄-alkyl, wie 2-Thiomorpholinoethyl, Morpholinocarbonyl-C₁-C₄-alkyl, wie 2-Morpholinocarbonylethyl, Carbamoyl-C₁-C₄-alkyl, wie 3-Carbamoylpropyl oder 2-Carbamoyl-2-methyl-ethyl, C₁-C₄-Alkylcarbamoyl-C₁-C₄-alkyl, wie 2-Methylcarbamoyl-2-methyl-ethyl, Di-C₁-C₄-alkylcarbamoyl-C₁-C₄-alkyl, wie 2-Dimethylcarbamoylethyl, Piperidinocarbonyl-C₁-C₄-alkyl, wie Piperidinocarbonylmethyl, Piperazinocarbonyl-C₁-C₄-alkyl, N'-C₁-C₄-Alkylpiperazinocarbonyl-C₁-C₄-alkyl, N'-C₁-C₄-Alkanoylpiperazinocarbonyl-C₁-C₄-alkyl, wie N'-Acetylpiperazinocarbonylmethyl, N'-C₁-C₄-Alkylpiperazinocarbonyl-C₁-C₄-alkyl, wie N'-Methylpiperazinocarbonylmethyl, oder Morpholinocarbonyl-C₁-C₄-alkyl, wie 2-Morpholinocarbonylethyl,
darstellt, und ihre Salze.

Die Erfindung betrifft in allererster Linie Verbindungen der Formel I, insbesondere der Formel la 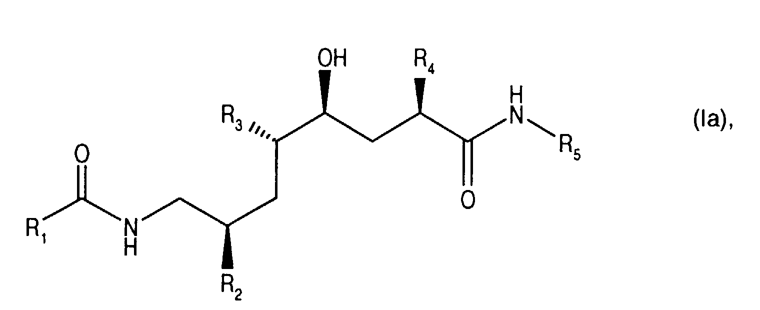 worin

R₁ einen 2-R_{A}-4-R_{C}-Phenylrest, 2-R_{A}-Pyddin-3-ylrest oder 3-R_{A}-Pyddin-2ylrest bedeutet, wobei
R_{A} C₁-C₄-Alkoxy-C₁-C₄-alkyl, wie Propyloxymethyl, Morpholino-C₁-C₄-alkyl, wie 2-Morpholinoethyl oder 3-Morpholinopropyl, C₁-C₇-Alkanoylpiperazino-C₁-C₄-alkyl, wie N'-Acetylpiperazinomethyl, C₁-C₇-Alkoxy, wie Propyloxy, C₁-C₄-Alkoxy-C₁-C₄-alkoxy, wie 2-Methoxyethoxy, 3-Methoxypropyloxy, 4-Methoxybutyloxy oder 5-Methoxypentyloxy, C₁-C₄-Alkoxy-C₁-C₄-alkenyloxy, wie 4-Methoxy-but-2-enyloxy,
C₁-C₄-Alkoxy-C₁-C₄-alkoxy-C₁-C₄-alkoxy, wie 2-(Methoxymethoxy)ethoxy oder 2-(2-Methoxyethoxy)ethoxy, Amino-C₁-C₄-alkoxy, wie 2-Aminoethoxy oder 3-Aminopropyloxy, Di-C₁-C₄-alkylamino-C₁-C₄-alkoxy, wie 3-Dimethylaminopropyloxy, Carbamoyl-C₁-C₄-alkoxy, wie 2-Carbamoylethoxy, oder Carbamoyl bedeutet und
R_{C} Wasserstoff, Di-C₁-C₄-alkylamino-C₁-C₄-alkyl, wie Dimethylaminomethyl, Piperidino-C₁-C₄-alkyl, wie Piperidinomethyl, Pyrrolidino-C₁-C₄-alkyl, wie Pyrrolidinomethyl, Morpholino-C₁-C₄-alkyl, wie Morpholinomethyl, C₁-C₇-Alkanoylpiperazino-C₁-C₄-alkyl, wie N'-Acetylpiperazinomethyl, oder C₁-C₄-Alkylpiperazino-C₁-C₄-alkyl, wie N'-Methylpiperazinomethyl, Morpholino, C₁-C₄-Alkoxy, wie Methoxy, Morpholino-C₁-C₄-alkoxy, wie 2-Morpholinoethoxy oder 3-Morpholinopropyloxy, Morpholino-C₁-C₄-alkylcarbamoyl-C₁-C₄-alkoxy, wie 2-Morpholinoethylcarbamoylmethoxy, Piperidino-C₁-C₄-alkoxy, wie 2-Pipeddinoethoxy, Carboxy, Carbamoyl, C₁-C₄-Alkylcarbamoyl, wie Methylcarbamoyl, Carboxy-C₁-C₄-alkoxy, wie Carboxymethoxy, Di-C₁-C₄-alkylamino-C₁-C₄-alkoxy, wie 3-Dimethylaminopropyloxy, C₁-C₇-Alkylcarbamoyl-C₁-C₄-alkoxy, wie Butylcarbamoylmethoxy, oder Tetrazolyl-C₁-C₄-alkoxy, wie Tetrazol-5-ylmethoxy, darstellt,
X₁ für Carbonyl und X₂ für Methylen steht,
R₂ und R₄ unabhängig voneinander C₁-C₄-Alkyl, wie Methyl oder Isopropyl, bedeuten, R₃ für Amino steht und
R₅ C₁-C₄-Alkyl, wie Butyl, Morpholino-C₁-C₄-alkyl, wie 2-Morpholinoethyl oder 3-Morpholinopropyl, Thiomorpholino-C₁-C₄-alkyl, wie 2-Thiomorpholinoethyl, Morpholinocarbonyl-C₁-C₄-alkyl, wie 2-Morpholinocarbonylethyl, Carbamoyl-C₁-C₄-alkyl, wie 3-Carbamoylpropyl oder 2-Carbamoyl-2-methyl-ethyl, C₁-C₄-Alkylcarbamoyl-C₁-C₄-alkyl, wie 2-Methylcarbamoyl-2-methyl-ethyl, Di-C₁-C₄-alkylcarbamoyl-C₁-C₄-alkyl, wie 2-Dimethylcarbamoylethyl, N'-C₁-C₄-Alkylpiperazino-C₁-C₄-alkyl, wie N'-Methylpiperazinomethyl, N'-C₁-C₄-Alkoxycarbonylpiperazino-C₁-C₄-alkyl, wie N'-Methoxycarbonylpiperazinomethyl, oder N'-C₁-C₇-ASkanoykpiperazino-C₁-C₄-alkyl, wie N'-Acetylpiperazinomethyl, bedeutet, und ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Das erfindungsgemäße Verfahren zur Herstellung von Verbindungen der Formel I ist dadurch gekennzeichnet, daß man
a) eine Verbindung der Formel II 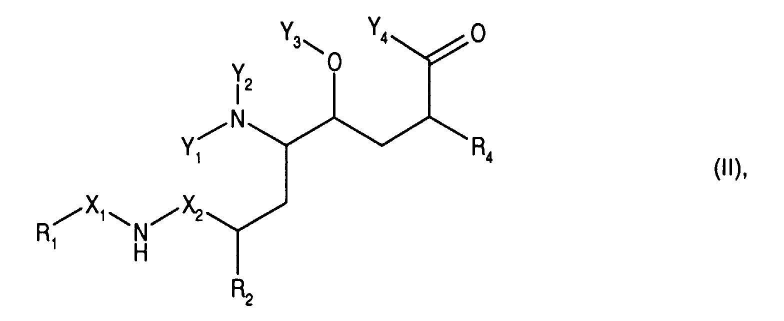 worin Y₁ Niederalkyl, Niederalkanoyl oder eine Aminoschutzgruppe bedeutet, Y₂ Wasserstoff oder gemeinsam mit Y₃ eine bivalente Schutzgruppe darstellt, Y₃ Wasserstoff, eine Hydroxyschutzgruppe oder gemeinsam mit Y₂ eine bivalente Schutzgruppe darstellt bzw. gemeinsam mit Y₄ für eine direkte Bindung steht, Y₄ gegebenenfalls reaktionsfähig verethertes oder verestertes Hydroxy bedeutet oder gemeinsam mit Y₃ eine direkte Bindung darstellt und R₁, R₂, R₃, R₄, R₅, X₁ und X₂ die unter der Formel I angegebenen Bedeutungen haben, mit einem Amin der Formel III

   H₂N-R₅ (III),

   worin R₅ eine der unter der Formel I angegebenen Bedeutungen hat, unter Bildung einer Amidbindung umsetzt und vorhandene Schutzgruppen abspaltet, oder
b) Verbindunqen der Formeln IV und V

   R₁-Y₅ (IV)

   und 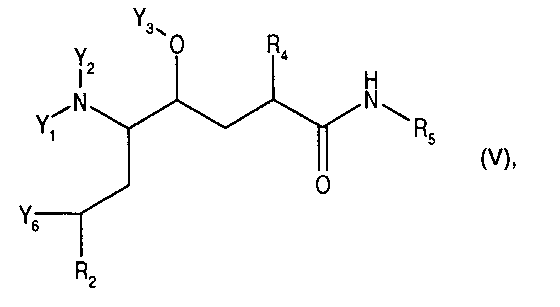 worin Y₁ Niederalkyl, Niederalkanoyl oder eine Aminoschutzgruppe bedeutet, Y₂ Wasserstoff oder gemeinsam mit Y₃ eine bivalente Schutzgruppe darstellt, Y₃ Wasserstoff, eine Hydroxyschutzgruppe oder gemeinsam mit Y₂ eine bivalente Schutzgruppe darstellt, einer der Reste Y₅ und Y₆ eine Aminomethylgruppe und der andere eine gegebenenfalls funktionell abgewandelte Carboxygruppe bedeutet und R₁, R₂, R₃, R₄ und R₅ die unter der Formel I angegebenen Bedeutungen haben, miteinander kondensiert und vorhandene Schutzgruppen abspaltet oder
c) zur Herstellung von Verbindungen der Formel I, worin R₃ Amino bedeutet, in einer Verbindung der Formel VI 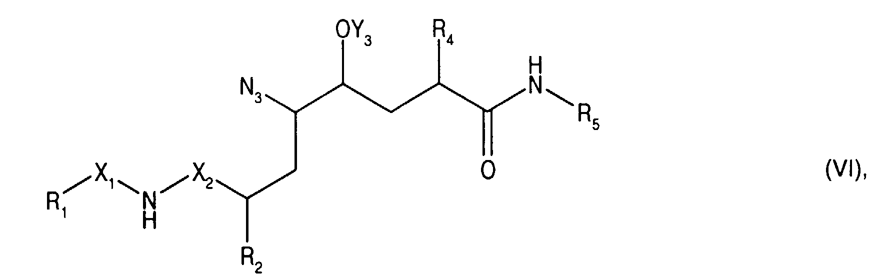 in der und R₁, R₂, R₄, R₅, X₁ und X₂ die unter der Formel I angegebenen Bedeutungen haben und Y₃ Wasserstoff oder eine Hydroxyschutzgruppe darstellt, die Azidogruppe zu Amino reduziert und vorhandene Schutzgruppen abspaltet und jeweils gewünschtenfalls eine nach einem der vorstehend genannten Verfahren erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemäße Verbindung der Formel I in eine andere erfindungsgemäße Verbindung der Formel I umwandelt.

Funktionelle Gruppen in Ausgangsmaterialien, deren Umsetzung vermieden werden soll, insbesondere Carboxy-, Amino- und Hydroxygruppen, können durch geeignete Schutzgruppen (conventional protecting groups) geschützt sein, die üblicherweise bei der Synthese von Peptidverbindungen, aber auch von Cephalosporinen und Penicillinen sowie Nucleinsäurederivaten und Zuckem verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, daß sie leicht, d.h. ohne unerwünschte Nebenreaktionen abspaltbar sind, beispielsweise solvolytisch, reduktiv, photolytisch oder auch enzymatisch, z.B. unter physiologischen Bedingungen. Schutzgruppen können aber auch in den Endstoffen vorhanden sein. Verbindungen der Formel I mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben Weyl, 4. Auflage, Band 15/1, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: MonoSaccharin und Derivate", Georg Thieme Verlag, Stuttgart 1974, beschrieben.

Aminoschutzgruppen Y₁ sind beispielsweise von Niederalkanoyl verschiedenen Acylgruppen, ferner Arylmethyl, Niederalkylthio, 2-Acyl-niederalk-1-enyl oder Silyl. Die Gruppe Y₁-N(Y₂)- kann auch als Azidogruppe vorliegen.

Von Niederalkanoyl verschiedenen Acylgruppen sind beispielsweise Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, beispielsweise Tertiärniederalkoxycarbonyl, wie Tertiärbutyloxycarbonyl, Arylmethoxycarbonyl mit einem oder zwei Arylresten, die gegebenenfalls, z.B. durch Niederalkyl, z.B. tertiäres Niederalkyl, wie Tertiärbutyl, Niederalkoxy, wie Methoxy, Hydroxy, Halogen, wie Chlor, und/oder Nitro mono- oder polysubstituiertes Phenyl darstellen, z.B. Benzyloxycarbonyl, gegebenenfalls substituiertes Benzyloxycarbonyl, wie 4-Nitrobenzyloxycarbonyl, Diphenylmethoxycarbonyl, Fluorenylmethoxycarbonyl oder substituiertes Diphenylmethoxycarbonyl, wie Di-(4-methoxy phenyl)methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Jodethoxycarbonyl, 2-(trisubstituiertes Silyl)-nieder alkoxycarbonyl, z. B.2-Triniederalkylsilylniederalkoxycarbonyl, z.B. 2-Trimethylsilylethoxycarbonyl oder 2-(Di-n-butyl-methyl-silyl)-ethoxycarbonyl, oder Triarylsilylniederalkoxycarbonyl, z.B. 2-Triphenylsilylethoxycarbonyl.

In einem als Aminoschutzgruppe verwendbaren 2-Acyl-niederalk-1-enylrest ist Acyl z.B. der entsprechende Rest einer Niederalkancarbonsäure, einer gegebenenfalls z.B. durch Niederalkyl, wie Methyl oder Tertiärbutyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder Niederalkoxycarbonyl-prop-1-en-2-yl, z.B. 1-Ethoxycarbonyl-prop-1-en-2-yl.

Eine Silylaminogruppe ist beispielsweise eine Triniederalkylsilylaminogruppe, z.B. Trimethylsilylamino. Das Siliziumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Eine Aminogruppe kann auch durch Überführung in die protonierte Form geschützt wer den; als entsprechende Anionen kommen in erster Linie diejenigen von starken anorganischen Säuren, wie von Schwefelsäure, Phosphorsäure oder Halogenwasserstoffsäuren, z.B. das Chlor- oder Bromanion, oder von organischen Sulfonsäuren, wie p-Toluolsulfonsäure, in Frage.

Bevorzugte Aminoschutzgruppen Y₁ sind Acylreste von Kohlensäurehalbestern, wie Niederalkoxycarbonyl, insbesondere Tertiärbutyloxycarbonyl oder Fluorenylmethoxycarbonyl, gegebenenfalls durch Niederalkyl, Niederalkoxy, Nitro und/oder Halogen substituiertes α-Phenyl- oder α,α-Diphenylniederalkoxycarbonyl, wie Benzyloxycarbonyl, p-Nitrobenzyloxycarbonyl oder Diphenylmethoxycarbonyl, oder 2-Halogenniederalkoxycarbonyl, z.B. 2,2,2-Trichlorethoxycarbonyl, ferner Trityl.

Hydroxyschutzgruppen Y₃ sind beispielsweise Acylgruppen, z.B. durch Halogen, wie Chlor, substituiertes Niederalkanoyl, z.B. 2,2-Dichloracetyl, oder insbesondere für geschützte Aminogruppen genannte Acylreste eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Als weitere Hydroxyschutzgruppe Y₃ kommen Triniederalkylsilyl, z.B. Trimethylsilyl, Triisopropylsilyl oder Dimethyl-tertiärbutylsilyl, leicht abspaltbare verethernde Gruppen, z.B. eine Alkylgruppe, wie tertiäres Niederalkyl, z.B. Tertiärbutyl, oxa- oder thiaaliphatische oder -cycloaliphatische, insbesondere 2-oxa- oder 2-thiaaliphatische oder -cycloaliphatische, Kohlenwasserstoffreste, beispielsweise 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, z.B. 2-Tetrahydrofuryl oder 2-Tetrahydropyranyl, oder entsprechende Thiaanaloge sowie 1-Phenyiniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, in Betracht

Durch Y₂ und Y₃ gemeinsam dargestellte bivalente Schutzgruppen sind beispielsweise durch ein oder zwei Alkylreste oder einen Alkylenrest substituierte Methylengruppen und bedeuten somit unsubstituiertes oder substituiertes Alkyliden, wie Niederalkyliden, z.B. Isopropyliden, Cycloalkyliden, z.B. Cyclohexyliden, ferner Carbonyl oder Benzyliden.

### Verfahrensvariante a):

Bedeutet Y₄ in Ausgangsstoffen der Formel II reaktionsfähig verethertes oder verestertes Hydroxy, stellt die endständige Gruppe -(=O)-Y₄ eine reaktionsfähig funktionell abgewandelte Carbonsäurefunktion dar und liegt beispielsweise in einer aktivierten Ester- oder Anhydridform vor. Die reaktionsfähigen Säurederivate können auch *in situ* gebildet werden.

Derartige aktivierte Ester von Verbindungen der Formel II sind insbesondere innere Ester, z.B. γ-Laktone, ferner am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylester (erhältlich z.B. durch Behandeln der entsprechenden Säure mit einem Isoxazoliumreagens; 1,2-Oxazolium- oder Woodward-Methode), oder 1-Niederalkoxyvinylester (erhältlich z.B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid- Methode), oder N,N-disubstituierte Amidinoester (erhältlich z.B. durch Behandeln der entsprechenden Säure mit einem N,N-disubstituierten Cyananmid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyaminobzw. N-Hydroxyamidoverbindung, z.B. N-Hydroxysuccinimid, N-Hydroxypiperidin, N-Hydroxyphthalimid, N-Hydroxy-5-norbornen-2,3-dicarbonsäureimid, 1-Hydroxybenz triazol oder 3-Hydroxy-3,4-dihydro-1,2,3-benztriazin-4-on, z.B. nach der Anhydrid- oder Carbodiimid-Methode; Methode der aktivierten N-Hydroxyester).

Die Kondensation von innere Ester, insbesondere γ-Laktone, d.h. Verbindungen der Formel II, worin Y₃ und Y₄ gemeinsam für eine direkte Bindung stehen, erfolgt vorteilhaft in Gegenwart eines basischen Kondensationsmittels, vorzugsweise von 2-Hydroxypyridin bei erhöhter Temperatur. Diese Verfahrensvariante ist insbesondere vorzüglich geeignet für die Umsetzung mit sterisch gehinderten Aminen.

Anhydride von Säuren der Formel II können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimides oder von 1-Diethylaminopropin; Methode der symmetrischen Anhydride).

Für die Herstellung der Ausgangsstoffe der Formel II können mehrere Bildungsweisen herangezogen werden. So erhält man Verbindungen der Formel lla 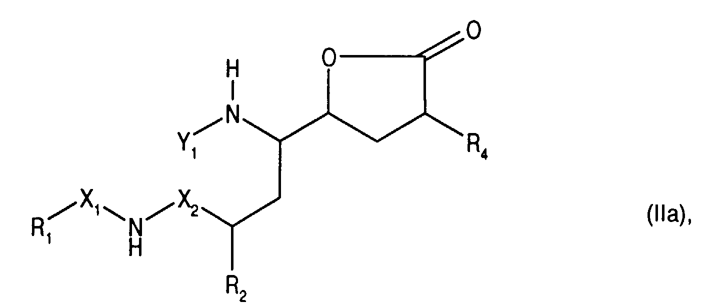 worin X₁ Methylen, X₂ Carbonyl und Y₁ eine Aminoschutzgruppe, insbesondere Tertiärbutyloxycarbonyl, bedeutet, beispielsweise, indem man E-1,4-Dibrombut-2-en zunächst mit einer Verbindung der Formel VII 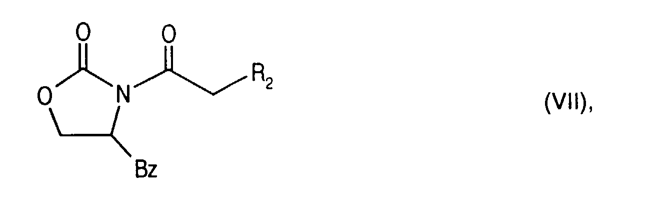 und anschließend mit einer Verbindung der Formel VIII 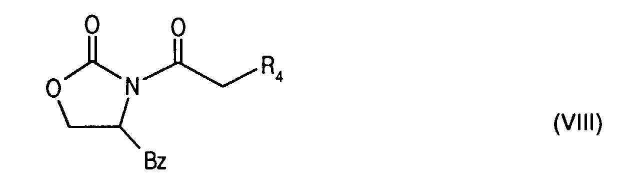 zur entsprechenden Verbindung der Formel IX 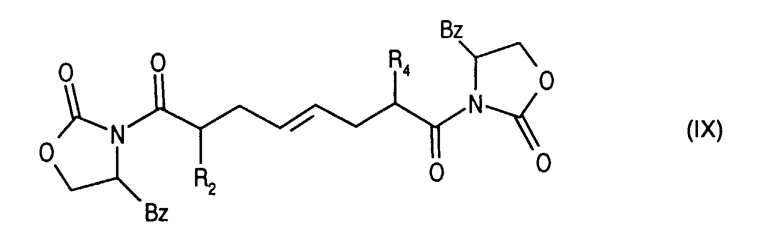 umsetzt, diese, beispielsweise durch Behandeln mit einem üblichen Halogenierungsmittel, wie elementarem Halogen, insbesondere Brom oder Jod, oder vorzugsweise mit einem N-Halogensuccinimid, insbesondere N-Bromsuccinimid in 1,2-Dimethoxyethan (DME), in die entsprechende Verbindung der Formel X 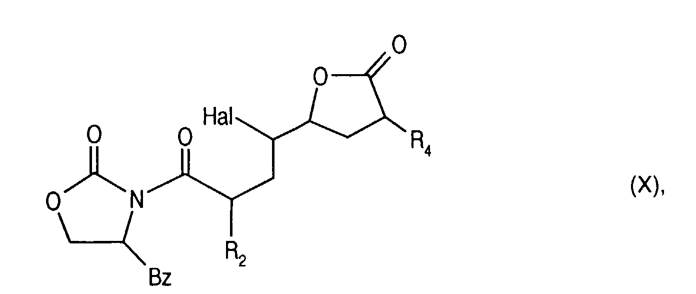 worin Hal Halogen bedeutet, überführt, das gewünschte Isomere in Bezug auf R₂ bzw. R₄ abtrennt und in diesem das Halogenatom, beispielsweise durch Behandeln mit Tetrabenzylammoniumazid in Toluol, durch Azido ersetzt und in der erhaltenen Verbindung der Formel XI 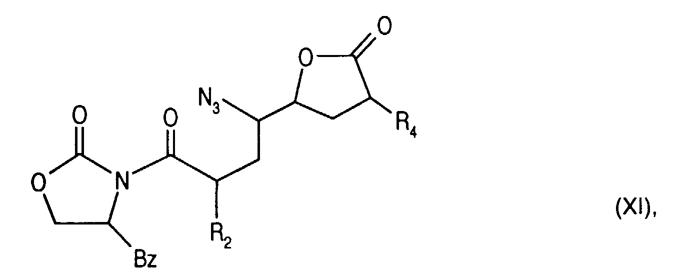 worin R₂ und R₄ die vorstehend angegebenen Bedeutungen haben und Bz Benzyl bedeutet, die 4-Benzyl-2-oxo-oxazolidin-1-ylcarbonylgruppe selektiv zu Carboxy hydrolysiert, einen dabei gegebenenfalls geöffneten Laktonring unter Verwendung eines sauren Katalysators wieder schließt, in der erhaltenen Verbindungen der Formel XII 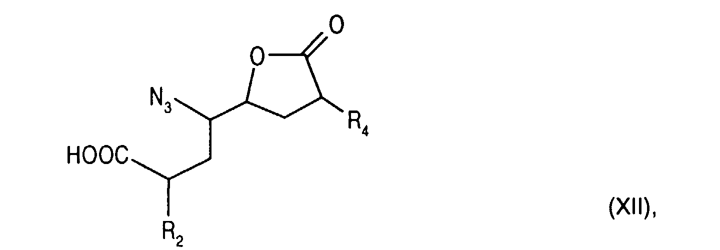 die Azidogruppe in üblicher Weise, beispielsweise mittels Wasserstoff auf Palladium/Kohle, zu Amino reduziert, die gebildete Aminogruppe, beispielsweise durch Umsetzung mit Ditertiärbutyldicarbonat, intermediär durch eine Aminoschutzgruppe, beispielsweise durch Tertiärbutyloxycarbonyl, Y₁ schützt und die erhaltene Verbindungen der Formel XIII 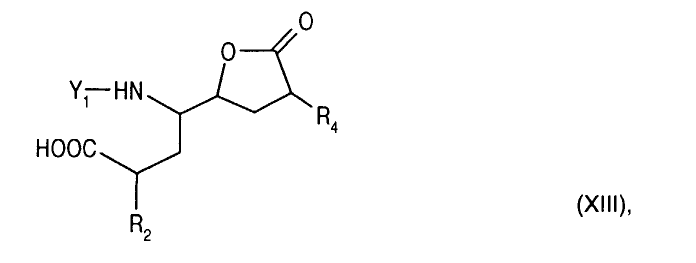 in üblicher Weise, beispielsweise wie nachstehend unter der Verfahrensvariante c) beschrieben, mit einer Verbindungen der Formel IV

R₁-Y₅ (IV),

worin Y₅ für Aminomethyl steht, kondensiert.

Zwischenprodukte der Formel IIa, worin X₁ Carbonyl, X₂ Methylen und Y₁ eine Aminoschutzgruppe, z.B. Tertiärbutyloxycarbonyl, darstellt, kann man aus wie vorstehend beschrieben erhältlichen Verbindungen der Formel XII erhalten, indem man zunächst die Carboxygruppe, beispielsweise durch Umsetzung mit einem Chlorameisensäureester und anschließende Behandlung mit Natriumborhydrid, zu Hydroxymethyl und nachfolgend die Azidogruppe, beispielsweise mittels Wasserstoff in Gegenwart von Palladium/Kohle, zu Amino reduziert, die gebildete Aminogruppe, beispielsweise durch Umsetzung mit Ditertiärbutyldicarbonat, durch eine Aminoschutzgruppe, beispielsweise durch Tertiärbutyloxycarbonyl, Y₁ schützt, in der erhaltenen Verbindung der Formel XIV 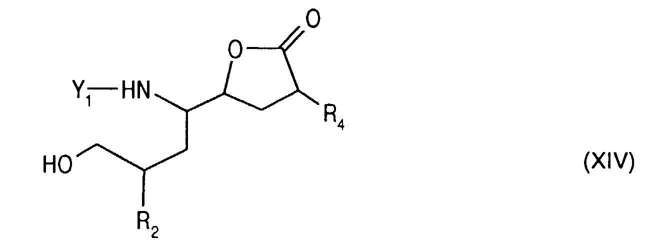 die terminale Hydroxygruppe in üblicher Weise, beispielsweise durch Behandlung zunächst mit Methansulfonylchlorid und anschließend mit Natriumazid, durch Azido ersetzt, in der erhaltenen Verbindungen der Formel XV 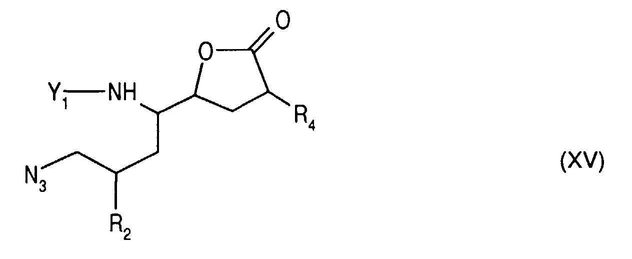 die Azidogruppe in üblicher Weise, beispielsweise wie vorstehend beschrieben, zu Amino reduziert und anschließend durch Umsetzung mit einer Carbonsäure der Formel IV, worin Y₅ Carboxy bedeutet durch den gewünschten Rest -X₁-R₁ substituiert.

Ausgangsstoffe der Formel llb 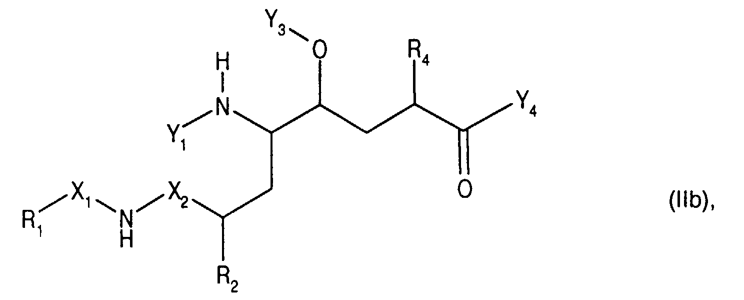 worin Y₁ eine Aminoschutzgruppe, insbesondere Tertiärbutyl oxycarbonyl, Y₃ eine Hydroxyschutzgruppe wie Triniederalkylsilyl, Y₄ Hydroxy X₁ Carbonyl und X₂ Methylen bedeutet, können aus Aziden der Formel XV hergestellt werden, beispielsweise durch Behandlung mit einem Alkalimetallhydroxid, wie Lithiumhydroxid, anschließende Umsetzung mit Tertiärbutyl(dimethyl)silylchlorid, anschließende übliche Reduktion der Azidogruppe zu Amino und abschließende Umsetzung mit einer Verbindung der Formel IV

R₁-Y₅ (IV),

worin Y₅ für gegebenenfalls reaktionsfähig funktionell abgewandeltes Carboxy steht.

### Verfahrensvariante b):

Gegebenenfalls funktionell abgewandeltes Carboxy Y₅ bzw. Y₆ in Ausgangsstoffen der Formel IV bzw. V ist beispielsweise freies oder in einer Ester- oder Anhydridform vorliegendes Carboxy. Die reaktionsfähigen Säurederivate können auch *in situ* gebildet werden.

Ester von Säuren der Formel IV bzw. V, worin Y₅ bzw. Y₆ Carboxy ist, sind beispielsweise deren aliphatische, araliphatische oder aromatische Ester, wie Niederalkylester, im Phenylteil gegebenenfalls, beispielsweise durch Niederalkyl, Niederalkoxy, Halogen und/oder Nitro substituierte Phenylniederalkylester oder gegebenenfalls, beispielsweise durch Niederalkyl, Niederalkoxy, Halogen und/oder Nitro substituierte Phenylester. Weiterhin in Betracht kommen aktivierte Ester. Solche sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylester (erhältlich z.B. durch Behandeln der entsprechenden Säure mit einem Isoxazoliumreagens; 1,2-Oxazolium- oder Woodward-Methode), oder 1-Niederalkoxyvinylester (erhältlich z.B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid- Methode), oder N,N-disubstituierte Amidinoester (erhältlich z.B. durch Behandeln der entsprechenden Säure mit einem N,N-disubstituierten Cyananmid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino- bzw. N-Hydroxyamidoverbindung, z.B. N-Hydroxysuccinimid, N-Hydroxypiperidin, N-Hydroxyphthalimid, N-Hydroxy-5-norbomen-2,3-dicarbonsäureimid, 1-Hydroxybenz Triazol oder 3-Hydroxy-3,4-dihydro-1 ,2,3-benztriazin-4-on, z.B. nach der Anhydrid- oder Carbodiimid-Methode; Methode der aktivierten N-Hydroxyester).

Anhydride von Säuren der Formel IV bzw. V, worin Y₅ bzw. Y₆ Carboxy ist, können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin (Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimides oder von 1-Diethylaminopropin; Methode der symmetrischen Anhydride).

Die Kondensation von Verbindungen der Formeln IV und V kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure mit dem entsprechenden Amin kann vorzugsweise in Gegenwart eines der üblichen Kondensationsmittel durchgeführt werden. Übliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3'-sulfonat und 2-TertiärbButyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoroamidochloridat, Bis-(2-oxo-3-oxazolidinyl)-phosphinsäurechlorid oder 1-Benztriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Ethyldiisopropylamin, und/oder eine heterocyclische Base, z.B. Pyridin, N-Methylmorpholin oder bevorzugt 4-Dimethylaminopyridin.

Die Kondensation aktivierter Ester, reaktionsfähiger Anhydride oder reaktionsfähiger cyclischer Amide mit den entsprechenden Aminen wird üblicherweise in Gegenwart einer organischen Base, z.B. einfachen Triniederalkylaminen, z.B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z.B. in Gegenwart von anorganischen Carbonaten, z.B. Ammonium- oder Alkalimetallcarbonaten oder -hydrogencarbonaten, wie Natrium- oder Kaliu mcarbonat oder -hydrogencarbonat (üblicherweise zusammen mit einem Sulfat), erfolgen.

Carbonsäurechloride, beispielsweise die von der Säure der Formel II abgeleiteten Chlorkohlensäurederivate werden mit den entsprechenden Aminen vorzugsweise in Gegenwart eines organischen Amins, z.B. der vorstehend genannten Triniederalkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, einem cyclischen Ether, z.B. Tetrahydrofuran, einem Ester, z.B. Essigsäureethylester, oder einem Nitril, z.B. Acetonitril, oder in einer Mischung davon, gegebenenfalls bei erniedrigter oder erhöhter Temperatur, z.B. in einem Temperaturbereich von etwa -40° C bis etwa +100° C, bevorzugt von etwa -10° C bis etwa +50° C, im Falle der Verwendung von Arylsulfonylestern auch bei etwa +100° C bis +200° C, und gegebenenfalls unter Inertgas-, z.B. Stickstoff- oder Argonatmosphäre.

Auch wässrige, beispielsweise alkoholische, z.B. Ethanol, oder aromatische Lösungsmittel, z.B. Benzol oder Toluol, sind möglich. Bei Gegenwart von Alkalihydroxiden als Basen kann gegebenenfalls auch Aceton zugesetzt werden.

Die Kondensation kann auch gemäß der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 (1985) beschrieben ist.

In einer bevorzugten Variante dieses Verfahrens, die sich insbesondere für die Herstellung von Verbindungen der Formel I eignet, worin X₁ Carbonyl, X₂ Methylen und R₁ beispielsweise einen 1-R_{D}-Indol-3-ylrest darstellt, geht man von einer Carbonsäure der Formeln IV aus und setzt diese in Gegenwart eines Cyanphosphonsäurediesters, z.B. von Cyanphosphonsäurediethylester, oder Benzotriazolyloxy-tris(diniederalkyl amino)-phophoniumsalz, z.B. von 1-Benztriazolyloxy-tris-(dimethylamino)-phosphoniumhexafluorophosphat oder -chlorid, und eines tertiären organischen Amins, wie eines Triniederalkylamins, z.B. von Trimethylamin, und in einem polaren Lösungsmittel, z.B. einem Nitril, wie Acetonitril, einem Amid, wie Dimethylformamid, N,N-Dimethylacetamid, N-Methylpyrrolidon oder Hexamethylphosphorsäuretriamid, einem Hamstoff, z.B. N,N'-Dimethyl-N,N'-propylenylharnstoff, einem Niederalkoxy-niederalkanol, z.B. Diethylenglycolmonomethylether, in Dimethylsulfoxid oder einem Gemisch der genannten Solventien oder einem Gemisch eines oder mehrerer der genannten Solventien mit Wasser bei Temperaturen von -30° C bis 100° C, bevorzugt von 20° C bis 80°C, mit der Aminkomponente der Formel V um, wobei für die Schutzgruppen das oben Gesagte gilt.

Ausgangsstoffe der Formel IV sind bekannt oder können in Analogie zur Bildungsweise von bekannten Verbindungen der Formel IV hergestellt werden.

Ausgangsstoffe der Formel V, worin Y₆ Amino bedeutet, Y₁ beispielsweise Tertiärbutyloxycarbonyl ist und Y₂ und Y₃ gemeinsam beispielsweise für Isopropyliden stehen, können beispielsweise hergestellt werden, indem man nach an sich bekannten Methoden eine Verbindung der Formel XVI 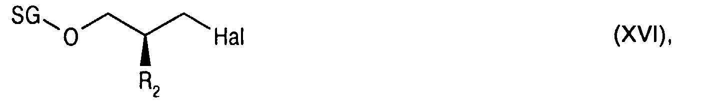 worin SG eine Hydroxyschutzgruppe, wie α-Phenylniederalkyl, insbesondere Benzyl, bedeutet, Hal für Halogen steht und R₂ die angegebene Bedeutung hat, mit einer Verbindung der Formel XVII 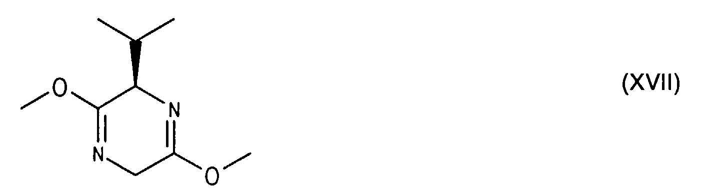 kondensiert, die erhaltene Verbindung der Formel XVIII 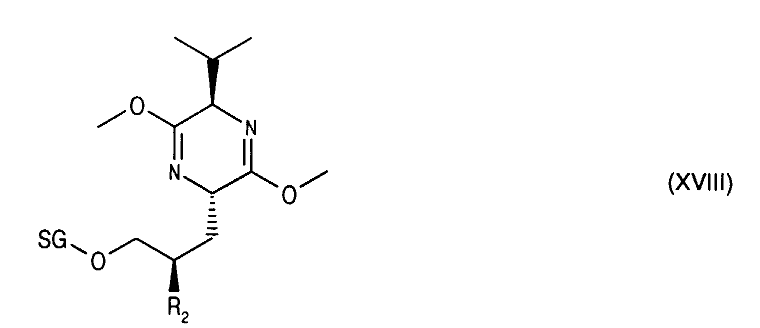 zunächst in üblicher Weise, z.B. in Gegenwart von verdünnter Salzsäure hydrolysiert und anschließend mit Ditertiärbutyldicarbonat umsetzt, die erhaltene Verbindung der Formel XIX 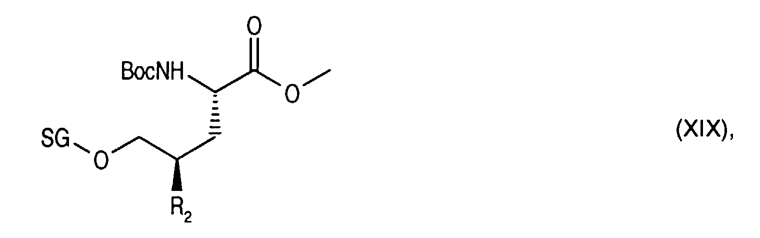 worin Boc Tertiärbutyloxycarbonyl bedeutet, nacheinander mit Dibutylaluminiumhydrid, mit einem N-R₅-Methacrylamid, Butyllithium und Triisopropyloxytitanchlorid, nach Auftrennung des erhaltenen Stereoisomerengemisches mit Wasserstoff in Gegenwart von [Ru₂Cl₄-(S)-(BINAP)₂]NEt₃ und mit Dimethoxypropen sowie mit p-Toluolsulfonsäure umsetzt und in der erhaltenen Verbindung der Formel XX 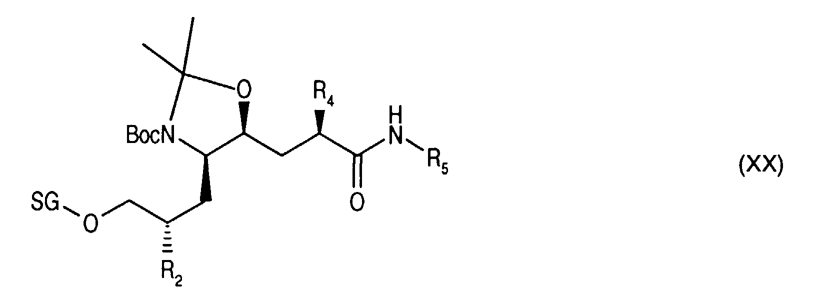 die geschützte Hydroxygruppe SG-O- in üblicher Weise, beispielsweise durch hydrogenolytische Entbenzylierung, beispielsweise mittels Wasserstoff in Gegenwart von Palladiumkohle, Umsetzung mit einem Sulfonylhalogenid, wie Methansulfonylchlorid, Weiterumsetzung mit einem Alkalimetallazid, wie Natriumazid, und emeute Hydrierung, beispielsweise mittels Wasserstoff in Gegenwart von Palladiumkohle in Amino überführt.

### Verfahrensvariante c): (Reduktion der Azidogruppe):

In Ausgangsmaterialien der Formel VI sind funktionelle Gruppen, die nicht an der Reaktion teilnehmen sollen, durch eine der unter Verfahren a) genannten Schutzgruppen geschützt.

Zur Reduktion der Azidogruppe eignen sich solche Reduktionsmittel, die unter den Reaktionsbedingungen des Verfahrens eine gegebenenfalls funktionalisierte Hydroxygruppe oder Azidogruppe selektiv oder schneller als die in Verbindungen der Formel I vorhandenen Amidgruppen reduzieren.

Die Reduktion wird vorzugsweise mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, durchgeführt.

Zwischenprodukte der Formel VI können beispielsweise hergestellt werden, indem man E-1,4-Dibrombut-2-en zunächst mit einer Verbindung der Formel VII 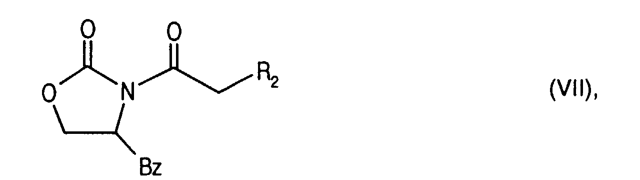 und anschließend mit einer Verbindung der Formel VIII 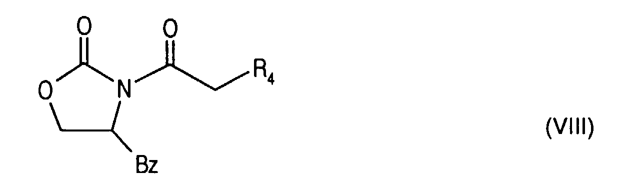 zur entsprechenden Verbindung der Formel IX 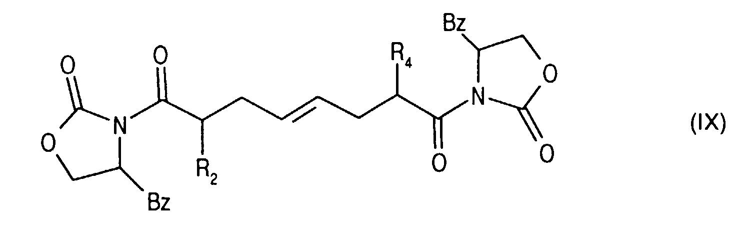 umsetzt, diese, beispielsweise durch Behandeln mit einem üblichen Halogenierungsmittel, wie elementarem Halogen, insbesondere Brom oder Jod, oder vorzugsweise mit einem N-Halogensuccinimid, insbesondere N-Bromsuccinimid in 1,2-Dimethoxyethan (DME), in die entsprechende Verbindung der Formel X 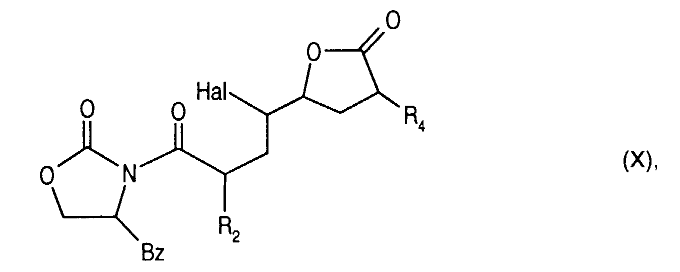 worin Hal Halogen bedeutet, überführt, das gewünschte Isomere in Bezug auf R₂ bzw. R₄ abtrennt und in diesem das Halogenatom, beispielsweise durch Behandeln mit Tetrabenzylammoniumazid in Toluol, durch Azido ersetzt und in der erhaltenen Verbindung der Formel XI 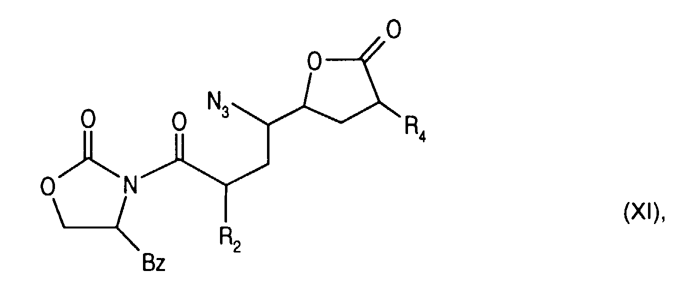 worin R₂ und R₄ die vorstehend angegebenen Bedeutungen haben und Bz Benzyl bedeutet, die 4-Benzyl-2-oxo-oxazolidin-1-ylcarbonylgruppe selektiv zu Carboxy hydrolysiert, einen dabei gegebenenfalls geöffneten Laktonring unter Verwendung eines sauren Katalysators wieder schließt, die erhaltene Verbindung der Formel XII 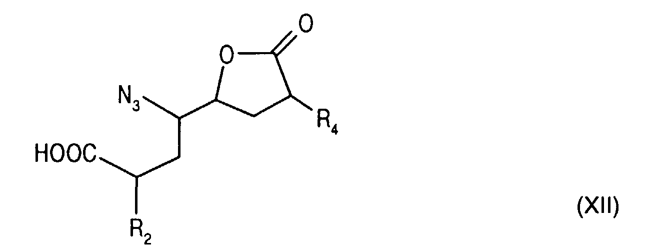 bzw. ein reaktionsfähiges funktionelles Carboxyderivat davon mit einer Verbindungen der Formel IV

R₁-Y₅ (IV),

worin Y₅ für Aminomethyl steht, kondensiert und die erhaltene Verbindung der Formel XXI 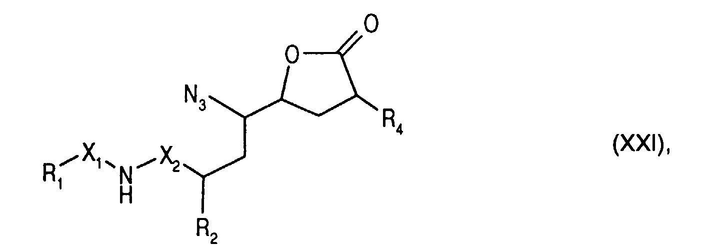 worin X₁ für Methylen und X₂ für Carbonyl steht, in üblicher Weise, beispielsweise wie unter der Verfahrensvariante a) beschrieben, mit einem Amin der Formel III

H₂N-R₅ (III),

worin R₅ eine der unter der Formel I angegebenen Bedeutungen hat, kondensiert.

Zwischenprodukte der Formel VI, worin X₁ Carbonyl und X₂ Methylen ist, kann man beispielsweise herstellen, indem man auf der Stufe der Verbindung der Formel XII die Carboxygruppe in Aminomethyl überführt, insbesondere in analoger Weise wie für die Herstellung von Verbindungen der Formel llb beschrieben.

Die sich an die vorstehend beschriebenen Verfahrensvarianten gegebenenfalls anschließende Abspaltung der Schutzgruppen, die nicht Bestandteil des gewünschten Endproduktes der Formel I sind, z.B. der Carboxy-, Amino-, Hydroxy- und/oder Mercaptoschutzgruppen, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemischer Reduktion, sowie Photolyse, gegebenenfalls stufenweise oder gleichzeitig, wobei auch enzymatische Methoden verwendet werden können. Die Abspaltung der Schutzgruppen ist beispielsweise in den weiter vom im Abschnitt über "Schutzgruppen" genannten Standardwerken beschrieben.

So kann beispielsweise geschütztes Carboxy, z.B. tertiäres Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z.B. mittels Hydrogenolyse, d.h. durch Behandeln mit Wasserstoff in Gegenwart eines metallischen Hydrierungskatalysators, wie eines Palladiumkatalysators, freigesetzt werden. Femer kann geeignet substituiertes Benzyloxycarbonyl, wie 4-Nitrobenzyloxycarbonyl, auch durch Reduktion, z.B. durch Behandeln mit einem Alkalimetall, wie Natriumdithionit, oder mit einem reduzierenden Metall, z.B. Zink, oder reduzierenden Metallsalz, wie einem Chrom-ll-salz, z.B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glykolsäure, Diphenylglykolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-lodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagenz, wie Natriumthiophenolat oder Natriumjodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines makrocyclischen Polyethers ("Kronenether"), oder mit einem Fluorid einer organischen quaternären Base, wie Tetraniederalkylammoniumfluorid oder Triniederalkylarylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z.B. Trimethylsilyloxycarbonyl, geschütztes Carboxy kann in üblicher Weise solvolytisch, z.B. durch Behandeln mit Wasser, einem Alkohol oder Säure, oder außerdem Fluorid, wie oben beschrieben, freigesetzt werden. Verestertes Carboxy kann auch enzymatisch freigesetzt werden, beispielsweise durch Esterasen oder geeignete Peptidasen.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-lodniederalkoxycarbonyl aminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wäßriger Essigsäure, gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einem Alkalimetall, z.B. Natriumdithionit, gespalten werden. Gegebenenfalls substituiertes Diphenylmethoxycarbonylamino, Tertiämiederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierungskatalysators, wie eines Palladiumkatalysators, gegebenenfalls substituiertes Triarylmethylamino oder Formylamino z.B. durch Behandeln mit einer Säure, wie Mineralsäure, z.B.

Chlorwasserstoffsäure, oder einer organischen Säure, z.B. Ameisen-, Essig- oder Trifluoressigsäure, gegebenenfalls in Gegenwart von Wasser, und eine als Silylamino geschützten Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschließende Solvolyse, wie Alkoholyse oder Hydrolyse, des entstandenen Kondensationsproduktes freigesetzt werden. Eine durch 2-(trisubstituiertes Silyl)-niederalkoxycarbonyl, wie 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechenden geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino überführt, beispielsweise durch katalytische Hydrierung mit Wasserstoff in Gegenwart eines Hydrierungskatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20° C bis 25° C, oder auch unter Kühlen oder Erwärmen, durchgeführt. Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thiaaliphatischen oder -cycloaliphatischen Kohlenwasserstoffrest geschützte Hydroxy bzw. Mercaptogruppe durch Acidolyse, z.B. durch Behandeln mit einer Mineralsäure oder einer starken Carbonsäure, z.B. Trifluoressigsäure, freigesetzt. Durch Pyridyldiphenylmethyl geschütztes Mercapto kann z.B. durch Quecksilber-II-salze bei pH 2-6, der durch Zink/Essigsäure oder elektrolytische Reduktion, Acetamidomethyl und Isobutyrylamidomethyl z.B. durch Reaktion mit Quecksilber-II-salzen bei pH 2-6, 2-Chloracetamidomethyl z.B. durch 1-Piperidinothiocarboxamid, S-Ethylthio, S-tert-Butylthio und S-Sulfo z.B. durch Thiolyse mit Thiophenol, Thioglykolsäure, Natriumthiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z.B. einer durch Alkyl ein- oder zweifach substituierten Methylengruppe, wie durch Niederalkyliden, z.B. Isopropyliden, Cycloyalkyliden, z.B. Cyclohexyliden, oder Benzyliden, geschützt sind, können durch saure Solvolyse, besonders in Gegenwart einer Mineralsäure oder einer starken organischen Säure, freigesetzt werden. 2-Halogenniederalkoxycarbonyl wird auch durch die oben genannten Reduktionsmittel, z.B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt.

Beim Vorhandensein von mehreren geschützten funktionellen Gruppen können, wenn erwünscht, die Schutzgruppen so gewählt werden, daß gleichzeitig mehr als eine solche Gruppe abgespalten werden kann, beispielsweise acidolytisch, wie durch Behandeln mit Trifluoressigsäure, oder mit Wasserstoff und einem Hydrierungskatalysator, wie einem Palladium/Kohle-Katalysator. Umgekehrt können die Gruppen auch so gewählt werden, daß sie nicht alle gleichzeitig, sondem in gewünschter Reihenfolge oder nur teilweise abgespalten werden.

Bei jedem der oben genannten Verfahren können die Ausgangsverbindungen auch als Salze verwendet werden, sofern die Reaktionsbedingungen dies zulassen.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemäβen Verfahren erhältliche Verbindung unter den Verfahrensbedingungen erzeugt und in situ weiterverarbeitet. Dabei geht man vorzugsweise von solchen Ausgangsstoffen aus, die zu den oben als sehr bevorzugt oder ganz besonders bevorzugt beschriebenen Verbindungen führen.

Die neuen Ausgangsstoffe, die speziell für die Herstellung der erfindungsgemäßen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Verfahrensgemäss erhältliche Verbindungen der Formeln I können in üblicher Weise in andere Verbindungen der Formeln I überführt werden.

So kann man in einer verfahrensgemäß erhältlichen Verbindung der Formeln I eine in freier oder in reaktionsfähiger Form vorliegende Carboxygruppe verestem oder amidieren bzw. eine veresterte oder amidierte Carboxygruppe in eine freie Carboxygruppe überführen.

Zur Veresterung oder Amidierung einer Carboxygruppe in einer Verbindung der Formel I kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z.B. mit Diazomethan, Meerweinsalzen oder 1-substituierten 3-Aryltriazenen.

Zur Überführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung nach den im Organikum, 17. Auflage, VEB Deutscher Verlag der Wissenschaften, Berlin 1988, genannten Reaktionsbedingungen angewendet werden.

In einer verfahrensgemäß erhältlichen Verbindung der Formel I kann man eine veresterte Carboxygruppe durch Aminolyse mit Ammoniak oder einem primären oder sekundären Amin, gegebenenfalls in Gegenwart eines geeigneten Kondensationsmittels oder Katalysators, in eine gegebenenfalls substituierte Carboxamidgruppe überführen. Die Aminolyse kann nach den im Organikum, 15. Auflage, VEB Deutscher Verlag der Wissenschaften, Berlin (Ost) 1976, für derartige Umsetzungen genannten Reaktionsbedingungen erfolgen.

In einer verfahrensgemäß erhältlichen Verbindung der Formel I kann man eine vorhandene freie Aminogruppe acylieren oder alkylieren, beispielsweise zur Einführung eines Restes R₆ außer Wasserstoff. Die Acylierung und die Alkylierung können erfolgen nach einer der für Schutzgruppen genannten Methoden oder nach einem der im Organikum, 17. Auflage, VEB Deutscher Verlag der Wissenschaften, Berlin (Ost) 1988, genannten Verfahren.

Weiterhin kann man in einer verfahrensgemäβ erhältlichen Verbindung der Formel I eine vorhandene freie Hydroxygruppe, beispielsweise als Bestandteil des Restes R₅, acylieren. Die Acylierung kann erfolgen mit acylierenden Reagenzien nach einer der für Schutzgruppen genannten Methoden oder nach einem der im Organikum, 17. Auflage, VEB Deutscher Verlag der Wissenschaften, Berlin (Ost) 1988, genannten Verfahren.

In einer verfahrensgemäß erhältlichen Verbindung der Formel I kann man weiterhin aus einem Sulfid das entsprechende Sulfoxid oder Sulfon herstellen, d.h. eine Thiogruppe zur Sulfinyl- oder Sulfonylgruppe bzw. ein Sulfinylgruppe zu Sulfonyl, ebenso Thiomorpholino zu S-Oxy- oder S,S-Dioxythiomorpholino oxidieren.

Die Oxidation zum Sulfon kann mit den meisten der üblichen Oxidationsmittel durchgeführt werden. Besonders bevorzugt verwendet man solche Oxidationsmittel, die die Thiogruppe oder den Sulfidschwefel selektiv in Gegenwart anderer funktioneller Gruppen der jeweiligen Verbindung der Formel I, z.B. von Amino oder Hydroxygruppen, oxidieren, beispielsweise aromatische oder aliphatische Peroxycarbonsäuren, z.B. Peroxybenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffen, z.B. Methylenchlorid oder Chloroform, Ethern, wie Diethylether, Estern, wie Essigester oder dergleichen, bei Temperaturen zwischen -78° C und Raumtemperatur, z.B. zwischen -20° C und +10° C, bevorzugt um 0° C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z.B. Methanol/Wasser oder Ethanol/Wasser, oder in wäßriger Essigsäure bei Temperaturen zwischen -70° C und +30° C, z.B. zwischen -20° C und Raumtemperatur, ferner Natriummetaperjodat in Methanol oder Methanol/Wasser-Gemischen bei Temperaturen zwischen 0° C und 50° C, z.B. um Raumtemperatur. Bei Einsatz stöchiometrischer Mengen der genannten Oxidationsmittel können auch die entsprechenden Sulfoxide erhalten werden.

Gewünschtenfalls kann durch Reduktion einer Sulfonylgruppe oder eines Sulfonrestes in einer erhältlichen Verbindung der Formel I die entsprechende Thioverbindung oder das entsprechende Sulfid erhalten werden, beispielsweise mit Diisobutylaluminiumhydrid in Ether oder Tetrahydrofuran.

In Verbindungen der Formeln I kann man ferner eine freie oder veresterte Carboxygruppe in üblicher Weise, beispielsweise mittels eines Dileichtmetallhydrides, wie Lithiumaluminiumhydrid oder Natriumboranat, in einem inerten Lösungsmittel, wie einem Ether, z.B. in Tetrahydrofuran, zu Hydroxymethyl reduzieren.

In Verbindungen der Formeln I kann man ferner Hydroxy R_{A}, R_{B} und/oder R_{C} durch eine der unter den Formeln I genannten veretherten Hydroxygruppen ersetzen, indem man die entsprechende Verbindung der Formeln I, worin R_{A}, R_{B} und/oder R_{C} Hydroxy ist, in üblicher Weise, beispielsweise in Gegenwart eines basischen Kondensationsmittels, mit einer Verbindung der Formeln R_{A}-Y, R_{B}-Y und/oder R_{C}-Y umsetzt, worin einer der Reste R_{A} und R_{B} einen aliphatischen, araliphatischen oder heteroaraliphatischen Rest, z.B. einen gegebenenfalls N-niederalkanoylierten, N-mono- oder N,N-diniederalkylierten oder durch Niederalkylen, Hydroxy-, Niederalkoxy- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituierten Aminoniederalkoxyrest, Niederalkoxy, Hydroxyniederalkoxy, Niederalkanoyloxyniederalkoxy, Niederalkoxyniederalkoxy, Niederalkoxyniederalkoxyniederalkoxy, Polyhalogenniederalkoxy, Cyanniederalkoxy, gegebenenfalls substituiertes Phenyl- oder Pyridylniederalkoxy, Niederalkoxyniederalkenyloxy, gegebenenfalls S-oxidiertes Niederalkylthioniederalkoxy oder gegebenenfalls N-niederalkanoyliertes, N-mono- oder N,N-diniederalkyliertes oder durch Niederalkylen, Hydroxy-, Niederalkoxy- oder Niederalkoxyniederalkoxyniederalkylen oder gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkoxy und der andere Wasserstoff, Niederalkyl, Carbamoyl, Hydroxy, Niederalkoxy oder Polyhalogenniederalkoxy, bedeutet, R_{C} einen aliphatische, araliphatischen, heteroaraliphatischen oder heteroarylaliphatischen Rest, z.B. Hydroxy, Niederalkoxy, Hydroxyniederalkoxy, Niederalkoxyniederalkoxy, Morpholinoniederalkycarbamoyl niederalkoxy, eine gegebenenfalls N-niederalkanoylierte, N-mono- oder N,N-diniederalkylierte oder durch Niederalkylen, Hydroxy-, Niederalkoxy-, Niederalkoxycarbonyl- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituierte Aminoniederalkoxygruppe, eine gegebenenfalls amidierte Carboxy- oder Carboxyniederalkoxygruppe oder Tetrazolylniederalkoxy, bedeutet und Y reaktionsfähiges verestertes Hydroxy, insbesondere mit einer Mineralsäure, mit Schwefelsäure oder mit einer organischen Sulfonsäure veresterte Hydroxygruppen, wie Halogen, vorzugsweise Chlor, Brom oder Jod, Gruppen der Formeln -O-SO₂-O-R_{A}, -O-SO₂-O-R_{B} oder -O-SO₂-O-R_{A} oder Niederalkan- oder gegebenenfalls substituiertes Benzolsulfonyloxy, insbesondere Methan-, Ethan-, Benzol-, p-Toluol- oder p-Brombenzolsulfonyl, veresterte Hydroxy bedeutet.

Die Umsetzung erfolgt, wie erwähnt, vorzugsweise in Gegenwart eines basischen Kondensationsmittels, wie einem Alkalimetallcarbonat, beispielsweise von Kaliumcarbonat, in einem inerten Lösungsmittel, wie einem Niederalkanol, wie Methanol, Ethanol, Butanol, Tertiärbutanol oder insbesondere Amylalkohol, vorteilhaft bei erhöhter Temperatur, beispielsweise im Temperaturbereich von etwa 40° bis 140°C, erforderlichenfalls unter, beispielsweise azeotroper, Abdestillation des gebildeten Reaktionswassers.

Ferner kann man verfahrensgemäß erhältliche Salze von Verbindungen der Formeln I in an sich bekannter Weise in die freien Verbindungen umwandeln, z.B. durch Behandeln mit einer Base, wie einem Alkalimetallhydroxid, einem Metallcarbonat oder --hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formeln I, einschließlich ihrer Salze, können auch in Form von Hydraten erhalten werden oder das zur Kristallisation verwendete Lösungsmittel einschließen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z.B. optisch aktiven Säuren oder Basen, durch Chromatographie an mit optisch aktiven Verbindungen belegten Säulenmaterialien oder durch enzymatische Methoden, z.B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

An einzelnen Chiralitätszentren in einer Verbindung der Formel I kann die Konfiguration gezielt umgekehrt werden. Beispielsweise kann man die Konfiguration asymmetrischer Kohlenstoffatome, welche nukleophile Substituenten, wie Amino oder Hydroxy, tragen, durch nukleophile Substitution zweiter Ordnung, gegebenenfalls nach Überführung des gebundenen nukleophilen Substituenten in eine geeignete nukleofuge Abgangsgruppe und Reaktion mit einem den ursprünglichen Substituenten einführenden Reagenz, umkehren, oder man kann die Konfiguration an Kohlenstoffatomen mit Hydroxygruppen durch Oxidation und Reduktion, analog dem Verfahren in der Europäischen Patentanmeldung EP-A-0 236 734, umkehren.

Vorteilhaft ist auch die reaktionsfähig funktionelle Abwandlung der Hydroxygruppe und anschließender Ersatz derselben durch Hydroxy unter Konfigurationsumkehr. Dazu werden die in Formel I eingezeichnete Amino- und Hydroxygruppe durch eine bivalente Gruppe, insbesondere Carbonyl überbrückt, wobei man eine Verbindung der Formel XXII 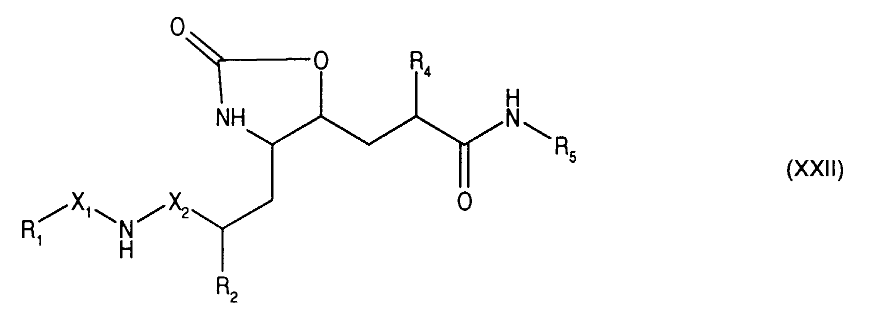 erhält, die bei Behandlung mit Thionylchlorid unter Konfigurationsumkehr wieder gespalten werden kann.

Die Erfindung betrifft ferner pharmazeutische Präparate enthaltende Verbindungen der Formel I.

Die pharmakologisch verwendbaren Verbindungen der vorliegenden Erfindung können z.B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemäßen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die pharmazeutischen Präparate enthalten von etwa 1 % bis etwa 95 %, vorzugsweise von etwa 20 % bis etwa 90 % des Wirkstoffes. Erfindungsgemäße pharmazeutische Präparate können z.B. in Dosiseinheitsform, wie Ampullen, Vials, Suppositorien, Dragees, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z.B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z.B. Ölsäure, Elaidinsäure, Erucasäure, Brasidinsäure oder Linolsäure, enthalten, gegebenenfalls unter Zusatz von Antioxidantien, wie z.B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Ethanol, Propanol, Butanol oder Pentanol oder deren Isomere, vor allem aber Glykol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Myglyol 812" (Triglycerid gesättigter Fettsäuren der Kettenlänge C₈ bis C₁₂ der Firma Chemische Werke Witten/Ruhr, Deutschland), besonders aber vegetabile Öle wie Baumwollsaatöl, Mandelöl, Olivenöl, Rizinusöl, Sesamöl, Sojabohnenöl und vor allem Erdnußöl.

Die Herstellung der Injektionspräparate erfolgt in üblicher Weise unter sterilen Bedingungen, ebenso das Abfüllen in Ampullen oder Vialen sowie das Verschließen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch, wenn erwünscht oder notwendig, nach Zugabe von geeigneten Hilfsstoffen, zu Tabletten, Dragee-Kemen oder Kapseln verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate, und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fließregulier- und Schmiermittel, z.B. Kieselsäure, Talk, Stearinsäure oder Salze davon, wie Magnesium- oder Calciumstearat, und/oder Polyethylenglykol. Dragee-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropyl methylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulates, z.B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragee-Überzügen und den Kapselhüllen können Farbstoffe oder Pigmente, z.B. zur Identifizierung oder zur Kennzeichnung verschiedener Wirkstoffdosen, beigefügt werden.

Die Erfindung betrifft auch die Verwendung von Verbindungen der Formel I zur Behandlung von auf Reninhemmung ansprechenden Erkrankungen, wie der eingangs genannten, insbesondere von Bluthochdruck und/oder Glaukom.

Die am Warmblüter, z.B. Menschen, von beispielsweise etwa 70 kg Körpergewicht, zu verabreichenden Dosismengen, insbesondere die zur Hemmung des Enzyms Renin, zur Blutdrucksenkung und/oder zur Besserung der Glaukomsymptomatik wirksamen Dosen, liegen zwischen etwa 3 mg und etwa 3 g, vorzugsweise zwischen etwa 10 mg und etwa 1 g, z.B. bei ungefähr 20 bis 200 mg pro Person und Tag, verteilt auf vorzugsweise 1 bis 4 Einzeldosen, die z.B. gleich groß sein können. Üblicherweise erhalten Kinder etwa die halbe Dosis von Erwachsenen. Die individuell notwendige Dosierung kann z.B. durch Messung der Serumkonzentration des Wirkstoffes überwacht und optimal eingestellt werden.

Die nachfolgen Beispiele dienen zur Illustration der Erfindung; Temperaturen sind in Celsiusgraden, Drucke in mbar angegeben.

### DC-Fließmittelsysteme:

- A: Hexan - Essigsäureethylester ( 9:1)
- B: Hexan - Essigsäureethylester ( 4:1)
- C: Hexan - Essigsäureethylester ( 3:1)
- D: Hexan - Essigsäureethylester ( 2:1)
- E: Hexan - Essigsäureethylester ( 1:1)
- F: Hexan - Essigsäureethylester ( 1:2)
- G: Hexan - Essigsäureethylester - Eisessig (50:50:1)
- H: Essigsäureethylester - Methanol - Ammoniak Konz. (98: 1:1)
- l: Essigsäureethylester - Methanol - Ammoniak Konz. (90:10:1)
- J: Essigsäureethylester - Methanol - Ammoniak Konz. (80:15:5)
- K: Essigsäureethylester - Methanol - Ammoniak Konz. (40:45:5)
- L: Dichlormethan - Methanol (90:10)
- M: Dichlormethan - Methanol (92: 8)
- N: Dichlormethan - Methanol (95: 5)
- O: Dichlormethan - Methanol (96: 4)
- P: Dichlormethan - Methanol (97: 3)
- Q: Dichlormethan - Methanol (98: 2)
- R: Dichlormethan - Methanol (99: 1)
- S: Dichlormethan - Methanol - Ammoniak Konz. (99: 1:1)
- T: Dichlormethan - Methanol - Ammoniak Konz. (98: 2:1)
- U: Dichlormethan - Methanol - Ammoniak Konz. (96: 4:1)
- V: Dichlormethan - Methanol - Ammoniak Konz. (97: 3:1)
- W: Dichlormethan - Methanol - Ammoniak Konz. (90:10:1)
- X: Dichlormethan - Methanol - Essigsäure (90:10:1)
- Y: Dichlormethan - Methanol - Essigsäure (95: 5:1)

### HPLC-Fließmittelgradienten auf C18-Nucleosil® (5 µM), Säulenlänge 25 cm:

20% Acetonitril/80% Wasser/0.1% Trifluoressigsäure nach 100% Acetonitril/0% Wasser/0.1% Trifluoressigsäure in 20 min, anschließend 100 % Acetonitril/0.1% Trifluoressigsäure in 8 min.

Die Abkürzung "R_{f}(A)" bedeutet beispielsweise, daß der R_{f}-Wert im Lösungsmittelsystem A ermittelt wird. Das Mengenverhältnis von Lösungsmitteln zueinander ist stets in Volumenteilen angegeben.

Für die Bezeichnung der Fließmittelsysteme werden bei der Flash-Chromatographie und der Mitteldruckchromatographie die gleichen Abkürzungen verwendet.

### Die verwendeten Kurzbezeichnungen und Abkürzungen haben die folgende Bedeutung:

- RT: Raumtemperatur
- HV: Hochvakuum
- bar: Druck in Bar
- C18-Nucleosil®: Handelsname für mit Octadecyl resten belegtes "Reversed Phase"-Säulenmaterial fü r HPLC
- FAB-MS: Fast-Atom-Bombardment Mass-Spectroscopy
- HRMS(FAB): High Resolution Fast-Atom-Bombardment Mass-Spectroscopy
- DC: Dünnschicht-Chromatographie
- FC: Flash-Säulenchromatographie
- HPLC: Hochleistungs-Flüssigchromatographie
- Hyflo®: Handelsname für Filterhilfsmittel (Fluka, Buchs, Schweiz)
- h: Stunde(n)
- min: Minute(n)
- Kp: Siedepunkt beim in Torr angegebenen Druck
- mL: Milliliter
- R_{f}: Verhältnis von Laufstrecke einer Substanz zu Entfernung der Laufmittelfront vom Startpunkt bei DC
- Rₜ: Retentionszeit einer Substanz bei HPLC
- Schmp.: Schmelzpunkt

### Beispiel 1:

Die Mischung von 2-(3-Methoxypropoxy)-benzoesäure (0.105 g), Bis-(2-oxo-3-oxazolidinyl)-phosphinsäurechlorid (0.127 g) und Triethylamin (0.140 mL) in Dichlormethan (2 mL) wird bei RT während 1 h gerührt. Anschließend werden eine Lösung aus (2R,4'S,5'S,2''S)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid (0.111 g) in Dichlormethan (2 mL) und 4-Dimethylaminopyridin (0.024 g) addiert, und das Reaktionsgemisch wird über Nacht gerührt. Nach dem Abdampfen des Lösungsmittels wird der Rückstand mit gesättigter Natriumhydrogencarbonat-Lösung (30 mL) versetzt und anschließend mit Essigsäureethylester (3x 30 mL) extrahiert. Die organischen Phasen werden über Magnesiumsulfat getrocknet, eingedampft und durch FC (20 g KG, Fließmittel F) gereinigt. Man erhält das (2S,4'S,5'S,2''R)-N-{2-[5'-(2''-Butylcarbamoylpropyl)-3'-(*tert*-butoxycarbonyl)-2',2'-dimethyl-1,3-oxazolidin-4'-ylmethyl]-3-methylbutyl}-2-(3-methoxypropoxy)-benzamid (0.112 g) als farbloses Öl: R_{f}(F) = 0.28. HPLC Rₜ = 21.0 min .

Das als Ausgangsmaterial eingesetzte (2R,4'S,5'S,2''S)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid wird folgendermaßen hergestellt:
a) (2R,4'S,5'S,2''S)-3-[4''-(2''-Aminomethyl-3'-methylbutyl)-3'-(*tert*-butoxycarbonyl)-2',2'dimethyl-1,3-oxazolidin-5'-yl]-2-methylpropionsäure-N-(butyl)amid: (2R,4'S,5'S,2''S)-3-[4'-(2''-Azidomethyl-3''-methylbutyl)-3'-(*tert*-butoxycarbonyl)-2',2'-dimethyl-1,3-oxazolidin-5'-yl]-2-methylpropionsäure-N-(butyl)amid (0.435 g) gelöst in Essigsäureethylester (25 mL), wird in Gegenwart von Pd/C 10% (0.100 g) bei RT unter Normaldruck über 2 h hydriert. Nach Filtration über Hyflo® und Entfernen des Lösungsmittels erhält man 0.41 g der rohen Titelverbindung als blaßgelbes Öl: Rf (Dichlormethan-Methanol-Ammoniak konz. 350:50:1) = 0.19 . HPLC Rₜ = 13.6 min . MS(FAB) *m*/e 442 (M⁺ +1).
b) (2R,4'S,5'S,2''S)-3-[4''-(2''-Azidomethyl-3''-methylbutyl)-3'-(*tert*-butoxycarbonyl)-2',2'-dimethyl-1,3-oxazolidin-5'-yl]-2-methylpropionsäure-N-(butyl)amid: Die Mischung von (2S,4'S,5'S,2''R)-Methansulfonsäure-{2-[5'-(2''-butylcarbamoylpropyl)-3'-(*tert*-butoxycarbonyl)-2',2'-dimethyl-1,3-oxazolidin-4'-ylmethyl]-3-methylbutyl}ester (0.52 g) und Natriumazid (0.65 g) in 1,3-Dimethyl-3,4,5,6-tetrahydro-2(1 *H*)-pyrimidinon (5 mL) wird bei 50° C während 5 h gerührt. Das abgekühlte Reaktionsgemisch wird auf Wasser (100 mL) gegossen und mit Diethylether (3x 100 mL) extrahiert. Die organischen Phasen werden mit Wasser (2x 100 mL) und Sole (100 mL) gewaschen, über Magnesiumsulfat getrocknet und eingedampft. Aus dem Eindampfrückstand erhält man nach FC (35 g KG, Fließmittel D) die Titelverbindung (0.438 g) als blaßgelbes Öl: R_{f} (E) = 0.49 . HPLC Rₜ = 21.0 min . MS(FAB) *m/e* 468 (M⁺ +1).
c) (2S,4'S,5'S,2''R)-Methansulfonsäure-{2-[5'-(2''-butylcarbamoylpropyl)-3'-(*tert*-butoxycarbonyl)-2',2'-dimethyl-1,3-oxazolidin-4'-ylmethyl]-3-methylbutyl}ester: Zu einer gerührten Lösung von (2R,4'S,5'S,2''S)-3-[4'-(2''-Hydroxymethyl-3''-methyl-butyl)-3'-(*tert*-butoxycarbonyl)-2',2'-dimethyl-1,3-oxazolidin-5'-yl]-2-methylpropionsäure-N-(butyl)amid (0.442 g) in Dichlormethan (15 mL) werden bei 0° C zunächst Triethylamin (0.418 mL) und anschließend Methansulfonsäurechlorid (0.117 mL) addiert. Das Reaktionsgemisch wird 1 h bei 0° C gerührt und anschließend das Lösungsmittel zur Hälfte eingeengt. Durch FC (25 g KG, Fließmittel E ) erhält man die Titelverbindung (0.51 g) als farbloses Öl. R_{f}(E) = 0.27 . HPLC Rₜ = 18.5 min. MS(FAB) *m/e* 521 (M⁺ +1).
d) (2R,4'S,5'S,2''S)-3-[4'-(2''-Hydroxymethyl-3''-methyl-butyl)-3'-(*tert*-butoxycarbonyl)-2',2'-dimethyl-1,3-oxazolidin-5'-yl]-2-methylpropionsäure-N-(butyl)amid: (2R,4'S,5'S,2''S)-3-[4'-(2''-Benzyloxymethyl-3''-methyl-butyl)-3'-(*tert*-butoxycarbonyl)-2',2'-dimethyl-1,3-oxazolidin-5'-yl]-2-methylpropionsäure-N-(butyl)amid (2.20 g), gelöst in Tetrahydrofuran (60 mL), wird in Gegenwart von Pd/C 10% Degussa E 101 N (0.220 g) bei RT und Normaldruck über 0.5 h hydriert. Nach Filtration über Hyflo® und Entfernen des Lösungsmittels erhält man die Titelverbindung (1.79 g) als farbloses Öl: R_{f}(E) = 0.23 . HPLC Rₜ = 18.3 min . MS(FAB) *m/e* 443 (M⁺ +1).
e) (2R,4'S,5'S,2''S)-3-[4'-(2''-Benzyloxymethyl-3''-methyl-butyl)-3'-(*tert*-butoxycarbonyl)-2',2'-dimethyl-1,3-oxazolidin-5'-yl]-2-methylpropionsäure-N-(butyl)amid: Die Lösung aus (2RS,4S,5S,7S)-7-Benzyloxymethyl-5-(*tert*-butoxycarbonyl)amino-4-hydroxy-2,8-dimethylnonansäure-N(butyl)amid (3.0 g) in Dichlormethan (30 mL) wird unter Rühren nacheinander mit 2',2'-Dimethoxypropan (5 mL) und p-Toluolsulfonsäure (0.01 g) versetzt und das Reaktionsgemisch anschließend während 20 h stehen gelassen. Das Lösungsmittel wird eingeengt und das Rohprodukt bestehend aus den beiden (2S,4'S,5'S,2"S)- und (2R,4'S,5'S,2''S)-Diastereomeren im Verhältnis 2 : 8 (HPLC Rₜ = 23.3/23.5 min) chromatographiert (FC an 160 g KG, Fließmittel C). Man erhält die stereoisomerenreine Titelverbindung (2.22 g) als farbloses Öl: R_{f}(E) = 0.47 . HPLC Rₜ = 23.5 min .
f) (2RS,4S,5S,7S)-7-Benzyloxymethyl-5-(*tert*-butoxycarbonyl)amino-4-hydroxy-2,8-dimethylnonansäure-N-(butyl)amid: Eine Lösung von (2S,3S,5S)-2-[3-(*tert*-Butoxycarbonyl)amino-5-(benzyloxymethyl)-2-hydroxy-6-methylheptyl]-N-(butyl)acrylamid (A) (3.0 g) in wasserfreiem Methanol (25 mL) wird in Gegenwart von [Ru₂Cl₄[(S)-BINAP]₂]NEt₃ (39.5 mg) bei RT und 25 bar Druck während 22 h hydriert. Das Reaktionsgemisch wird eingedampft und anschließend durch FC (100 g KG, Fließmittel E) gereinigt. Man erhält die Titelverbindung (3.0 g) als blaßgelbes Öl: R_{f} (E) = 0.15 . HPLC Rₜ = 19.4 min .
g) (2S,3S,5S)-2-[3-(*tert*-Butoxycarbonyl)amino-5-(benzyloxymethyl)-2-hydroxy-6-methylheptyl]-N-(butyl)acrylamid (A) und (2R,3S,5S)-2-[3-(*tert*-Butoxycarbonyl)amino-5-(benzyloxymethyl)-2-hydroxy-6-methylheptyl]-N-(butyl)acrylamid (B): Eine Lösung von Methacrylsäure-N-(butyl)amid (7.92 g) in Tetrahydrofuran (125 mL) wird unter Rühren mit einer 1.6 M n-Butyllithium-Lösung in Hexan (73.3 mL) bei -75° C während 15 min versetzt. Das Reaktionsgemisch wird nach beendeter Zugabe 30 min bei 0° C gerührt, auf -75° C abgekühlt und mit einer M Chlortitantriisopropoxid-Lösung in Hexan (89.3 mL) tropfenweise über 40 min versetzt. Es wird noch 15 min bei -75° C gerührt und anschließend die Lösung von (2S,4S)-2-(*tert*-Butoxycarbonyl)amino-4-(benzyloxymethyl)-5-methyl-hexanal (9.10 g) in Tetrahydrofuran (90 mL) bei gleicher Temperatur über 15 min zugetropft. Das Reaktionsgemisch wird 75 min bei -75° C nachgerührt und anschließend bei -20° C mit gesättigter Ammoniumchlorid-Lösung (150 mL) versetzt. Die wässrige Phase wird mit Diethylether (3x 600 mL) extrahiert, und die vereinte organische Phase nacheinander mit Wasser (600 mL) und gesättigter Natriumchlorid-Lösung (600 mL) gewaschen, über Magnesiumsulfat getrocknet und eingedampft. Das Rohprodukt wird an 1.3 kg KG (Fließmittel C) unter Auftrennung des Diastereomerengemisches chromatographiert. Man erhält die Titelverbindung A (3.01 g) als blaßgelbes Öl: R_{f}(D) = 0.22. HPLC Rₜ = 20.1 min. Ferner erhält man die Titelverbindung B (5.70 g ) als blaßgelbes Öl: R_{f} (D) = 0.17. HPLC Rₜ = 19.87 min.
h) (2S,4S)-2-(*tert*-Butoxycarbonyl)amino-4-(benzyloxymethyl)-5-methyl-hexanal: Eine Lösung von (2S,4S)-2-(*tert*-Butoxycarbonyl)amino-4-(benzyloxymethyl)-5-methylhexansäuremethylester (9.70 g) in Toluol (100 mL) wird unter Rühren bei -75° C langsam mit einer 1.2 M Diisobutylaluminiumhydrid-Lösung in Toluol (51 mL) versetzt. Das Reaktionsgemisch wird noch 45 min bei gleicher Temperatur gerührt und anschließend vorsichtig mit Methanol (20 mL) versetzt. Die erhaltene Mischung wird auf 1 N Salzsäure/Eis (500 mL) gegossen und mit Essigsäureethylester (3x 500 mL) extrahiert. Die organischen Phasen werden nacheinander mit Wasser (2x 500 mL) und gesättigter Natriumchlorid-Lösung (500 mL) gewaschen, über Hyflo® klarfiltriert und über Magnesiumsulfat getrocknet. Das Lösungsmittel wird abgedampft und der Rückstand im HV getrocknet . Man erhält die rohe Titelverbindung (8.91 g) als farbloses Öl: R_{f} (B) = 0.25 . HPLC Rₜ = 19.2 min .
i) (2S,4S)-2-(*tert*-Butoxycarbonyl)amino-4-(benzyloxymethyl)-5-methylhexansäuremethylester: Eine Lösung von (2S,4S)-2-Amino-4-(benzyloxymethyl)-5-methylhexansäuremethylester (21.9 g) in Dichlormethan (0.5 L) wird unter Rühren bei 0° C nacheinander mit Ethyldiisopropylamin (17.4 mL) und einer Lösung von Di- tert-butyldicarbonat (18.8 g) in Dichlormethan (0.1 L) versetzt. Das Reaktionsgemisch wird noch 16 h bei RT gerührt und anschließend eingedampft. Aus dem Eindampfrückstand wird nach FC (2.4 kg KG, Essigsäureethylester-Hexan 1:6) die Titelverbindung (27.0 g ) als leicht gelbliches Öl erhalten. R_{f} (B) = 0.32 . HPLC Rₜ = 27.2 min .
j) (2S,4S)-2-Amino-4-(benzyloxymethyl)-5-methyl-hexansäuremethylester Eine Lösung von (2S,2'S,5R)-2-[2'-(Benzyloxymethyl)-3'-methylbutyl]-2,5-dihydro-5-isopropyl-3,6-dimethoxypyrazin (36.2 g) in Acetonitril (400 mL) wird bei RT mit einer 1 N Salzsäure-Lösung (400 mL) versetzt und während 2 h gerührt. Danach wird der Reaktionsansatz auf ein Gemisch von gesättigter Natriumhydrogencarbonat-Lösung und Eis (1 L) gegossen und mit Dichlormethan (3x 0.8 L) extrahiert. Die organischen Phasen werden mit Wasser (1 L) gewaschen, über Magnesiumsulfat getrocknet und eingedampft. Der Eindampfrückstand wird durch FC (2.4 kg KG, Dichlormethan-Methanol-Ammoniak konz. 95:5:0.1) gereinigt. Man erhält die Titelverbindung (21.9 g) als farbloses Öl: R_{f} (Dichlormethan-Methanol-Ammoniak konz. 700:50:1) = 0.34 . HPLC Rₜ = 13.6 min .
k) (2S,2'S,5R)-2-[2'-(Benzyloxymethyl)-3'-methylbutyl]-2,5-dihydro-5-isopropyl-3,6-dimethoxypyrazin: Eine Lösung von (2R)-2,5-Dihydro-2-isopropyl-3,6-dimethoxypyrazin (29.5 g) in absolutem Tetrahydrofuran (530 mL) wird bei -75° C tropfenweise mit einer 1.6 M n-Butyllithium-Lösung in Hexan (100 mL) versetzt. Das Reaktionsgemisch wird weitere 30 min bei -75° C gerührt und anschließend während 20 min mit einer Lösung von 2(S)-(Benzyloxymethyl)-3-methyl-butylbromid (29 g) in Tetrahydrofuran (130 mL) versetzt. Die Reaktionslösung wird noch 2 h bei -75° C gerührt und anschließend während 64 h bei -18° C stehen gelassen. Das Reaktionsgemisch wird eingedampft, mit Wasser (500 mL) versetzt und mit Diethylether (3x 500 mL) extrahiert. Die organischen Phasen werden mit gesättigter Natriumchlorid-Lösung (500 mL) gewaschen, über Magnesiumsulfat getrocknet und eingedampft. Der Eindampfrückstand wird durch FC (2.4 kg KG, Essigsäureethylester-Hexan 1:15) gereinigt, und man erhält die Titelverbindung (36.2 g) als gelbliches Öl: R_{f} (B) = 0.58. HPLC Rₜ = 25.8 min.

Die als Ausgangsstoff eingesetzte 2-(3-Methoxypropoxy)-benzoesäure wird folgendermaßen hergestellt:
a) Zu einer Lösung von 2-(3-Methoxypropoxy)-benzoesäureethylester (2.4 g) in Ethanol (20 mL) und Wasser (10 mL) wird 1 N Natronlauge (11.1 mL) addiert und das Reaktionsgemisch bei 50° C über 7 h gerührt. Der Ansatz wird eingeengt und die angesäuerte wässrige Phase mit Dichlormethan extrahiert (3x 40 mL). Die organische Phase wird mit gesättigter Natriumchlorid-Lösung gewaschen, über Magnesiumsulfat getrocknet und eingeengt. Man erhält die 2-(3-Methoxypropoxy)-benzoesäure, R_{f} (Hexan-Essigsäureethylester-Eisessig 1:2:0.1) = 0.43, als gelbliches Öl.
b) 2-(3-Methoxypropoxy)-benzoesäureethylester: Zu einer Lösung von Salicylsäureethylester (3.5 g) in wasserfreiem Aceton (50 mL) wird unter Rühren getrocknetes Kaliumcarbonat-Pulver (3.49 g) addiert und anschließend eine Lösung von 3-Methoxypropylbromid (4.83 g) in wasserfreiem Aceton (15 mL) rasch bei RT zugetropft. Die Suspension wird über 38 h unter Rückfluß erhitzt. Nach dem Abkühlen wird filtriert, das Filtrat eingeengt und der Rückstand durch FC (200 g KG, Fließmittel A) gereinigt. Man erhält die Titelverbindung, R_{f} (Hexan-Essigsäureethylester-Eisessig 1:1:0.1) = 0.39, als farbloses Öl.

### Beispiel 2:

In analoger Weise wie in Beispiel 1) beschrieben, werden die nachfolgenden Verbindungen hergestellt:
a) Aus 128 mg (2R,4'S,5'S,2''S)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 139 mg 3-Methoxy-2-(3-methoxypropoxy)-benzoesäure das (2S,4'S,5'S,2''R)-N-{2-[5'-(2''- Butylcarbamoylpropyl)-3'-(*tert*-butoxycarbonyl)-2',2'-dimethyl-1,3-oxazolidin-4'-ylmethyl]-3-methylbutyl}-3-methoxy-2-(3-methoxypropoxy)-benzamid, R_{f} (L) = 0.65; HPLC Rₜ = 20.9 min; MS(FAB) *m/e* 664 (M⁺ +1), als farbloses Öl.
b) Aus 128 mg (2R,4'S,5'S,2''S)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 139 mg 4-Methoxy-2-(3-methoxypropoxy)-benzoesäure das (2S,4'S,5'S,2''R)-N-{2-[5'-(2''-Butylcarbamoylpropyl)-3'-(*tert*-butoxycarbonyl)-2',2'-dimethyl-1,3-oxazolidin-4'-ylmethyl]-3-methylbutyl}-4-methoxy-2-(3-methoxypropoxy)-benzamid, R_{f} (N) = 0.17; HPLC Rₜ = 21.4 min; MS(FAB) *m/e* 664 (M⁺ +1), als farbloses Öl.
c) Aus 111 mg (2R,4'S,5'S,2''S)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 105 mg 3-(3-Methoxypropoxy)-benzoesäure das (2S,4'S,5'S,2''R)-N-{2-[5'-(2''-Butyl carbamoylpropyl)-3'-(*tert*-butoxycarbonyl)-2',2'-dimethyl-1,3-oxazolidin-4'-ylmethyl]-3-methylbutyl}-3-(3-methoxypropoxy)-benzamid, R_{f} (N) = 0.26; HPLC Rₜ = 20.7 min; MS(FAB) *m/e* 634 (M⁺ +1), als weißer Schaum.

Die als Ausgangsstoff eingesetzte 3-(3-Methoxypropoxy)-benzoesäure wird folgendermaßen hergestellt:
a) Durch alkalische Hydrolyse von 3-(3-Methoxypropoxy)-benzoesäuremethylester, analog wie in Beispiel 1) beschrieben, erhält man die Titelverbindung, R_{f} (Hexan-Essigsäureethylester-Eisessig 3:1:0.01) = 0.18; Schmp. 82-84° C, als Feststoff.
b) 3-(3-Methoxypropoxy)-benzoesäuremethylester Zu einer Lösung von 3-Hydroxybenzoesäuremethylester (2.04 g) in Tetrahydrofuran (50 mL) wird Natriumhydrid 80 %ige Dispersion in Öl (0.39 g) unter Rühren bei 0° C addiert. Nach 30 min Rühren wird eine Lösung von 3-Methoxypropylbromid (3.08 g) in Tetrahydrofuran (15 mL) bei 0° C zugetropft. Man erwärmt langsam auf 50° C und rührt die weiße Suspension weitere 30 h nach. Der Ansatz wird auf Eiswasser (40 mL) gegossen und die wässrige Phase mit Dichlormethan (3x 40 mL) extrahiert. Die vereinten organischen Phasen werden mit gesättigter Natriumchlorid-Lösung gewaschen, über Magnesiumsulfat getrocknet und eingeengt. FC Reinigung des Rohproduktes (100 g KG, Fließmittel A) ergibt die Titelverbindung, R_{f} (C) = 0.36, als gelbliches Öl.

Die weiter oben als Ausgangsmaterial eingesetzte 4-Methoxy-2-(3-methoxypropoxy)-benzoesäure und 3-Methoxy-2-(3-methoxypropoxy)-benzoesäure wird in analoger Weise wie in Beispiel 1) beschrieben hergestellt.

### Beispiel 3:

Die Lösung von (2S,4'S,5'S,2''R)-N-{2-[5'-(2''-Butylcarbamoylpropyl)-3'-(*tert*-butoxycarbonyl)-2',2'-dimethyl-1 ,3-oxazolidin-4'-ylmethyl]-3-methylbutyl}-2-(3-methoxypropoxy)-benzamid (105 mg) in Methanol (3 mL) wird unter Rühren mit p-Toluolsulfonsäure (2 mg) versetzt und während 24 h bei RT nachgerührt. Das Lösungsmittel wird bei RT abgedampft und der Rückstand durch FC (40 g KG, Fließmittel N) gereinigt. Man erhält das (2S,4S,5S,7R)-N-[4-(tert-Butoxycarbonyl)amino-7-butylcarbamoyl-5-hydroxy-2-isopropyl-octyl]-2-(3-methoxypropoxy)-benzamid (86 mg) als weißen Schaum: R_{f} (F) = 0.09. HPLC Rₜ = 17.2 min.

### Beispiel 4:

Analog wie in Beispiel 3) beschrieben, werden die nachfolgenden Verbindungen hergestellt:
a) Aus 150 mg (2S,4'S,5'S,2"R)-N-{2-[5'-(2"-Butylcarbamoylpropyl)-3'-(*tert*-butoxycarbonyl)-2',2'-dimethyl-1,3-oxazolidin-4'-ylmethyl]-3-methylbutyl}-3-methoxy-2-(3-methoxypropoxy)-benzamid das (2S,4S,5S,7R)-N-[4-(tert-Butoxycarbonyl)amino-7-butylcarbamoyl-5-hydroxy-2-isopropyl-octyl]-3-methoxy-2-(3-methoxypropoxy)-benzamid, R_{f} (F) = 0.05; HPLC Rₜ = 17.2 min; MS(FAB) m/e 624 (M⁺ +1), als farbloses Öl.
b) Aus 172 mg (2S,4'S,5'S,2''R)-N-{2-[5'-(2''-Butylcarbamoylpropyl)-3'-(*tert*-butoxycarbonyl)-2',2'-dimethyl-1,3-oxazolidin-4'-ylmethyl]-3-methylbutyl}-4-methoxy-2-(3-methoxypropoxy)benzamid das (2S,4S,5S,7R)-N-[4-(tert-Butoxycarbonyl)amino-7-butylcarbamoyl-5-hydroxy-2-isopropyl-octyl]-4-methoxy-2-(3-methoxypropoxy)-benzamid, R_{f} (N) = 0.30; HPLC Rₜ = 17.6 min; MS(FAB) m/e 624 (M⁺ +1), als farbloses Öl.
c) Aus 105 mg (2S,4'S,5'S,2''R)-N-{2-[5'-(2''-Butylcarbamoylpropyl)-3'-(*tert*-butoxycarbonyl)-2',2'-dimethyl-1,3-oxazolidin-4'-ylmethyl]-3-methylbutyl}-3-(3-methoxypropoxy)-benzamid das (2S,4S,5S,7R)-N-[4-(tert-Butoxycarbonyl)amino-7-butylcarbamoyl-5-hydroxy-2-isopropyl-octyl]-3-(3-methoxypropoxy)-benzamid, R_{f} (N) = 0.18; HPLC Rₜ = 17.0 min; MS(FAB) m/e 594 (M⁺ +1), als weißen Schaum.

### Beispiel 5:

(2S,4S,5S,7R)-N-[4-(tert-Butoxycarbonyl)amino-7-butylcarbamoyl-5-hydroxy-2-isopropyl-octyl]-2-(3-methoxypropoxy)-benzamid (82 mg) werden in 3 mL einer 4 N Salzsäure-Lösung in Dioxan bei 0° C gelöst und für 2 h bei 0° C gerührt. Das Reaktionsgemisch wird lyophilisiert und man erhält das (2S,4S,5S,7R)-N-(4-Amino-7-butylcarbamoyl-5-hydroxy-2-isopropyl-octyl)-2-(3-methoxypropoxy)-benzamid-hydrochlorid als weißen Schaum: R_{f} (L) = 0.12. HPLC Rₜ = 11.6 min. MS(FAB) m/e 494 (M⁺ +1).

### Beispiel 6:

In analoger Weise wie in Beispiel 5) beschrieben, werden die nachfolgenden Verbindungen durch De-Bocylierung hergestellt:
a) Aus 117 mg (2S,4S,5S,7R)-N-[4-(tert-Butoxycarbonyl)amino-7-butylcarbamoyl-5-hydroxy-2-isopropyl-octyl]-3-methoxy-2-(3-methoxypropoxy)-benzamid das (2S,4S,5S,7R)-N-(4-Amino-7-butylcarbamoyl-5-hydroxy-2-isopropyl-octyl)-3-methoxy-2-(3-methoxypropoxy)-benzamid-hydrochlorid: R_{f} (L) = 0.15. HPLC Rₜ = 11.7 min. MS(FAB) m/e 524 (M⁺ +1).
b) Aus 119 mg (2S,4S,5S,7R)-N-[4-(tert-Butoxycarbonyl)amino-7-butylcarbamoyl-5-hydroxy-2-isopropyl-octyl]-4-methoxy-2-(3-methoxypropoxy)-benzamid das (2S,4S,5S,7R)-N-(4-Amino-7-butylcarbamoyl-5-hydroxy-2-isopropyl-octyl)-4-methoxy-2-(3-methoxypropoxy)-benzamid-hydrochlorid: R_{f} (L) = 0.13. HPLC Rₜ = 12.3 min. MS(FAB) m/e 524 (M⁺ +1).
c) Aus 82 mg (2S,4S,5S,7R)-N-[4-(tert-Butoxycarbonyl)amino-7-butylcarbamoyl-5-hydroxy-2-isopropyl-octyl]-3-(3-methoxypropoxy)-benzamid das (2S,4S,5S,7R)-N-(4-Amino-7-butylcarbamoyl-5-hydroxy-2-isopropyl-octyl)-3-(3-methoxypropoxy)-benzamid-hydrochlorid: R_{f} (L) = 0.20. HPLC Rₜ = 11.4 min. MS(FAB) m/e 494 (M⁺ +1).

### Beispiel 7:

Zu einer Lösung von (2S,4S,5S,7R)-N-(4-Amino-7-butylcarbamoyl-5-hydroxy-2-isopropyl-octyl)-3-methoxy-2-(3-methoxypropoxy)-benzamid-hydrochlorid (Beispiel 6a) (67 mg) in Dichlormethan (4 mL) addiert man Triethylamin (0.034 mL) und Ameisensäure-4-nitrophenylester (28 mg) unter Rühren bei RT. Die entstandene Reaktionslösung wird noch 30 min nachgerührt und anschließend eingedampft. Der Rückstand wird mittels FC (18 g KG; Fließmittel E, dann Essigsäureethylester-Hexan-Methanol 5:5:1) gereinigt. Man erhält das (2S,4S,5S,7R)-N-(7-Butylcarbamoyl-4-formylamino-5-hydroxy-2-isopropyl-octyl)-3-methoxy-2-(3-methoxypropoxy)-benzamid als weißen Schaum: R_{f} (L) = 0.58. HPLC Rₜ = 13.2 min. MS(FAB) *m/e* 552 (M⁺ +1).

### Beispiel 8:

(2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid (100 mg) werden in Dichlormethan nach dem in Beispiel 1) beschriebenen Verfahren mit 1-Benzyl-1H-indol-3-carbonsäure (114 mg) in Gegenwart von Bis-(2-oxo-3-oxazolidinyl)-phosphinsäurechlorid (115 mg), Triethylamin (0.13 mL) und einer katalytischen Menge 4-Dimethylaminopyridin umgesetzt. Nach beendeter Reaktion wird das Lösungsmittel abgedampft und der Rückstand direkt durch FC (30 g Kieselgel, Fließmittel W) gereinigt. Man erhält das (2R,4'S,5'S,2''R)-1-Benzyl-1H-indol-3-carbonsäure-N-{2-[5'-(2''-butylcarbamoylpropyl)-2',2'-dimethyl-1,3-oxazolidin-4'-ylmethyl]-3-methylbutyl}-amid, R_{f} (L) = 0.61, als gelbliches Öl.

Das als Ausgangsmaterial eingesetzte (2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid wird folgendermaßen hergestellt:
a) (2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid: Man erhält die Titelverbindung aus (2R,4'S,5'S,2''R)-3-[4'-(2''-Azidomethyl-3''-methylbutyl)-3'-(*tert*-butoxycarbonyl)-2',2'-dimethyl-1,3-oxazolidin-5'-yl]-2-methylpropionsäure-N-(butyl)amid(0.6 g) durch Hydrogenolyse analog wie in Beispiel 1a) und anschließender FC Reinigung (100 g Kieselgel, Fließmittel V) als blaßgelbes Öl: R_{f} (W) = 0.32.
b) (2R,4'S,5'S,2''R)-3-[4'-(2''-Azidomethyl-3''-methylbutyl)-3'-(*tert*-butoxycarbonyl)-2',2'-dimethyl-1,3-oxazolidin-5'-yl]-2-methylpropionsäure-N-(butyl)amid: Durch Umsetzung von (2R,4S,5S,2'R)-Methansulfonsäure-{2-[5'-(2''-butylcarbamoylpropyl)-3'-(*tert*-butoxycarbonyl)-2',2'-dimethyl-1,3-oxazolidin-4'-ylmethyl]-3-methylbutyl}ester (1.30 g) mit Natriumazid (1.92 g) analog wie in Beispiel 1b) beschrieben und FC Reinigung (200 g Kieselgel, Fließmittel D) erhält man die Titelverbindung, R_{f} (E) = 0.61, als blaßgelbes Öl.
c) (2R,4'S,5'S,2''R)-Methansulfonsäure-{2-[5'-(2''-butylcarbamoylpropyl)-3'-(*tert*-butoxycarbonyl)-2',2'-dimethyl-1,3-oxazolidin-4'-ylmethyl]-3-methylbutyl}ester: Man erhält die Titelverbindung analog wie in Beispiel 1c) beschrieben ausgehend von (2R,4'S,5'S,2''R)-3-[4'-(2''-Hydroxymethyl-3''-methylbutyl)-3'-(*tert*-butoxycarbonyl)-2',2'-dimethyl-1,3-oxazolidin-5'-yl]-2-methylpropionsäure-N-(butyl)amid (1.10 g) als farbloses Öl (1.30 g): R_{f} (E) = 0.33.
d) (2R,4'S,5'S,2''R)-3-[4'-(2''-Hydroxymethyl-3''-methylbutyl)-3'-(*tert*-butoxycarbonyl)-2',2'-dimethyl-1 ,3-oxazolidin-5'-yl]-2-methylpropionsäure-N-(butyl)amid: Man erhält die Titelverbindung aus (2R,4'S,5'S,2"R)-3-[4'-(2"-Benzyloxymethyl-3"-methylbutyl)-3'-(*tert*-butoxycarbonyl)-2',2'-dimethyl-1,3-oxazolidin-5'-yl]-2-methylpropionsäure-N-(butyl)amid (1.64 g) durch Hydrierung analog wie in Beispiel 1d) beschrieben und anschließender Reinigung durch FC (50 g Kieselgel, Fließmittelgradient von E nach Essigsäureethylester) als weißen Feststoff (1.16 g): R_{f} (Hexan-Essigsäureethylester 1:3) = 0.44. Schmp. 110-112° C.
e) (2R,4'S,5'S,2''R)-3-[4'-(2''-Benzyloxymethyl-3''-methylbutyl)-3'-(*tert*-butoxycarbonyl)-2',2'-dimethyl-1,3-oxazolidin-5'-yl]-2-methylpropionsäure-N-(butyl)amid: Die Mischung aus (2RS,4S,5S,7R)-7-Benzyloxymethyl-5-(*tert*-butoxycarbonyl)amino-4-hydroxy-2,8-dirnethylnonansäure-N(butyl)amid (1.9 g) und para-Toluolsulfonsäure Hydrat (0.037 g) in 2',2'-Dimethoxypropan (35 mL) und Dichlormethan (35 mL) wird über 1 h bei RT gerührt. Anschließend addiert man Pyridin (16 µL) und Hexan (35 mL), engt das Lösungsmittel unter Vakuum ein und chromatographiert den öligen Rückstand an 100 g Kieselgel (Fließmittelgradient von Hexan-Essigsäureethylester 6:1 nach 4:1). Fraktionen mit reinem (2R,4'S,5'S,2''R)-Diastereomer werden zusammengefaßt, während Mischfraktionen erneut unter gleichen Bedingungen chromatographiert werden. Nach Zusammenfassung der entsprechenden Fraktionen, Einengen des Lösungsmittels und Trocknen des Rückstandes im HV erhält man die Titelverbindung (1.64 g; R_{f} (E) = 0.72; HPLC, Rₜ = 22.8 min; MS(FAB) *m/e* 533 (M⁺ +1)) neben einem 6:4-Gemisch aus den beiden (2R,4'S,5'S,2''R)- und (2R,4'S,5'S,2"S)-Diastereomeren (0.28 g; R_{f} (E) = 0.72/0.66; HPLC, Rₜ = 22.8 and 23.0 min).
f) (2RS,4S,5S,7R)-7-Benzyloxymethyl-5-(*tert*-butoxycarbonyl)amino-4-hydroxy-2,8-dimethylnonansäure-N(butyl)amid: Analog wie in Beispiel 1f) beschrieben erhält man aus dem stereoisomerenreinen (2S,3S,SR)-2-[3-(*tert*-Butoxycarbonyl)amino-5-(benzyloxymethyl)-2-hydroxy-6-methylheptyl]-N-(butyl)acrylamid (A) (2.5 g, 5.10 mmol) durch Hydrierung in Gegenwart von katalytischen Mengen [Ru₂Cl₄[(S)-BINAP]₂]NEt₃ (30 mg) die Titelverbindung als Gemisch von an Kieselgel nicht trennbaren Diastereoisomeren (1.91 g), wobei das (2R,4S,5S,2'R)-lsomere bevorzugt entsteht: gelbliches Öl, R_{f} (E) = 0.25, MS(FAB) *m/e* 493 (M⁺ +1).
g) (2S,3S,5R)-2-[3-(*tert*-Butoxycarbonyl)amino-5-(benzyloxymethyl)-2-hydroxy-6-methylheptyl]-N-(butyl)acrylamid (A) und (2R,3S,5R)-2-[3-(*tert*-Butoxycarbonyl)amino-5-(benzyloxymethyl)-2-hydroxy-6-methylheptyl]-N-(butyl)acrylamid (B): Analog wie in Beispiel 1g) beschrieben erhält man zunächst durch Reduktion von (2S,4R)-2-(*tert*-Butoxycarbonyl)amino-4-(benzyloxymethyl)-5-methyl-hexansäuremethylester (8.0 g), gelöst in wasserfreiem Toluol (120 mL), mit einer 1.2 M Diisobutylaluminiumhydrid-Lösung in Toluol (34.9 mL) das (2S,4R)-2-(*tert*-Butoxycarbonyl)amino-4-(benzyloxymethyl)-5-methyl-hexanal als blaßgelbes Öl (R_{f}(N) = 0.6). Der rohe Aldehyd wird ohne weitere Reinigung analog wie in Beispiel lg) beschrieben umgesetzt. Das nach wässriger Aufarbeitung erhaltene Rohprodukt (14.8 g) wird über 1.0 kg Kieselgel mit einem Fließmittelgradienten von B nach E unter Trennung der beiden (2S,3S,5R)- und (2R,3S,5R)-Diastereoisomeren chromatographiert. Man erhält die Titelverbindung A (2.52 g, 24 %) als hellgelbes Öl, R_{f} (E) = 0.55, HPLC Rₜ = 19.8 min, MS(FAB) *m/e* 491 (M⁺ +1); sowie die Titelverbindung B (3.76 g, 35 %) als hellgelbes Öl, R_{f} (E) = 0.42, HPLC Rₜ = 19.6 min. MS(FAB) *m/e* 491 (M⁺ +1).
h) (2S,4R)-2-(*tert*-Butoxycarbonyl)amino-4-(benzyloxymethyl)-5-methylhexansäuremethylester: Analog wie in den Beispielen 1i und 1j) beschrieben wird (2S,2'R,5R)-2-[2'-(Benzyloxymethyl)-3'-methylbutyl]-2,5-dihydro-5-isopropyl-3,6-dimethoxypyrazin (15.7 g) zunächst in Gegenwart von 1 N Salzsäure-Lösung (176 mL) hydrolysiert und anschließend das nach Aufarbeitung und Reinigung durch FC (1.0 kg KG, Fließmittel V) erhaltene Produkt (R_{f}(W) = 0.59) mit Di-*tert*iärbutyl-dicarbonat in Gegenwart von Hünigsbase umgesetzt. Nach Reinigung durch FC (1.0 kg KG, Hexan-Essigsäureethylester 6:1) erhält man die Titelverbindung (13.4 g) als blaßgelbes Öl: R_{f} (C) = 0.43.
i) (2S,2'R,5R)-2-[2'-(Benzyloxymethyl)-3'-methylbutyl]-2,5-dihydro-5-isopropyl-3,6-dimethoxypyrazin: Die Lösung von (2R)-2,5-Dihydro-2-isopropyl-3,6-dimethoxypyrazin (13.0 g) in absolutem Tetrahydrofuran (230 mL) wird in analoger Weise wie in Beispiel 1 k) beschrieben zunächst mit einer 1.6 M n-Butyllithium-Lösung in Hexan (44 mL) und anschließend mit 2(R)-(Benzyloxymethyl)-3-methylbutylbromid (12.8 g) in Tetrahydrofuran (60 mL) umgesetzt. Nach Aufarbeitung des Reaktionsgemisches wird das ölige Rohprodukt durch FC (1.0 kg KG, Essigsäureethylester-Hexan 1:20) gereinigt. Man erhält neben dem (2R,2'R,5R)-Diastereomeren (3.49 g; gelbliches Öl, R_{f} (Essigsäureethylester-Hexan 1:6) = 0.50) die stereoisomerenreine Titelverbindung (12.9 g) als gelbliches Öl: R_{f} (Essigsäureethylester-Hexan 1:6) = 0.62.

### Beispiel 9:

In analoger Weise wie in Beispiel 8) beschrieben werden die nachfolgenden Verbindungen hergestellt:
a) Aus 100 mg (2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 99 mg 1-(2-Methoxyethyl)-1H-indol-3-carbonsäure das (2R,4'S,5'S,2''R)-1-(2-Methoxyethyl)-1H-indol-3-carbonsäure-N-{2-[5'-(2''-butylcarbamoylpropyl)-2',2'-dimethyl-1,3-oxazolidin-4'-ylmethyl]-3-methylbutyl}-amid, R_{f} (L) = 0.68, als gelbliches Öl.
b) Aus 46 mg (2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 53 mg 1-Pyridin-2-yl-1H-indol-3-carbonsäure das (2S,4'S,5'S,2''R)-1-Pyridin-2-yl-1H-indol-3-carbonsäure-N-{2-[5'-(2''-butylcarbamoylpropyl)-2',2'-dimethyl-oxazolidin-4'-ylmethyl]-3-methylbutyl}-amid, R_{f} (L) = 0.81, als gelbliches Öl.
c) Aus 46 mg (2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 59 mg 1-(2-Methoxybenzyl)-1H-indol-3-carbonsäure das (2R,4'S,5'S,2''R)-1-(2-Methoxybenzyl)-1H-indol-3-carbonsäure-N-{2-[5'-(2''-butylcarbamoylpropyl)-2',2'-dimethyl-oxazolidin-4'-ylmethyl]-3-methylbutyl}-amid, R_{f} (L) = 0.73, als farbloses Öl.

Die als Ausgangsmaterial eingesetzte 1-(2-Methoxyethyl)-1H-indol-3-carbonsäure wird folgendermaßen hergestellt:
a) 1-(2-Methoxyethyl)-1H-indol-3-carbonsäure: Zu einer Lösung von 1-(2-Methoxyethyl)-1H-indol-3-carbonsäureethylester (1.26 g) in Ethanol (10 mL) und Wasser (5 mL) wird 1 N Natronlauge (5.1 mL) addiert und der Ansatz unter Rühren über 1 h auf 50° C erwärmt. Es wird erneut 1 N Natronlauge (7.1 mL) addiert und für weitere 5 h bei 80° C nachgerührt. Der Ansatz wird am Rotavap unter Entfernung des Ethanols eingeengt, die wässrige Phase durch Addition von 1 M Kaliumhydrogensulfat-Lösung angesäuert und mit Dichlormethan extrahiert. Die organische Phase wird mit gesättigter Natriumchlorid-Lösung gewaschen, über Natriumsulfat getrocknet und eingeengt. Man erhält die Titelverbindung (0.96 g), R_{f} (Hexan-Essigsäureethylester-Eisessig 67:33:1) = 0.15, als gelbliche Festsubstanz.
b) 1-(2-Methoxyethyl)-1H-indol-3-carbonsäureethylester: Zu einer Lösung von 1H-indol-3-carbonsäureethylester (1.0 g) in N,N-Dimethylformamid (25 mL) wird Natriumhydrid 80 %ige Dispersion in Öl (0.27 g) addiert und die Mischung bei RT über 30 min gerührt. Anschließend wird 3-Methoxyethyliodid (1.5 g) addiert und das Reaktionsgemisch zunächst über 1 h bei 50° C und dann bei 80° C über Nacht gerührt. Der Ansatz wird in Eiswasser (50 mL) gegossen, die wässrige Phase mit Dichlormethan extrahiert und die vereinte organische Phase mit gesättigter Natriumchlorid-Lösung gewaschen, über Natriumsulfat getrocknet und eingeengt. Das Rohprodukt wird durch FC (100 g KG, Fließmittelgradient von C nach D) gereinigt. Man erhält die Titelverbindung 1.26 g), R_{f} (C, zweifache Laufstrecke) = 0.44, als Öl.

### Beispiel 10:

In analoger Weise wie in Beispiel 3) beschrieben werden die nachfolgenden Verbindungen hergestellt:
a) Aus 126 mg (2R,4'S,5'S,2''R)-1-Benzyl-1H-indol-3-carbonsäure-N-(2-[5'-(2''-butylcarbamoylpropyl)-2',2'-dimethyl-1,3-oxazolidin-4'-ylmethyl]-3-methylbutyl}-amid das (2R,4S,5S,7R)-1-Benzyl-1H-indol-3-carbonsäure-N-[4-(*tert*-butoxycarbonyl)amino-7-butylcarbamoyl-5-hydroxy-2-isopropyl-octyl]-amid, R_{f} (L) = 0.55, als weißer Schaum.
b) Aus 146 mg (2R,4'S,5'S,2''R)-1-(2-Methoxyethyl)-1H-indol-3-carbonsäure-N-(2-[5'-(2''-butylcarbamoylpropyl)-2',2'-dimethyl-1,3-oxazolidin-4'-ylmethyl]-3-methylbutyl}-amid das (2R,4S,5S,7R)-1-(2-Methoxyethyl)-1H-indol-3-carbonsäure-N-[4-(*tert*-butoxycarbonyl)amino-7-butylcarbamoyl-5-hydroxy-2-isopropyl-octyl]-amid, R_{f} (L) = 0.52, als farbloses Öl.
c) Aus 77 mg (2R,4'S,5'S,2''R)-1-Pyridin-2-yl-1H-indol-3-carbonsäure-N-{2-[5'-(2''-butylcarbamoylpropyl)-2',2'-dimethyl-1,3-oxazolidin-4'-ylmethyl]-3-methylbutyl}-amid und FC Reinigung an 10 g KG (Fließmittel S) das (2R,4S,5S,7R)-1-Pyridin-2-yl-1H-indol-3-carbonsäu re-N-[4-(*tert*-butoxycarbonyl)amino-7-butylcarbamoyl-5-hydroxy-2-isopropyl-octyl]-amid, R_{f} (W) = 0.54, als farbloser Schaum.
d) Aus 67 mg (2R,4'S,5'S,2''R)-1-(2-Methoxybenzyl)-1H-indol-3-carbonsäure-N-(2-[5'-(2''-butylcarbamoylpropyl)-2',2'-dimethyl-1,3-oxazolidin-4'-ylmethyl]-3-methylbutyl}-amid das (2R,4S,5S,7R)-1-(2-Methoxybenzyl)-1H-indol-3-carbonsäure-N-[4-(*tert*-butoxycarbonyl)-amino-7-butylcarbamoyl-5-hydroxy-2-isopropyl-octyl]-amid, R_{f} (L) = 0.61, als farbloser Schaum.

### Beispiel 11:

In analoger Weise wie in Beispiel 5) beschrieben, werden die nachfolgenden Verbindungen durch De-Bocylierung hergestellt:
a) Aus 93 mg (2R,4S,5S,7R)-1-Benzyl-1H-indol-3-carbonsäure-N-[4-(*tert*-butoxycarbonyl)amino-7-butylcarbamoyl-5-hydroxy-2-isopropyl-octyl]-amid das (2R,4S,5S,7R)-1-Benzyl-1H-indol-3-carbonsäure-N-(4-amino-7-butylcarbamoyl-5-hydroxy-2-isopropyl-octyl)-amid-hydrochlorid: R_{f} (W) = 0.32. HPLC Rₜ = 13.8 min. MS(FAB) m/e 535 (M⁺ +1).
b) Aus 89 mg (2R,4S,5S,7R)-1-(2-Methoxyethyl)-1H-indol-3-carbonsäure-N-[4-(*tert*-butoxycarbonyl)amino-7-butylcarbamoyl-5-hydroxy-2-isopropyl-octyl]-amid das (2R,4S,5S,7R)-1-(2-Methoxyethyl)-1H-indol-3-carbonsäure-N-(4-amino-7-butylcarbamoyl-5-hydroxy-2-isopropyl-octyl)-amid-hydrochlorid: R_{f} (W) = 0.29. HPLC Rₜ = 11.7 min. MS(FAB) m/e 503 (M⁺ +1).
c) Aus 60 mg (2R,4S,5S,7R)-1-Pyridin-2-yl-1H-indol-3-carbonsäure-N-[4-(*tert*-butoxycarbonyl)amino-7-butylcarbamoyl-5-hydroxy-2-isopropyl-octyl]-amid das (2R,4S,5S,7R)-1-Pyridin-2-yl-1H-indol-3-carbonsäure-N-(4-amino-7-butylcarbamoyl-5-hydroxy-2-isopropyloctyl)-amid-hydrochlorid: R_{f} (W) = 0.24. HPLC Rₜ = 9.94 min. MS(FAB) m/e 536 (M⁺ +1).
d) Aus 50 mg (2R,4S,5S,7R)-1-(2-Methoxybenzyl)-1H-indol-3-carbonsäure-N-[4-(*tert*-butoxycarbonyl)amino-7-butylcarbamoyl-5-hydroxy-2-isopropyl-octyl]-amid das (2R,4S,5S,7R)-1-(2-Methoxybenzyl)-1H-indol-3-carbonsäure-N-(4-amino-7-butylcarbamoyl-5-hydroxy-2-isopropyl-octyl)-amid-hydrochlorid: R_{f} (W) = 0.32. HPLC Rₜ = 14.0 min. MS(FAB) m/e 565 (M⁺ +1).

### Beispiel 12:

Analog wie in Beispiel 5) beschrieben erhält man aus (2R,4S,5S,7R)-N-[4-(*tert*-Butoxycarbonyl)amino-7-butylcarbamoyl-5-hydroxy-2-isopropyl-octyl]-2-(3-methoxypropoxy)-benzamid (93 mg) das (2R,4S,5S,7R)-N-(4-Amino-7-butylcarbamoyl-5-hydroxy-2-isopropyloctyl)-2-(3-methoxypropoxy)-benzamid-hydrochlorid: R_{f}(W) = 0.28. HPLC Rₜ = 11.9 min. MS(FAB) m/e 494 (M⁺ +1).

Das als Ausgangsmaterial eingesetzte (2R,4S,5S,7R)-N-[4-(*tert*-Butoxycarbonyl)amino-7-butylcarbamoyl-5-hydroxy-2-isopropyl-octyl]-2-(3-methoxypropoxy)-benzamid wird folgendermaßen hergestellt:
a) Aus 135 mg (2R,4'S,5'S,2''R)-N-{2-[5'-(2''-Butylcarbamoylpropyl)-3'-(*tert*-butoxycarbonyl)-2',2'-dimethyl-1,3-oxazolidin-4'-ylmethyl]-3-methylbutyl}-2-(3-methoxypropoxy)-benzamid erhält man nach dem in Beispiel 3) beschriebenen Verfahren das (2R,4S,5S,7R)-N-[4-( *tert*-Butoxycarbonyl)amino-7-butylcarbamoyl-5-hydroxy-2-isopropyl-octyl]-2-(3-methoxypropoxy)-benzamid, R_{f} (N) = 0.28, als farbloses Öl.
b) (2R,4'S,5'S,2''R)-N-{2-[5'-(2''-Butylcarbamoylpropyl)-3'-(*tert*-butoxycarbonyl)-2',2'-dimethyl-1,3-oxazolidin-4'-ylmethyl]-3-methylbutyl}-2-(3-methoxypropoxy)-benzamid: Aus 100 mg (2R,4'S,5'S,2''R)-3-[4'-(2''-Aminomethyl-3''-methylbutyl)-3'-(*tert*-butoxycarbonyl)-2',2'-dimethyl-1,3-oxazolidin-5'-yl]-2-methylpropionsäure-N-(butyl)amid und 95 mg 2-(3-Methoxypropoxy)-benzoesäure erhält man nach dem in Beispiel 1) beschriebenen Verfahren die Titelverbindung, R_{f} (M) = 0.57, als blaßgelbes Öl.

### Beispiel 13:

Durch Hydrogenolyse von (2R,4'S,5'S,2''R)-3-[4'-(3''-Azido-2''-methylpropyl)-3'-(*tert*-butoxycarbonyl)-2',2'-dimethyl-1,3-oxazolidin-5'-yl]-2-methylpropionsäure-N-(butyl)amid (0.67 g) analog wie in Beispiel 1a) beschrieben erhält man das (2R,4'S,5'S,2''R)-3-[4'-(3''-Amino-2''-methylpropyl)-3'-(*tert*-butoxycarbonyl)-2',2'-dimethyl-1,3-oxazolidin-5'-yl]-2-methylpropionsäure-N-(butyl)amid als blaßgelbes Öl: R_{f} (W) = 0.33.

Das als Ausgangsmaterial eingesetzte (2R,4'S,5'S,2''R)-3-[4'-(3''-Azido-2''-methylpropyl)-3'-(*tert*-butoxycarbonyl)-2',2'-dimethyl-1,3-oxazolidin-5'-yl]-2-methylpropionsäure-N-(butyl)amid wird folgendermaßen hergestellt:
a) (2R,4'S,5'S,2''R)-3-[4'-(3''-Azido-2''-methylpropyl)-3'-(*tert*-butoxycarbonyl)-2',2'-dimethyl-1,3-oxazolidin-5'-yl]-2-methylpropionsäure-N-(butyl)amid: Aus (2R,4'S,5'S,2''R)-3-[3'-(*tert*-Butoxycarbonyl)-4'-(3''-hydroxy-2''-methylpropyl)-2',2'-dimethyl-1,3-oxazolidin-5'-yl]-2-methylpropionsäure-N-(butyl)amid (0.85 g) erhält man, analog wie in den Beispielen 1c) und 1b) beschrieben und nach FC Reinigung (100 g Kieselgel, Essigsäureethylester-Hexan 3:1) die Titelverbindung (0.86 g), R_{f} (E) = 0.71, als blaßgelbes Öl.
b) (2R,4'S,5'S,2''R)-3-[3''-(*tert*-Butoxycarbonyl)-4'-(3''-hydroxy-2''-methylpropyl)-2',2'-dimethyl-1,3-oxazolidin-5'-yl]-2-methylpropionsäure-N-(butyl)amid: Man erhält, in analoger Weise wie in den Beispielen 1d),1e) und1f) beschrieben durch (1) stereoselektive Hydrierung von (2S,3S,5R)-2-[6-(Benzyloxymethyl)-3-(*tert*-butoxycarbonyl)amino-2-hydroxy-5-methylhexyl]-N-(butyl)acrylamid (A) (3.17 g) in absolutem Methanol in Gegenwart von [Ru₂Cl₄[(S)-BINAP]₂]NEt₃ (0.057 g), gefolgt von (2) N,O-Acetalisierung durch Umsetzung mit 2',2'-Dimethoxypropan in Gegenwart von para-Toluolsulfonsäure und chromatographische Auftrennung des so erhaltenen ca. 9:1-Diastereoisomeren-Gemisches aus (2R,4'S,5'S,2''R)-3-[4'-(3''-Benzyloxy-2''-methylpropyl)-3'-(*tert*-butoxycarbonyl)-2',2'-dimethyl-1,3-oxazolidin-5'-yl]-2-methylpropionsäure-N-(butyl)amid (R_{f} (E) = 0.64) und (2S,4'S,5'S,2''R)-3-[4'-(3''-Benzyloxy-2''-methylpropyl)-3'-(*tert*-butoxycarbonyl)-2',2'-dimethyl-1,3-oxazolidin-5'-yl]-2-methylpropionsäure-N-(butyl)amid (R_{f} (E) = 0.54) mittels FC an 100 g Kieselgel (Fließmittelgradient von Hexan-Essigsäureethylester 5:1 nach Essigsäureethylester), und anschließend (3) Hydrogenolyse des so erhaltenen (2R,4'S,5'S,2''R)-3-[4'-(3''-Benzyloxy-2''-methylpropyl)-3'-(*tert*-butoxycarbonyl)-2',2'-dimethyl-1,3-oxazolidin-5'-yl]-2-methylpropionsäure-N-(butyl)amid an 10 % Palladium auf Kohle die stereoisomerenreine Titelverbindung: farbloses Öl (1.46 g; FC Reinigung des rohen Alkohols an 50 g Kieselgel, Fließmittelgradient von D nach Hexan-Essigsäureethylester 1:3), R_{f} (Hexan-Essigsäureethylester 1:3) = 0.29.
c) (2S,3S,5R)-2-[6-Benzyloxy-3-(*tert*-butoxycarbonyl)amino-2-hydroxy-5-methylhexyl]-N-(butyl)acrylamid (A) und (2R,3S,5R)-2-[6-Benzyloxy-3-(*tert*-butoxycarbonyl)amino-2-hydroxy-5-methylhexyl]-N-(butyl)acrylamid (B): Analog wie in Beispiel 1h) beschrieben erhält man zunächst durch Reduktion von (2S,4R)-5-Benzyloxy-2-(*tert*-butoxycarbonyl)amino-4-methylpentansäu remethylester (9.51 g) in Gegenwart einer 1.2 M Diisobutylaluminiumhydrid-Lösung in Toluol das (2S,4R)-5-Benzyloxy-2-(*tert*-butoxycarbonyl)amino-4-methyl-pentanal (8.2 g Rohprodukt) als blaßgelbes Öl. Der rohe Aldehyd wird ohne weitere Reinigung analog wie in Beispiel 1 g) beschrieben umgesetzt. Das so erhaltene Rohprodukt (17.1 g) enthält ein ca. 1:1.45-Gemisch aus den beiden Diastereoisomeren A und B. Säulenchromatographische Reinigung unter Trennung der beiden Stereoisomere an Kieselgel (0.6 kg, Fließmittelgradient von C nach E) ergibt die Titelverbindung A (2.05 g) als wachsartige Festsubstanz, R_{f} (E) = 0.43, MS(FAB) *m/e* 463 (M⁺ +1); sowie die Titelverbindung B (3.01 g) als hellgelbes Öl, R_{f} (E) = 0.35, MS(FAB) *m/e* 463 (M⁺ +1).
d) (2S,4R)-5-Benzyloxy-2-(*tert*-butoxycarbonyl)amino-4-methylpentansäuremethylester: Analog wie in den Beispielen 1i und 1j) beschrieben wird (2S,2'R,5R)-2-(3'-Benzyloxy-2'-methylpropyl)-2,5-dihydro-5-isopropyl-3,6-dimethoxypyrazin (18.4 g) zunächst in Gegenwart von 1 N Salzsäure-Lösung (215 mL) hydrolysiert und anschließend mit Di-*tert*-butyl-dicarbonat in Gegenwart von Hünigsbase umgesetzt. Nach Reinigung durch FC (0.9 kg KG, Fließmittel A) erhält man die Titelverbindung (16.0 g) als farbloses Öl: R_{f} (C) = 0.54.
e) (2S,2R',5R)-2-(3'-Benzyloxy-2'-methylpropyl)-2,5-dihydro-5-isopropyl-3,6-dimethoxypyrazin: Die Lösung von (2R)-2,5-Dihydro-2-isopropyl-3,6-dimethoxypyrazin (14.7 mL) in absolutem Tetrahydrofuran (230 mL) wird in analoger Weise wie in Beispiel 1 k) beschrieben zunächst mit einer 1.6 M n-Butyllithium-Lösung in Hexan (47.4 mL) und anschließend mit 2(S)-3-Benzyloxy-2-methylpropylbromid (20.0 g) in Tetrahydrofuran (115 mL) umgesetzt. Nach Aufarbeitung des Reaktionsgemisches wird das ölige Rohprodukt durch FC (0.9 kg KG, Essigsäureethylester-Hexan 5:95) gereinigt. Man erhält neben dem (2R,2'R,5R)-Diastereomeren (1.1 g; gelbliches Öl, R_{f} (Essigsäureethylester-Hexan 1:6) = 0.27) die stereoisomerenreine Titelverbindung (18.4 g) als gelbliches Öl: R_{f} (Essigsäureethylester-Hexan 1:6) = 0.33.

### Beispiel 14:

Analog wie in Beispiel 5) beschrieben erhält man aus (2R,4S,5S,7R)-N-[4-(*tert*-Butoxycarbonyl)amino-7-butylcarbamoyl-5-hydroxy-2-methyloctyl]-2-(3-methoxypropoxy)-benzamid (82 mg) das (2R,4S,5S,7R)-N-(4-Amino-7-butylcarbamoyl-5-hydroxy-2-methyloctyl)-2-(3-methoxypropoxy)-benzamid-hydrochlorid: R_{f}(W) = 0.25. HPLC Rₜ = 9.0 min. MS(FAB) m/e 466 (M⁺ +1).

Das als Ausgangsmaterial eingesetzte (2R,4S,5S,7R)-N-[4-(*tert*-Butoxycarbonyl)amino-7-butylcarbamoyl-5-hydroxy-2-methyloctyl]-2-(3-methoxypropoxy)-benzamid wird folgendermaßen hergestellt:
a) Aus 134 mg (2R,4'S,5'S,2''R)-N-{3-[5'-(2''-Butylcarbamoylpropyl)-3'-(*tert*-butoxycarbonyl)-2',2'-dimethyl-1,3-oxazolidin-4'-yl]-2-methylpropyl}-2-(3-methoxypropoxy)-benzamid erhält man nach dem in Beispiel 3) beschriebenen Verfahren das (2R,4S,5S,7R)-N-[4-(*tert*-Butoxycarbonyl)amino-7-butylcarbamoyl-5-hydroxy-2-methyloctyl]-2-(3-methoxypropoxy)-benzamid, R_{f}(L)= 0.36, als gelbliches Öl.
b) (2R,4'S,5'S,2''R)-N-{3-[5'-(2''-Butylcarbamoylpropyl)-3'-(*tert*-butoxycarbonyl)-2',2'-dimethyl-1,3-oxazolidin-4'-yl]-2-methylbutyl}-2-(3-methoxypropoxy)-benzamid: Aus 100 mg (2R,4'S,5'S,2''R)-3-[4'-(3''-Amino-2''-methylpropyl)-3'-(*tert*-butoxycarbonyl)-2',2'-dimethyl-1,3-oxazolidin-5'-yl]-2-methylpropionsäure-N-(butyl)amid und 102 mg 2-(3-Methoxypropoxy)-benzoesäure erhält man nach dem in Beispiel 1) beschriebenen Verfahren die Titelverbindung, R_{f}(L) = 0.43, als blaßgelbes Öl.

### Beispiel 15:

Die Mischung von 2-(3-Methoxypropoxy)-benzoesäure (1.7 g), Bis-(2-oxo-3-oxazolidinyl)-phosphinsäurechlorid (1.90 g) und Triethylamin (2.81 mL) in Dichlormethan (40 mL) wird bei RT über 60 min gerührt. Anschließend werden eine Lösung von (3S,5S,1S',3'S)-5-[3'-Aminomethyl-1'-(*tert*-butoxycarbonyl)amino-4'-methylpentyl]-3-isopropyl-dihydrofuran-2-on (1.80 g) in 40 mL Dichlormethan und 4-Dimethylaminopyridin (380 mg) addiert. Das Reaktionsgemisch wird über Nacht gerührt. Nach Zugabe von Dichlormethan (200 mL) wird die organische Phase nacheinander mit verdünnter Natronlauge (pH 9), verdünnter wässriger Salzsäure und gesättigter Natriumchlorid-Lösung gewaschen, über Magnesiumsulfat getrocknet und eingeengt. FC an Kieselgel (Fließmittel R) ergibt das (2S,2'S,2''S,4''S)-N-{2-[2'-(*tert*-Butoxycarbonyl)amino-2'-(4''-isopropyl-5''-oxotetrahydrofuran-2''-yl)-ethyl]-3-methylbutyl}-2-(3-methoxypropoxy)-benzamid als blaßgelbes Öl (2.70 g): R_{f} (E) = 0.30. MS(FAB) *m/e* 563 (M⁺ +1).

Das als Ausgangsstoff eingesetzte (3S,5S,1S',3'S)-5-[3'-Aminomethyl-1'-( *tert*-butoxycarbonyl)amino-4'-methylpentyl]-3-isopropyl-dihydrofuran-2-on wird wie folgt hergestellt:
a) (3S,5S,1'S,3'S)-5-[3'-Azidomethyl-1'-(*tert*-butoxycarbonyl)amino-4'-methylpentyl]-3-isopropyl-dihydrofuran-2-on (12.4 g), gelöst in Essigsäureethylester (500 mL), wird in Gegenwart von Pd/C 10 % (2.5 g) bei RT unter Normaldruck über 3 h hydriert. Nach Filtration über Hyflo® und Entfernen des Lösungsmittels erhält man die Titelverbindung als weiße Festsubstanz (11.3 g): R_{f} (W) = 0.34. Schmp. 136-8° C (umkristallisiert aus Dichlormethan-Hexan).
b) (3S,5S,1'S,3'S)-5-[3'-Azidomethyl-1'-(*tert*-butoxycarbonyl)amino-4'-methylpentyl]-3-isopropyl-dihydrofuran-2-on: Die Mischung von (2S,2'S,2''S,4''S)-Methansulfonsäure-{N-(*tert*-butoxycarbonyl)-2-[2'-amino-2'-(4''-isopropyl-5''-oxo-tetrahydrofuran-2''-yl)-ethyl]-3-methylbutyl}-ester (30.2 g) und Natriumazid (22.5 g) in 1,3-Dimethyl-3,4,5,6-tetrahydro-2(1*H*)-pyrimidinon (290 mL) wird bei 50° C über Nacht gerührt. Nach Abkühlen des Reaktionsansatzes wird Dichlormethan (650 mL) addiert und die organische Phase mit Wasser pH 8 (140 mL) und mit gesättigter Natriumchlorid-Lösung gewaschen, über Magnesiumsulfat getrocknet und eingeengt. Aus dem Rohprodukt erhält man nach FC an KG (2 kg, Fließmittel B) die Titelverbindung als weiße Festsubstanz (23.6 g): R_{f} (C) = 0.36. Schmp. 78-81° C. MS(FAB) m/e 383 (M⁺ +1).
c) (2S,2'S,2''S,4''S)-Methansulfonsäure-{N-(*tert*-butoxycarbonyl)-2-[2'-amino-2'-(4''-isopropyl-5''-oxo-tetrahydrofuran-2''-yl)-ethyl]-3-methylbutyl}-ester Zu einer Lösung von (3S,5S,1'S,3'S)-N-(*tert*-Butoxycarbonyl)-5-(1'-amino-3'-hydroxymethyl-4'-methylpentyl)-3-isopropyl-dihydrofuran-2-on (24.8 g) in Dichlormethan (750 mL) werden unter Rühren bei-10° C zunächst Triethylamin (14.5 mL) und anschließend über 10 min Methansulfochlorid (5.64 mL) addiert. Nach weiteren 30 min Rühren bei -10° C wird das Reaktionsgemisch vorsichtig auf Essigester (1 L) gegossen. Die organische Phase wird nacheinander mit 0.5 M wässriger H₃PO₄-Lösung, gesättigter Natriumhydrogencarbonat-Lösung und gesättigter Natriumchlorid-Lösung gewaschen, über Magnesiumsulfat getrocknet und eingeengt. Man erhält die Titelverbindung als Rohprodukt (31.1 g, farbloses Öl), das beim Stehenlassen langsam auskristallisiert. R_{f} (E) = 0.63.
d) (3S,5S,1'S,3'S)-N-(*tert*-Butoxycarbonyl)-5-(1'-amino-3'-hydroxymethyl-4'-methylpentyl)-3-isopropyl-dihydrofuran-2-on: Die Lösung von (3S,5S,1 'S,3'S)-5-(1'-Azido-3'-hydroxymethyl-4'-methylpentyl)-3-isopropyl-dihydrofuran-2-on (24.8 g) in Essigsäureethylester (250 ml) wird in Gegenwart von Pd-C 10 % (8.68 g) bei RT und Normaldruck über 24 h hydriert. Man filtriert über Hyflo©, wäscht mehrmals mit Essigsäureethylester nach und engt ein. Das so erhaltene rohe (3S,5S,1 'S,3'S)-5-(1'-Amino-3'-hydroxymethyl-4'-methylpentyl)-3-isopropyldihydrofuran-2-on (23.0 g; farbloses Oel, R_{f} (W) = 0.67) wird in Essigsäureethylester (500 mL) gelöst und bei 0-5° unter Rühren zunächst mit N-Ethyldiisopropylamin (23.7 mL) und dann tropfenweise mit einer Lösung von Di- *tert*-butyldicarbonat (21.0 g) in Essigsäureethylester (100 mL) versetzt. Nach Aufwärmen auf RT lässt man über Nacht weiterrühren. Das Reaktionsgemisch wird eingeengt und der ölige Rückstand durch FC (250 g KG, Fliessmittel D) gereinigt. Man erhält die Titelverbindung (24.9 g) als weisse Festsubstanz: R_{f} (Dichlormethan-Methanol 1:1) = 0.64. Schmp. 126-8° C (Diethylether). MS(FAB) *m/e* 358 (M⁺ +1).
e) (3S,5S,1'S,3'S)-5-(1'-Azido-3'-hydroxymethyl-4'-methylpentyl)-3-isopropyl-dihydrofuran-2-on: Zur Lösung von (2S,2'S,2''S,4''S-[2'-Azido-2'-(4''-isopropyl-5''-oxo-tetrahydrofuran-2''-yl)-ethyl]-3-methyl-buttersäure (8.0 g) in wasserfreiem Tetrahydrofuran (180 mL) wird bei-10° C nacheinander Triethylamin (5.62 mL) und Chlorameisensäuremethylester (2.59 mL) zugetropft. Die weisse Suspension wird zunächst bei -10° über 1 h und anschliessend bei 0° über 2 h nachgerührt. Der Ansatz wird mit Essigsäureethylester (100 mL) verdünnt und die organische Phase nacheinander mit eiskalter 0.5 N Salzsäure, gesättigter Natriumhydrogencarbonat-Lösung und Wasser gewaschen, über Natriumsulfat getrocknet und eingeengt. Der blassgelbe ölige Rückstand wird in Tetrahydrofuran (160 mL) aufgenommen, und unter Rühren bei -20° portionsweise Natriumborhydrid (1.12 g) addiert. Anschliessend wird über 10 min Methanol (1.5 mL) zugetropft (geringe Exothermie). Man lässt die leicht trübe Mischung langsam auf 0-5° aufwärmen und bei dieser Temperatur über Nacht weiterrühren, tropft anschliessend 1 N Salzsäure (39 mL) zu und extrahiert die wässerige Phase mit Essigsäureethylester (100 mL). Die organische Phase wird mit eiskalter 1 N Natriumcarbonat-Lösung (70 mL) und dann mit gesättigter Natriumchlorid-Lösung neutral gewaschen, über Magnesiumsulfat getrocknet und eingeengt. Nach Trocknen im Hochvakuum erhält man die Titelverbindung als blassgelbes Oel (7.18 g). Analysenreines Produkt (blassgelbes Oel) erhält man nach Flash-Chromatographie an Kieselgel (Fliessmittel-Gradient Hexan/Essigsäureethylester von 5:1 nach 3:1): R_{f} (Hexan/Essigsäureethylester 1:1) = 0.50.

### Beispiel 16:

Die Mischung von 2-(4-Methoxybutoxy)-benzoesäure (1.7 g), Bis-(2-oxo-3-oxazolidinyl)-phosphinsäurechlorid (1.90 g) und Triethylamin (2.81 mL) in Dichlormethan (40 mL) wird bei RT über 60 min gerührt. Anschließend werden eine Lösung von (3S,5S,1S',3'S)-5-[3'-Aminomethyl-1'-(*tert*-butoxycarbonyl)amino-4'-methylpentyl]-3-isopropyl-dihydrofuran-2-on (1.80 g) in Dichlormethan (40 mL) und 4-Dimethylaminopyridin (380 mg) addiert, und das Reaktionsgemisch wird über Nacht rühren gelassen. Nach Zugabe von Dichlormethan (200 mL) wird die organische Phase nacheinander mit verdünnter Natronlauge (pH 9), verdünnter wässriger Salzsäure und gesättigter Natriumchlorid-Lösung gewaschen, über Magnesiumsulfat getrocknet und eingeengt. FC an Kieselgel (Fliessmittel R) ergibt das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-benzamid als blaßgelbes Öl (2.70 g). R_{f} (E) = 0.30. MS(FAB) *m/e* 563 (M⁺ +1).

Die als Ausgangsstoff eingesetzte 2-(4-Methoxybutoxy)-benzoesäure wird folgendermassen hergestellt:
a) In analoger Weise wie in Beispiel 1) beschrieben, wird 2-(4-Methoxybutoxy)-benzoesäureethylester (4.35 g) mit 1 N Natronlauge (17.3 mL) in einem 2:1-Gemisch aus Ethanol und Wasser (30 mL) hydrolysiert. Nach beendeter Reaktion wird mit Dichlormethan (30 mL) versetzt, die wässrige Phase durch Zugabe einer 1 M Kaliumhydrogensulfat-Lösung angesäuert und mit Dichlormethan (3x 40 mL) extrahiert. Die organische Phase wird mit gesättigter Natriumchlorid-Lösung gewaschen, über Magnesiumsulfat getrocknet und eingeengt. Man erhält die Titelverbindung, R_{f} (E) = 0.39, als farbloses Öl, das beim Stehenlassen auskristallisiert.
b) 2-(4-Methoxybutoxy)-benzoesäureethylester: Zu einer Mischung aus Salicylsäureethylester (2.63 mL), gepulvertem Kaliumcarbonat (3.10 g) und Kaliumiodid (10 mg) in Acetonitril (50 mL) wird unter Rückfluss eine Lösung von 4-Methoxybutylbromid (4.5 g) in Acetonitril (15 mL) zugetropft und der Reaktionsansatz über Nacht weitergerührt. Nach dem Abkühlen wird filtriert, das Filtrat eingeengt und der Rückstand im HV getrocknet. Man erhält die Titelverbindung (4.4 g), R_{f} (C) = 0.28, als blaßgelbes Öl.

### Beispiel 17:

In analoger Weise wie in Beispiel 15) beschrieben und anschließender FC Reinigung an KG (Fliessmittel C oder D) werden, soweit nicht nachfolgend näher beschrieben, die folgenden Verbindungen hergestellt:
a) Aus 80 mg (3S,5S,1'S,3'S)-5-[3'-Aminomethyl-1'-(*tert*-butoxycarbonyl)amino-4'-methylpentyl]-3-isopropyl-dihydrofuran-2-on und 81 mg 2-Propoxybenzoesäure das (2S,2'S,2''S,4''S)-N-{2-[2'-(*tert*-Butoxycarbonyl)amino-2'-(4''-isopropyl-5''-oxotetrahydrofuran-2''-yl)-ethyl]-3-methylbutyl}-2-propoxy-benzamid, R_{f} (E) = 0.39, als gelbliches Öl.
b) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 110 mg 2-(2-Methoxyethoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(2-methoxyethoxy)-benzamid, R_{f} (E) = 0.28, als farbloses Öl.
c) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 102 mg 2-(Methoxymethoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(methoxymethoxy)-benzamid, R_{f} (E) = 0.40, als gelbliche Festsubstanz.
d) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 135 mg 2-[2-(2-Methoxyethoxy)-ethoxy]-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(2-methoxyethoxy)-ethoxy]-benzamid, R_{f} (E) = 0.20, als gelbliches Öl.
e) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 135 mg 4-Methoxy-2-(3-methoxypropoxy)benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-methoxy-2-(3-methoxypropoxy)-benzamid, R_{f} (L) = 0.80, als gelbliches Öl.
f) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 135 mg 4-Methoxy-3-(3-methoxypropoxy)-benzoesäure das (2S,2'S,2''S,4''S)-N-{2-[2'-( *tert*-Butoxycarbonyl)amino-2'-(4''-isopropyl-5''-oxo-tetrahydrofuran-2''-yl)-ethyl]-3-methylbutyl}-4-methoxy-3-(3-methoxypropoxy)-benzamid, R_{f} (L) = 0.71, als blaßgelbes Öl.
g) Aus 100 mg (3S,5S,1'S,3'S)-5-[3'-Aminomethyl-1'-( *tert*-butoxycarbonyl)amino-4'-methylpentyl]-3-isopropyl-dihydrofuran-2-on und 109 mg 2-(Propoxymethyl)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(propoxymethyl)-benzamid, R_{f} (E) = 0.46, als gelbliches Öl.
h) Aus 80 mg (3S,5S,1S',3'S)-5-[3'-Aminomethyl-1'-( *tert*-butoxycarbonyl)amino-4'-methylpentyl]-3-isopropyl-dihydrofuran-2-on und 101 mg 2-[2-(Methoxymethoxy)-ethoxy]-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(methoxymethoxy)-ethoxy]-benzamid, R₁ (E) = 0.38, als blaßgelbes Öl.
i) Aus 50 mg (3S,5S,1S',3'S)-5-[3'-Aminomethyl-1'-(*tert*-butoxycarbonyl)amino-4'-methylpentyl]-3-isopropyl-dihydrofuran-2-on und 75 mg 2-Acetamido-benzoesäure (Reaktion bei RT über 48 h und anschließend bei 50° C über 12 h) und anschließender FC Reinigung des Rohproduktes an 20 g KG (Fliessmittel D) das (2S,2'S,2"S,4"S)-N-{2-[2'-( *tert*-Butoxycarbonyl)amino-2'-(4''-isopropyl-5''-oxo-tetrahydrofuran-2''-yl)-ethyl]-3-methylbutyl}-2-acetamido-benzamid, R_{f} (E) = 0.25, als gelbliches Öl.
j) Aus 120 mg (3S,5S,1S',3'S)-5-[3'-Aminomethyl-1'-( *tert*-butoxycarbonyl)amino-4'-methylpentyl]-3-isopropyl-dihydrofuran-2-on und 143 mg 2-(3-Methoxypropoxy)-nicotinsäure und anschließender FC Reinigung des Rohproduktes an 30 g KG (Fliessmittel S) das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-methoxypropoxy)-nicotinamid, R_{f} (W) = 0.77, als gelbes Öl.
k) Aus 120 mg (3S,5S,1S',3'S)-5-[3'-Aminomethyl-1'-(*tert*-butoxycarbonyl)amino-4'-methylpentyl]-3-isopropyl-dihydrofuran-2-on und 151 mg 3-(4-Methoxybutoxy)-pyridin-2-carbonsäure und anschließender FC Reinigung des Rohproduktes an 30 g KG (Fliessmittel T) das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-3-(4-methoxybutoxy)-pyddin-2-carbonsäureamid, R_{f} (W ) = 0.70, als gelbes Öl.
l) Aus 50 mg (3S,5S,1'S,3'S)-5-[3'-Aminomethyl-1'-(*tert*-butoxycarbonyl)amino-4'-methylpentyl]-3-isopropyl-dihydrofuran-2-on und 63 mg 2-[2-(Acetamido)-ethoxy]-benzoesäure und anschließender Reinigung des Rohproduktes an 10 g KG (Fliessmittel T) das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(acetamido)-ethoxy]-benzamid, R_{f} (W) = 0.65, als Öl.
m) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 125 mg 2-(4-Methoxybut-2-enoxy)-benzoesäure und anschließender Reinigung des Rohproduktes an 10 g KG (Fliessmittel S) das (2S,2'S,2''S,4''S)-N-{2-[2'-(*tert*-ButoxycarbonyI)amino-2'-(4''-isopropyl-5''-oxo-tetrahydrofuran-2''-yl)-ethyl]-3-methylbutyl}-2-(4-methoxybut-2-enoxy)-benzamid, R_{f} (W) = 0.79, als Öl.
n) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 137 mg 2-(4-Methoxybutoxy)-4-methylbenzoesäure und anschließender Reinigung des Rohproduktes an 25 g KG (Fliessmittel S) das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-methyl-benzamid, R_{f} (W) = 0.81, als blaßgelbes Öl.
o) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 134 mg 2-(5-Methoxypentoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(5-methoxypentoxy)-benzamid, R_{f} (L) = 0.79, als blaßgelbes Öl.

Die als Ausgangsstoffe eingesetzten Benzoesäure-Derivate werden, soweit nicht nachfolgend näher beschrieben, aus entsprechenden Vorstufen analog wie in den Beispielen 1), 2), 16) und 50) beschrieben hergestellt oder nach allgemeinen Standardverfahren erhalten.
A) 2-[2-(2-(Methoxyethoxy)-ethoxy]-benzoesäureethylester: Zu einer Suspension von Salicylsäureethylester (1.86 g) und Kaliumcarbonat-Pulver (1.85 g) in wasserfreiem N,N-Dimethylformamid (50 mL) werden bei 85° C 2-[2-Methoxyethoxy]-ethylbromid (2.25 g) und eine katalytische Menge Kaliumiodid (20 mg) zugegeben. Der Ansatz wird bei 85° C über Nacht gerührt, nach dem Abkühlen filtriert und eingeengt. FC Reinigung (100 g KG, Fliessmittel C) ergibt ein blaßgelbes Öl (2.89 g): R_{f} (D) = 0.29 .
B) 2-Propoxymethyl-benzoesäure: Die Titelverbindung erhält man aus 2-Propoxymethylbenzoesäurepropylester durch alkalische Hydrolyse als blaßgelbe Festsubstanz: R_{f} (Hexan-Essigsäureethylester-Eisessig 50:50:1) = 0.63; MS(EI) *m/e* 194 (M⁺).

Der als Ausgangsmaterial eingesetzte 2-Propoxymethyl-benzoesäurepropylester wird folgendermaßen hergestellt:
a) Zu einer Lösung von Kalium-2-(hydroxymethyl)-benzoat (3.0 g), hergestellt nach dem in J. Am. Chem. Soc. (1989), 111, 1465-1473 beschriebenen Verfahren, in wasserfreiem N,N-Dimethylformamid (20 mL) wird unter Rühren Natriumhydrid 80 % Dispersion in Öl (0.38 g) bei RT addiert. Nach 30 min Rühren wird Propyliodid (8.05 g) tropfenweise zugegeben, der Ansatz auf 80° C erwärmt und 20 h nachgerührt. Nach dem Abkühlen auf RT wird das Reaktionsgemisch auf Eiswasser (50 mL) gegossen und die wässrige Phase mit Diethylether (3x 40 mL) extrahiert. Die organische Phase wird über Magnesiumsulfat getrocknet und eingeengt. FC Reinigung (80 g KG, Fliessmittel B) ergibt den 2-Propoxy-methyl-benzoesäurepropylester (0.69 g), R_{f} (D) = 0.30, als gelbes Öl.
C) 2-[2-( Methoxymethoxy)-ethoxy]-benzoesäureethylester: Zu einer Mischung aus Salicylsäureethylester (4.43 mL) und Kaliumcarbonat-Pulver (4.99 g) in wasserfreiem Aceton (50 mL) und wasserfreiem Dimethylsulfoxid (100 mL) werden 2-(2-Methoxymethoxy)-ethylchlorid (5.62 g), gelöst in Aceton (30 mL), und Kaliumiodid (4.5 g) addiert. Der Ansatz wird bei 70° C über zwei Tage gerührt. Die Suspension wird nach dem Abkühlen filtriert, das Filtrat eingeengt und der Rückstand durch FC (400 g KG, Hexan-Essigsäurethylester 5:1) gereinigt. Man erhält die Titelverbindung als gelbliches Öl (3.8 g), R_{f} (C) = 0.35, das geringe Mengen an einem nicht identifizierten Nebenprodukt enthält.
D) 2-(4-Methoxybut-2-enoxy)-benzoesäuremethylester: Zu 2-(4-Brom-but-2-enoxy)-benzoesäuremethylester (12.1 g) in absolutem Methanol (70 mL) wird eine 30 %ige methanolische Natriummethylat-Lösung (8.83 mL) während 30 min bei 60° C zugetropft und der Ansatz über 5 h gerührt. Nach üblicher Aufarbeitung und FC Reinigung (Hexan-Essigsäureethylester 8:1) erhält man die Titelverbindung als blaßgelbes Öl (6.77 g): R_{f} (C) = 0.36.

Der als Ausgangsmaterial eingesetzte 2-(4-Brom-but-2-enoxy)-benzoesäuremethylester wird folgendermaßen hergestellt: Zu einem Gemisch aus Salicylsäuremethylester (20.0 g) und wasserfreiem Kaliumcarbonat (27.3 g) in Acetonitril (350 mL) wird 1,4-Dibrombuten (28.1 g) addiert. Man rührt unter Rückfluss über 4 h, filtriert und engt das Filtrat ein. Nach FC (400 g KG, Fliessmittel C) erhält man die Titelverbindung, R_{f} (C) = 0.34, als Öl.
E) 2-(4-Methoxybutoxy)-4-methyl-benzoesäure: Durch alkalische Hydrolyse von 2-(4-Methoxybutoxy)-4-methyl-benzoesäuremethylester erhält man die Titelverbindung, R_{f} (Hexan-Essigsäureethylester-Eisessig 50:50:1) = 0.38, als blaßgelbes Öl.

Der als Ausgangsmaterial eingesetzte 2-(4-Methoxybutoxy)-4-methyl-benzoesäuremethylester wird folgendermaßen hergestellt: Analog wie in Beispiel 50f) beschrieben, erhält man aus 2-(4-Brombutoxy)-4-methyl-benzoesäuremethylester (R_{f} (C) = 0.47) die Titelverbindung, R_{f} (C) = 0.31, als Öl.

Die weiter oben als Ausgangsstoff eingesetzte 2-(3-Methoxypropoxy)-nicotinsäure wird folgendermaßen hergestellt:
a) Durch alkalische Hydrolyse analog wie in Beispiel 1) beschrieben erhält man aus 2-(3-Methoxypropoxy)-nicotinsäureethylester die Titelverbindung, R_{f} (Hexan-Essigsäureethylester-Eisessig 50:25:3) = 0.30, als gelbes Öl.
b) 2-(3-Methoxypropoxy)-nicotinsäureethylester: 2-Hydroxy-nicotinsäureethylester (1.67 g), 3-Methoxypropylbromid (2.3 g) und Silbercarbonat (1,38 g) in Toluol (80 mL) werden nach dem von Labaudinière et al. beschriebenen Verfahren (J . *Med. Chem.* 1992, 35, 4315-4324) umgesetzt. Reinigung des Rohproduktes durch FC (Dichlormethan-Methanol-Ammoniak konz. 95:5:1) ergibt das 1-(3-Methoxypropoxy)-3-carbethoxy-2(1 *H*)-pyridinon (1.17 g), R_{f} (Dichlormethan-Methanol-Ammoniak konz. 95:5:1) = 0.59, als blaßgelbes Öl; sowie die Titelverbindung (0.93 g), R_{f} (Dichlormethan-Methanol-Ammoniak konz. 95:5:1) = 0.79, als gelbliches Öl.

Die weiter oben als Ausgangsstoff eingesetzte 3-(4-Methoxybutoxy)-picolinsäure wird folgendermaßen hergestellt:
a) Durch alkalische Hydrolyse wie in Beispiel 1) beschrieben erhält man aus 3-(4-Methoxybutoxy)-picolinsäureethylester die Titelverbindung als Feststoff.
b) 3-(4-Methoxybutoxy)-picolinsäureethylester: Analog dem Verfahren nach Labaudinière et al. (J. *Med. Chem.* 1992, 35, 4315-4324) erhält man aus 3-Hydroxypicolinsäureethylester (2.0 g) und 4-Methoxybutylbromid (2.99 g) und anschließender FC Reinigung (Dichlormethan-Methanol-Ammoniak konz. 95:5:1) die Titelverbindung (0.98 g), R_{f} (Hexan-Essigsäureethylester-Eisessig 1:1:0.01) = 0.21, als gelbes Öl.

### Beispiel 18:

Die Lösung von (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-methoxypropoxy)-benzamid (60 mg) in n-Butylamin (2 mL) wird bei 50° C über 40 h gerührt. Der Ansatz wird eingeengt und der ölige Rückstand durch FC (10 g KG, Fliessmittelgradient von E nach Hexan-Essigsäureethylester1:3) gereinigt. Man erhält das (2S,4S,5S,7S)-N-[4-(tert-Butoxycarbonyl)amino-7-butylcarbamoyl-5-hydroxy-2-isopropyl-8-methyl-nonyl]-2-(3-methoxypropoxy)-benzamid, R_{f} (E) = 0.07; HPLC R_{f} = 18.4 min; MS(FAB) *m/e* 622 (M⁺ +1), als gelbliches Öl.

### Beispiel 19:

Analog wie in Beispiel 18) beschrieben, erhält man durch Umsetzung von (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-benzamid (71 mg) in n-Butylamin (2 mL) bei 50° C über 48 h und anschließende FC Reinigung des Rohproduktes an 10 g KG (Fliessmittelgradient von E nach Hexan-Essigsäureethylester1:4) das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-benzamid, R_{f} (E) = 0.14, als schaumige Festsubstanz.

### Beispiel 20:

Analog wie in Beispiel 18) beschrieben, werden durch Laktonöffnung mit n-Butylamin die nachfolgenden Verbindungen erhalten:
a) Aus 116 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-propoxy-benzamid das (2S,4S,5S,7S)-N-[4-(tert-Butoxycarbonyl)amino-7-butylcarbamoyl-5-hydroxy-2-isopropyl-8-methyl-nonyl]-2-propoxy-benzamid, R_{f} (E) = 0.19, als Öl.
b) Aus 75 mg (2S,2'S,2''S,4''S)-N-{2-[2'-(*tert*-Butoxycarbonyl)amino-2"-(4''-isopropyl-5''-oxo-tetrahydrofuran-2''-yl)-ethyl]-3-methylbutyl}-2-(2-methoxyethoxy)-benzamid das (2S,4S,5S,7S)-N-[4-(tert-Butoxycarbonyl)amino-7-butylcarbamoyl-5-hydroxy-2-isopropyl-8-methyl-nonyl]-2-(2-methoxyethoxy)-benzamid, R_{f} (E) = 0.06, als schaumige Festsubstanz.
c) Aus 88 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(methoxymethoxy)-benzamid das (2S,4S,5S,7S)-N-[4-(tert-Butoxycarbonyl)amino-7-butylcarbamoyl-5-hydroxy-2-isopropyl-8-methyl-nonyl]-2-(2-methoxymethoxy)-benzamid, R_{f} (E) = 0.09, als schaumige Festsubstanz.
d) Aus 50 mg (2S,2'S,2''S,4''S)-N-{2-[2'-(*tert*-Butoxycarbonyl)amino-2''-(4''-isopropyl-5''-oxo-tetrahydrofuran-2''-yl)-ethyl]-3-methylbutyl}-2-[2-(2-methoxyethoxy)-ethoxy]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(2-methoxyethoxy)-ethoxy]-benzamid, R_{f} (F) = 0.11, als Öl.
e) Aus 107 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-methoxy-2-(3-methoxypropoxy)-benzamid und FC Reinigung an 25 g KG (Fliessmittelgradient von R nach P) das (2S,4S,5S,7S)-N-[4-(tert-Butoxycarbonyl)amino-7-butylcarbamoyl-5-hydroxy-2-isopropyl-8-methyl-nonyl]-4-methoxy-2-(3-methoxypropoxy)-benzamid, R_{f} (L) = 0.63, als gelbes Öl.
f) Aus 96 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-methoxy-3-(3-methoxypropoxy)-benzamid und FC Reinigung, wie in Beispiel 20e) beschrieben, das (2S,4S,5S,7S)-N-[4-(tert-Butoxycarbonyl)amino-7-butylcarbamoyl-5-hydroxy-2-isopropyl-8-methyl-nonyl]-4-methoxy-3-(3-methoxypropoxy)-benzamid, R_{f} (L) = 0.53, als schaumige Festsubstanz.
g) Aus 70 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-propoxymethyl-benzamid und FC Reinigung (25 g KG, Fliessmittel R) das (2S,4S,5S,7S)-N-[4-(tert-Butoxycarbonyl)amino-7-butylcarbamoyl-5-hydroxy-2-isopropyl-8-methyl-nonyl]-2-propoxymethyl-benzamid, R_{f} (L) = 0.56, als schaumige Festsubstanz.
h) Aus 60 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(methoxymethoxy)-ethoxy]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(methoxymethoxy)-ethoxy]-benzamid, R_{f} (L) = 0.56, als gelbliches Öl.
i) Aus 50 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-acetamido-benzamid das (2S,4S,5S,7S)-N-[4-(tert-Butoxycarbonyl)amino-7-butylcarbamoyl-5-hydroxy-2-isopropyl-8-methyl-nonyl]-2-acetamido-benzamid, R_{f} (L) = 0.64, als Öl.
j) Aus 60 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-methoxypropoxy)-nicotinamid und FC Reinigung des Rohproduktes an 25 g KG (Fliessmittelgradient von O nach P) das (2S,4S,5S,7S)-N-[4-(tert-Butoxycarbonyl)amino-7-butylcarbamoyl-5-hydroxy-2-isopropyl-8-methyl-nonyl]-2-(3-methoxypropoxy)-nicotinamid, R_{f} (L) = 0.56, als farbloses Öl.
k) Aus 65 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-3-(4-methoxybutoxy)-pyridin-2-carbonsäureamid und FC Reinigung des Rohproduktes an 25 g KG (Fliessmittel V) das (2S,4S,5S,7S)-N-[4-(tert-Butoxycarbonyl)amino-7-butylcarbamoyl-5-hydroxy-2-isopropyl-8-methyl-nonyl]-3-(4-methoxybutoxy)-pyridin-2-carbonsäureamid, R_{f} (W) = 0.56, als gelbes Öl.
l) Aus 75 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(acetamido)-ethoxy]-benzamid und FC Reinigung des Rohproduktes an 25 g KG (Fliessmittelgradient von T nach V) das (2S,4S,5S,7S)-N-[4-(tert-Butoxycarbonyl)amino-7-butylcarbamoyl-5-hydroxy-2-isopropyl-8-methyl-nonyl]-2-[2-(acetamido)-ethoxy]-benzamid, R_{f} (W) = 0.41, als Öl.
m) Aus 75 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybut-2-enoxy)-benzamid und FC Reinigung des Rohproduktes an 25 g KG (Fliessmittelgradient von S nach V) das (2S,4S,5S,7S)-N-[4-(tert-Butoxycarbonyl)amino-7-butylcarbamoyl-5-hydroxy-2-isopropyl-8-methyl-nonyl]-2-(4-methoxybut-2-enoxy)-benzamid, R_{f} (W) = 0.57, als Festsubstanz .
n) Aus 75 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-methyl-benzamid das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-methyl-benzamid, R_{f} (W) = 0.63, als Festsubstanz.

### Beispiel 21:

Zu (2S,4S,5S,7S)-N-[4-(tert-Butoxycarbonyl)amino-7-butylcarbamoyl-5-hydroxy-2-isopropyl-8-methyl-nonyl]-2-(3-methoxypropoxy)-benzamid (50 mg) wird bei 0° C eine 4 N Salzsäure-Lösung in Dioxan (2 mL) addiert. Der Reaktionsansatz wird bei 0° C über 2 h gerührt (DC Kontrolle) und anschließend das Lösungsmittel direkt am HV unter starkem Rühren bis zum Gefrieren eingeengt und im weiteren durch Lyophilisation entfernt. Nach Trocknen am HV erhält man das (2S,4S,5S,7S)-N-(4-Amino-7-butylcarbamoyl-5-hydroxy-2-isopropyl-8-methyl-nonyl)-2-(3-methoxypropoxy)-benzamid-hydrochlorid als schaumige Festsubstanz: R_{f} (W) = 0.31. HPLC Rₜ = 12.8 min. MS(FAB) *m/e* 522 (M⁺ +1).

### Beispiel 22:

Analog wie in Beispiel 21) beschrieben, erhält man durch Umsetzung von (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-benzamid (61 mg ) das (2S,4S,5S,7S)-N-(4-Amino-7-butylcarbamoyl-5-hydroxy-2-isopropyl-8-methyl-nonyl)-2-(4-methoxybutoxy)-benzamid-hydrochlorid: R_{f} (W) = 0.29. HPLC Rₜ = 13.3 min. MS(FAB) *m/e* 536 (M⁺ +1).

### Beispiel 23:

Analog wie in Beispiel 21) beschrieben, werden durch De-Bocylierung die nachfolgenden Verbindungen hergestellt:
a) Aus 100 mg (2S,4S,5S,7S)-N-[4-(tert-Butoxycarbonyl)amino-7-butylcarbamoyl-5-hydroxy-2-isopropyl-8-methyl-nonyl]-2-propoxy-benzamid das (2S,4S,5S,7S)-N-(4-Amino-7-butylcarbamoyl-5-hydroxy-2-isopropyl-8-methyl-nonyl)-2-propoxy-benzamid-hydrochlorid: R_{f} (W) = 0.37. HPLC Rₜ = 13.95 min. MS(FAB) *m/e* 492 (M⁺ +1).
b) Aus 60 mg (2S,4S,5S,7S)-N-[4-(tert-Butoxycarbonyl)amino-7-butylcarbamoyl-5-hydroxy-2-isopropyl-8-methyl-nonyl]-2-(2-methoxyethoxy)-benzamid das (2S,4S,5S,7S)-N-(4-Amino-7-butylcarbamoyl-5-hydroxy-2-isopropyl-8-methyl-nonyl)-2-(2-methoxyethoxy)-benzamid-hydrochlorid: R_{f} (W) = 0.38. HPLC Rₜ = 12.6 min. MS(FAB) *m/e* 508 (M⁺ +1).
c) Aus 38 mg (2S,4S,5S,7S)-N-[4-(tert-Butoxycarbonyl)amino-7-butylcarbamoyl-5-hydroxy-2-isopropyl-8-methyl-nonyl]-2-[2-(2-methoxyethoxy)-ethoxy]-benzamid das (2S,4S,5S,7S)-N-(4-Amino-7-butylcarbamoyl-5-hydroxy-2-isopropyl-8-methyl-nonyl)-2-[2-(2-methoxyethoxy)-ethoxy]-benzamid-hydrochlorid: R_{f} (W) = 0.19. HPLC Rₜ = 12.4 min. MS(FAB) *m/e* 552 (M⁺ +1).
d) Aus 93 mg (2S,4S,5S,7S)-N-[4-(tert-Butoxycarbonyl)amino-7-butylcarbamoyl-5-hydroxy-2-isopropyl-8-methyl-nonyl]-4-methoxy-2-(3-methoxypropoxy)-benzamid das (2S,4S,5S,7S)-N-(4-Amino-7-butylcarbamoyl-5-hydroxy-2-isopropyl-8-methyl-nonyl)-4-methoxy-2-(3-methoxypropoxy)-benzamid-hydrochlorid: R_{f} (W) = 0.25. HPLC Rₜ =13.4 min. MS(FAB) *m/e* 552 (M⁺ +1).
e) Aus 76 mg (2S,4S,5S,7S)-N-[4-(tert-Butoxycarbonyl)amino-7-butylcarbamoyl-5-hydroxy-2-isopropyl-8-methyl-nonyl]-4-methoxy-3-(3-methoxypropoxy)-benzamid das (2S,4S,5S,7S)-N-(4-Amino-7-butylcarbamoyl-5-hydroxy-2-isopropyl-8-methyl-nonyl)-4-methoxy-3-(3-methoxypropoxy)-benzamid-hydrochlorid: R_{f} (W) = 0.28. HPLC Rₜ = 12.1 min. MS(FAB) *m/e* 552 (M⁺ +1).
f) Aus 58 mg (2S,4S,5S,7S)-N-[4-(tert-Butoxycarbonyl)amino-7-butylcarbamoyl-5-hydroxy-2-isopropyl-8-methyl-nonyl]-2-propoxymethyl-benzamid das (2S,4S,5S,7S)-N-(4-Amino-7-butylcarbamoyl-5-hydroxy-2-isopropyl-8-methyl-nonyl)-2-(propoxymethyl)-benzamid-hydrochlorid: R_{f} (W) = 0.25. HPLC Rₜ = 13.4 min. MS(FAB) *m/e* 506 (M⁺ +1).
g) Aus 40 mg (2S,4S,5S,7S)-N-[4-(tert-Butoxycarbonyl)amino-7-butylcarbamoyl-5-hydroxy-2-isopropyl-8-methyl-nonyl]-2-acetamido-benzamid und FC Reinigung des Rohproduktes an 10 g KG (Fliessmittel M) das (2S,4S,5S,7S)-N-(4-Amino-7-butylcarbamoyl-5-hydroxy-2-isopropyl-8-methyl-nonyl)-2-acetamido-benzamid-hydrochlorid: R_{f} (W) = 0.45. HPLC Rₜ = 10.0 min. MS(FAB) *m/e* 473 [(M⁺ +1) - H₂O].
h) Aus 62 mg (2S,4S,5S,7S)-N-[4-(tert-Butoxycarbonyl)amino-7-butylcarbamoyl-5-hydroxy-2-isopropyl-8-methyl-nonyl]-2-[2-(acetamido)-ethoxy]-benzamid das (2S,4S,5S,7S)-N-(4-Amino-7-butylcarbamoyl-5-hydroxy-2-isopropyl-8-methyl-nonyl)-2-[2-(acetamido)-ethoxy]-benzamid-hydrochlorid: R_{f} (W) = 0.25. HPLC Rₜ = 10.4 min. MS(FAB) *m/e* 535 (M⁺ +1). i) Aus 54 mg (2S,4S,5S,7S)-N-[4-(tert-Butoxycarbonyl)amino-7-butylcarbamoyl-5-hydroxy-2-isopropyl-8-methyl-nonyl]-2-(4-methoxybut-2-enoxy)-benzamid das (2S,4S,5S,7S)-N-(4-Amino-7-butylcarbamoyl-5-hydroxy-2-isopropyl-8-methyl-nonyl)-2-(4-methoxybut-2-enoxy)-benzamid-hydrochlorid: R_{f} (W) = 0.38. HPLC Rₜ = 12.6 min. MS(FAB) *m/e* 534 (M⁺ +1).
j) Aus 59 mg (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-methyl-benzamid das (2S,4S,5S,7S)-N-(4-Amino-7-butylcarbamoyl-5-hydroxy-2-isopropyl-8-methyl-nonyl)-2-(4-methoxybutoxy)-4-methyl-benzamid-hydrochlorid: R_{f} (W) = 0.33. HPLC Rₜ = 14.2 min. MS(FAB) *m/e* 550 (M⁺ +1).
k) Aus 54 mg (2S,4S,5S,7S)-N-[4-(tert-Butoxycarbonyl)amino-7-butylcarbamoyl-5-hydroxy-2-isopropyl-8-methyl-nonyl]-2-(3-methoxypropoxy)-nicotinamid und anschließender FC Reinigung des Rohproduktes (Fliessmittelgradient von V nach U) das (2S,4S,5S,7S)-N-[4-Amino-7-butylcarbamoyl-5-hydroxy-2-isopropyl-8-methyl-nonyl]-2-(3-methoxypropoxy)-nicotinamid-hydrochlorid: R_{f} (W) = 0.50. HPLC Rₜ = 12.4 min. MS(FAB) *m/e* 523 (M⁺ +1).
l) Aus 45 mg (2S,4S,5S,7S)-N-[4-(tert-Butoxycarbonyl)amino-7-butylcarbamoyl-5-hydroxy-2-isopropyl-8-methyl-nonyl]-3-(4-methoxybutoxy)-pyddin-2-carbonsäureamid das (2S,4S,5S,7S)-N-[4-Amino-7-butylcarbamoyl-5-hydroxy-2-isopropyl-8-methyl-nonyl]-3-(4-methoxybutoxy)-pyridin-2-carbonsäureamid-hydrochlorid: R_{f} (W) = 0.33. HPLC Rₜ = 10.2 min. MS(FAB) *m/e* 537 (M⁺ +1).

### Beispiel 24:

Zur Lösung von (2S,4S,5S,7S)-N-[4-(tert-Butoxycarbonyl)amino-7-butylcarbamoyl-5-hydroxy-2-isopropyl-8-methyl-nonyl]-2-(methoxymethoxy)-benzamid (46 mg) in Dichlormethan (2 mL) wird bei 0° C unter Rühren Trifluoressigsäure (0.5 mL) addiert. Nach beendeter Reaktion (ca. 30 min) wird mit Toluol (2 mL) versetzt und der Reaktionsansatz eingeengt. Das nach kurzem Trocknen am HV erhaltene Rohprodukt wird durch FC an 6 g KG (Fliessmittel Q) gereinigt, und man erhält das (2S,4S,5S,7S)-N-(4-Amino-7-butylcarbamoyl-5-hydroxy-2-isopropyl-8-methyl-nonyl)-2-hydroxy-benzamid-trifluoracetat: R_{f} (W) = 0.34. HPLC Rₜ = 12.1 min. MS(FAB) *m/e* 450 (M⁺ +1).

### Beispiel 25:

In analoger Weise wie in Beispiel 24) beschrieben, wird durch De-Bocylierung aus 44 mg (2S,4S,5S,7S)-N-[4-(tert-Butoxycarbonyl)amino-7-butylcarbamoyl-5-hydroxy-2-isopropyl-8-methyl-nonyl]-2-[2-(methoxymethoxy)-ethoxy]-benzamid das (2S,4S,5S,7S)-N-(4-Amino-7-butylcarbamoyl-5-hydroxy-2-isopropyl-8-methyl-nonyl)-2-[2-(methoxymethoxy)-ethoxy]-benzamid-trifluoracetat, R_{f} (W) = 0.29; HPLC Rₜ = 12.3 min; MS(FAB) *m/e* 538 (M⁺ +1), erhalten.

### Beispiel 26:

In analoger Weise wie in Beispiel 27) beschrieben und anschließender FC Reinigung des Rohproduktes jeweils an 10 bis 25 g KG (Fliessmittelsystem Dichlormethan-Methanol-Ammoniak konz.) werden durch Laktonöffnung mit N-(2-Aminoethyl)-morpholin (0.5 mL) bei 80° C über Nacht die nachfolgenden Verbindungen erhalten:
a) Aus 75 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-benzamid das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(4-methoxybutoxy)-benzamid, R_{f} (W) = 0.35, als farbloses Öl.
b) Aus 68 mg (2S,2'S,2''S,4''S)-N-{2-[2'-(*tert*-Butoxycarbonyl)amino-2'-(4''-isopropyl-5''-oxo-tetrahydrofuran-2"-yl)-ethyl]-3-methylbutyl}-2-(2-methoxyethoxy)-benzamid das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(2-methoxyethoxy)-benzamid, R_{f} (W) = 0.24, als gelbliches Öl.
c) Aus 60 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-methoxypropoxy)-nicotinamid und FC Reinigung des Rohproduktes an 25 g KG (Fliessmittelgradient von P nach O) das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(3-methoxypropoxy)-nicotinamid, R_{f} (L) = 0.35, als gelbes Öl.
d) Aus 65 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-3-(4-methoxybutoxy)-pyridin-2-carbonsäureamid und FC Reinigung des Rohproduktes an 25 g KG (Fliessmittel V) das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-3-(4-methoxybutoxy)-pyridin-2-carbonsäureamid, R_{f} (W) = 0.38, als schaumige Festsubstanz.
e) Aus 45 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybut-2-enoxy)-benzamid das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(4-methoxybut-2-enoxy)-benzamid, R_{f} (W) = 0.50, als Öl.
f) Aus 75 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-methyl-benzamid und Reinigung des Rohproduktes an 25 g KG (Fliessmittelgradient von Q nach M) das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(4-methoxybutoxy)-4-methyl-benzamid, R_{f} (L) = 0.38, als Öl.
g) Aus 90 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(5-methoxypentoxy)-benzamid (Beispiel 17o) das (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(5-methoxypentoxy)-benzamid, R_{f} (L) = 0.55, als farbloses Öl.

### Beispiel 27:

Die Mischung aus (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-benzamid (150 mg) und N-(3-Aminopropyl)-morpholin (0.5 mL) wird bei 80° C über Nacht gerührt. Nach dem Abkühlen auf RT wird das Reaktionsgemisch direkt über 25 g KG (Fliessmittel V) chromatographiert. Man erhält das (2S,4S,5S,7S)-N-[4-(*tert*-Butoxycarbonyl)amino-5-hydroxy-2-isopropyl-8-methyl-7-(3-morpholin-4-ylpropylcarbamoyl)-nonyl]-2-(4-methoxybutoxy)-benzamid, R_{f} (W) = 0.43, als Öl.

### Beispiel 28:

(2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(3-methoxypropoxy)-benzamid (64 mg) werden analog wie in Beispiel 21) beschrieben in 2 mL einer 4 N Salzsäure-Lösung in Dioxan bei 0° C über 1 h gerührt. Nach Entfernen des Lösungsmittels und Trocknen am HV erhält man das (2S,4S,5S,7S)-N-[4-Amino-5-hydroxy-2-isopropyl-8-methyl-7-(2-morpholin-4-ylethylcarbamoyl)-nonyl]-2-(3-methoxypropoxy)-benzamid-dihydrochlorid: R_{f} (W) = 0.13. HPLC Rₜ = 9.59 min. MS(FAB) *m/e* 579 (M⁺ +1).

### Beispiel 29:

In analoger Weise wie in Beispiel 21) beschrieben, werden durch De-Bocylierung die nachfolgenden Verbindungen erhalten:
a) Aus 80 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(4-methoxybutoxy)-benzamid das (2S,4S,5S,7S)-N-[4-Amino-5-hydroxy-2-isopropyl-8-methyl-7-(2-morpholin-4-ylethylcarbamoyl)-nonyl]-2-(4-methoxybutoxy)-benzamid-dihydrochlodd: R_{f} (W) = 0.26. HPLC Rₜ = 9.9 min. MS(FAB) *m/e* 593 (M⁺ +1).
b) Aus 72 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(2-methoxyethoxy)-benzamid das (2S,4S,5S,7S)-N-[4-Amino-5-hydroxy-2-isopropyl-8-methyl-7-(2-morpholin-4-ylethylcarbamoyl)-nonyl]-2-(2-methoxyethoxy)-benzamid-dihydrochlorid: R_{f} (W) = 0.40. HPLC Rₜ = 8.9 min. MS(FAB) *m/e* 565 (M⁺ +1).
c) Aus 63 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(3-methoxypropoxy)-nicotinamid das (2S,4S,5S,7S)-N-[4-Amino-5-hydroxy-2-isopropyl-8-methyl-7-(2-morpholin-4-yl-ethylcarbamoyl)-nonyl]-2-(3-methoxypropoxy)-nicotinamid-dihydrochlorid: R_{f} (W) = 0.27. HPLC Rₜ = 8.4 min. MS(FAB) *m/e* 580 (M⁺ +1).
d) Aus 46 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-3-(4-methoxybutoxy)-pyridin-2-carbonsäureamid das (2S,4S,5S,7S)-N-[4-Amino-5-hydroxy-2-isopropyl-8-methyl-7-(2-morpholin-4-yl-ethylcarbamoyl)-nonyl]-3-(4-methoxybutoxy)-pyridin-2-carbonsäureamiddihydrochlorid: R_{f} (W) = 0.16. MS(FAB) *m/e* 593 (M⁺ +1).
e) Aus 50 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(4-methoxybut-2-enoxy)-benzamid das (2S,4S,5S,7S)-N-[4-Amino-5-hydroxy-2-isopropyl-8-methyl-7-(2-morpholin-4-ylethylcarbamoyl)-nonyl]-2-(4-methoxybut-2-enoxy)-benzamid-dihydrochlorid: R_{f} (W) = 0.17. HPLC Rₜ = 9.15 min. MS(FAB) *m/e* 591 (M⁺ +1).
f) Aus 75 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(4-methoxybutoxy)-4-methyl-benzamid das (2S,4S,5S,7S)-N-[4-Amino-5-hydroxy-2-isopropyl-8-methyl-7-(2-morpholin-4-ylethylcarbamoyl)-nonyl]-2-(4-methoxybutoxy)-4-methyl-benzamid-dihydrochlorid: R_{f} (W) = 0.28. HPLC Rₜ = 10.6 min. MS(FAB) *m/e* 607 (M⁺ +1).
g) Aus 75 mg (2S,4S,5S,7S)-N-[4-(*tert*-Butoxycarbonyl)amino-5-hydroxy-2-isopropyl-8-methyl-7-(2-morpholin-4-ylethylcarbamoyl)-nonyl]-2-(5-methoxypentoxy)-benzamid das (2S,4S,5S, 7S)-N-[4-Amino-5-hydroxy-2-isopropyl-8-methyl-7-(2-morpholin-4-ylethylcarbamoyl)-methyl-nonyl]-2-(5-methoxypentoxy)-benzamid-dihydrochlorid: R_{f} (W) = 0.29. HPLC Rₜ = 10.2 min. MS(FAB) *m/e* 607 (M⁺ +1).
h) Aus 96 mg (2S,4S,5S,7S)-N-[4-(*tert*-Butoxycarbonyl)amino-5-hydroxy-2-isopropyl-8-methyl-7-(3-morpholin-4-ylpropylcarbamoyl)-nonyl]-2-(4-methoxybutoxy)-benzamid das (2S,4S,5S,7S)-N-[4-Amino-5-hydroxy-2-isopropyl-8-methyl-7-(3-morpholin-4-ylpropylcarbamoyl)-nonyl]-2-(4-methoxybutoxy)-benzamid-dihydrochlorid: R_{f} (W) = 0.14. HPLC Rₜ = 10.0 min. MS(FAB) *m/e* 607 (M⁺ +1).

### Beispiel 30:

In analoger Weise wie in Beispiel 1) beschrieben, werden die folgenden Verbindungen hergestellt:
a) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 172 mg 2-(4-Methoxybutoxy)-4-(morpholin-4-ylmethyl)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(morpholin-4-ylmethyl)-benzamid, R_{f} (W) = 0.55, als gelbes Öl.
b) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 198 mg 2-(4-Methoxybutoxy)-4-(2-morpholin-4-ylethoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(2-morpholin-4-ylethoxy)-benzamid, R_{f} (W) = 0.65, als gelbes Öl.
c) Aus 50 mg (3S,SS,1'S,3'S)-5-[3'-Aminomethyl-1'-(*tert*-butoxycarbonyl)amino-4'-methylpentyl]-3-isopropyl-dihydrofuran-2-on und 91 mg 4-[3-(Dimethylamino)-propoxy]-2-(4-methoxybutoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-[3-(dimethylamino)-propoxy]-2-(4-methoxybutoxy)-benzamid, R_{f} (W) = 0.44, als Öl.
d) Aus 50 mg (3S,5S,1'S,3'S)-5-[3'-Aminomethyl-1'-(*tert*-butoxycarbonyl)amino-4'-methylpentyl]-3-isopropyl-dihydrofuran-2-on und 90 mg 2-(4-Methoxybutoxy)-4-(piperidin-1-yl)-methyl-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(piperidin-1-yl)methyl-benzamid, R_{f} (W) = 0.60, als Öl.
e) Aus 50 mg (3S,5S,1'S,3'S)-5-[3'-Aminomethyl-1'-(*tert*-butoxycarbonyl)amino-4'-methylpentyl]-3-isopropyl-dihydrofuran-2-on und 86 mg 2-(4-Methoxybutoxy)-4-(pyrrolidin-1-yl)-methyl-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(pyrrolidin-1-yl)methyl-benzamid, R_{f} (W) = 0.56, als Öl.
f) Aus 125 mg (3S,5S,1'S,3'S)-5-[3'-Aminomethyl-1'-(*tert*-butoxycarbonyl)amino-4'-methylpentyl]-3-isopropyl-dihydrofuran-2-on und 246 mg 2-(4-Methoxybutoxy)-4-(2-piperidin-1-ylethoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(2-piperidin-1-ylethoxy)-benzamid, R_{f} (W) = 0.58, als Öl.
g) Aus 80 mg (3S,5S,1'S,3'S)-5-[3'-Aminomethyl-1'-(*tert*-butoxycarbonyl)amino-4'-methylpentyl]-3-isopropyl-dihydrofuran-2-on und 252 mg 4-Dimethylaminomethyl-2-(4-methoxy-butoxy)-benzoesäure und anschließender FC Reinigung (Fliessmittel N) das (2S,2'S,2''S,4''S)-N-{2-[2'-(*tert*-Butoxycarbonyl)amino-2'-(4''-isopropyl-5''-oxo-tetrahydrofuran-2"-yl)-ethyl]-3-methylbutyl}-4-dimethylaminomethyl-2-(4-methoxybutoxy)-benzamid, R_{f} (L) = 0.41, als Öl.
h) Aus 80 mg (3S,5S,1'S,3'S)-5-[3'-Aminomethyl-1'-(*tert*-butoxycarbonyl)amino-4'-methylpentyl]-3-isopropyl-dihydrofuran-2-on und 274 mg 2-(4-Methoxybutoxy)-4-(4-methylpiperazin-1-yl)methyl-benzoesäure und anschließender FC Reinigung (Fliessmittelgradient von P nach N) das (2S,2'S,2''S,4''S)-N-{2-[2'-(*tert*-Butoxycarbonyl)amino-2'-(4''-isopropyl-5''-oxo-tetrahydrofu ran-2''-yl)-ethyl]-3-methylbutyl}-2-(4-methoxybutoxy)-4-(4-methylpiperazin-1-yl)methyl-benzamid, R_{f} (W) = 0.28, als gelbliche Festsubstanz.
i) Aus 80 mg (3S,5S,1'S,3'S)-5-[3'-Aminomethyl-1'-(*tert*-butoxycarbonyl)amino-4'-methylpentyl]-3-isopropyl-dihydrofuran-2-on und 255 mg 4-(4-Acetylpiperazin-1-yl)-methyl-2-(4-methoxybutoxy)-benzoesäure und anschließender FC Reinigung (Fliessmittel O) das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-(4-acetylpiperazin-1 -yl)-methyl-2-(4-methoxybutoxy)-benzamid, R_{f} (W) = 0.55, als gelbliche Festsubstanz.

Die als Ausgangsstoffe eingesetzten Benzoesäuren werden wie nachfolgend beschrieben hergestellt:
A) 2-(4-Methoxybutoxy)-4-(morpholin-4-ylmethyl)-benzoesäure: Die Titelverbindung erhält man durch Hydrolyse von 2-(4-Methoxybutoxy)-4-(morpholin-4-ylmethyl)-benzoesäuremethylester (1.08 g) mit 1 N Natronlauge (4.75 mL) in einem 2:1 Gemisch aus Ethanol und Wasser (15 mL) bei 50° C und üblicher Aufarbeitung als gelbes Öl: R_{f} (L) = 0.36.
   Der eingesetzte 2-(4-Methoxybutoxy)-4-(morpholin-4-ylmethyl)-benzoesäuremethylester wird folgendermaßen hergestellt: Das Gemisch aus 2-(4-Methoxybutoxy)-4-methyl-benzoesäuremethylester (1.0 g), N-Bromsuccinimid (0.70 g), 2',2''-Azoisobutyronitril (23 mg) und Dibenzoylperoxid (34 mg) in Tetrachlorkohlenstoff (10 mL) wird über 5 h unter Rückfluss gerührt. Nach Abkühlen auf RT wird vom Niederschlag abfiltriert, das Filtrat mit Morpholin (1.03 mL) versetzt und über 2 h bei RT nachgerührt. Das nach Filtration und Einengen erhaltene Rohprodukt wird durch FC an KG (40 g, Fliessmittelgradient von C nach F) gereinigt. Man erhält die Titelverbindung, R_{f} (F) = 0.14, als gelbliches Öl .
B) 2-(4-Methoxybutoxy)-4-(2-morpholin-4-ylethoxy)-benzoesäure: Die Mischung aus 2-(4-Methoxybutoxy)-4-(2-morpholin-4-ylethoxy)-benzoesäuremethylester (2.95 g) und 1 N Natronlauge (8.83 mL) in Ethanol (10 mL) und Wasser (5mL) wird bei 50° C über Nacht gerührt. Nach üblicher Aufarbeitung erhält man die Titelverbindung (2.50 g), R_{f} (L) = 0.51, als gelbliches Öl.
   Der als Ausgangsmaterial eingesetzte 2-(4-Methoxybutoxy)-4-(2-morpholin-4-ylethoxy)-benzoesäuremethylester wird folgendermaßen hergestellt:
   a) Die Suspension aus 4-Hydroxy-2-(4-methoxybutoxy)-benzoesäuremethylester (2.0 g), 2-Chlorethylmorpholin (11.8 g) und Cesiumcarbonat (12.8 g) in Aceton (30 mL) wird 2 h unter Rückfluss gerührt. Filtration und FC Reinigung (80 g KG, Fliessmittel F und Essigsäureethylester-Ammoniak konz. 10:0.1) ergibt den 2-(4-Methoxybutoxy)-4-(2-morpholin-4-ylethoxy)-benzoesäuremethylester (2.96 g), R_{f} (N) = 0.73, als blaßgelbes Öl.
   b) 4-Hydroxy-2-(4-methoxybutoxy)-benzoesäuremethylester: Die Lösung von 4-Benzyloxy-2-(4-methoxybutoxy)-benzoesäuremethylester(13.8 g) in Essigsäureethylester (130 mL) wird in Gegenwart von 10 % Pd/C (1.37 g) bei RT über 2 h hydriert. Man erhält die Titelverbindung (10.1 g), R_{f} (C) = 0.09; Schmp. 62-63° C, als weisse Festsubstanz .
C) 2-(4-Methoxybutoxy)-4-(3-dimethylaminopropoxy)-benzoesäure: 2-(4-Methoxybutoxy)-4-(3-dimethylaminopropoxy)-benzoesäuremethylester (2.12 g), gelöst in einem Gemisch aus Ethanol (10 mL) und Wasser (5 mL), wird in Gegenwart von 1 N Natronlauge (6.87 mL) hydrolysiert. Nach beendeter Reaktion wird Dichlormethan (100 mL) addiert und die wässrige Phase durch Zugabe von 1 M Kaliumhydrogensulfat-Lösung auf pH 6 gestellt. Nach wiederholter Extraktion der wässrigen Phase mit Dichlormethan wird die vereinte organische Phase mit Sole (20 mL) gewaschen, über Magnesiumsulfat getrocknet und eingeengt. Man erhält die Titelverbindung im Gemisch mit anorganischen Salzen als gelbliches Öl, das ohne weitere Reinigung weiter umgesetzt wird.
   Den als Ausgangsmaterial eingesetzten 2-(4-Methoxybutoxy)-4-(3-dimethylaminopropoxy)-benzoesäuremethylester erhält man in analoger Weise wie in Beispiel 30 Aa) beschrieben aus 2-(4-Methoxybutoxy)-4-methyl-benzoesäuremethylester (2.0 g) und Dimethylaminopropylchlorid (2.4 g) und anschließender FC Reinigung an 40 g KG (Fliessmittel F und Essigsäureethylester-Ammoniak konz. 99:1) als Öl.
D) 2-(4-Methoxybutoxy)-4-(piperidin-1-ylmethyl)-benzoesäure: 2-(4-Methoxybutoxy)-4-(piperidin-1-ylmethyl)-benzoesäuremethylester (1.35 g), gelöst in Ethanol (20 mL) und Wasser (10 mL), wird mit 1 N Natronlauge (4.8 mL) versetzt und bei RT über Nacht gerührt. Durch Zugabe von 1 M Kaliumhydrogensulfat-Lösung wird der Reaktionsansatz auf pH 6 gestellt und weitgehend eingeengt. Der Rückstand wird in Dioxan (30 mL) aufgenommen, die Lösung in einem Trockeneisbad eingefroren und am HV lyophilisiert. Man erhält die Titelverbindung im Gemisch mit anorganischen Salzen als hellbraune Festsubstanz (1.60 g), die ohne weitere Reinigung umgesetzt wird: R_{f} (L) = 0.05.
   Der eingesetzte 2-(4-Methoxybutoxy)-4-(piperidin-1-ylmethyl)-benzoesäuremethylester wird analog wie in Beispiel 30 Aa) beschrieben aus 2-(4-Methoxybutoxy)-4-methyl-benzoesäuremethylester und Piperidin und anschließender Reinigung des Rohproduktes durch FC (Fliessmittel C und M) hergestellt: braunes Öl, R_{f} (N) = 0.34.
E) 2-(4-Methoxybutoxy)-4-(pyrrolidin-1-ylmethyl)-benzoesäure: Die Titelverbindung wird analog wie in Beispiel 30 D) beschrieben aus 2-(4-Methoxybutoxy)-4-(pyrrolidin-1-ylmethyl)-benzoesäuremethylester im Gemisch mit anorganischen Salzen erhalten.
   Der eingesetzte 2-(4-Methoxybutoxy)-4-(pyrrolidin-1-ylmethyl)-benzoesäuremethylester wird analog wie in Beispiel 30 Aa) beschrieben aus 2-(4-Methoxybutoxy)-4-methyl-benzoesäuremethylester und Pyrrolidin und anschließende Reinigung des Rohproduktes durch FC (Fliessmittel C und M) hergestellt: braun-schwarzes Öl, R_{f} (N) = 0.22.
F) 2-(4-Methoxybutoxy)-4-(piperidin-1-ylethoxy)-benzoesäure: Die Titelverbindung wird aus 2-(4-Methoxybutoxy)-4-(piperidin-1-ylethoxy)-benzoesäuremethylester durch alkalische Hydrolyse, analog wie in Beispiel 30D) beschrieben, und anschließender FC Reinigung (Fliessmittelgradient von N nach Dichlormethan-Methanol 8:2) als gelbliches Öl erhalten, das beim Stehenlassen langsam auskristallisiert: R_{f} (Dichlormethan-Methanol 8:2) = 0.50; Schmp. 91-94° C.
   Der als Ausgangsmaterial eingesetzte 2-(4-Methoxybutoxy)-4-(piperidin-1-ylethoxy)-benzoesäuremethylester wird folgendermaßen hergestellt:
   a) Die Lösung von 2-(4-Methoxybutoxy)-4-(piperidin-1-ylcarbamoylmethoxy)-benzoesäuremethylester (2.29 g) in Tetrahydrofuran (10 mL) wird zu einer 1 M Boran THF-Komplexlösung in Tetrahydrofuran (10.0 mL) bei 0-5° C über 15 min zugetropft. Anschließend erwärmt man auf Rückflusstemperatur und rührt den Ansatz über weitere 4 h. Es werden erneut 2.0 mL einer 1 M Boran THF-Komplexlösung addiert. Nach 1 h unter Rückfluss lässt man abkühlen, entfernt das Lösungsmittel und versetzt den Rückstand mit wasserfreiem Methanol (0.97 mL) und 3.75 N Salzsäure-Lösung in Diethylether (1.61 mL). Das Gemisch wird nach Rühren bei RT über Nacht eingeengt, und das Rohprodukt durch FC (Fliessmittelgradient von P nach N) gereinigt. Man erhält den 2-(4-Methoxybutoxy)-4-(piperidin-1-ylethoxy)-benzoesäuremethylester, R_{f} (L) = 0.38, als braunes Öl (1.39 g).
   b) 2-(4-Methoxybutoxy)-4-(piperidin-1-ylcarbamoylmethoxy)-benzoesäuremethylester: Zu einer Suspension von 4-Carboxymethoxy-2-(4-methoxybutoxy)-benzoesäuremethylester (1.64 g) in wasserfreiem Dichlormethan (20 mL) werden Cyanphosphonsäurediethylester (0.88 mL), Piperidin (0.57 mL) und Triethylamin (0.73 mL) bei 0° C addiert. Das Reaktionsgemisch wird 4 h bei 0° C und 1 h bei RT gerührt. Es werden nochmals Cyanphosphonsäurediethylester (0.40 mL) und Piperidin (0.25 mL) addiert, weitere 45 min bei RT gerührt und anschließend mit Dichlormethan (50 mL) verdünnt. Die organische Phase wird mit einer 1 M Kaliumhydrogensulfat-Lösung, gesättigter Natriumhydrogencarbonat-Lösung und gesättigter Natriumchlorid-Lösung gewaschen, über Magnesiumsulfat getrocknet und eingeengt. FC Reinigung (Fliessmittelgradient von E nach Hexan-Essigsäureethylester 1:3) ergibt die Titelverbindung (1.95 g) als blaßgelbe Festsubstanz: R_{f} (E) = 0.13; MS(EI) *m/e* 365 (M⁺).
   c) 4-Carboxymethoxy-2-(4-methoxybutoxy)-benzoesäuremethylester: 4-*tert*-Butoxycarbonylmethoxy-2-(4-methoxybutoxy)-benzoesäuremethylester (3.0 g), das analog wie in Beispiel 30 Ba) beschrieben aus 4-Hydroxy-2-(4-methoxybutoxy)-benzoesäuremethylester und *tert*-Butylbromacetat als blaßgelbes Öl (R_{f} (C) = 0.13) erhalten wird, wird in 4 N Salzsäure-Lösung in Dioxan (25 mL) gelöst und die Mischung über Nacht bei RT gerührt. Das Lösungsmittel wird anschlissend im HV entfernt und der feste Rückstand in heissem Essigsäureethylester (10 mL) gelöst. Es wird Hexan (ca. 20 mL) bis zur beginnenden Trübung addiert. Nach dem Abkühlen auf RT wird der weisse Niederschlag abfiltriert, mit Hexan gewaschen und getrocknet. Man erhält die Titelverbindung (1.64 g) als weisse Festubstanz: R_{f} (Hexan-Essigsäureethylester-Eisessig 50:50:1) = 0.12.
G) 2-(4-Methoxybutoxy)-4-[(4-methyl-piperazin-1-yl)methyl]-benzoesäuremethylester: Die Titelverbindung wird analog wie in Beispiel 30 Aa) beschrieben aus 2-(4-Methoxybutoxy)-4-methyl-benzoesäuremethylester und N-Methylpiperidin und anschließende Reinigung des Rohproduktes durch FC (Fliessmittel T) hergestellt: gelbes Öl, R_{f} (V) = 0.37.
H) 4-[(4-Acetyl-piperazin-1-yl)methyl]-2-(4-methoxybutoxy)-benzoesäuremethylester: Die Titelverbindung wird analog wie in Beispiel 30Aa) beschrieben aus 2-(4-Methoxybutoxy)-4-methyl-benzoesäuremethylester und N-Acetylpiperazin und anschließender Reinigung durch FC (Fliessmittel T) hergestellt: gelbes Öl, R_{f} (V) = 0.30 .

### Beispiel 31:

In analoger Weise wie in Beispiel 18) beschrieben, und anschließender FC Reinigung des Rohproduktes jeweils an 25 - 50 g KG (Fliessmittelsystem Dichlormethan-Methanol-Ammoniak konz.) werden durch Laktonöffnung die folgenden Verbindungen hergestellt:
a) Aus 202 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(morpholin-4-ylmethyl)-benzamid das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(morpholin-4-ylmethyl)-benzamid, R_{f} (W) = 0.60, als schaumige Festsubstanz.
b) Aus 185 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-[2-(morpholin-4-yl)-ethoxy]-benzamid das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-[2-(morpholin-4-yl)-ethoxy]-benzamid, R_{f} (W) = 0.54, als Öl.
c) Aus 70 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-[3-(dimethylamino)-propoxy]-2-(4-methoxybutoxy)-benzamid das (2S,4S,5S,7S)-N-[4-(tert-Butoxycarbonyl)amino-7-butylcarbamoyl-5-hydroxy-2-isopropyl-8-methyl-nonyl]-4-[3-(dimethylamino)-propoxy]-2-(4-methoxybutoxy)-benzamid, R_{f} (W) = 0.29, als Öl.
d) Aus 84 mg (2S,2'S,2''S,4''S)-N-{2-[2'-(*tert*-Butoxycarbonyl)amino-2'-(4''-isopropyl-5''-oxo-tetrahydrofuran-2"-yl)-ethyl]-3-methylbutyl}-2-(4-methoxybutoxy)-4-(piperidin-1-yl)methyl-benzamid das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(piperidin-1-yl)methyl-benzamid, R_{f} (W) = 0.66; MS(FAB) *m/e* 733 (M⁺ +1), als Öl.
e) Aus 60 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(pyrrolidin-1-yl)methyl-benzamid das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(pyrrolidin-1-yl)methyl-benzamid, R_{f} (W) = 0.54; MS(FAB) *m/e* 719 (M⁺ +1), als Öl.
f) Aus 68 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(2-piperidin-1-ylethoxy)-benzamid das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(2-pipridin-1-ylethoxy)-benzamid, R_{f} (W) = 0.54, als farblose Festsubstanz.
g) Aus 100 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-dimethylaminomethyl-2-(4-methoxybutoxy)-benzamid das (2S,4S,5S,7S)-N-[4-(tert-Butoxycarbonyl)amino-7-butylcarbamoyl-5-hydroxy-2-isopropyl-8-methyl-nonyl]-4-dimethylaminomethyl-2-(4-methoxybutoxy)-benzamid, R_{f} (W) = 0.48, als gelbliche Festsubstanz.
h) Aus 151 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(4-methylpiperazin-1-yl)methyl-benzamid und FC Reinigung (25 g KG, Fliessmittel V) das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(4-methylpiperazin-1-yl)methyl-benzamid, R_{f} (W) = 0.37; HPLC Rₜ = 13.8 min, als Öl.
i) Aus 130 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-(4-acetylpiperazin-1-yl)methyl-2-(4-methoxybutoxy)-benzamid und FC Reinigung (Fliessmittel T) das (2S,4S,5S,7S)-N-[4-(tert-Butoxycarbonyl)amino-7-butylcarbamoyl-5-hydroxy-2-isopropyl-8-methyl-nonyl]-4-(4-acetylpiperazin-1-yl)methyl-2-(4-methoxybutoxy)-benzamid, R_{f} (W) = 0.46; HPLC Rₜ = 17.9 min, als gelbliche Festsubstanz.

### Beispiel 32:

In analoger Weise wie in Beispiel 21) beschrieben, werden durch De-Bocylierung die folgenden Verbindungen hergestellt:
a) Aus 144 mg (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(morpholin-4-ylmethyl)-benzamid das (2S,4S,5S,7S)-N-(4-Amino-7-butylcarbamoyl-5-hydroxy-2-isopropyl-8-methyl-nonyl)-2-(4-methoxybutoxy)-4-(morpholin-4-ylmethyl)-benzamid-dihydrochlorid: R_{f} (W) = 0.25. HPLC Rₜ = 9.6 min. MS(FAB) *m/e* 635 (M⁺ +1).
b) Aus 155 mg (25,45,55,75)-N-[4-(tert-Butoxycarbonyl)amino-7-butylcarbamoyl-5-hydroxy-2-isopropyl-8-methyl-nonyl]-2-(4-methoxybutoxy)-4-[2-(morpholin-4-yl)-ethoxy]-benzamid das (2S,4S,5S,7S)-N-(4-Amino-7-butylcarbamoyl-5-hydroxy-2-isopropyl-8-methyl-nonyl)-2-(4-methoxybutoxy)-4-[2-(morpholin-4-yl)-ethoxy]-benzamid-dihydrochlorid, R, (W) = 0.18. HPLC Rₜ = 10.0 min. MS(FAB) *m/e* 665 (M⁺ +1).
c) Aus 36 mg (2S,4S,5S,7S)-N-[4-(tert-Butoxycarbonyl)amino-7-butylcarbamoyl-5-hydroxy-2-isopropyl-8-methyl-nonyl]-4-[3-(dimethylamino)-propoxy]-2-(4-methoxybutoxy)-benzamid das (2S,4S,5S,7S)-N-(4-Amino-7-butylcarbamoyl-5-hydroxy-2-isopropyl-8-methyl-nonyl)-4-[3-(dimethylamino)-propoxy]-2-(4-methoxybutoxy)-benzamid-dihydrochlorid: R_{f} (W) = 0.11. HPLC Rₜ = 9.3 min. MS(FAB) *m/e* 637 (M⁺ +1).
d) Aus 50 mg (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(pipeddin-1-yl)methyl-benzamid das (2S,4S,5S,7S)-N-(4-Amino-7-butylcarbamoyl-5-hydroxy-2-isopropyl-8-methyl-nonyl)-2-(4-methoxybutoxy)-4-(piperidin-1-yl)methyl-benzamid-dihydrochlorid: R_{f} (W) = 0.41. HPLC Rₜ = 10.5 min. MS(FAB) *m/e* 633 (M⁺ +1).
e) Aus 48 mg (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(pyrrolidin-1-yl)methyl-benzamid das (2S,4S,5S,7S)-N-(4-Amino-7-butylcarbamoyl-5-hydroxy-2-isopropyl-8-methyl-nonyl)-2-(4-methoxybutoxy)-4-(pyrrolidin-1-yl)methyl-benzamid-dihydrochlorid: R_{f} (W) = 0.32. HPLC Rₜ = 10.2 min. MS(FAB) *m/e* 619 (M⁺ +1).
f) Aus 53 mg (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(2-pipeddin-1-ylethoxy)-benzamid das (2S,4S,5S,7S)-N-(4-Amino-7-butylcarbamoyl-5-hydroxy-2-isopropyl-8-methyl-nonyl)-2-(4-methoxybutoxy)-4-(2-piperidin-1-ylethoxy)-benzamid-dihydrochlorid: R_{f} (W) = 0.16. HPLC Rₜ = 9.98 min. MS(FAB) *m/e* 663 (M⁺ +1).
g) Aus 79 mg (2S,4S,5S,7S)-N-[4-(tert-Butoxycarbonyl)amino-7-butylcarbamoyl-5-hydroxy-2-isopropyl-8-methyl-nonyl]-4-dimethylaminomethyl-2-(4-methoxybutoxy)-benzamid das (2S,4S,5S,7S)-N-(4-Amino-7-butylcarbamoyl-5-hydroxy-2-isopropyl-8-methyl-nonyl)-4-dimethylaminomethyl-2-(4-methoxybutoxy)-benzamid-dihydrochlorid: R_{f} (W) = 0.21. HPLC Rₜ = 9.57 min. MS(FAB) *m/e* 593 (M⁺ +1).
h) Aus 124 mg (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(4-methylpiperazin-1-yl)methyl-benzamid das (2S,4S,5S,7S)-N-(4-Amino-7-butylcarbamoyl-5-hydroxy-2-isopropyl-8-methyl-nonyl)-2-(4-methoxybutoxy)-4-(4-methylpiperazin-1-yl)methyl-benzamid-trihydrochlorid: R_{f} (W) = 0.21. HPLC Rₜ = 10.2 min. MS(FAB) *m/e* 648 (M⁺ +1).
i) Aus 83 mg (2S,4S,5S,7S)-N-[4-(tert-Butoxycarbonyl)amino-7-butylcarbamoyl-5-hydroxy-2-isopropyl-8-methyl-nonyl]-4-(4-acetylpiperazin-1-yl)methyl-2-(4-methoxybutoxy)-benzamid das (2S,4S,5S,7S)-N-(4-Amino-7-butylcarbamoyl-5-hydroxy-2-isopropyl-8-methyl-nonyl)-4-(4-acetylpiperazin-1-yl)methyl-2-(4-methoxybutoxy)-benzamid-dihydrochlorid: R_{f} (W) = 0.29. HPLC Rₜ = 10.6 min. HRMS(FAB) *m/e* 676.5017 (M⁺ +1).

### Beispiel 33:

In analoger Weise wie in Beispiel 1) beschrieben, werden die nachfolgenden Verbindungen hergestellt:
a) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 124 mg 2-(3-Azidopropoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-azidopropoxy)-benzamid, R_{f} (Hexan-Diethylether 1:4) = 0.46; HPLC Rₜ = 19.2 min.
b) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 116 mg 2-(2-Azidoethoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(2-azidoethoxy)-benzamid, R_{f} (Hexan-Diethylether 1:4) = 0.41; HPLC Rₜ = 18.6 min.
c) Aus 150 mg (3S,5S,1'S,3'S)-5-[3'-Aminomethyl-1'-(*tert*-butoxycarbonyl)amino-4'-methylpentyl]-3-isopropyl-dihydrofuran-2-on und 246 mg 2-[2-(4-Acetylpiperazin-1-yl)-ethoxy]-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(4-acetylpiperazin-1-yl)-ethoxy]-benzamid, R_{f} (J) = 0.45; HPLC Rₜ = 13.6 min.
d) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 132 mg 2-[2-(Morpholin-4-yl)-ethyl]-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(morpholin-4-yl)-ethyl]-benzamid, R_{f} (L) = 0.50; HPLC Rₜ = 15.8 min.
e) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 125 mg 2-(3-Dimethylaminopropoxy)-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-dimethylaminopropoxy)-benzamid, R_{f} (J) = 0.70; HPLC Rₜ = 14.6 min.
f) Aus 50 mg (3S,5S,1'S,3'S)-5-[3'-Aminomethyl-1'-(*tert*-butoxycarbonyl)amino-4'-methylpentyl]-3-isopropyl-dihydrofuran-2-on und 72 mg 2-[3-(Morpholin-4-yl)-propoxy]-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[3-(morpholin-4-yl)-propoxy]-benzamid, R_{f} (H) = 0.25; HPLC Rₜ = 14.4 min.
g) Aus 50 mg (3S,5S,1'S,3'S)-5-[3'-Aminomethyl-1'-(*tert*-butoxycarbonyl)amino-4'-methylpentyl]-3-isopropyl-dihydrofuran-2-on und 70 mg 2-[2-(Morpholin-4-yl)-ethoxy]-benzoesäure das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(morpholin-4-yl)-ethoxy]-benzamid, R_{f} (H) = 0.43; HPLC Rₜ = 14.2 min.

Die als Ausgangsstoffe eingesetzten Benzoesäure-Derivate werden, soweit nicht nachfolgend näher beschrieben, nach üblichen Literaturverfahren hergestellt:
A) 2-(3-Azidopropoxy)-benzoesäure: Zu einer Lösung von 2-(3-Azidopropoxy)-benzoesäuremethylester (0.5 g) in Methanol (7 mL) wird 1N Natronlauge (3 mL) addiert und das Reaktiongemisch bei Rückfluss über 15 min gerührt. Der Ansatz wird eingedampft, Wasser (25 mL) zugeben und bei 0° C mit 1N Salzsäure auf pH 6 gestellt. Die wässrige Lösung wird mit Dichlormethan (2x 100 mL) extrahiert, die organische Phase über Natriumsulfat getrocknet und eingedampft. Man erhält die Titelverbindung, R_{f} (L) = 0.62.
   a) 2-(3-Azidopropoxy)-benzoesäuremethylester: Zu einer Lösung von 2-(3-Brompropoxy)-benzoesäuremethylester (1.5 g; hergestellt nach dem von Smith et al. in J. Chem. Soc. Perkin Trans I (1988) 77 beschriebenen Verfahren) in N,N-Dimethylformamid (10 mL) wird Natriumazid (0.45 g) addiert und die Suspension über 15 h bei 50° C erhitzt. Der Reaktionsansatz wird eingedampft und zwischen Wasser (50 mL) und Dichlormethan (100 mL) verteilt. Die organische Phase wird mit Wasser (2x 50 mL) gewaschen, über Natriumsulfat getrocknet und eingedampft. Der Eindampfrückstand wird durch FC (200 g KG, Fliessmittel A) gereinigt. Man erhält die Titelverbindung, R_{f} (B) = 0.25.
B) 2-(2-Azidoethoxy)-benzoesäure: In analoger Weise wie in Beispiel 33A) beschrieben aus 1.3 g 2-(2-Azidoethoxy)-benzoesäuremethylester als Öl, R_{f} (L) = 0.59.
   a) 2-(2-Azidoethoxy)-benzoesäuremethylester: Zu einer Lösung von 2-(3-Bromethoxy)-benzoesäuremethylester (2.0 g; hergestellt nach dem von W. A. Jacobs und M. Heidelberger in J. Biol. Chem. (1915) 21, 448 beschriebenen Verfahren) in 1,3-Dimethyl-3,4,5,6-tetrahydro-2(1*H*)-pyrimidinon (20 mL) wird Natriumazid (0.6 g) addiert und die Suspension über 5 h bei 50° C erhitzt. Der Reaktionsansatz wird eingedampft und zwischen Wasser (100 mL) und Diethylether (200 mL) verteilt. Die organische Phase wird mit Wasser (50 mL) gewaschen, über Natriumsulfat getrocknet und eingedampft. Der Eindampfrückstand wird durch FC (360 g KG, Fliessmittel E) gereinigt. Man erhält die Titelverbindung, R_{f} (E) = 0.43.
C) 2-[2-(4-Acetylpiperazin-1-yl)-ethoxy]-benzoesäure: In analoger Weise aus 2-[2-(4-Acetylpiperazin-1-yl)-ethoxy]-benzoesäuremethylester (2.5 g) als Öl: R_{f} (Dichlormethan-Methanol 4:1) = 0.20.
   a) 2-[2-(4-Acetylpiperazin-1-yl)-ethoxy]-benzoesäuremethylester: Zu einer Lösung von 2-(2-Brompropoxy)-benzoesäuremethylester (2.0 g) in Acetonitril (50 mL) wird N-Acetylpiperazin (3.0 g) addiert und das Reaktionsgemisch bei 50° C erhitzt. Nach beendeter Reaktion wird der Ansatz eingedampft und das Rohprodukt durch FC (150 g KG, Dichlormethan-Methanol 4:1) gereinigt. Man erhält die Titelverbindung, R_{f} (Dichlormethan-Methanol 4:1) = 0.76.
D) 2-[2-(Morpholin-4-yl)-ethyl]-benzoesäure: In analoger Weise aus 2-[2-(Morpholin-4-yl)-ethyl]-benzoesäureethylester (1.0 g) als Öl: R_{f} (Essigsäureethylester-Methanol 9:1) = 0.05.
   a) 2-[2-(Morpholin-4-yl)-ethyl]-benzoesäureethylester: Die Titelverbindung erhält man aus 2-(2-Bromethyl)-benzoesäureethylester (1.0 g) analog wie in Beispiel 33Ca) beschrieben: R_{f} (Essigsäureethylester-Methanol 9:1) = 0.55.
   b) 2-(2-Bromethyl)-benzoesäureethylester: Zu einer Lösung von 1-Oxo-isochroman (8.0 g) in Tetrachlorkohlenstoff (80 mL) wird Phosphortribromid (5.58 mL) und Brom (3.33 mL) bei - 15° C addiert. Der Ansatz wird bei RT über Nacht und anschließend bei 60° C über 3 h gerührt. Die Mischung wird mit Ethanol (16 mL) bei RT versetzt und 1 h verrührt. Der Reaktionsansatz wird schliesslich zwischen Dichlormethan (500 mL) und Wasser (50 mL) verteilt und die organische Phase mit Wasser (50 mL) gewaschen, über Natriumsulfat getrocknet und eingedampft. Der Rückstand wird durch FC (Fliessmittel E) gereinigt. Man erhält die Titelverbindung, R_{f} (E) = 0.70.
E) In analoger Weise wie in den Beispielen 33A-D) beschrieben erhält man die nachfolgenden Verbindungen:
   a) Aus 2-[3-(Morpholin-4-yl)-propoxy]-benzoesäuremethylester die 2-[3-(Morpholin-4-yl)-propoxy]-benzoesäure, R_{f} (Dichlormethan-Methanol 7:3) = 0.46, als Öl.
   b) Aus 2-[2-(Morpholin-4-yl)-ethoxy]-benzoesäuremethylester die 2-[2-(Morpholin-4-yl)-ethoxy]-benzoesäure, R_{f} (Dichlormethan-Methanol 7:3) = 0.47, als Öl.
   c) Aus 2-[2-(4-Methoxypiperidin-1-yl)-ethyl]-benzoesäureethylester (R_{f} (Essigsäureethylester-Methanol 9:1) = 0.22) die 2-[2-(4-Methoxypiperidin-1-yl)-ethyl]-benzoesäure, R_{f} (Dichlormethan-Methanol 7:3) = 0.60, als Öl.
   d) Aus 2-[2-(4-Acetylpiperazin-1-yl)-ethyl]-benzoesäureethylester (R_{f} (Essigsäureethylester-Methanol 9:1) = 0.22) die 2-[2-(4-Acetylpiperazin-1-yl)-ethyl]-benzoesäure, R_{f} (Dichlormethan-Methanol 7:3) = 0.60, als Öl.

### Beispiel 34:

Die Mischung von (3S,5S,1'S,3'S)-5-[3'-Aminomethyl-1'-(*tert*-butoxycarbonyl)-amino-4'-methylpentyl]-3-isopropyl-dihydrofuran-2-on (100 mg), 2-[2-(4-Methoxypiperidin-1-yl)-ethyl]-benzoesäure (89 mg), o-Benzotriazol-1-yl-N,N,N',N'-tetramethyl-uroniumhexafluorophosphat (128 mg) und Triethylamin (59 mL ) in Acetonitril (5 mL) wird bei RT über 24 h gerührt. Der Ansatz wird eingedampft und der Rückstand zwischen Dichlormethan und Wasser verteilt. Die organische Phase wird über Natriumsulfat getrocknet und eingedampft. FC an KG (50 g, Fliessmittel I) ergibt das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(4-methoxypiperidin-1-yl)-ethyl]-benzamid, R_{f} (L) = 0.44; HPLC Rₜ = 16.1 min.

### Beispiel 35:

In analoger Weise wie in Beispiel 34) beschrieben erhält man aus (3S,5S,1'S,3'S)-5-[3'-Aminomethyl-1'-(*tert*-butoxycarbonyl)amino-4'-methylpentyl]-3-isopropyl-dihydrofuran-2-on (100 mg), 2-[2-(4-Acetylpiperazin-1-yl)-ethyl]-benzoesäure (93 mg), o-Benzotriazol-1-yl-N,N,N',N'-tetramethyl-uronium-hexafluorophosphat (128 mg) und Triethylamin (59 mL) in Acetonitril (5 mL) und Reinigung des Rohproduktes durch FC (50 g KG, Essigsäureethylester-Methanol 9:1) das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(4-acetylpiperazin-1-yl)-ethyl]-benzamid, R_{f} (Essigsäureethylester-Methanol 9:1) = 0.26; HPLC R_{f} = 14.4 min.

### Beispiel 36:

In analoger Weise wie in Beispiel 31) beschrieben, werden die nachfolgenden Verbindungen durch Laktonöffnung mit n-Butylamin hergestellt:
a) Aus 110 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-azidopropoxy)-benzamid das (2S,4S,5S,7S)-N-[4-(tert-Butoxycarbonyl)amino-7-butylcarbamoyl-5-hydroxy-2-isopropyl-8-methyl-nonyl]-2-(3-azidopropoxy)-benzamid, R_{f} (E) = 0.14; HPLC Rₜ = 18.8 min.
b) Aus 100 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(2-azidoethoxy)-benzamid das (2S,4S,5S,7S)-N-[4-(tert-Butoxycarbonyl)amino-7-butylcarbamoyl-5-hydroxy-2-isopropyl-8-methyl-nonyl]-2-(2-azidoethoxy)-benzamid, R_{f} (Essigsäureethylester) = 0.11; HPLC Rₜ = 18.0 min.
c) Aus 200 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(4-acetylpiperazin-1-yl)-ethoxy]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(4-acetylpiperazin-1-yl)-ethoxy]-benzamid, R_{f} (Dichlormethan-Methanol 4:1) = 0.70; HPLC Rₜ = 13.4 min.
d) Aus 54 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(morpholin-4-yl)-ethyl]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(morpholin-4-yl)-ethyl]-benzamid, R_{f} (L) = 0.38; HPLC Rₜ = 15.7 min.
e) Aus 60 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(3-dimethylaminopropoxy)-benzamid das (2S,4S,5S,7S)-N-[4-(tert-Butoxycarbonyl)amino-7-butylcarbamoyl-5-hydroxy-2-isopropyl-8-methyl-nonyl]-2-(3-dimethylaminopropoxy)-benzamid, R_{f} (J) = 0.65; HPLC Rₜ = 14.3 min.
f) Aus 49 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[3-(morpholin-4-yl)-propoxy]-benzamid das (2S,4S,5S,7S)-N-[4-(tert-Butoxycarbonyl)amino-7-butylcarbamoyl-5-hydroxy-2-isopropyl-8-methyl-nonyl]-2-[3-(morpholin-4-yl)-propoxy]-benzamid, R_{f} (W) = 0.70; HPLC Rₜ = 14.2 min.
g) Aus 65 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(morpholin-4-yl)-ethoxy]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(morpholin-4-yl)-ethoxy]-benzamid, R_{f} (H) = 0.16; HPLC Rₜ = 14.0 min.
h) Aus 135 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(4-methoxypipeddin-1-yl)-ethyl]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(4-methoxypiperidin-1-yl)-ethyl]-benzamid, R_{f} (Essigsäureethylester-Methanol-Ammoniak konz. 90:15:5) = 0.72; HPLC R_{f} = 15.3 min.
i) Aus 165 mg (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-[2-(4-acetylpiperazin-1-yl)-ethyl]-benzamid das (2S,4S,5S,7S)-N-[4-(tert-Butoxycarbonyl)amino-7-butylcarbamoyl-5-hydroxy-2-isopropyl-8-methyl-nonyl]-2-[2-(4-acetylpiperazin-1-yl)-ethyl]-benzamid, R_{f} (L) = 0.45; HPLC Rₜ = 13.9 min.

### Beispiel 37:

In analoger Weise wie in Beispiel 1a) beschrieben, werden die nachfolgenden Verbindungen hergestellt:
a) Aus 75 mg (2S,4S,5S,7S)-N-[4-(*tert*-Butoxycarbonyl)amino-7-butylcarbamoyl-5-hydroxy-2-isopropyl-8-methyl-nonyl]-2-(3-azidopropoxy)-benzamid das (2S,4S,5S,7S)-N-[4-(*tert*-Butoxycarbonyl)amino-7-butylcarbamoyl-5-hydroxy-2-isopropyl-8-methyl-nonyl]-2-(3-aminopropoxy)-benzamid, R_{f} (J) = 0.18; HPLC Rₜ = 13.8 min.
b) Aus 47 mg (2S,4S,5S,7S)-N-[4-(*tert*-Butoxycarbonyl)amino-7-butylcarbamoyl-5-hydroxy-2-isopropyl-8-methyl-nonyl]-2-(2-azidoethoxy)-benzamid das (2S,4S,5S,7S)-N-[4-(*tert*-Butoxycarbonyl)amino-7-butylcarbamoyl-5-hydroxy-2-isopropyl-8-methyl-nonyl]-2-(2-amino-ethoxy)-benzamid, R_{f} (J) = 0.25; HPLC Rₜ = 13.4 min.

### Beispiel 38:

In analoger Weise wie in Beispiel 21) beschrieben, werden die nachfolgenden Verbindungen durch Debocylierung hergestellt:
a) Aus 58 mg (2S,4S,5S,7S)-N-[4-(*tert*-Butoxycarbonyl)amino-7-butylcarbamoyl-5-hydroxy-2-isopropyl-8-methyl-nonyl]-2-(3-aminopropoxy)-benzamid das (2S,4S,5S,7S)-N-(4-Amino-7-butylcarbamoyl-5-hydroxy-2-isopropyl-8-methyl-nonyl)-2-(3-aminopropoxy)-benzamid-dihydrochlorid: R_{f} (Essigsäureethylester-Methanol-Ammoniak konz. 50:45:5) = 0.15. HPLC Rₜ = 8.8 min. MS(FAB) *m/e* 507 (M⁺ +1).
b) Aus 22 mg (2S,4S,5S,7S)-N-[4-(*tert*-Butoxycarbonyl)amino-7-butylcarbamoyl-5-hydroxy-2-isopropyl-8-methyl-nonyl]-2-(2-aminoethoxy)-benzamid das (2S,4S,5S,7S)-N-(4-Amino-7-butylcarbamoyl-5-hydroxy-2-isopropyl-8-methyl-nonyl)-2-(2-aminoethoxy)-benzamid-dihydrochlorid: R_{f} (Essigsäureethylester-Methanol-Ammoniak konz. 50:45:5) = 0.14. HPLC Rₜ = 8.4 min. MS(FAB) *m/e* 493 (M⁺ +1).
c) Aus 185 mg (2S,4S,5S,7S)-N-[4-(*tert*-Butoxycarbonyl)amino-7-butylcarbamoyl-5-hydroxy-2-isopropyl-8-methyl-nonyl]-2-[2-(4-acetylpiperazin-1-yl)-ethoxy]-benzamid das (2S,4S,5S,7S)-N-(4-Amino-7-butylcarbamoyl-5-hydroxy-2-isopropyl-8-methyl-nonyl)-2-[2-(4-acetylpiperazin-1-yl)-ethoxy]-benzamid-dihydrochlorid: R_{f} (J) = 0.35. HPLC Rₜ = 10.0 min. MS(FAB) *m/e* 604 (M⁺ +1).
d) Aus 48 mg (2S,4S,5S,7S)-N-[4-(*tert*-Butoxycarbonyl)amino-7-butylcarbamoyl-5-hydroxy-2-isopropyl-8-methyl-nonyl]-2-[2-(morpholin-4-yl)-ethyl]-benzamid das (2S,4S,5S,7S)-N-(4-Amino-7-butylcarbamoyl-5-hydroxy-2-isopropyl-8-methyl-nonyl)-2-[2-(morpholin-4-yl)-ethyl]-benzamid-dihydrochlorid: R_{f} (J) = 0.54. HPLC Rₜ = 10.7 min. MS(FAB) *m/e* 547 (M⁺ +1).
e) Aus 32 mg (2S,4S,5S,7S)-N-[4-(*tert*-Butoxycarbonyl)amino-7-butylcarbamoyl-5-hydroxy-2-isopropyl-8-methyl-nonyl]-2-(3-dimethylaminopropoxy)-benzamid das (2S,4S,5S,7S)-N-(4-Amino-7-butylcarbamoyl-5-hydroxy-2-isopropyl-8-methyl-nonyl)-2-(3-dimethyl aminopropoxy)-benzamid-dihydrochlorid: R_{f} (J) = 0.31. HPLC Rₜ = 9.2 min. MS(FAB) *m/e* 535 (M⁺ +1).
f) Aus 40 mg (2S,4S,5S,7S)-N-[4-(*tert*-Butoxycarbonyl)amino-7-butylcarbamoyl-5-hydroxy-2-isopropyl-8-methyl-nonyl]-2-[3-(morpholin-4-yl)-propoxy]-benzamid das (2S,4S,5S,7S)-N-(4-Amino-7-butylcarbamoyl-5-hydroxy-2-isopropyl-8-methyl-nonyl)-2-[3-(morpholin-4-yl)-propoxy]-benzamid-dihydrochlorid: R_{f} (W) = 0.20. HPLC Rₜ = 9.4 min. MS(FAB) *m/e* 577 (M⁺ +1).
g) Aus 53 mg (2S,4S,5S,7S)-N-[4-(*tert*-Butoxycarbonyl)amino-7-butylcarbamoyl-5-hydroxy-2-isopropyl-8-methyl-nonyl]-2-[2-(morpholin-4-yl)-ethoxy]-benzamid das (2S,4S,5S,7S)-N-(4-Amino-7-butylcarbamoyl-5-hydroxy-2-isopropyl-8-methyl-nonyl)-2-[2-(morpholin-4-yl)-ethoxy]-benzamid-dihydrochlorid: R_{f} (J) = 0.54. HPLC Rₜ = 9.3 min. MS(FAB) *m/e* 563 (M⁺ +1).
h) Aus 32 mg (2S,4S,5S,7S)-N-[4-(*tert*-Butoxycarbonyl)amino-7-butylcarbamoyl-5-hydroxy-2-isopropyl-8-methyl-nonyl]-2-[2-(4-methoxypiperidin-1-yl)-ethyl]-benzamid das (2S,4S,5S,7S)-N-(4-Amino-7-butylcarbamoyl-5-hydroxy-2-isopropyl-8-methyl-nonyl)-2-[2-(4-methoxypiperidin-1-yl)-ethyl]-benzamid-dihydrochlorid: R_{f} (Essigsäureethylester-Methanol-Ammoniak konz. 90:15:5) = 0.29. HPLC Rₜ = 10.4 min. MS(FAB) *m/e* 575 (M⁺ +1).
i) Aus 137 mg (2S,4S,5S,7S)-N-[4-(*tert*-Butoxycarbonyl)amino-7-butylcarbamoyl-5-hydroxy-2-isopropyl-8-methyl-nonyl]-2-[2-(4-acetylpiperazin-1-yl)-ethyl]-benzamid das (2S,4S,5S,7S)-N-(4-Amino-7-butylcarbamoyl-5-hydroxy-2-isopropyl-8-methyl-nonyl)-2-[2-(4-acetylpiperazin-1-yl)-ethyl]-benzamid-dihydrochlorid: R_{f} (Essigsäureethylester-Methanol-Ammoniak konz. 90:15:5) = 0.19. HPLC Rₜ = 9.5 min. MS(FAB) *m/e* 588 (M⁺ +1).

### Beispiel 39:

Zu einer Lösung von (2S,2'S,2''S,4''S)-2-[2'-Azido-2'-(4''-isopropyl-5''-oxo-tetrahydrofuran-2''-yl)-ethyl]-3-methylbuttersäure (208 mg) (**Case 4-19919**/**P1**) in Dichlormethan (17 mL) werden unter Rühren bei 0 ° C nacheinander Triethylamin (117µL) und Cyanphosphonsäurediethylester (137 µL) zugetropft. Das Reaktionsgemisch wird 10 min bei 0° C weitergerührt und dann tropfenweise mit der Lösung von 2-(3-Methoxypropoxy)-benzylamin (164 mg) in Dichlormethan (2 mL) versetzt. Der Ansatz wird 16 h bei RT nachgerührt, anschließend mit Dichlormethan (100 mL) verdünnt und die organische Phase mit 10%iger Citronensäure-Lösung (50 mL), gesättigter Natriumhydrogencarbonat-Lösung (50 mL) und gesättigter Natriumchlorid-Lösung (50 mL) gewaschen. Die wässrigen Phasen werden jeweils mit Dichlormethan (2x 50 mL) zurückextrahiert. Aus den vereinten organischen Phasen erhält man nach Trocknen über Magnesiumsulfat, Eindampfen und Reinigen des Rückstandes mittels FC (18 g Kieselgel, Fliessmittel E) das (2S,2'S,2''S,4''S)-2-[2'-Azido-2'-(4''-isopropyl-5''-oxo-tetrahydrofuran-2''-yl)-ethyl]-N-[2-(3-methoxypropoxy)-benzyl]-3-methylbuttersäureamid, R_{f} (E) = 0.32; HPLC Rₜ = 18.0 min; MS(FAB) *m/e* 475 (M⁺ +1), als hellgelbes Öl.

### Beispiel 40:

In analoger Weise wie in Beispiel 39) beschrieben, werden die nachfolgenden Verbindungen hergestellt:
a) Aus 208 mg (2S,2'S,2''S,4''S)-2-[2'-Azido-2'-(4''-isopropyl-5''-oxo-tetrahydrofuran-2''-yl)-ethyl]-3-methylbuttersäure und 164 mg 3-(3-Methoxypropoxy)-benzylamin das (2S,2'S,2''S,4''S)-2-[2'-Azido-2'-(4''-isopropyl-5''-oxo-tetrahydrofuran-2''-yl)-ethyl]-N-[3-(3-methoxypropoxy)-benzyl]-3-methylbuttersäureamid, R_{f} (Hexan-Essigsäureethylester-Eisessig 66:33:1) = 0.17; MS(FAB) *m/e* 475 (M⁺ +1), als gelbes Öl.
b) Aus 210 mg (2S,2'S,2''S,4''S)-2-[2'-Azido-2'-(4''-isopropyl-5''-oxo-tetrahydrofuran-2''-yl)-ethyl]-3-methylbuttersäure und 177 mg 2-(4-Methoxybutoxy)-benzylamin das (2S,2'S,2''S,4''S)-2-[2'-Azido-2'-(4''-isopropyl-5''-oxo-tetrahydrofuran-2''-yl)-ethyl]-N-[2-(4-methoxybutoxy)-benzyl]-3-methylbuttersäureamid, R_{f} (Hexan-Essigsäureethylester-Eisessig 66:33:1) = 0.2; HPLC Rₜ = 18.3 min; MS(FAB) *m/e* 489 (M⁺ +1), als hellgelbes Öl.
c) Aus 194 mg (2S,2'S,2''S,4''S)-2-[2'-Azido-2'-(4''-isopropyl-5''-oxo-tetrahydrofuran-2''-yl)-ethyl]-3-methylbuttersäure und 175 mg 2-(5-Methoxypentoxy)-benzylamin das (2S,2'S,2''S,4''S)-2-[2'-Azido-2'-(4''-isopropyl-5''-oxo-tetrahydrofuran-2''-yl)-ethyl]-N-[2-(5-methoxypentoxy)-benzyl]-3-methylbuttersäureamid, R_{f} (Hexan-Essigsäureethylester-Eisessig 66:33:1) = 0.38; HPLC Rₜ = 19.1 min; MS(FAB) *m/e* 503 (M⁺ +1), als farbloses Öl.

### Beispiel 41:

Eine Lösung von (2S,2'S,2''S,4''S)-2-[2'-Azido-2'-(4''-isopropyl-5''-oxo-tetrahydrofuran-2''-yl)-ethyl]-N-[2-(3-methoxypropoxy)-benzyl]-3-methylbuttersäureamid (100 mg) in n-Butylamin (0.5 mL) wird über 16 h bei 50-55° C gerührt und dann zur Trockene eingedampft. Die Reinigung des Rückstandes mittels FC (5.5 g KG, Fliessmittel F) ergibt das (2S,4S,5S,7S)-N-[4-Azido-5-hydroxy-2,7-diisopropyl-octandisäure-8-butylamid-1-[2-(3-methoxypropoxy)-benzyl]amid, R_{f} (F) = 0.14; MS(FAB) *m/e* 548 (M⁺ +1), als farbloses Öl.

### Beispiel 42:

In analoger Weise wie in Beispiel 41) beschrieben, werden die nachfolgenden Verbindungen hergestellt:
a) Aus 150 mg (2S,2'S,2''S,4S)-2-[2'-Azido-2'-(4''-isopropyl-5''-oxo-tetrahydrofuran-2''-yl)-ethyl]-N-[3-(3-methoxypropoxy)-benzyl]-3-methylbuttersäureamid das (2S,4S,5S,7S)-N-[4-Azido-5-hydroxy-2,7-diisopropyl-octandisäure-8-butylamid-1-[3-(3-methoxypropoxy)-benzyl]amid, R_{f} (F) = 0.28; MS(FAB) *m/e* 548 (M⁺ +1), als gelbes Öl.
b) Aus 282 mg (2S,2'S,2''S,4S)-2-[2'-Azido-2'-(4''-isopropyl-5''-oxo-tetrahydrofuran-2''-yl)-ethyl]-N-[2-(4-methoxybutoxy)-benzyl]-3-methylbuttersäureamid das (2S,4S,5S,7S)-N-[4-Azido-5-hydroxy-2,7-diisopropyl-octandisäure-8-butylamid-1-[2-(4-methoxybutoxy)-benzyl]amid, R_{f} (F) = 0.21; HPLC Rₜ = 17.6 min; MS(FAB) *m/e* 562 (M⁺ +1), als hellgelber Schaum.
c) Aus 274 mg (2S,2'S,2''S,4S)-2-[2'-Azido-2'-(4''-isopropyl-5''-oxo-tetrahydrofuran-2''-yl)-ethyl]-N-[2-(5-methoxypentoxy)-benzyl]-3-methylbuttersäureamid das (2S,4S,5S,7S)-N-[4-Azido-5-hydroxy-2,7-diisopropyl-octandisäure-8-butylamid-1-[2-(5-methoxypentoxy)-benzyl]amid, R_{f} (F) = 0.5; HPLC Rₜ = 18.3 min; MS(FAB) *m/e* 576 (M⁺ +1), als hellgelbes Öl.

### Beispiel 43:

Die Lösung von (2S,4S,5S,7S)-N-[4-Azido-5-hydroxy-2,7-diisopropyl-octandisäure-8-butylamid-1-[2-(3-methoxypropoxy)-benzyl]amid (38 mg) in Methanol (8 mL) wird in Gegenwart von Pd/C 10% (20 mg) während 4 h bei RT und Normaldruck hydriert, wobei der pH-Wert zu Zugabe von 0.1 N methanolischer Salzsäure-Lösung konstant bei pH 6 gehalten wird. Anschließend wird das Reaktionsgemisch über Kieselgur filtriert und eingedampft. Der Rückstand wird zweimal in wenig Toluol gelöst und wieder eingedampft, dann mit 1 Tropfen 4 N Salzsäure-Lösung in Dioxan versetzt und erneut am HV eingedampft. Aus dem Rohprodukt erhält man nach Reinigen mittels FC (1.4 g KG, Fliessmittel L) das (2S,4S,5S,7S)-4-Amino-5-hydroxy-2,7-diisopropyl-octandisäure-8-butylamid-1-[2-(3-methoxypropoxy)-benzyl]amid-hydrochlorid als gelbliches amorphes Pulver: R_{f} (Dichlormethan-Methanol 8:2) = 0.37; HPLC Rₜ = 12.8 min; MS(FAB) *m/e* 522 (M⁺ +1).

### Beispiel 44:

In analoger Weise wie in Beispiel 43) beschrieben, werden die nachfolgenden Verbindungen hergestellt:
a) Aus 135 mg (25,45,5S,7S)-N-[4-Azido-5-hydroxy-2,7-diisopropyl-octandisäure-8-butylamid-1-[3-(3-methoxypropoxy)-benzyl]amid das (2S,4S,5S,7S)-4-Amino-5-hydroxy-2,7-diisopropyl-octandisäure-8-butylamid-1-[3-(3-methoxypropoxy)-benzyl]amid-hydrochlorid als gelblicher amorpher Feststoff: R_{f} (Dichlormethan-Methanol 8:2) = 0.62; HPLC Rₜ = 12.4 min; MS(FAB) *m/e* 522 (M⁺ +1).
b) Aus 234 mg (2S,4S,5S,7S)-N-[4-Azido-5-hydroxy-2,7-diisopropyl-octandisäure-8-butylamid-1-[2-(4-methoxybutoxy)-benzyl]amid das (2S,4S,5S,7S)-4-Amino-5-hydroxy-2,7-diisopropyl-octandisäure-8-butylamid-1-[2-(4-methoxybutoxy)-benzyl]amid-hydrochlorid als farbloses amorphes Pulver: R_{f} (L) = 0.27; HPLC Rₜ = 13.2 min; MS(FAB) *m/e* 536 (M⁺ +1).
c) Aus 228 mg (25,45,55,75)-N-[4-Azido-5-hydroxy-2,7-diisopropyl-octandisäure-8-butylamid-1 -[2-(5-methoxypentoxy)-benzyl]amid das (2S,4S,SS,7S)-4-Amino-5-hydroxy-2,7-diisopropyl-octandisäure-8-butylamid-1-[2-(5-methoxypentoxy)-benzyljamid-hydrochlorid als gelbliches amorphes Pulver: R_{f} (L) = 0.33; HPLC Rₜ = 13.2 min; MS(FAB) *m/e* 550 (M⁺ +1).

### Beispiel 45:

Durch Umsetzung von (3S,5S,1'S,3'S)-5-[3'-Aminomethyl-1'-(*tert*-butoxycarbonyl)amino-4'-methylpentyl]-3-isopropyl-dihydrofuran-2-on (100 mg) mit 3-(4-Methoxybutoxy)-terephthalsäure-N-(methyl)amid (119 mg), analog wie in Beispiel 1) beschrieben, erhält man das (2S,2'S,2''S,4''S)-N1-{2-[2'-(*tert*-Butoxycarbonyl)amino-2'-(4''-isopropyl-5''-oxo-tetrahydrofuran-2''-yl)-ethyl]-3-methylbutyl}-N4-methyl-2-(4-methoxybutoxy)-terephthalamid, R_{f} (L) = 0.59; MS(FAB) *m/e* 620 (M⁺ +1), als amorphes weisses Pulver.

### Beispiel 46:

Analog wie in Beispiel 45) beschrieben, werden die nachfolgenden Verbindungen hergestellt:
a) Aus 400 mg (3S,5S,1'S,3'S)-5-[3'-Aminomethyl-1'-(*tert*-butoxycarbonyl)amino-4'-methylpentyl]-3-isopropyl-dihydrofuran-2-on und 546 mg 3-(4-Methoxybutoxy)-terephthalsäure-(*tert*-butyl)ester das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-3-(4-methoxybutoxy)-terephthalamidsäure-(*tert*-butyl)ester, R_{f} (E) = 0.48 ; MS(FAB) *m/e* 664 (M⁺ +1), als leicht gelbliches Öl.
b) Aus 100 mg (3S,5S,1'S,3'S)-5-[3'-Aminomethyl-1'-(*tert*-butoxycarbonyl)amino-4'-methylpentyl]-3-isopropyl-dihydrofuran-2-on und 160 mg 3-(4-Methoxybutoxy)-terephthalsäure-N-[2-morpholin-4-yl)-ethyl]amid das (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-N4-[2-morpholin-4-yl)-ethyl]-2-(4-methoxybutoxy)-terephthaldiamid, R_{f} (L) = 0.63; MS(FAB) *m/e* 719 (M⁺ +1), als gelbliches Öl.
c) Aus 120 mg (3S,5S,1'S,3'S)-5-[3'-Aminomethyl-1'-(*tert*-butoxycarbonyl)amino-4'-methylpentyl]-3-isopropyl-dihydrofuran-2-on und 135 mg 3-(4-Methoxybutoxy)-terephthalsäuremonoamid das (2S,2'S,2''S,4''S)-N1-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-terephthalsäurediamid, R_{f} (L ) = 0.58; MS(FAB) *m/e* 606 (M⁺ +1), als gelbes amorphes Pulver.

Die als Ausgangsstoffe eingesetzten Terephthalsäure-Derivate werden, soweit nicht nachfolgend näher beschrieben, nach üblichen Literaturverfahren hergestellt:
A) 2-(4-Methoxybutoxy)-N-methyl-terephthalamidsäure: Durch alkalische Hydrolyse von 300 mg 2-(4-Methoxybutoxy)-N-methyl-terephthalamidsäure-methylester in der in Beispiel 30) beschriebenen Weise erhält man die Titelverbindung, R_{f} (L) = 0.15, als weisses Pulver.
   a) 2-(4-Methoxybutoxy)-N-methyl-terephthalamidsäure-methylester: 2-(4-Methoxybutoxy)-terephthalsäure-1-methylester (564 mg) und Thionylchlorid (3 ml) werden während 1 h unter Rückfluss gerührt. Nach dem Eindampfen wird das erhaltene Säurechlorid in Tetrahydrofuran (5 ml) gelöst und zu einer 40 %igen wässrigen Methylamin-Lösung (5 ml) bei -10° C zudosiert. Nach beendeter Zugabe wird das Lösungsmittel eingedampft und der Rückstand zwischen Essigsäureethylester und einer 2 N wässrigen Salzsäure-Lösung verteilt. Die organische Phase wird abgetrennt, mit Wasser gewaschen, über Magnesiumsulfat getrocknet und eingedampft. Reinigung mittels FC (Fliessmittel Q) ergibt die Titelverbindung, R_{f} (N) = 0.55, als gelbliches amorphes Pulver.
   b) 2-(4-Methoxybutoxy)-terephthalsäure-1-methylester: Durch alkalische Hydrolyse von 2-(4-Methoxybutoxy)-terephthalsäuredimethylester (5 g) in der in Beispiel 16a) beschriebenen Weise erhält man die Titelverbindung, R (L) = 0.32, als weisses Pulver.
   c) 2-(4-Methoxybutoxy)-terephthalsäuredimethylester: Durch Alkylierung von 2-Hydroxy-terephthalsäuredimethylester (10 g) mit 4-Methoxybutylbromid in wasserfreiem Aceton in Gegenwart von getrocknetem Kaliumcarbonat und Kaliumjodid analog der in Beispiel 16a) beschriebenen Weise erhält man die Titelverbindung, R_{f} (B) = 0.20, als leicht gelbliches Öl.
B) 2-(4-Methoxybutoxy)-terephthalsäure-4-*tert*-butylester-1-methylester: Die Lösung von 2-(4-Methoxybutoxy)-terephthalsäure-1-methylester (2.8 g) in N,N-Dimethylformamid (10 mL) wird mit 1,1'-Carbonyl-diimidazol (1.65 g) versetzt und während 1 h bei 40° C gerührt. Nach Zugabe von *tert*-Butanol (1.48 g) und 1,8-Diazabicyclo[5.4.0]undec-7-en (1.52 g) wird während 24 h bei 40° C weitergerührt und das Reaktionsgemisch anschließend eingedampft. Der Rückstand wird zwischen Essigsäureethylester und Wasser verteilt, die organische Phase abgetrennt, über Magnesiumsulfat getrocknet und eingedampft. Reinigung mittels FC (Fliessmittel B) ergibt die Titelverbindung, R_{f} (B) = 0.3, als gelbliches Öl.
C) 2-(4-Methoxybutoxy)-N-(2-morpholin-4-ylethyl)-terephthalamidsäure: Durch alkalische Hydrolyse von 300 mg 2-(4-Methoxybutoxy)-N-(2-morpholin-4-ylethyl)-terephthalamidsäure-methylester in der in Beispiel 16a) beschriebenen Weise erhält man die Titelverbindung, R_{f} (L) = 0.33, als weisses Pulver.
   a) 2-(4-Methoxybutoxy)-N-(2-morpholin-4-ylethyl)-terephthalamidsäure-methylester: Durch Umsetzung von 500 mg 2-(4-Methoxybutoxy)-terephthalsäure-1-methylester mit Thionylchlorid und anschließender Reaktion mit einer Lösung von 4-(2-Aminoethyl)-morpholin und Triethylamin in Dichlormethan, analog wie in Beispiel 46Aa) beschrieben, erhält man die Titelverbindung, R_{f} (L) = 0.6, als weisses amorphes Pulver.
D) 2-(4-Methoxybutoxy)-terephthalamidsäure: Durch alkalische Hydrolyse von 2-(4-Methoxybutoxy)-terephthalamidsäure-methylester (300 mg) in der in Beispiel 1 6a) beschriebenen Weise erhält man die Titelverbindung, R_{f} (Dichlormethan-Methanol-Essigsäure-Wasser 90:10:0.5:1) = 0.33, als weisses Pulver.
   a) 2-(4-Methoxybutoxy)-terephthalamidsäure-methylester: Durch Umsetzung von 2-(4-Methoxybutoxy)-terephthalsäure-1-methylester (500 mg) mit Thionylchlorid und anschließender Reaktion mit 25 %igem wässrigem Ammoniak, analog wie in Beispiel 46Aa) beschrieben, erhält man die Titelverbindung, R_{f} (N) = 0.38, als weisses amorphes Pulver.

### Beispiel 47:

Analog wie in Beispiel 18) beschrieben, werden durch Laktonöffnung die nachfolgenden Verbindungen erhalten:
a) Aus 100 mg (2S,2'S,2''S,4''S)-N1-{2-[2'-(*tert*-Butoxycarbonyl)amino-2'-(4''-isopropyl-5''-oxo-tetrahydrofuran-2''-yl)-ethyl]-3-methylbutyl}-N4-methyl-2-(4-methoxybutoxy)-terephthaldiamid das (2S,4S,5S,7S)-N1-(4-(*tert*-Butoxycarbonyl)amino-7-butylcarbamoyl-5-hydroxy-2-isopropyl-8-methyl-nonyl]-N4-methyl-2-(4-methoxybutoxy)-terephthaldiamid, R_{f} (L) = 0.34; MS(FAB) *m/e* 693 (M⁺ +1), als weisses amorphes Pulver.
b) Aus 200 mg (2S,2'S,2''S,4''S)-N1-{2-[2'-(*tert*-Butoxycarbonyl)amino-2'-(4''-isopropyl-5''-oxo-tetrahydrofuran-2''-yl)-ethyl]-3-methylbutyl}-3-(4-methoxybutoxy)-terephthalamidsäure-(tert-butyl)ester das (2S,4S,5S,7S)-N1-(4-(*tert*-Butoxycarbonyl)amino-7-butylcarbamoyl-5-hydroxy-2-isopropyl-8-methyl-nonyl]-3-(4-methoxybutoxy)-terephthalamidsäure-(tert-butyl)ester, R_{f} (E) = 0.20; MS(FAB) *m/e* 737 (M⁺ +1), als gelbes Öl.
c) Aus 170 mg (2S,2'S,2''S,4''S)-N1-{2-[2'-(*tert*-Butoxycarbonyl)amino-2'-(4''-isopropyl-5''-oxo-tetrahydrofuran-2''-yl)-ethyl]-3-methylbutyl}-N4-[2-morpholin-4-yl)-ethyl]-2-(4-methoxybutoxy)-terephthaldiamid das (2S,4S,5S,7S)-N-(4-(*tert*-Butoxycarbonyl)amino-7-butylcarbamoyl-5-hydroxy-2-isopropyl-8-methyl-nonyl]-N4-[2-morpholin-4-yl)-ethyl]-2-(4-methoxybutoxy)-terephthaldiamid, R_{f} (L) = 0.43; MS(FAB) *m/e* 792 (M⁺ +1), als gelbes amorphes Pulver.
d) Aus 200 mg (2S,2'S,2''S,4''S)-N1-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-terephthalsäurediamid das (2S,4S,5S,7S)-N1-[4-(*tert*-Butoxycarbonyl)amino-7-butylcarbamoyl-5-hydroxy-2-isopropyl-8-methyl-nonyl]-2-(4-methoxybutoxy)-terephthalsäurediamid, R_{f} (L) = 0.38; MS(FAB) *m/e* 679 (M⁺ +1).

### Beispiel 48:

Analog wie in Beispiel 21) beschrieben, werden durch De-Bocylierung die nachfolgenden Verbindungen erhalten:
a) Aus 100 mg (2S,4S,5S,7S)-N1-[4-(*tert*-Butoxycarbonyl)amino-7-butylcarbamoyl-5-hydroxy-2-isopropyl-8-methyl-nonyl]-N4-methyl-2-(4-methoxybutoxy)-terephthaldiamid das (2S,4S,5S,7S)-N1-(4-Amino-7-butylcarbamoyl-5-hydroxy-2-isopropyl-8-methyl-nonyl)-N4-methyl-2-(4-methoxybutoxy)-terephthaldiamid-hydrochlodd: R_{f} (L) = 0.13. HPLC Rₜ = 11.6 min. MS(FAB) *m/e* 593 (M⁺ +1).
b) Aus 170 mg (2S,4S,5S,7S)-N1-(4-(*tert*-Butoxycarbonyl)amino-7-butylcarbamoyl-5-hydroxy-2-isopropyl-8-methyl-nonyl]-N4-[2-morpholin-4-yl)-ethyl]-2-(4-methoxybutoxy)-terephthaldiamid das (2S,4S,5S,7S)-N1-(4-Amino-7-butylcarbamoyl-5-hydroxy-2-isopropyl-8-methyl-nonyl)-N4-[2-morpholin-4-yl)-ethyl]-2-(4-methoxybutoxy)-terephthaldiamid-dihydrochlorid: HPLC Rₜ = 9.73 min. MS(FAB) *m/e* 692 (M⁺ +1).
c) Aus 175 mg (2S,4S,5S,7S)-N1-(4-(*tert*-Butoxycarbonyl)amino-7-butylcarbamoyl-5-hydroxy-2-isopropyl-8-methyl-nonyl]-2-(4-methoxybutoxy)-terephthalsäurediamid das (2S,4S,5S,7S)-N1-(4-Amino-7-butylcarbamoyl-5-hydroxy-2-isopropyl-8-methyl-nonyl)-2-(4-methoxybutoxy)-terephthalsäurediamid-hydrochlorid: R_{f} (L) = 0.1. HPLC Rₜ = 11.0 min. MS(FAB) *m/e* 579 (M⁺ +1).

### Beispiel 49:

Durch Umsetzung von (2S,4S,5S,7S)-N1-[4-(*tert*-Butoxycarbonyl)amino-7-butylcarbamoyl-5-hydroxy-2-isopropyl-8-methyl-nonyl]-3-(4-methoxybutoxy)-terephthalamidsäure-(*tert*-butyl)ester (205 mg) in 3 mL eines 1:1-Gemisches aus Dichlormethan und Trifluoressigsäure bei 0° C erhält man das (2S,4S,5S,7S)-N4-(4-Amino-7-butylcarbamoyl-5-hydroxy-2-isopropyl-8-methyl-nonyl)-3-(4-methoxybutoxy)-terephthalamidsäure-trifluoracetat: HPLC Rₜ = 11.9 min. MS(FAB) *m/e* 580 (M⁺ +1).

### Beispiel 50:

Auf analoge Weise wie in Beispiel 21) beschrieben, wird durch Umsetzung von (2S,4S,5S,7S)-N-[4-(tert-Butoxycarbonyl)amino-7-butylcarbamoyl-5-hydroxy-2-isopropyl-8-methyl-nonyl]-4-butylcarbamoylmethoxy-2-(4-methoxybutoxy)-benzamid (80 mg ) in 4 N Salzsäure-Lösung in Dioxan bei 0° C über 1 h, rasches Einengen des Lösungsmittels am HV und Lyophilisation das (2S,4S,5S,7S)-N-(4-Amino-7-butylcarbamoyl-5-hydroxy-2-isopropyl-8-methyl-nonyl)-4-butylcarbamoylmethoxy-2-(4-methoxybutoxy)-benzamid-hydrochlorid hergestellt: R_{f} (W) = 0.23. HPLC Rₜ = 13.8 min. MS(FAB) *m/e* 665 (M⁺ +1). Das als Ausgangsstoff eingesetzte (2S,4S,5S,7S)-N-[4-(tert-Butoxycarbonyl)amino-7-butylcarbamoyl-5-hydroxy-2-isopropyl-8-methyl-nonyl]-4-butylcarbamoylmethoxy-2-(4-methoxybutoxy)-benzamid wird wie folgt hergestellt:
a) Die Lösung von (2S,2'S,2''S,4''S)-4-{2-[2'-(*tert*-Butoxycarbonyl)amino-2'-(4''-isopropyl-5''-oxo-tetrahydrofuran-2''-yl)-ethyl]-3-methylbutylcarbamoyl}-3-(4-methoxybutoxy)-phenoxy)-essigsäure-(tert-butyl)ester (239 mg) in n-Butylamin (3 mL) wird bei 50° C über 18 h gerührt. Das Reaktionsgemisch wird eingeengt und der Rückstand chromatographiert (FC an 50 g KG, Fliessmittel T). Man erhält das (2S,4S,5S,7S)-N-[4-(tert-Butoxycarbonyl)amino-7-butylcarbamoyl-5-hydroxy-2-isopropyl-8-methyl-nonyl]-4-butylcarbamoylmethoxy-2-(4-methoxybutoxy)-benzamid (83 mg; R_{f} (W) = 0.46 ; HPLC Rₜ = 18.7 min) und 94 mg des (2S,4S,5S,7S)-{4-[4-(*tert*-Butoxycarbonyl)amino-7-butylcarbamoyl-5-hydroxy-2-isopropyl-8-methyl-nonylcarbamoyl]-3-(4-methoxybutoxy)-phenoxy}-essigsäure-(*tert*-butyl)ester (R_{f} (W) = 0.50 ; HPLC Rₜ = 20.1 min), sowie 26 mg einer Mischfraktion aus beiden Produkten.
b) (2S,2'S,2''S,4''S)-4-{2-[2'-(*tert*-Butoxycarbonyl)amino-2'-(4''-isopropyl-5''-oxo-tetrahydrofuran-2''-yl)-ethyl]-3-methylbutylcarbamoyl}-3-(4-methoxybutoxy)-phenoxy)-essigsäure-(*tert*-butyl)ester: Zu einer Lösung von (2S,2'S,2''S,4''S)-N-{2-[2'-( *tert*-Butoxycarbonyl)amino-2'-(4''-isopropyl-5''-oxo-tetrahydrofuran-2''-yl)-ethyl]-3-methylbutyl}-4-hydroxy-2-(4-methoxybutoxy)-benzamid (200 mg) in Aceton (10 mL) werden bei RT *tert*-Butylbromacetat (76 µL) und Cesiumcarbonat (169 mg) addiert. Die weisse Suspension wird unter Rückfluss über 2 h gerührt, nach dem Abkühlen filtriert und das Filtrat eingeengt. Nach Trocknen im HV erhält man die Titelverbindung als gelbes Öl (255 mg). R_{f} (L) = 0.73. HPLC Rₜ = 19.9 min.
c) (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-hydroxy-2-(4-methoxybutoxy)-benzamid: (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-benzyloxy-2-(4-methoxybutoxy)-benzamid (2.46 g), gelöst in Essigsäureethylester (60 mL), werden in Gegenwart von Pd/C 5 % (Degussa) (250 mg) bei RT über 15 h hydriert. Nach Filtration über Celite 545 und Einengen des Filtrats erhält man die Titelverbindung (2.05 g). R_{f} (L) = 0.47. HPLC Rₜ = 16.0 min.
d) (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-benzyloxy-2-(4-methoxybutoxy)-benzamid: Durch Umsetzung von (3S,5S,1'S,3'S)-5-[3'-Aminomethyl-1'-(*tert*-butoxycarbonyl)amino-4'-methylpentyl]-3-isopropyl-dihydrofuran-2-on (3.33 g) mit 4-Benzyloxy-2-(4-methoxybutoxy)-benzoesäure (3.70 g), analog wie in Beispiel 1) beschrieben, und anschließender Reinigung durch FC (Fliessmittel Q) erhält man die Titelverbindung, R_{f} (L) = 0.79, als Öl.

Die als Ausgangsmaterial eingesetzte 4-Benzyl oxy-2-(4-methoxybutoxy)-benzoesäure wird folgendermaßen hergestellt:
a) 4-Benzyloxy-2-(4-methoxybutoxy)-benzoesäure: Die Titelverbindung erhält man durch alkalische Hydrolyse von 4-Benzyloxy-2-(4-methoxybutoxy)-benzoesäuremethylester als blaßgelbes Öl: R_{f} (G) = 0.38.
b) 4-Benzyloxy-2-(4-methoxybutoxy)-benzoesäuremethylester: Zu einer Lösung von 4-Benzyloxy-2-(4-brombutoxy)-benzoesäuremethylester (29.6 g) in wasserfreiem Methanol (250 mL) wird unter Rückfluss eine 30 %ige methanolische Natriummethylat-Lösung (21 mL) während 30 min zugetropft und der Ansatz über Nacht weitergerührt. Nach dem Abkühlen wird auf die Hälfte des Volumens eingeengt, mit Wasser (50 mL) versetzt und durch Zugabe von 1 M Kaliumhydrogensulfat-Lösung auf pH 2 gestellt. Extraktion mit Dichlormethan und FC Reinigung des Rohproduktes (2 kg KG, Hexan-Essigsäureethylester 7:1) ergibt die Titelverbindung als Festsubstanz (18.8 g): R_{f} (C) = 0.24. Schmp. 72-4°C.
c) 4-Benzyloxy-2-(4-brombutoxy)-benzoesäuremethylester: 4-Benzyloxy-2-hydroxy-benzoesäuremethylester (20.0 g) (hergestellt nach dem in J. Med. Chem. (1985), 28, 717-727 angegebenen Verfahren) und 1,4-Dibrombutan (91.2 mL), gelöst in Aceton (200 mL), werden in Gegenwart von wasserfreiem gepulvertem Kaliumcarbonat (16.0 g) unter Rückfluss über 30 h gerührt. Nach Filtration und Einengen wird das Rohprodukt mittels FC (400 g KG, Fliessmittel A) gereinigt. Man erhält die Titelverbindung als gelbliche Festsubstanz (29.7 g): R_{f} (C) = 0.35.

### Beispiel 51:

Nach Umsetzung von (2S,4S,5S,7S)-{4-[4-(*tert*-Butoxycarbonyl)amino-7-butylcarbamoyl-5-hydroxy-2-isopropyl-8-methyl-nonylcarbamoyl]-3-(4-methoxybutoxy)-phenoxy}-essigsäure-(*tert*-butyl)ester (93 mg) in einer 4 N Salzsäure-Lösung in Dioxan (2 mL) bei 0° C über 45 min und anschließend bei RT über 13 h wird das Lösungsmittel im HV rasch unter starkem Rühren bis zum Gefrieren eingeengt und im weiteren durch Lyophilisation entfernt. Man erhält die (2S,4S,5S,7S)-[4-(4-Amino-7-butylcarbamoyl-5-hydroxy-2-isopropyl-8-methyl-nonylcarbamoyl)-3-(4-methoxybutoxy)-phenoxy]-essigsäure: HPLC Rₜ = 11.7 min. MS(FAB) *m/e* 610 (M⁺ +1).

### Beispiel 52:

Analog wie in Beispiel 21) beschrieben, erhält man aus 85 mg (2S,4S,5S,7S)-N-{4-(*tert*-Butoxycarbonyl)amino-7-[2-(morpholin-4-yl)-ethylcarbamoyl]-5-hydroxy-2-isopropyl-8-methyl-nonyl}-2-(4-methoxybutoxy)-4-[2-(morpholin-4-yl)-ethylcarbamoyl-methoxy]-benzamid das (2S,4S,5S,7S)-N-{4-Amino-5-hydroxy-2-isopropyl-8-methyl-7-[2-(morpholin-4-yl)-ethylcarbamoyl]-nonyl}-2-(4-methoxybutoxy)-4-[2-(morpholin-4-yl)-ethylcarbamoylmethoxy]-benzamid-trihydrochlorid: R_{f} (W) = 0.07. HPLC Rₜ = 7.69 min. HRMS(FAB) *m/e* 779.5264.

Das als Ausgangsverbindung eingesetzte (2S,4S,5S,7S)-N-{4-( *tert*-Butoxycarbonyl)amino-7-[2-(morpholin-4-yl)-ethylcarbamoyl]-5-hydroxy-2-isopropyl-8-methyl-nonyl}-2-(4-methoxybutoxy)-4-[2-(morpholin-4-yl)-ethylcarbamoylmethoxy]-benzamid wird folgendermaßen hergestellt:
a) Die Lösung von (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-carbamoylmethoxybenzamid (98 mg) in N-(2-Aminoethyl)morpholin (0.5 mL) wird über Nacht bei 80° C gerührt. Der Reaktionsansatz wird anschließend direkt an 25 g Kieselgel (Fliessmittelgradient von P nach L) chromatographiert. Man erhält die Titelverbindung, R_{f} (W) = 0.41; HPLC Rₜ = 9.85 min; MS(FAB) *m/e* 880 (M⁺ +1), als gelblichen Schaum.
b) (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-carbamoylmethoxybenzamid: Die Mischung von (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-hydroxy-2-(4-methoxybutoxy)-benzamid (200 mg), 2-Bromacetamid (72 mg) und Cesiumcarbonat (169 mg) in wasserfreiem Aceton (10 mL) wird über 2 h unter Rückfluss gerührt. Nach dem Abkühlen wird filtriert, das Filtrat eingeengt und der Rückstand im HV getrocknet. Man erhält 169 mg der Titelverbindung: R_{f} (L) = 0.59; MS(FAB) *m/e* 636 (M⁺ +1), als weisse Festsubstanz.

### Beispiel 53:

Auf analoge Weise wie in Beispiel 21) beschrieben, wird durch Umsetzung von (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(1H-tetrazol-5-ylmethoxy)-benzamid (88 mg) in einer 4 N Salzsäure-Lösung in Dioxan bei 0° C über 6 h, rasches Einengen des Lösungsmittels am HV und Lyophilisation das (2S,4S,5S,7S)-N-(4-Amino-7-butylcarbamoyl-5-hydroxy-2-isopropyl-8-methyl-nonyl)-2-(4-methoxybutoxy)-4-(1H-tetrazol-5-ylmethoxy)-benzamid-hydrochlorid erhalten: R_{f} (W) = 0.06. HPLC Rₜ = 11.5 min. MS(FAB) *m/e* 634 (M⁺ +1).

Das als Ausgangsstoff eingesetzte (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(1H-tetrazol-5-ylmethoxy)-benzamid wird wie folgt hergestellt:
a) Aus(2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(1H-tetrazol-5-ylmethoxy)-benzamid (104 mg) analog wie in Beispiel 18) beschrieben, durch Umsetzung in n-Butylamin bei 50° C über 20 h. Das Reaktionsgemisch wird eingeengt und der Rückstand in Dichlormethan (50 mL) aufgenommen. Die organische Phase wird mit Eiswasser (pH 4) gewaschen, und die wässrige Phase mit Dichlormethan noch zweimal zurückextrahiert. Man wäscht die vereinten organischen Phasen mit gesättigter Natriumchlorid-Lösung, trocknet über Magnesiumsulfat und entfernt das Lösungsmittel unter Vakuum. Nach Trocknen im HV erhält man das (2S,4S,5S,7S)-N-[4-(tert-Butoxycarbonyl)amino-7-butylcarbamoyl-5-hydroxy-2-isopropyl-8-methyl-nonyl]-2-(4-methoxybutoxy)-4-(1H-tetrazol-5-ylmethoxy)-benzamid, R_{f} (Dichlormethan-Methanol-Eisessig 9:1:0.1) = 0.31, als gelbliche Festsubstanz.
b) (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(1H-tetrazol-5-ylmethoxy)-benzamid: Aus(3S,5S,1'S,3'S)-5-[3'-Aminomethyl-1'-(*tert*-butoxycarbonyl)-amino-4'-methylpentyl]-3-isopropyl-dihydrofuran-2-on (80 mg) und 2-(4-Methoxybutoxy)-4-(1 H-tetrazol-5-ylmethoxy)-benzoesäure (145 mg) erhält man nach FC Reinigung des Rohproduktes an 25 g KG (Fliessmittel Dichlormethan-Methanol-Eisessig 95:5:1; Mischfraktionen werden wiederholt unter gleichen Bedingungen bzw. mit Dichlormethan-Methanol-Ammoniak konz. 95:5:1 chromatographiert) die Titelverbindung, R_{f} (W) = 0.32, als Festsubstanz.

Die als Ausgangsstoff eingesetzte 2-(4-Methoxybutoxy)-4-(1H -tetrazol-5-ylmethoxy)-benzoesäure wird wie folgt hergestellt:
a) 2-(4-Methoxybutoxy)-4-(1H-tetrazol-5-ylmethoxy)-benzoesäuremethylester (1.0 g) wird mit 1 N Natronlauge (3.6 mL) in Methanol (10 mL) und Wasser (5 mL) hydrolysiert. Das Reaktionsgemisch wird mit Dichlormethan (50 mL) verdünnt und die wässrige Phase mit einer 1 M Kaliumhydrogensulfat-Lösung auf pH 2 gebracht. Man extrahiert wiederholt mit Dichlormethan, wäscht die vereinten organischen Phasen mit gesättigter Natriumchlorid-Lösung, trocknet über Magnesiumsulfat und entfernt das Lösungsmittel. Man erhält die Titelverbindung (780 g), R_{f} (Dichlormethan-Methanol-Eisessig 40:10:1) = 0.61, als weisses Pulver.
b) 2-(4-Methoxybutoxy)-4-(1H-tetrazol-5-ylmethoxy)-benzoesäuremethylester: Die Mischung aus 2-(4-Methoxybutoxy)-4-(cyanomethoxy)-benzoesäuremethylester (1.0 g), Natriumazid (1.02 g) und Ammoniumchlorid (0.84 g) in absolutem N,N-Dimethylformamid (30 mL) wird bei 135° C über Nacht gerührt. Die braune Suspension wird eingeengt, und der Eindampfrückstand durch FC (80 g KG, Fliessmittel Dichlormethan-Methanol-Ammoniak konz. 40:10:1) gereinigt. Man erhält die Titelverbindung, R_{f} (Dichlormethan-Methanol-Eisessig 40:10:1) = 0.71; R_{f} (Dichlormethan-Methanol-Ammoniak konz. 40:10:1) = 0.29, als braunes Öl.
c) 2-(4-Methoxybutoxy)-4-(cyanomethoxy)-benzoesäuremethylester: Man erhält die Titelverbindung aus 4-Hydroxy-2-(4-methoxybutoxy)-benzoesäuremethylester (3.0 g), Chloracetonitril (1.9 mL) und Cesiumcarbonat (5.8 g), analog wie in Beispiel 30Ba) beschrieben als Öl: R_{f} (E) = 0.38.

### Beispiel 54:

Zur Lösung von (2S,4S,5S,7S)-5-(*tert*-Butoxycarbonyl)amino-4-*(tert*-butyl)-dimethylsilyloxy-2-isopropyl-7-{[2-(4-methoxybutoxy)-benzoylamino]-methyl}-8-methylnonansäure (100 mg) und Triethylamin (48 µL) in N,N-Dimethylformamid (4 mL) werden bei 0° ^{c} unter Rühren Cyanphosphonsäurediethylester (26 µL) und 2-Aminopropionsäure-N,N-(dimethyl)amid (26 mg) addiert. Nach 30 min lässt man auf RT aufwärmen und rührt über Nacht weiter. Der Ansatz wird eingeengt und der Rückstand in Essigsäureethylester aufgenommen. Nach Waschen der organischen Phase mit 10 %iger Citronensäure-Lösung, gesättigter Natriumhydrogencarbonat-Lösung und gesättigter Natriumchlorid-Lösung, wird über Magnesiumsulfat getrocknet und eingeengt. FC Reinigung (25 g KG, Fliessmittel V) ergibt das (2S,4S,5S,7S)-N-(4-(*tert*-Butoxycarbonyl)amino-5-(*tert*-butyl)dimethylsilyloxy-7-[2-(dimethylaminocarbamoyl)-ethylcarbamoyl]-2-isopropyl-8-methyl-nonyl)-2-(4-methoxybutoxy)-benzamid, R_{f} (Dichlormethan-Methanol-Ammoniak konz. 95:5:1) = 0.21; MS(FAB) *m/e* 794 (M⁺ +1), als gelbes Öl.

Die als Ausgangsstoff eingesetzte (2S,4S,5S,7S)-5-(*tert*--Butoxycarbonyl)amino-4-(*tert*-butyl)dimethylsilyloxy-2-isopropyl-7-{[2-(4-methoxybutoxy)-benzoylamino]-methyl}-8-methyl-nonansäure wird wie folgt hergestellt:
a) (2S,4S,5S,7S)-5-(*tert*-Butoxycarbonyl)am ino-4-(*tert*-butyl)dimethylsilyloxy-2-isopropyl-7-{[2-(4-methoxybutoxy)-benzoylamino]-methyl}-8-methyl-nonansäure: Die Lösung von (2S,4S,5S,7S)-5-(*tert*-Butoxycarbonyl)amino-4-hydroxy-2-isopropyl-7-{[2-(4-methoxybutoxy)-benzoylamino]-methyl}-8-methyl-nonansäure (2.96 g), *tert*-Butyldimethylsilylchlorid (1.69 g) und Imidazol (1.46 g) in N,N-Dimethylformamid (30 mL) wird bei RT über 3 Tage gerührt. Anschließend wird der Reaktionsansatz eingeengt und der Rückstand zwischen Eiswasser und Essigsäureethylester verteilt. Nach Extraktion der wässrigen Phase mit Essigsäureethylester wird die eiskalte organische Phase mit 10 %iger Citronensäure-Lösung, gesättigter Natriumhydrogencarbonat-Lösung und Wasser gewaschen, über Magnesiumsulfat getrocknet und eingeengt. Der rohe Silylester (3.81 g, gelbes Öl) wird in einer Mischung aus Tetrahydrofuran (15 mL), Wasser (6 mL) und Eisessig (15 mL) über Nacht bei RT gerührt. Einengen des Reaktionsansatzes und übliche extraktive Aufarbeitung mit Essigsäureethylester ergibt ein gelbes Öl, aus dem man nach FC (400 g KG, Elution zunächst mit Fliessmittelgradient von D nach F, anschließend vollständige Elution des Produktes mit Fliessmittel L) die Titelverbindung (2.01 g) als schaumige Festsubstanz erhält: R_{f} (E) = 0.32. MS(FAB) *m/e* 695 (M⁺ +1).
b) (2S,4S,5S,7S)-5-(*tert*-Butoxycarbonyl)amino-4-hydroxy-2-isopropyl-7-{[2-(4-methoxybutoxy)-benzoylamino]-methyl}-8-methyl-nonansäure: Zu einer Lösung von (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-benzamid (3.04 g) in einem 2:1-Gemisch aus 1,2-Dimethoxyethan - Wasser (150 mL) werden 21.6 mL einer 1 M Lithiumhydroxid-Lösung addiert und der Reaktionsansatz 1 h bei RT gerührt. Nach Entfemen des Ethers am Rotavap (Badtemperatur 35° C) wird mit eiskalter 10 %iger Citronensäure-Lösung (45 mL) angesäuert und mit Dichlormethan extrahiert. Die organische Phase wird über Magnesiumsulfat getrocknet und unter Vakuum bei bei RT eingeengt. Man erhält 2.96 g der Titelverbindung, R_{f} (L) = 0.26, als blaßgelbe schaumige Festsubstanz.

### Beispiel 55:

In analoger Weise wie in Beispiel 54) beschrieben, werden die nachfolgenden Verbindungen hergestellt:
a) Aus 100 mg (2S,4S,5S,7S)-5-(*tert*-Butoxycarbonyl)amino-4-(*tert*-butyl)dimethylsilyloxy-2-isopropyl-7-{[2-(4-methoxybutoxy)-benzoylamino]-methyl}-8-methyl-nonansäure und 24 mg 3-Aminobuttersäureamid und nachfolgende FC Reinigung (Fliessmittelgradient von U nach Dichlormethan-Methanol-Ammoniak konz. 95:5:1) das (2S,4S,5S,7S)-N-(4-(*tert*-Butoxycarbonyl)amino-5-(*tert*-butyl)dimethylsilyloxy-7-(3-carbamoylpropylcarbamoyl)-2-isopropyl-8-methyl-nonyl)-2-(4-methoxybutoxy)-benzamid, R_{f} (W) = 0.48, als gelbes Öl.
b) Aus 100 mg (2S,4S,5S,7S)-5-(*tert*-Butoxycarbonyl)amino-4-(*tert*-butyl)dimethylsilyloxy-2-isopropyl-7-{[2-(4-methoxybutoxy)-benzoylamino]-methyl}-8-methyl-nonansäure und 26.3 mg 3-Amino-2,2-dimethylpropionsäureamid das (2S,4S,5S,7S)-N-(4-(*tert*-Butoxycarbonyl)-amino-5-(tert-butyl)dimethylsilyloxy-7-(2-carbamoyl-2-methylpropylcarbamoyl)-2-isopropyl-8-methyl-nonyl)-2-(4-methoxybutoxy)-benzamid, R_{f} (W) = 0.59, als farbloses Öl.
c) Aus 100 mg (2S,4S,5S,7S)-5-(*tert*-Butoxycarbonyl)amino-4-(*tert*-butyl)dimethylsilyloxy-2-isopropyl-7-{[2-(4-methoxybutoxy)-benzoylamino]-methyl}-8-methyl-nonansäure und 48 mg 3-Aminopropionsäure-N-(morpholin)amid-hydrochlorid das (2S,4S,5S,7S)-N-{4-(*tert*-Butoxycarbonyl)amino-5-(*tert*-butyl)dimethylsilyloxy-2-isopropyl-8-methyl-7-[3-(morpholin-4-yl)-3-oxo-propylcarbamoyl]-nonyl}-2-(4-methoxybutoxy)-benzamid, R_{f} (W) = 0.62, als farbloses Öl.
d) Aus 100 mg (2S,4S,5S,7S)-5-(*tert*-Butoxycarbonyl)amino-4-(*tert*-butyl)dimethylsilyloxy-2-isopropyl-7-{[2-(4-methoxybutoxy)-benzoylamino]-methyl}-8-methyl-nonansäure und 41 mg 1-[4-(2-Aminoethyl)-piperidin-1-yl]-ethanon das (2S,4S,5S,7S)-N-{7-[2-(4-Acetylpiperidin-1-yl)-ethylcarbamoyl]-4-(*tert*-butoxycarbonyl)amino-5-(*tert*-butyl)dimethylsilyloxy-2-isopropyl-8-methyl-nonyl}-2-(4-methoxybutoxy)-benzamid, R_{f} (W) = 0.58, als Öl.
e) Aus 100 mg (2S,4S,5S,7S)-5-(*tert*-Butoxycarbonyl)amino-4-(*tert*-butyl)dimethylsilyloxy-2-isopropyl-7-{[2-(4-methoxybutoxy)-benzoylamino]-methyl}-8-methyl-nonansäure und 31 mg 2-Aminoethyl-thiomorpholin und FC Reinigung (25 g KG, Fliessmittel T) das (2S,4S,5S,7S)-N-[7-(2-thiomorpholin-4-ylethylcarbamoyl)-4-(*tert*-butoxycarbonyl)amino-5-(*tert*-butyl)-dimethylsilyloxy-2-isopropyl-8-methyl-nonyl]-2-(4-methoxybutoxy)-benzamid, R_{f} (Dichlormethan-Methanol-Ammoniak konz. 95:5:1) = 0.36, als Öl.

### Beispiel 56:

Analog wie in Beispiel 54) beschrieben, werden (2S,4S,5S,7S)-5-(*tert*-Butoxycarbonyl)amino-4-(*tert*-butyl)dimethylsilyloxy-2-isopropyl-7-{[2-(4-methoxybutoxy)-4-[2-(morpholin-4-yl)-ethoxy)-benzoylamino]-methyl}-8-methyl-nonansäure (258 mg), Cyanphosphonsäurediethylester (0.138 mL), 3-Amino-2,2-dimethylpropionsäureamid-hydrochlorid (139 mg) und Triethylamin (0.20 mL) in wasserfreiem N,N-Dimethylformamid (10 mL) umgesetzt. FC Reinigung (Fliessmittel V) ergibt das (2S,4S,5S,7S)-N-(4-(*tert*-Butoxycarbonyl)amino-5-(*tert*-butyl)dimethylsilyloxy-7-(2-carbamoyl-2-methylpropylcarbamoyl)-2-isopropyl-8-methyl-nonyl)-2-(4-methoxybutoxy)-4-(2-morpholin-4-ylethoxy)-benzamid, R_{f} (W) = 0.50, als weisse Festsubstanz.

Die als Ausgangsstoff eingesetzte (2S,4S,5S,7S)-5-( *tert*-Butoxycarbonyl)amino-4-(*tert*-butyl)dimethylsilyloxy-2-isopropyl-7-{[2-(4-methoxybutoxy)-4-(2-morpholin-4-ylethoxy)-benzoylamino]-methyl}-8-methyl-nonansäure wird wie folgt hergestellt:
a) (2S,4S,5S,7S)-5-(*tert*-Butoxycarbonyl)amino-4-(*tert*-butyl)dimethylsilyloxy-2-isopropyl-7-{[2-(4-methoxybutoxy)-4-(2-morpholin-4-ylethoxy)-benzoylamino]-methyl}-8-methylnonansäure: Analog wie in Beispiel 54a) beschrieben, werden (2S,4S,5S,7S)-5-( *tert*-Butoxycarbonyl)amino-4-hydroxy-2-isopropyl-7-{[2-(4-methoxybutoxy)-4-(2-morpholin-4-ylethoxy)-benzoylamino]-methyl}-8-methyl-nonansäure (629 mg), *tert*-Butyldimethylsilylchlorid (294 mg ) und Imidazol (253 mg) in wasserfreiem N,N-Dimethylformamid (5 mL) über 5 Tage bei RT gerührt. Nach Aufarbeitung erhält man 611 mg eines gelblichen Öls, das in einem 2:1-Gemisch aus Tetrahydrofuran - Wasser (4 mL) und Eisessig (3 mL) gelöst und über Nacht bei RT gerührt wird. Nach Einengen des Reaktionsansatzes und extraktivem Aufarbeitung mit Essigsäureethylester erhält man 920 mg der Titelverbindung, R_{f} (W) = 0.26, als gelbes Öl.
b) (2S,4S,5S,7S)-5-(*tert*-Butoxycarbonyl)amino-4-hydroxy-2-isopropyl-7-{[2-(4-methoxybutoxy)-4-(2-morpholin-4-ylethoxy)-benzoylamino]-methyl}-8-methyl-nonansäure: Analog wie in Beispiel 54b) beschrieben wird (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-4-(2-morpholin-4-ylethoxy)-benzamid (622 mg ) (Beispiel 30b), gelöst in 30 mL eines 2:1-Gemisch aus 1,2-Dimethoxyethan - Wasser, mit einer 1 M Lithiumhydroxid-Lösung (3.6 mL) umgesetzt. Nach Aufarbeitung erhält man die Titelverbindung (630 mg) als blaßgelbe schaumige Festsubstanz, R_{f} (L) = 0.29, die sofort weiter umgesetzt wird.

### Beispiel 57:

Durch Umsetzung von (2S,4S,5S,7S)-5-(*tert*-Butoxycarbonyl)amino-7-aminomethyl-4-(*tert*-butyl)dimethylsilyloxy-2-isopropyl-8-methyl-nonansäure-N-(2-carbamoyl-2-methylpropyl)amid (100 mg) und 2-(4-Methoxybutoxy)-4-(morpholin-4-ylmethyl)-benzoesäure (116 mg) analog wie in Beispiel 15) beschrieben und FC Reinigung (30 g KG, Fliessmittel T) erhält man das (2S,4S,5S,7S)-N-(4-(*tert*-Butoxycarbonyl)amino-5-(*tert*-butyl)dimethylsilyloxy-7-(2-carbamoyl-2-methylpropylcarbamoyl)-2-isopropyl-8-methyl-nonyl)-2-(4-methoxybutoxy)-4-(morpholin-4-ylmethyl)-benzamid, R_{f} (W) = 0.57, als weisse Festsubstanz.

Das als Ausgangsmaterial eingesetzte (2S,4S,5S,7S)-5-(*tert*-Butoxycarbonyl)amino-7-aminomethyl-4-(*tert*-butyl)dimethylsilyloxy-2-isopropyl-8-methyl-nonansäure-N-(2-carbamoyl-2-methylpropyl)amid wird folgendermaßen hergestellt:
a) Nach Hydrierung von (2S,4S,5S,7S)-5-(*tert*-Butoxycarbonyl)amino-7-azidomethyl-4-(*tert*-butyl)dimethylsilyloxy-2-isopropyl-8-methyl-nonansäure-N-(2-carbamoyl-2-methyl propyl)-amid (534 mg), gelöst in Essigsäureethylester (30 mL), in Gegenwart von 10 % Pd/C (106 mg) über 5 h bei RT wird über Celite 545 filtriert, das Filtrat eingeengt und das so erhaltene Rohprodukt erneut in Gegenwart von frischem Katalysator (106 mg 10 % Pd/C) über 24 h hydriert. FC Reinigung (25 g KG, Fliessmittelgradient Dichlormethan-Methanol-Ammoniak konz. von 94:6:1 nach T) ergibt das (2S,4S,5S,7S)-5-( *tert*-Butoxycarbonyl)amino-7-amino-methyl-4-(*tert*-butyl)dimethylsilyloxy-2-isopropyl-8-methyl-nonansäure-N-(2-carbamoyl-2-methylpropyl)amid, R_{f} (W) = 0.28, als weisse Festsubstanz.
b) (2S,4S,5S,7S)-5-(*ter*t-Butoxycarbonyl)amino-7-azidomethyl-4-(*tert*-butyl)dimethylsilyloxy-2-isopropyl-8-methyl-nonansäure-N-(2-carbamoyl-2-methylpropyl)amid: Umsetzung von (2S,4S,5S,7S)-5-(*tert-*Butoxycarbonyl)amino-7-azidomethyl-4-(*tert*-butyl)dimethylsilyloxy-2-isopropyl-8-methyl-nonansäure (490 mg) mit 3-Amino-2,2-dimethylpropionsäureamid-hydrochlorid (290 mg), analog wie in Beispiel 56) beschrieben, und FC Reinigung (50 g KG, Fliessmittel T) ergibt die Titelverbindung, R_{f} (W) = 0.67 ; MS(FAB) *m/e* 613 (M⁺ +1), als weisse Festsubstanz.
c) Die (2S,4S,5S,7S)-5-(*tert*-Butoxycarbonyl)amino-7-azidomethyl-4-(*tert*-butyl)-dimethylsilyloxy-2-isopropyl-8-methyl-nonansäure erhält man analog wie in den Beispielen 54a) und 54b) beschrieben aus 500 mg (3S,5S,1'S,3'S)-5-[3'-Azidomethyl-1'-( *tert*-butoxycarbonyl)amino-4'-methylpentyl]-3-isopropyl-dihydrofuran-2-on (Beispiel 15b) über die (2S,4S,5S,7S)-5-(*tert*-Butoxycarbonyl)amino-7-azidomethyl-4-hydroxy-2-isopropyl-8-methyl-nonansäure (R_{f} (L) = 0.43) nach FC Reinigung (50 g KG, Fliessmittel Q) als weisse Festsubstanz (enthält geringen Anteil an Edukt aufgrund von Relaktonisierung): R_{f} (L) = 0.64.

### Beispiel 58:

Die Umsetzung von (2S,4S,5S,7S)-5-(*tert*-Butoxycarbonyl)amino-7-amino-methyl-4-(*tert*-butyl)dimethylsilyloxy-2-isopropyl-8-methyl-nonansäure-N-(2-carbamoyl-2-methylpropyl)amid (100 mg) und 2-(2-Morpholin-4-ylethoxy)-benzoesäure (89 mg) analog wie in Beispiel 54) beschrieben, und FC Reinigung ergibt das (2S,4S,5S,7S)-N-[4-( *tert*-Butoxycarbonyl)amino-5-(*tert*-butyl)dimethylsilyloxy-7-(2-carbamoyl-2-methylpropyl-carbamoyl)-2-isopropyl-8-methyl-nonyl]-2-(2-morpholin-4-ylethoxy)-benzamid als Öl: R_{f} (L) = 0.40; HPLC Rt = 17.3 min.

### Beispiel 59:

Zu einer Lösung von (2S,4S,5S,7S)-N-(4-(*tert*-Butoxycarbonyl)amino-5-(*tert*-butyl)dimethylsilyloxy-7-[2-(dimethylaminocarbamoyl)-ethylcarbamoyl]-2-isopropyl-8-methylnonyl)-2-(4-methoxybutoxy)-benzamid (71 mg) in wasserfreiem N,N-Dimethylformamid (5 mL) wird Tetrabutylammoniumfluorid-Trihydrat (31 mg) addiert und der Ansatz bei RT über Nacht gerührt. Das Reaktionsgemisch wird eingeengt und der Rückstand zwischen gesättigter Natriumhydrogencarbonat-Lösung (20 mL) und Essigsäureethylester (30 mL) verteilt. Die abgetrennte wässrige Phase wird mit Essigsäureethylester extrahiert, und die vereinigten organischen Phasen mit gesättigter Natriumchlorid-Lösung gewaschen, über Magnesiumsulfat getrocknet und eingeengt. Durch FC (25 g KG, Fliessmittel O) erhält man das (2S,4S,5S,7S)-N-[4-(tert-Butoxycarbonyl)amino-7-[2-(dimethylaminocarbamoyl)-ethylcarbamoyl]-5-hydroxy-2-isopropyl-8-methyl-nonyl)-2-(4-methoxybutoxy)-benzamid, R_{f} (N) = 0.32, als gelbliches Öl.

### Beispiel 60:

In analoger Weise wie in Beispiel 59) beschrieben, werden durch Abspaltung der Silyloxy-Schutzgruppe die nachfolgenden Verbindungen hergestellt:
a) Aus 76 mg (2S,4S,5S,7S)-N-(4-(*tert*-Butoxycarbonyl)amino-5-(*tert*-butyl)dimethylsilyloxy-7-(3-carbamoylpropylcarbamoyl)-2-isopropyl-8-methyl-nonyl)-2-(4-methoxybutoxy)-benzamid und FC Reinigung (25 g KG, Fliessmittelgradient von N nach L) das (2S,4S,5S,7S)-N-[4-(tert-Butoxycarbonyl)amino-7-(3-carbamoylpropylcarbamoyl)-5-hydroxy-2-isopropyl-8-methyl-nonyl)-2-(4-methoxybutoxy)-benzamid, R_{f} (L) = 0.45, als Öl.
b) Aus 66 mg (2S,4S,5S,7S)-N-(4-(*tert*-Butoxycarbonyl)amino-5-(*tert*-butyl)dimethylsilyloxy-7-(2-carbamoyl-2-methylpropylcarbamoyl)-2-isopropyl-8-methyl-nonyl)-2-(4-methoxybutoxy)-benzamid das (2S,4S,5S,7S)-N-[4-(tert-Butoxycarbonyl)amino-7-(2-carbamoyl-2-methylpropylcarbamoyl)-5-hydroxy-2-isopropyl-8-methyl-nonyl)-2-(4-methoxybutoxy)-benzamid, R_{f} (L) = 0.48, als Öl.
c) Aus 101 mg (2S,4S,5S,7S)-N-{4-(*tert*-Butoxycarbonyl)amino-5-(*tert*-butyl)-dimethylsilyloxy-2-isopropyl-8-methyl-7-[3-(morpholin-4-yl)-3-oxo-propylcarbamoyl]-nonyl}-2-(4-methoxybutoxy)-benzamid das (2S,4S,5S,7S)-N-{4-(*tert*-Butoxycarbonyl)amino-5-hydroxy-2-isopropyl-8-methyl-7-[3-(morpholin-4-yl)-3-oxo-propylcarbamoyl]-nonyl}-2-(4-methoxybutoxy)-benzamid, R_{f} (W) = 0.54; HPLC Rₜ = 15.7 min, als Öl.
d) Aus 134 mg (2S,4S,5S,7S)-N-{7-[2-(4-Acetylpipeddin-1-yl)-ethylcarbamoyl]-4-(*tert*-butoxycarbonyl)amino-5-(*tert*-butyl)dimethylsilyloxy-2-isopropyl-8-methyl-nonyl}-2-(4-methoxybutoxy)-benzamid das (2S,4S,5S,7S)-N-{7-[2-(4-Acetylpiperidin-1-yl)-ethylcarbamoyl]-4-(*tert*-butoxycarbonyl)amino-5-hydroxy-2-isopropyl-8-methyl-nonyl}-2-(4-methoxybutoxy)-benzamid, R_{f} (W) = 0.46; HPLC Rₜ = 17.1 min, als weisse Festsubstanz.
e) Aus 76 mg (2S,4S,5S,7S)-N-[4-(*tert*-Butoxycarbonyl)amino-5-(*tert*-butyl)dimethylsilyloxy-2-isopropyl-8-methyl-7-(2-thiomorpholin-4-ylethylcarbamoyl)-nonyl]-2-(4-methoxybutoxy)-benzamid das (2S,4S,5S,7S)-N-[4-(*tert*-Butoxycarbonyl)amino-5-hydroxy-2-isopropyl-8-methyl-7-(2-thiomorpholin-4-ylethylcarbamoyl)-nonyl]-2-(4-methoxybutoxy)-benzamid, R_{f} (N) = 0.28; HPLC Rₜ = 14.7 min, als Öl.
f) Aus 150 mg (2S,4S,5S,7S)-N-(4-(*tert*-Butoxycarbonyl)amino-5-(*tert*-butyl)dimethylsilyloxy-7-(2-carbamoyl-2-methylpropylcarbamoyl)-2-isopropyl-8-methyl-nonyl)-2-(4-methoxybutoxy)-4-(2-morpholin-4-ylethoxy)-benzamid das (2S,4S,5S,7S)-N-[4-(tert-Butoxycarbonyl)amino-7-(2-carbamoyl-2-methylpropylcarbamoyl)-5-hydroxy-2-isopropyl-8-methyl-nonyl)-2-(4-methoxybutoxy)-4-(2-morpholin-4-ylethoxy)-benzamid, R_{f} (W) = 0.40, als Öl.
g) Aus 116 mg (2S,4S,5S,7S)-N-(4-(*tert*-Butoxycarbonyl)amino-5-(*tert*-butyl)-dimethylsilyloxy-7-(2-carbamoyl-2-methylpropylcarbamoyl)-2-isopropyl-8-methyl-nonyl)-2-(4-methoxybutoxy)-4-(morpholin-4-ylmethyl)-benzamid das (2S,4S,5S,7S)-N-[4-(tert-Butoxycarbonyl)amino-7-(2-carbamoyl-2-methylpropylcarbamoyl)-5-hydroxy-2-isopropyl-8-methyl-nonyl)-2-(4-methoxybutoxy)-4-(morpholin-4-ylmethyl)-benzamid, R_{f} (W) = 0.33; HPLC Rₜ = 11.5 min, als Öl.
h) Aus 96 mg (2S,4S,5S,7S)-N-[4-(*tert*-Butoxycarbonyl)amino-5-(*tert*-butyl)dimethylsilyloxy-7-(2-carbamoyl-2-methylpropylcarbamoyl)-2-isopropyl-8-methyl-nonyl]-2-(4-methoxybutoxy)-4-(2-morpholin-4-ylethoxy)-benzamid das (2S,4S,5S,7S)-N-[4-(*tert*-Butoxycarbonyl)amino-7-(2-carbamoyl-2-methylpropylcarbamoyl)-5-hydroxy-2-isopropyl-8-methyl-nonyl]-2-(2-morpholin-4-ylethoxy)-benzamid, R_{f} (L) = 0.35; HPLC Rₜ = 11.6 min, als Öl.

### Beispiel 61:

Analog wie in Beispiel 21) beschrieben, erhält man aus 71 mg (2S,4S,5S,7S,2'R)-N-[4-(*tert*-Butoxycarbonyl)amino-7-(2'-methylcarbamoyl-propylcarbamoyl)-5-hydroxy-2-isopropyl-8-methyl-nonyl]-2-(4-methoxybutoxy)-benzamid das (2S,4S,5S,7S,2 R')-N-[4-Amino-7-(2'-methylcarbamoyl-propylcarbamoyl)-5-hydroxy-2-isopropyl-8-methyl-nonyl]-2-(4-methoxybutoxy)-benzamid-hydrochlorid: R_{f} (W) = 0.33. HPLC Rₜ = 12.7 min. MS(FAB) *m/e* 579 (M⁺ +1).

Das als Ausgangsmaterial eingesetzte (2S,4S,5S,7S,2'R)-N-[4-(*tert*-Butoxycarbonyl)amino-7-(2'-methylcarbamoyl-propylcarbamoyl)-5-hydroxy-2-isopropyl-8-methyl-nonyl]-2-(4-methoxybutoxy)-benzamid wird folgendermaßen hergestellt:
a) Analog wie in Beispiel 59) beschrieben werden (2S,4S,5S,7S,2'R)-N-[4-(*tert*-Butoxycarbonyl)amino-(*tert*-butyl)dimethylsilyloxy-7-(2'-methylcarbamoyl-propylcarbamoyl)-2-isopropyl-8-methyl-nonyl]-2-(4-methoxybutoxy)-benzamid (132 mg ) und Tetrabutylammoniumfluorid-trihydrat (52 mg) in N,N-Dimethylformamid (5mL) bei RT über 20 h gerührt. Nach wässriger Aufarbeitung und Reinigung durch FC (25 g KG, Fliessmittel V) erhält man das (2S,4S,5S,7S,2'R)-N-[4-(*tert*-Butoxycarbonyl)amino-7-(2'-methylcarbamoylpropylcarbamoyl)-5-hydroxy-2-isopropyl-8-methyl-nonyl]-2-(4-methoxybutoxy)-benzamid, R_{f} (W) = 0.51; HPLC Rₜ = 17.6 min, als farblosen Schaum.
b) (2S,4S,5S,7S,2'R)-N-[4-(*tert*-Butoxycarbonyl)amino-(*tert*-butyl)dimethylsilyloxy-7-(2'-methylcarbamoyl-propylcarbamoyl)-2-isopropyl-8-methyl-nonyl]-2-(4-methoxybutoxy)-benzamid: Die Lösung von (2R,2'S,4'S,5'S,7'S)-3-{5'-( *tert*-Butoxycarbonyl)amino-4'-(*tert*-butyl)dimethylsilyloxy-2'-isopropyl-7'-[2-(4-methoxybutoxy)-benzylcarbamoyl]-8-methyl-nonanoylamino}-2-methylpropionsäuremethylester (150 mg) in einer 33 %igen ethanolischen Methylamin-Lösung (6 mL) wird bei 40° C über 40 h gerührt. Nach Einengen des Reaktionsansatzes wird der Rückstand durch FC (Fliessmittel W) gereinigt. Man erhält die Titelverbindung, R_{f} (W) = 0.54, als gelbes Öl.
c) (2R,2'S,4'S,5'S,7'S)-3-{5'-(*tert*-Butoxycarbonyl)amino-4'-(*tert*-butyl)dimethylsilyloxy-2'-isopropyl-7'-[2-(4-methoxybutoxy)-benzylcarbamoyl]-8-methyl-nonanoylamino}-2-methylpropionsäuremethylester: Analog wie in Beispiel 54) beschrieben, werden (2S,4S,5S,7S)-5-(*tert*-Butoxycarbonyl)amino-4-(*tert*-butyl)dimethylsilyloxy-2-isopropyl-7-{[2-(4-methoxy-butoxy)-benzoylamino]-methyl}-8-methyl-nonansäure (300 mg), (2R)-3-Amino-2-methyl-propionsäuremethylester-hydrochlorid (112 mg), Cyanphosphonsäurediethylester (110 µL) und Triethylamin (204 µL) in N,N-Dimethylformamid (10 mL) umgesetzt. Das Reaktionsgemisch wird eingeengt und der Rückstand in Essigsäureethylester aufgenommen. Die organische Phase wird mit 1 M Citronensäure-Lösung, gesättigter Natriumhydrogencarbonat-Lösung und gesättigter Natriumchlorid-Lösung gewaschen, über Magnesiumsulfat getrocknet und eingeengt. Nach FC Reinigung des Rohproduktes (80 g KG, Fliessmittel S) erhält man die Titelverbindung, R_{f} (W) = 0.82, als Öl.

### Beispiel 62:

In analoger Weise wie in Beispiel 21) beschrieben, werden durch De-Bocylierung die nachfolgenden Verbindungen hergestellt:
a) Aus 35 mg (2S,4S,5S,7S)-N-[4-(tert-Butoxycarbonyl)amino-7-[2-(dimethyl aminocarbamoyl)-ethylcarbamoyl]-5-hydroxy-2-isopropyl-8-methyl-nonyl)-2-(4-methoxybutoxy)-benzamid das (2S,4S,5S,7S)-N-(4-Amino-7-[2-(dimethylaminocarbamoyl)-ethylcarbamoyl]-5-hydroxy-2-isopropyl-8-methyl-nonyl)2-(4-methoxybutoxy)-benzamid-hydrochlorid: R_{f} (W) = 0.26. HPLC Rₜ = 11.5 min. MS(FAB) *m/e* 579 (M⁺ +1).
b) Aus 45 mg (2S,4S,SS,7S)-N-[4-(*tert*-Butoxycarbonyl)amino-7-(3-carbamoylpropylcarbamoyl)-5-hydroxy-2-isopropyl-8-methyl-nonyl]-2-(4-methoxybutoxy)-benzamid das (2S,4S,5S,7S)-N-[4-Amino-7-(3-carbamoylpropylcarbamoyl)-5-hydroxy-2-isopropyl-8-methyl-nonyl]-2-(4-methoxybutoxy)-benzamid-hydrochlorid: R_{f} (W) = 0.21. HPLC Rₜ = 10.3 min. MS(FAB) *m/e* 565 (M⁺ +1).
c) Aus 46 mg (2S,4S,5S,7S)-N-[4-(tert-Butoxycarbonyl)amino-7-(2-carbamoyl-2-methylpropylcarbamoyl)-5-hydroxy-2-isopropyl-8-methyl-nonyl)-2-(4-methoxybutoxy)-benzamid das (2S,4S,5S,7S)-N-[4-Amino-7-(2-carbamoyl-2-methylpropylcarbamoyl)-5-hydroxy-2-isopropyl-8-methyl-nonyl]-2-(4-methoxybutoxy)-benzamid-hydrochlorid: R_{f} (W) = 0.27. HPLC Rₜ = 11.1 min. MS(FAB) *m/e* 579 (M⁺ +1).
d) Aus 65 mg (2S,4S,5S,7S)-N-{4-(*tert*-Butoxycarbonyl)amino-5-hydroxy-2-isopropyl-8-methyl-7-[3-(morpholin-4-yl)-3-oxo-propylcarbamoyl]-nonyl}-2-(4-methoxybutoxy)-benzamid das (2S,4S,5S,7S)-N-{4-Amino-5-hydroxy-2-isopropyl-8-methyl-7-[3-(morpholin-4-yl)-3-oxo-propylcarbamoyl]-nonyl}-2-(4-methoxybutoxy)-benzamid-hydrochlorid: R_{f} (W) = 0.25. HPLC Rₜ = 11.3 min. MS(FAB) *m/e* 621 (M⁺ +1).
e) Aus 71 mg (2S,4S,5S,7S)-N-{7-[2-(4-Acetylpiperidin-1-yl)-ethylcarbamoyl]-4-(*tert*-butoxycarbonyl)amino-5-hydroxy-2-isopropyl-8-methyl-nonyl}-2-(4-methoxybutoxy)-benzamid das (2S,4S,5S,7S)-N-{7-[2-(4-Acetylpiperidin-1-yl)-ethylcarbamoyl]-4-amino-5-hydroxy-2-isopropyl-8-methyl-nonyl}-2-(4-methoxybutoxy)-benzamid-hydrochlorid: R_{f} (W) = 0.29. HPLC R_{f} = 12.7 min. MS(FAB) *m/e* 633 (M⁺ +1).
f) Aus 40 mg (2S,4S,5S,7S)-N-[4-(*tert*-Butoxycarbonyl)amino-5-hydroxy-2-isopropyl-8-methyl-7-(2-thiomorpholin-4-ylethylcarbamoyl)-nonyl]-2-(4-methoxybutoxy)-benzamid das (2S,4S,5S,7S)-N-[4-Amino-5-hydroxy-2-isopropyl-8-methyl-7-(2-thiomorpholin-4-ylethylcarbamoyl)-methyl-nonyl]-2-(4-methoxybutoxy)-benzamid-dihydrochlorid: R_{f} (W) = 0.43. HPLC Rₜ = 10.7 min. MS(FAB) *m/e* 609 (M⁺ +1).
g) Aus 102 mg (2S,4S,5S,7S)-N-[4-(tert-Butoxycarbonyl)amino-7-(2-carbamoyl-2-methylpropylcarbamoyl)-5-hydroxy-2-isopropyl-8-methyl-nonyl)-2-(4-methoxybutoxy)-4-(2-morpholin-4-ylethoxy)-benzamid das (2S,4S,5S,7S)-N-(4-Amino-7-(2-carbamoyl-2-methylpropylcarbamoyl)-5-hydroxy-2-isopropyl-8-methyl-nonyl)-2-(4-methoxybutoxy)-4-(2-morpholin-4-ylethoxy-benzamid-dihydrochlorid: R_{f} (W) = 0.18. HPLC Rₜ = 8.48 min. MS(FAB) *m/e* 708 (M⁺ +1).
h) Aus 78 mg (2S,4S,5S,7S)-N-[4-(tert-Butoxycarbonyl)amino-7-(2-carbamoyl-2-methylpropylcarbamoyl)-5-hydroxy-2-isopropyl-8-methyl-nonyl)-2-(4-methoxybutoxy)-4-(morpholin-4-ylmethyl)-benzamid das (2S,4S,5S,7S)-N-(4-Amino-7-(2-carbamoyl-2-methyl propylcarbamoyl)-5-hydroxy-2-isopropyl-8-methyl-nonyl)-2-(4-methoxybutoxy)-4-(morpholin-4-ylmethyl)-benzamid-dihydrochlorid: R_{f} (W) = 0.17. HPLC Rₜ = 7.83 min. MS(FAB) *m/e* 678 (M⁺ +1).
i) Aus 62 mg (2S,4S,5S,7S)-N-[4-(*tert*-Butoxycarbonyl)amino-7-(2-carbamoyl-2-methylpropylcarbamoyl)-5-hydroxy-2-isopropyl-8-methyl-nonyl]-2-(2-morpholin-4-ylethoxy)-benzamid das (2S,4S,5S,7S)-N-[4-Amino-7-(2-carbamoyl-2-methylpropylcarbamoyl)-5-hydroxy-2-isopropyl-8-methyl-nonyl]-2-(2-morpholin-4-ylethoxy)-benzamid-dihydrochlorid: R_{f} (J) = 0.20. HPLC Rₜ = 7.0 min. MS(FAB) *m/e* 606 (M⁺ +1).
j) Aus 490 mg (2S,4S,5S,7S)-N-{4-(*tert*-Butoxycarbonyl)amino-5-hydroxy-2-isopropyl-7-[2-(4-methoxycarbonylpiperidin-1-yl)-ethylcarbamoyl]-8-methyl-nonyl}-2-(4-methoxybutoxy)-benzamid (Beispiel 66) das (2S,4S,5S,7S)-N-{4-Amino-5-hydroxy-2-isopropyl-7-[2-(4-methoxycarbonylpiperidin- 1-yl)-ethylcarbamoyl]-8-methyl-nonyl}-2-(4-methoxybutoxy)-benzamid-hydrochlorid: R_{f} (W) = 0.30. HPLC Rₜ = 11.8 min. MS(FAB) *m/e* 649 (M⁺ +1).

### Beispiel 63:

Die Lösung von (2S,4S,5S,7R)-N-{4-(*tert*-Butoxycarbonyl)amino-5-hydroxy-2-isopropyl-7-[(2-morpholin-4-ylethyl)-carbamoyl]-octyl}-2-(3-methoxypropoxy)-benzamid (105 mg) in 4N Salzsäure-Lösung in Dioxan (4 mL) wird während 1 h bei 0° C gerührt. Der Reaktionsansatz wird anschließend lyophilisiert. Man erhält das (2S,4S,5S,7R)-N-[4-Amino-5-hydroxy-2-methyl-7-[(2-morpholin-4-ylethyl)-carbamoyl]-octyl}-2-(3-methoxypropoxy)-benzamid-dihydrochlorid als beiges Pulver: R_{f} (Dichlormethan-Methanol 8:2) = 0.28. HPLC Rₜ = 7.73 min. MS(FAB) *m/e* 551 (M⁺ +1).

Das als Ausgangsmaterial eingesetzte (2S,4S,5S,7R)-N-{4-(*tert*-Butoxycarbonyl)amino-5-hydroxy-2-isopropyl-7-[(2-morpholin-4-ylethyl)-carbamoyl]-octyl}-2-(3-methoxypropoxy)-benzamid wird folgendermaßen hergestellt:
a) (2S,4S,5S,7R)-N-{4-(*tert*-Butoxycarbonyl)amino-5-hydroxy-2-isopropyl-7-[(2-morpholin-4-ylethyl)-carbamoyl]-octyl}-2-(3-methoxypropoxy)-benzamid: Eine Mischung von (2S,2'S,2''S,4''R)-N-{2-[2'-(*tert*-Butoxycarbonyl)amino-2'-(4''-isopropyl-5''-oxo-tetrahydrofuran-2''-yl)-ethyl]-3-methylbutyl}-2-(3-methoxypropoxy)-benzamid (104 mg) und 4-(2-Aminoethyl)morpholin (2 mL) wird während 2 h bei 80° C gerührt. Anschließend wird das überschüssige 4-(2-Aminoethyl)-morpholin abdestilliert und der Eindampfrückstand durch FC (100 g KG, Fliessmittel L) gereinigt. Man erhält die Titelverbindung (110 mg) als weissen Schaum: R_{f} (L) = 0.36; HPLC Rₜ = 12.1 min.
b) (2S,2'S,2''S,4''R)-N-{2-[2'-(*tert*-Butoxycarbonyl)amino-2'-(4''-isopropyl-5''-oxo-tetrahydrofuran-2''-yl)-ethyl]-3-methylbutyl}-2-(3-methoxypropoxy)-benzamid: Die Lösung von (2S,4S,5S,7R)-N-[4-(tert-Butoxycarbonyl)amino-7-butylcarbamoyl-5-hydroxy-2-isopropyl-octyl]-2-(3-methoxypropoxy)-benzamid (380 mg) in Chloroform (20 mL) wird bei 0° C mit p-Toluolsulfonsäure-monohydrat (134 mg) versetzt. Der Ansatz wird über 20 h bei RT gerührt. Das Lösungsmittel wird abgedampft und der Rückstand mittels FC (60 g KG, Fliessmittel E) gereinigt. Man erhält die Titelverbindung als farbloses Öl: R (F) = 0.36; HPLC Rₜ = 17.1 min; MS(FAB) m/e 521 (M⁺ +1).

### Beispiel 64:

In analoger Weise wie in Beispiel 21) beschrieben, wird aus (2S,4S,5S,7S)-N-{4-(*tert*-Butoxycarbonyl)amino-5-hydroxy-2-isopropyl-8-methyl-7-[2-(morpholin-4-yl)-ethylcarbamoyl]-nonyl}-4-carbamoylmethoxy-2-(4-methoxybutoxy)-benzamid (103 mg) das (2S,4S,5S,7S)-N-{4-Amino-5-hydroxy-2-isopropyl-8-methyl-7-[2-(morpholin-4-yl)-ethylcarbamoyl]-nonyl}-4-carbamoylmethoxy-2-(4-methoxybutoxy)-benzamid-dihydrochlorid hergestellt: R_{f} (W) = 0.23. HPLC Rₜ = 9.81 min. MS(FAB) *m/e* 666 (M⁺ +1).

Das als Ausgangsmaterial eingesetzte (2S,4S,5S,7S)-N-{4-(*tert*-Butoxycarbonyl)amino-5-hydroxy-2-isopropyl-8-methyl-7-[2-(morpholin-4-yl)-ethylcarbamoyl]-nonyl}-4-carbamoylmethoxy-2-(4-methoxybutoxy)-benzamid wird folgendermaßen hergestellt:
a) Die Mischung aus (2S,4S,5S,7S)-N-{4-(*tert*-Butoxycarbonyl)amino-5-hydroxy-2-isopropyl-8-methyl-7-[2-(morpholin-4-yl)-ethylcarbamoyl]-nonyl}-4-hydroxy-2-(4-methoxybutoxy)-benzamid (100 mg), 2-Bromacetamid (24 mg) und Cesiumcarbonat (69 mg) in wasserfreiem Aceton (5 mL) wird über 2 h unter Rückfluss gerührt. Das nach Filtration und Einengen des Lösungsmittels erhaltene Rohprodukt wird durch FC an 25 g KG (Fliessmittelgradient von V nach Dichlormethan-Methanol-Ammoniak konz. 95:5:1) gereinigt. Man erhält die Titelverbindung, R_{f} (Dichlormethan-Methanol-Ammoniak konz. 95:5:1; zweifache Laufstrecke) = 0.24 ; HPLC Rₜ = 13.7 min; MS(FAB) *m/e* 766 (M⁺ +1), als weisse Festsubstanz.
b) (2S,4S,5S,7S)-N-{4-(*tert*-Butoxycarbonyl)amino-5-hydroxy-2-isopropyl-8-methyl-7-[2-(morpholin-4-yl)-ethylcarbamoyl]-nonyl}-4-hydroxy-2-(4-methoxybutoxy)-benzamid: Die Lösung von (2S,4S,5S,7S)- N-{4-(*tert*-Butoxycarbonyl)amino-5-hydroxy-2-isopropyl-8-methyl-7-[2-(morpholin-4-yl)-ethylcarbamoyl]-nonyl}-4-benzyloxy-2-(4-methoxybutoxy)-benzamid (0.86 g) in Essigsäureethylester (30 mL) wird in Gegenwart von Pd/C 5 % (Degussa) (170 mg) bei RT über 6 h hydriert. Das Rohprodukt wird durch FC (Fliessmittelgradient von Dichlormethan-Methanol-Ammoniak konz. 96:4:1 nach W) gereinigt und ergibt die Titelverbindung (0.76 g) als weisse schaumige Festsubstanz: R_{f} (L) = 0.27.
c) (2S,4S,5S,7S)-N-{4-(*tert*-Butoxycarbonyl)amino-5-hydroxy-2-isopropyl-8-methyl-7-[2-(morpholin-4-yl)-ethylcarbamoyl]-nonyl}-4-benzyloxy-2-(4-methoxybutoxy)-benzamid: Analog wie in Beispiel 52a) beschrieben, wird die Titelverbindung durch Umsetzung von (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-4-benzyloxy-2-(4-methoxybutoxy)-benzamid (0.79 g) in N-(2-Aminoethyl)-morpholin (5 mL) und anschließender FC Reinigung (Fliessmittel V) hergestellt: R_{f} (W) = 0.50.

### Beispiel 65:

Die Mischung von (2S,2'S,2''S,4''S)-N-{2-[2'-(*tert*-Butoxycarbonyl)amino-2'-(4"-isopropyl-5''-oxo-tetrahydrofuran-2''-yl)-ethyl]-3-methylbutyl}-2-(4-methoxybutoxy)-benzamid (1.0 g), 1-[4-(2-Aminoethyl)-piperidin-1-yl]-ethanon (0.91 g) und 2-Hydroxypyridin (169 mg) in Triethylamin (7.5 mL) wird bei 80° C über 16 h gerührt (zwei Phasen). Die obere Phase wird auf etwa 25 % des Volumens eingeengt, und das Reaktionsgemisch für weitere 3 h bei 80° C gerührt. Nach dem Abkühlen wird der Ansatz mit Dichlormethan verdünnt, die organische Phase mit gesättigter Natriumhydrogencarbonat-Lösung gewaschen, über Magnesiumsulfat getrocknet und eingeengt. Das Rohprodukt wird durch FC an Kieselgel (Fliessmittel V) gereinigt. Man erhält das (2S,4S,5S,7S)-N-{7-[2-(4-Acetylpipeddin-1-yl)-ethylcarbamoyl]-4-(*tert*-butoxycarbonyl)amino-5-hydroxy-2-isopropyl-8-methyl-nonyl)-2-(4-methoxybutoxy)-benzamid nach Lyophilisation aus einer Lösung in Dioxan als weisses Pulver: R_{f} (W) = 0.46.

### Beispiel 66:

In analoger Weise wie in Beispiel 65) beschrieben wird die nachfolgende Verbindung hergestellt:
a) Aus (2S,2'S,2''S,4''S)-N-{2-[2'-(*tert*-Butoxycarbonyl)amino-2'-(4''-isopropyl-5''-oxo-tetrahydrofuran-2''-yl)-ethyl]-3-methylbutyl}-2-(4-methoxybutoxy)-benzamid (400 mg), 4-Aminoethyl-1-methoxycarboxypiperidin (397 mg) und 2-Hydroxypyridin (68 mg) in Triethylamin (5 mL) das (2S,4S,5S,7S)-N-{4-(*tert*-Butoxycarbonyl)amino-5-hydroxy-2-isopropyl-7-[2-(4-methoxycarbonylpiperidin-1-yl)-ethylcarbamoyl]-8-methyl-nonyl}-2-(4-methoxybutoxy)-benzamid als Öl: R_{f} (L) = 0.50; HPLC Rₜ = 18.0 min.

### Beispiel 66:

Nach den in den Beispielen 1 bis 65 beschriebenen Verfahren werden in analoger Weise die nachfolgenden Verbindungen hergestellt:
a) (2R,4S,5S,7R)-1-(2-Methoxypropyl)-1H-indol-3-carbonsäure-N-(4-amino-7-butylcarbamoyl-5-hydroxy-2-isopropyl-octyl)-amid-hydrochlorid
b) (2R,4S,5S,7R)-1-(2-Ethoxypropyl)-1H-indol-3-carbonsäure-N-(4-amino-7-butylcarbamoyl-5-hydroxy-2-isopropyl-octyl)-amid-hydrochlorid
c) (2R,4S,5S,7R)-N-(4-Amino-7-butylcarbamoyl-5-hydroxy-2-isopropyl-8-methyl-nonyl)-2-benzyloxy-benzamid-hydrochlorid
d) (2S,4S,5S,7S)-N-(4-Amino-7-butylcarbamoyl-5-hydroxy-2-isopropyl-8-methyl-nonyl)-2-[2-methoxyethoxy)-ethyl]-benzamid
e) (2S,4S,5S,7S)-N-(4-Amino-7-butylcarbamoyl-5-hydroxy-2-isopropyl-8-methyl-nonyl)-2-(2-ethoxyethyl)-benzamid-hydrochlorid
f) (2R,4S,5S,7R,2'S)-N-{4-Amino-5-hydroxy-2-isopropyl-8-methyl-7-[(5'-oxo-pyrrolidin-2'-ylmethyl)-carbamoyl]-nonyl}-2-(4-methoxybutoxy)-benzamid-hydrochlorid
g) (2R,4S,5S,7R,2'R)-N-{4-Amino-5-hydroxy-2-isopropyl-8-methyl-7-[(5'-oXo-pyrrolidin-2'-ylmethyl)-carbamoyl]-nonyl}-2-(4-methoxybutoxy)-benzamid-hydrochlorid
h) (2R,4S,5S,7R)-N-{4-Amino-5-hydroxy-2-isopropyl-8-methyl-7-[2-methyl-2-(morpholin-4-yl)-propylcarbamoyl]-nonyl}-2-(4-methoxybutoxy)-benzamid-dihydrochlorid
i) (2R,4S,5S,7R,'R)-N-[4-Amino-5-hydroxy-2-isopropyl-8-methyl-7-(1'-methyl-2-methylcarbamoyl-ethylcarbamoyl)-nonyl]-2-(4-methoxybutoxy)-benzamid-hydrochlorid
j) (2R,4S,5S,7R,'S)-N-[4-Amino-5-hydroxy-2-isopropyl-8-methyl-7-(1'-methyl-2-methylcarbamoyl-ethylcarbamoyl)-nonyl]-2-(4-methoxybutoxy)-benzamid-hydrochlorid
k) (2R,4S,5S,7R,2'S)-N-[4-Amino-5-hydroxy-2-isopropyl-7-(2'-carbamoyl-propylcarbamoyl)-8-methyl-nonyl]-2-(4-methoxybutoxy)-benzamid-hydrochlorid
l) (2R,4S,55,7R,2'R)-N-[4-Amino-5-hydroxy-2-isopropyl-7-(2'-carbamoyl-propylcarbamoyl)-8-methyl-nonyl]-2-(4-methoxybutoxy)-benzamid-hydrochlorid
m) (2R,4S,5S,7R)-N-[4-Amino-5-hydroxy-2-isopropyl-7-(dimethylcarbamoylmethylcarbamoyl)-8-methyl-nonyl]-2-(4-methoxybutoxy)-benzamid-hydrochlorid
n) (2S,4S,5S,7S,1'S)-N-{4-Amino-7-[1'-methyl-2'-(morpholin-4-yl)-2'-oxo-ethylcarbamoyl]-5-hydroxy-2-isopropyl-8-methyl-nonyl}-2-(4-methoxybutoxy)-benzamid-hydrochlorid
o) (2S,4S,5S,7S,2R')-N-[4-Amino-7-(2'-methyl-2'-methylcarbamoyl-propylcarbamoyl)-5-hydroxy-2-isopropyl-8-methyl-nonyl]-2-(4-methoxybutoxy)-benzamid-hydrochlorid
p) (2S,4S,5S,7S)-3-{5-Amino-4-hydroxy-7-[2-(4-methoxybutoxy)-benzoylamino)-methyl]-2-isopropyl-8-methyl-nonanoylamino}-2,2-dimethyl-propionsäure
q) (2S,4S,5S,7S)-N-[4-Amino-7-(3-methoxypropylcarbamoyl)-5-hydroxy-2-isopropyl-8-methyl-nonyl]-2-(4-methoxybutoxy)-benzamid-hydrochlorid
r) (2S,4S,5S,7S,1'S,2'S)-N-[4-Amino-7-(1'-carbamoyl-2'-methyl-butylcarbamoyl)-5-hydroxy-2-isopropyl-8-methyl-nonyl]-2-(4-methoxybutoxy)-benzamid-hydrochlorid
s) (2S,4S,5S,7S)-N-[4-Amino-7-(3-methylcarbamoyl-propylcarbamoyl)-5-hydroxy-2-isopropyl-8-methyl-nonyl]-2-(4-methoxybutoxy)-benzamid-hydrochlorid
t) (2R,4S,5S,7R,1'R)-N-[4-Amino-5-hydroxy-2-isopropyl-8-methyl-7-(1'-methyl-2'-carbamoyl-ethylcarbamoyl)-nonyl]-2-(4-methoxybutoxy)-benzamid-hydrochlorid
u) (2R,4S,5S,7R,1'S)-N-[4-Amino-5-hydroxy-2-isopropyl-8-methyl-7-(1'-methyl-2'-carbamoyl-ethylcarbamoyl)-nonyl]-2-(4-methoxybutoxy)-benzamid-hydrochlorid
v) (2S,4S,5S,7S,1'R)-N-[4-Amino-7-(1'-isopropyl-2'-carbamoyl-ethylcarbamoyl)-5-hydroxy-2-isopropyl-8-methyl-nonyl]-2-(4-methoxybutoxy)-benzamid-hydrochlorid
w) (2S,4S,5S,7S)-N-[4-Amino-7-(3-dimethylcarbamoyl-propylcarbamoyl)-5-hydroxy-2-isopropyl-8-methyl-nonyl]-2-(4-methoxybutoxy)-benzamid-hydrochlorid
x) (2S,4S,5S,7S,2S')-N-[4-Amino-7-(2'-methylcarbamoyl-propylcarbamoyl)-5-hydroxy-2-isopropyl-8-methyl-nonyl]-2-(4-methoxybutoxy)-benzamid-hydrochlorid

### Beispiel 67: Gelatine-Lösung:

Eine sterilfiltrierte wässrige Lösung mit 20 % Cyclodextrinen als Lösungsvermittler von einer der in den vorausgehenden Beispielen genannten Verbindungen der Formel I als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

| | |
|---|---|
| Wirkstoff | 3 mg |
| Gelatine | 150.0 mg |
| Phenol | 4.7 mg |
| dest. Wasser mit 20 % Cyclodextrinen als Lösungsvermittler | 1.0 ml |

### Beispiel 68: Sterile Trockensubstanz zur Injektion:

Man löst 5 mg einer der in den vorausgehenden Beispielen genannten Verbindungen der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 69: Nasenspray:

In einer Mischung von 3.5 ml "Myglyol 812®'' und 0.08 g Benzylalkohol werden 500 mg fein gemahlenes (<5.0 µm) Pulver einer der in den vorausgehenden Beispielen genannten Verbindungen der Formel I als Wirkstoff suspendiert. Diese Suspension wird in einen Be hälter mit Dosierventil eingefüllt. Es werden 5.0 g "Freon 12 ®'' unter Druck durch das Ventil in einen Behälter abgefüllt. Durch Schütteln wird das "Freon ®'' in der Myglyol®-Benzylalkoholmischung gelöst. Dieser Spraybehälter enthält ca. 100 Einzeldosen, die einzeln appliziert werden können.

### Beispiel 70: Lacktabletten

Für die Herstellung von 10 000 Tabletten enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45° während 30 Minuten im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

## Patentansprüche

1. Verbindungen der Formel I 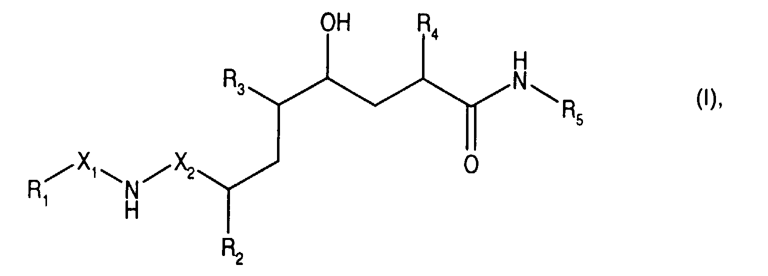 worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyddin-2-ylrest oder 1-R_{D}-Indol-3-ylrest bedeutet, wobei
einer der Reste R_{A} und R_{B} einen aliphatischen oder hetero cycloaliphatisch-aliphatischen Rest oder gegebenenfalls aliphatisch, araliphatisch oder heteroaraliphatisch verethertes Hydroxy und der andere Wasserstoff, einen aliphatischen Rest oder gegebenenfalls verestertes oder amidiertes Carboxy darstellt,
R_{C} Wasserstoff, einen aliphatischen Rest, gegebenenfalls aliphatisch, araliphatisch, heteroarylaliphatisch oder heterocycloaliphatisch-aliphatisch verethertes Hydroxy oder eine gegebenenfalls heteroaliphatisch substituierte Aminogruppe ist und
R_{D} für einen aliphatischen, araliphatischen oder heteroaliphatischen Rest steht, einer der Reste X₁ und X₂ Carbonyl und der andere Methylen darstellt,
R₂ ein aliphatischer Rest ist,
R₃ gegebenenfalls aliphatisch substituiertes Amino darstellt,
R₄ einen aliphatischen oder araliphatischen Rest bedeutet und
R₅ einen aliphatischen oder cycloaliphatisch-aliphatischen Rest oder einen gegebenenfalls hydrierten und/oder oxosubstituierten Heteroarylrest oder einen durch einen über ein C-Atom gebundenen und gegebenenfalls hydrierten und/oder oxosubstituierten Heteroaryl- oder Heteroaliphatylrest substituierten Niederalkylrest darstellt,
und ihre Salze.

2. Verbindungen gemäß Anspruch 1 der Formel I, worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyddin-2-ylrest oder 1-R_{D}-Indol-3-ylrest bedeutet, wobei
einer der Reste R_{A} und R_{B} Niederalkyl, Hydroxyniederalkyl, Niederalkanoyloxyniederalkyl, Niederalkoxyniederalkyl, Niederalkoxyniederalkoxyniederalkyl, gegebenenfalls N-niederalkanoyliertes, N-mono- oder N,N-diniederalkyliertes oder durch Niederalkylen, Hydroxy-, Niederalkoxy- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkyl, Hydroxy, Niederalkoxy, Hydroxyniederalkoxy, Niederalkanoyloxyniederalkoxy, Niederalkoxyniederalkoxy, Niederalkoxyniederalkoxyniederalkoxy, Polyhalogenniederalkoxy, Cyanniederalkoxy, gegebenenfalls substituiertes Phenyl- oder Pyridylniederalkoxy, Niederalkoxyniederalkenyloxy, gegebenenfalls S-oxidiertes Niederalkylthioniederalkoxy oder gegebenenfalls N-niederalkanoyliertes, N-mono- oder N,N-diniederalkyliertes oder durch Niederalkylen, Hydroxy-, Niederalkoxy- oder Niederalkoxyniederalkoxyniederalkylen oder gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkoxy und der andere Wasserstoff, Niederalkyl, Carbamoyl, Hydroxy, Niederalkoxy oder Polyhalogenniederalkoxy darstellt,
R_{C} Wasserstoff, Niederalkyl, Hydroxy, Niederalkoxy, Hydroxyniederalkoxy, Nieder alkoxyniederalkoxy, Morpholinoniederalkycarbamoylniederalkoxy, Niederalkoxyniederalkoxyniederalkyl, eine gegebenenfalls N-niederalkanoylierte, N-mono- oder N,N-diniederalkylierte oder durch Niederalkylen, Hydroxy-, Niederalkoxy-, Niederalkoxycarbonyl- oder Niederalkoxyniederalkoxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Amino-, Aminoniederalkyl- oder Aminonieder alkoxygruppe, eine gegebenenfalls amidierte Carboxy- oder Carboxyniederalkoxygruppe oder Tetrazolylniederalkoxy ist und
R_{D} für Niederalkyl, Hydroxyniederalkyl, Niederalkoxyniederalkyl, Niederalkoxyniederalkoxyniederalkyl, Hydroxyniederalkoxyniederalkyl, eine gegebenenfalls amidierte Carboxy- oder Carboxyniederalkylgruppe, eine gegebenenfalls substituierte Phenyl- oder Pyridylniederalkylgruppe steht,
einer der Reste X₁ und X₂ Carbonyl und der andere Methylen darstellt,
R₂ Niederalkyl ist,
R₃ gegebenenfalls N-niederalkanoyliertes oder N-mono- oder N,N-diniederalkyliertes Amino darstellt,
R₄ Niederalkyl oder Phenylniederalkyl bedeutet und
R₅ Niederalkyl, Cydoalkylniederalkyl, Hydroxyniederalkyl, Niederalkoxyniederalkyl, Niederalkanoyloxyniederalkyl, gegebenenfalls N-niederalkanoyliertes, N-mono- oder N,N-diniederalkyliertes oder durch Niederalkylen, Hydroxy-, Niederalkoxy-, Niederalkoxyniederalkyl- oder Niederalkanoyloxyniederalkylen, gegebenenfalls N'-niederalkanoyliertes, durch Niederalkoxycarbonyl oder Niederalkoxyniederalkyl N'-substituiertes bzw. N'-niederalkyliertes Azaniederalkylen, Oxaniederalkylen oder gegebenenfalls S-oxidiertes Thianiederalkylen N,N-disubstituiertes Aminoniederalkyl, gegebenenfalls verestertes oder amidiertes Carboxyniederalkyl, Cyanoniederalkyl, gegebenenfalls verestertes oder amidiertes Dicarboxyniederalkyl, gegebenenfalls verestertes oder amidiertes Carboxy-(hydroxy)niederalkyl, gegebenenfalls verestertes oder amidiertes Carboxycyl oalkylniederalkyl, Niederalkansulfonylniederalkyl, gegebenenfalls N-mono- oder N,N-diniederalkyliertes Thiocarbamoylniederalkyl, gegebenenfalls N-mono- oder N,N-diniederalkyliertes Sulfamoylniederalkyl oder einen gegebenenfalls hydrierten und/oder oxosubstituierten Heteroarylrest oder einen durch einen über ein C-Atom gebundenen und gegebenenfalls hydrierten und/oder oxosubstituierten Heteroarylrest substituiertes Niederalkyl darstellt,
und ihre Salze

3. Verbindungen gemäß Anspruch 1 der Formel I,
worin R₁ einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-Indol-3-ylrest bedeutet, wobei
einer der Reste R_{A} und R_{B} Niederalkyl, Hydroxyniederalkyl, Niederalkanoyloxyniederalkyl, Niederalkoxyniederalkyl, Niederalkoxyniederalkoxyniederalkyl, Aminoniederalkyl, Niederalkanoylaminoniederalkyl, Niederalkylaminoniederalkyl, Diniederalkylaminoniederalkyl, gegebenenfalls durch Hydroxy, Niederalkoxy oder Niederalkoxyniederalkyl substituiertes Piperidino- oder Pyrrolidinoniederalkyl, gegebenenfalls N'-niederalkyliertes, N'-niederalkanoyliertes oder durch Niederalkoxycarbonyl oder Niederalkoxyniederalkyl N'-substituiertes Piperazinoniederalkyl, gegebenenfalls niederalkyliertes Morpholinoniederalkyl, gegebenenfalls S-oxidiertes Thiomorpholinoniederalkyl, Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Niederalkoxyniederalkyl substituiertes Piperidino- oder Pyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, gegebenenfalls niederalkyliertes Morpholinoniederalkoxy, gegebenenfalls S-oxidiertes Thiomorpholinoniederalkoxy, Hydroxy, Niederalkoxy, Hydroxyniederalkoxy, Niederalkanoyloxyniederalkoxy, Niederalkoxyniederalkoxy, Niederalkoxyniederalkoxyniederalkoxy, Polyhalogenniederalkoxy, Cyanniederalkoxy, gegebenenfalls durch Niederalkyl, Niederalkoxy, Hydroxy, Nitro, Amino, Niederalkylamino, Diniederalkylamino, Halogen und/oder Trifluormethyl substituiertes Phenyl- oder Pyridylniederalkoxy, Niederalkoxyniederalkenyloxy, Niederalkylthioniederalkoxy, Niederalkansulfinylniederalkoxy, Niederalkansulfonylniederalkoxy, Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Niederalkoxyniederalkyl substituiertes Piperidino- oder Pyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, gegebenenfalls niederalkyliertes Morpholinoniederalkoxy oder gegebenenfalls S-oxidiertes Thiomorpholinoniederalkoxy und der andere Wasserstoff, Carbamoyl, Hydroxy, Niederalkoxy oder Polyhalogenniederalkoxy darstellt,
R_{C} Wasserstoff, Niederalkyl, Niederalkoxyniederalkoxyniederalkyl, Aminoniederalkyl, Niederalkanoylaminoniederalkyl, Niederalkylaminoniederalkyl, Diniederalkyl aminoniederalkyl, gegebenenfalls durch Hydroxy, Niederalkoxy oder Niederalkoxyniederalkyl substituiertes Piperidino- oder Pyrrolidinoniederalkyl, gegebenenfalls N'-niederalkyliertes, N'-niederalkanoyliertes oder durch Niederalkoxycarbonyl oder Niederalkoxyniederalkyl N'-substituiertes Piperazinoniederalkyl, gegebenenfalls niederalkyliertes Morpholinoniederalkyl, gegebenenfalls S-oxidiertes Thiomorpholinoniederalkyl, Diniederalkylamino, eine gegebenenfalls durch Hydroxy, Niederalkoxy oder Niederalkoxyniederalkyl substituiertes Piperidino- oder Pyrrolidinogruppe, gegebenenfalls N'-niederalkyliertes, N'-niederalkanoyliertes oder durch Niederalkoxycarbonyl oder Niederalkoxyniederalkyl N'-substituiertes Piperazino, gegebenenfalls niederalkyliertes Morpholino, gegebenenfalls S-oxidiertes Thiomorpholino, Hydroxy, Niederalkoxy, Hydroxyniederalkoxy, Niederalkoxyniederalkoxy, Morpholinoniederalkycarbamoylniederalkoxy, Aminoniederalkoxy, Niederalkanoylaminoniederalkoxy, Niederalkylaminoniederalkoxy, Diniederalkylaminoniederalkoxy, gegebenenfalls durch Hydroxy, Niederalkoxy oder Niederalkoxyniederalkyl substituiertes Piperidino- oder Pyrrolidinoniederalkoxy, gegebenenfalls N'-niederalkyliertes, N'-niederalkanoyliertes oder durch Niederalkoxycarbonyl oder Niederalkoxyniederalkyl N'-substituiertes Piperazinoniederalkoxy, gegebenenfalls niederalkyliertes Morpholinoniederalkoxy, gegebenenfalls S-oxidiertes Thiomorpholinoniederalkoxy, Carboxyniederalkoxy, Carbamoylniederalkoxy, Niederalkylcarbamoylniederalkoxy, Diniederalkylcarbamoylniederalkoxy, gegebenenfalls durch Hydroxy, Niederalkoxy oder Niederalkoxyniederalkyl substituiertes Piperidino- oder Pyrrolidinocarbonylniederalkoxy, gegebenenfalls N'-niederalkyliertes, N'-niederalkanoyliertes oder durch Niederalkoxycarbonyl oder Niederalkoxyniederalkyl N'-substituiertes Piperazinocarbonylniederalkoxy, gegebenenfalls niederalkyliertes Morpholinocarbonylniederalkoxy, gegebenenfalls S-oxidiertes Thiomorpholinocarbonylniederalkoxy, Tetrazolylniederalkoxy, Carboxy, Carbamoyl, Niederalkylcarbamoyl oder Diniederalkylcarbamoyl ist und
R_{D} für Niederalkyl, Hydroxyniederalkyl, Niederalkoxyniederalkyl, Niederalkoxyniederalkoxyniederalkyl, Hydroxyniederalkoxyniederalkyl, Carboxy, Niederalkoxycarbonyl, Carboxyniederalkyl, Niederalkoxycarbonylniederalkyl, Carbamoylniederalkyl, Niederalkylcarbamoylniederalkyl, Diniederalkylcarbamoylniederalkyl, gegebenenfalls durch Hydroxy, Niederalkoxy oder Niederalkoxyniederalkyl substituiertes Piperidino- oder Pyrrolidino carbonylniederalkyl, gegebenenfalls N'-niederalkyliertes, N'-niederalkanoyliertes oder durch Niederalkoxycarbonyl oder Niederalkoxyniederalkyl N'-substituiertes Piperazinocarbonylniederalkyl, gegebenenfalls niederalkyliertes Morpholinocarbonylniederalkyl, gegebenenfalls S-oxidiertes Thiomorpholinocarbonylniederalkyl, Carboxyniederalkyl, Niederalkoxycarbonylniederalkyl oder eine gegebenenfalls durch Niederalkyl, Niederalkoxy, Hydroxy, Nitro, Amino, Niederalkylamino, Diniederalkylamino, Halogen und/oder Trifluormethyl substituierte Phenyl- oder Pyddylniederalkylgruppe steht, einer der Reste X₁ und X₂ Carbonyl und der andere Methylen darstellt, R₂ Niederalkyl ist, R₃ Amino, Niederalkanoylamino, Niederalkylamino oder Diniederalkylamino darstellt, R₄ Niederalkyl oder Phenylniederalkyl bedeutet und R₅ Niederalkyl, Cydoalkylniederalkyl, Hydroxyniederalkyl, Niederalkoxyniederalkyl, Niederalkanoyloxyniederalkyl, gegebenenfalls durch Hydroxy, Niederalkoxy oder Niederalkoxyniederalkyl substituiertes Piperidino- oder Pyrrolidinocarbonylniederalkyl, gegebenenfalls N'-niederalkyliertes, N'-niederalkanoyliertes oder durch Niederalkoxycarbonyl oder Niederalkoxyniederalkyl N'-substituiertes Piperazinocarbonylniederalkyl, gegebenenfalls niederalkyliertes Morpholinocarbonylniederalkyl, gegebenenfalls S-oxidiertes Thiomorpholinocarbonylniederalkyl, Carboxyniederalkyl,
Niederal koxycarbonylniederalkyl, Carbamoylniederalkyl, Niederalkylcarbamoylniederalkyl, Diniederalkylcarbamoylniederalkyl, gegebenenfalls durch Hydroxy, Niederalkoxy oder Niederalkoxyniederalkyl substituiertes Piperidino- oder Pyrrolidinocarbonylniederalkyl, gegebenenfalls N'-niederalkyliertes, N'-niederalkanoyliertes oder durch Niederalkoxycarbonyl oder Niederalkoxyniederalkyl N'-substituiertes Piperazinocarbonylniederalkyl, gegebenenfalls niederalkyliertes Morpholinocarbonylniederalkyl, gegebenenfalls S-oxidiertes Thiomorpholinocarbonylniederalkyl,
Cyanoniederalkyl, Dicarboxyniederalkyl, Niederalkoxycarbonyl(carboxy)niederalkyl, Diniederalkoxycarbonylniederalkyl, Dicarbamoylniederalkyl, Carbamoyl(carboxy)niederalkyl, Di(niederalkylcarbamoyl)niederalkyl, Di(diniederalkylcarbamoyl)niederalkyl, Carboxy(hydroxy)niederalkyl, Niederalkoxycarbonyl(hydroxy)niederalkyl, Carbamoyl(hydroxy)niederalkyl, Niederalkylcarbamoyl(hydroxy)niederalkyl oder Diniederalkyl carbamoyl(hydroxy)niederalkyl, Carboxycycloalkylniederalkyl, Niederalkoxycarbonyl cycloalkylniederalkyl, Carbamoylcycloalkylniederalkyl, Niederalkylcarbamoylcydoalkylniederalkyl, Diniederalkylcarbamoylcydoalkylniederalkyl Niederalkansulfonylniederalkyl, Thiocarbamoyl niederalkyl, N-Niederalkylthiocarbamoylniederalkyl oder N,N-Diniederalkylthiocarbamoylniederalkyl, Sulfamoylniederalkyl, Niederalkylsulfamoylniederalkyl oder Diniederalkyl sulfamoylniederalkyl, gegebenenfalls oxosubstituiertes Pyrrolidinyl, Imidazolyl, Benzimidazolyl, Oxadiazolyl, Pyridyl, Oxopiperidinyl, Dioxopiperidinyl, Oxothiazolyl, Oxo-oxazolinyl oder Chinolinyl, gegebenenfalls oxosubstituiertes Pyrrolidinylniederalkyl, Imidazolylniederalkyl, Benzimidazolylniederalkyl, Oxadiazolylniederalkyl, Pyridylniederalkyl, Oxopiperidinylniederalkyl, Dioxopiperidinylniederalkyl, Oxothiazolylniederalkyl, Oxo-oxazolinylniederalkyl oder Chinolinylniederalkyl, Morpholinocarbonylniederalkyl oder gegebenenfalls N-niederalkanoyliertes Piperidylniederalkyl oder gegebenenfalls N-nieder alkanoyliertes Piperidyl, darstellt, und ihre Salze.

4. Verbindungen gemäß Anspruch 1 der Formel I, worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyridin-2-ylrest oder 1-R_{D}-Indol-3-ylrest bedeutet, wobei
einer der Reste R_{A} und R_{B} C₁-C₄-Alkyl, Hydroxy-C₁-C₄-alkyl, C₁-C₄-Alkanoyloxy-C₁-C₄-alkyl, C₁-C₄-Alkoxy-C₁-C₄-alkyl, C₁-C₄-Alkoxy-C₁-C₄-alkoxy-C₁-C₄-alkyl, Amino-C₁-C₄-alkyl, C₁-C₄-Alkanoylamino-C₁-C₄-alkyl, C₁-C₄-Alkylamino-C₁-C₄-alkyl, Di-C₁-C₄-alkylamino-C₁-C₄-alkyl, Piperidino-C₁-C₄-alkyl, Hydroxypiperidino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, C₁-C₇-Alkoxy, wie Propyloxy, Amino-C₁-C₇-alkoxy, C₁-C₄-Alkanoylamino-C₁-C₄-alkoxy, C₁-C₄-Alkylaminc-C₁-C₄-alkoxy, Di-C₁-C₄-alkylamino-C₁-C₄-alkoxy, Piperidino-C₁-C₄-alkoxy, Hydroxypipendino-C₁-C₄-alkoxy, C₁-C₄-Alkoxypiperidino-C₁-C₄-alkoxy, C₁-C₄-Alkoxy-C₁-C₄-alkoxypeddino-C₁-C₄-alkoxy, Pyrrolidino-C₁-C₄-alkoxy, Hydroxypyrrolidino-C₁-C₄-alkoxy, C₁-C₄-Alkoxypyrrolidino-C₁-C₄-alkoxy, C₁-C₄-Alkoxy-C₁-C₄-alkoxypyrrolidino-C₁-C₄-alkoxy, Piperazino-C₁-C₄-alkoxy, N'-C₁-C₄-Alkylpiperazino-C₁-C₄-alkoxy, N'-C₁-C₄-Alkanoylpiperazino-C₁-C₄-alkoxy, N'-C₁-C₄-Alkoxycarbonylpiperazino-C₁-C₄-alkoxy, N'-C₁-C₄-Alkoxy-C₁-C₄-alkylpiperazino-C₁-C₄-alkoxy, Morpholino-C₁-C₄-alkoxy oder C₁-C₄-Alkylmorpholino-C₁-C₄-alkoxy, Thiomorpholino-C₁-C₄-alkoxy, S-Oxythiomorpholino-C₁-C₄-alkoxy, S,S-Dioxythiomorpholino-C₁-C₄-alkoxy, Hydroxy, Hydroxy-C₁-C₄-aSkoxyl C₁-C₄-Alkanoyloxy-C₁-C₄-alkoxy, C₁-C₄-Alkoxy-C₁-C₄-alkoxy, C₁-C₄-Alkoxy-C₁-C₄-alkoxy-C₁-C₄-alkoxy, Polyhalogen-C₁-C₄-alkoxy, Cyan-C₁-C₄-alkoxy, Carbamoyl C₁-C₄-alkoxy, gegebenenfalls durch C₁-C₄-Alkyl, C₁-C₄-Alkoxy, Hydroxy, Nitro, Amino, C₁-C₄-Alkyamino, Di-C₁-C₄-alkylamino, Halogen und/oder Trifluormethyl substituiertes Phenyl- oder Pyridyl-C₁-C₄-alkoxy, C₁-C₄-Alkoxy-C₁-C₄-alkenyloxy, C₁-C₄-Alkylthio-C₁-C₄-alkoxy, C₁-C₄-Alkansulfinyl-C₁-C₄-alkoxy, C₁-C₄-Alkansulfonyl-C₁-C₄-alkoxy, Amino-C₁-C₇-alkoxy, C₁-C₄-Alkanoylamino-C₁-C₄-alkoxy, C₁-C₄-Alkylamino-C₁-C₄-alkoxy, Di-C₁-C₄-alkylamino-C₁-C₄-alkoxy, Piperidino-C₁-C₄-alkoxy, Hydroxypiperidino-C₁-C₄-alkoxy, C₁-C₄-Alkoxypipeddino-C₁-C₄-alkoxy, C₁-C₄-Alkoxy-C₁-C₄-alkoxyperidino-C₁-C₄-alkoxy, Pyrrolidino-C₁-C₄-alkoxy, Hydroxypyrrolidino-C₁-C₄-alkoxy, C₁-C₄-Alkoxypyrrolidino-C₁-C₄-alkoxy, C₁-C₄-Alkoxy-C₁-C₄-alkoxypyrrolidino-C₁-C₄-alkoxy, Piperazino-C₁-C₄-alkoxy, N'-C₁-C₄-Alkylpiperazino-C₁-C₄-alkoxy, N'-C₁-C₄-Alkanoylpiperazino-C₁-C₄-alkoxy, N'-C₁-C₄-Alkoxycarbonylpiperazino-C₁-C₄-alkoxy, N'-C₁-C₄-Alkoxy-C₁-C₄-alkylpiperazino-C₁-C₄-alkoxy, Morpholino-C₁-C₄-alkoxy oder C₁-C₄-Alkylmorpholino-C₁-C₄-alkoxy oder Thiomorpholino-C₁-C₄-alkoxy und der andere Wasserstoff, Carbamoyl, C₁-C₄-Alkyl, Hydroxy, C₁-C₄-Alkoxy oder Trihalogen-C₁-C₄-alkoxy darstellt,
R_{C} Wasserstoff, Hydroxy, Di-C₁-C₄-Alkylamino, Piperidino, Pyrrolidino, Morpholino, Thiomorpholino, S-Oxythiomorpholino, S,S-Dioxythiomorpholino, C₁-C₄-Alkoxy, Hydroxy-C₁-C₄-alkoxy, C₁-C₄-Alkoxy-C₁-C₄-alkoxy, Morpholino-C₁-C₄-alkylcarbamoyl-_{C}1-_{C}4-alkoxy, C₁-C₄-Alkoxy-C₁-C₄-alkoxy-C₁-C₄-alkyl, Amino-C₁-C₄-alkyl, C₁-C₄-Alkanoylamino-C₁-C₄-alkyl, C₁-C₄-Alkylamino-C₁-C₄-alkyl, Di-C₁-C₄-alkylamino-C₁-C₄-alkyl, gegebenenfalls durch Hydroxy, C₁-C₄-Alkoxy oder C₁-C₄-Alkoxy-C₁-C₄-alkyl substituiertes Piperidino- oder Pyrrolidino-C₁-C₄-alkyl, Amino-C₁-C₄-alkyl, C₁-C₄-Alkanoylamino-C₁-C₄-alkyl, C₁-C₄-Alkylamino-C₁-C₄-alkyl, Di-C₁-C₄-Alkylamino-C₁-C₄-alkyl, Pipeddino-C₁-C₄-alkyl, Hydroxypipeddino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C4-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, Amino-C₁-C₇-alkoxy, C₁-C₄-Alkanoylamino-C₁-C₄-alkoxy, C₁-C₄-Alkylamino-C₁-C₄-alkoxy, Di-C₁-C₄-alkylamino-C₁-C₄-alkoxy, Piperidino-C₁-C₄-alkoxy, Hydroxypiperidino-C₁-C₄-alkoxy, C₁-C₄-Alkoxypiperidino-C₁-C₄-alkoxy, C₁-C₄-Alkoxy-C₁-C₄-alkoxypeddino-C₁-C₄-alkoxy, Pyrrolidino-C₁-C₄-alkoxy, Hydroxypyrrolidino-C₁-C₄-alkoxy, C₁-C₄-Alkoxypyrrolidino-C₁-C₄-alkoxy, C₁-C₄-Alkoxy-C₁-C₄-alkoxypyrrolidino-C₁-C₄-alkoxy, Piperazino-C₁-C₄-alkoxy, N'-C₁-C₄-Alkylpiperazino-C₁-C₄-alkoxy, N'-C₁-C₄-Alkanoylpiperazino-C₁-C₄-alkoxy, N'-C₁-C₄-Alkoxyearbonylpiperazino-C₁-C₄-alkoxy, N'-C₁-C₄-Alkoxy-C₁-C₄-alkylpiperazino-C₁-C₄-alkoxy, Morpholino-C₁-C₄-alkoxy oder C₁-C₄-Alkylmorpholino-C₁-C₄-alkoxy, Thiomorpholino-C₁-C₄-alkoxy, S-Oxythiomorpholino-C₁-C₄-alkoxy, S,S-Dioxythiomorpholino-C₁-C₄-alkoxy, Carboxy-C₁-C₄-aSkoxyl Carbamoyl-C₁-C₄-alkoxy, C₁-C₄-Alkylcarbamoyl-C₁-C₄-alkoxy, Di-C₁-C₄-alkylcarbamoyl-C₁-C₄-alkoxy, Di-C₁-C₄-alkylamino-C₁-C₄-alkoxy, Piperidinocarbonyl-C₁-C₄-alkoxy, Hydroxypiperidinocarbonyl-C₁-C₄-alkoxy, C₁-C₄-Alkoxypiperidinocarbonyl-C₁-C₄-alkoxy, C₁-C₄-Alkoxy-C₁-C₄-alkoxyperidinocarbonyl-C₁-C₄-alkoxy, Pyrrolidinocarbonyl-C₁-C₄-alkoxy, Hydroxypyrrolidinocarbonyl-C₁-C₄-alkoxy, C₁-C₄-Alkoxypyrrolidinocarbonyl-C₁-C₄-alkoxy, C₁-C₄-Alkoxy-C₁-C₄-alkoxypyrrolidinocarbonyl-C₁-C₄-alkoxy, Piperazinocarbonyl-C₁-C₄-alkoxy, N'-C₁-C₄-Alkylpiperazinocarbonyl-C₁-C₄-alkoxy, N'-C₁-C₄-Alkanoylpiperazinocarbonyl-C₁-C₄-alkoxy, N'-C₁-C₄-Alkoxycarbonylpiperazinocarbonyl oder N'-C₁-C₄-Alkoxy-C₁-C₄-alkylpiperazinocarbonyl-C₁-C₄-alkoxy, Morpholinocarbonyl-C₁-C₄-alkoxy, C₁-C₄-Alkylmorpholinocarbonyl-C₁-C₄-alkoxy, Thiomorpholinocarbonyl-C₁-C₄-alkoxy, S-Oxythiomorpholinocarbonyl, S,S-Dioxythiomorpholinocarbonyl-C₁-C₄-alkoxy, Tetrazolyl-C₁-C₄-alkoxy, Carboxy, Carbamoyl, C₁-C₄-Alkylcarbamoyl ist und
R_{D} für C₁-C₄-Alkyl, Hydroxy-C₁-C₄-alkyl, C₁-C₄-Alkoxy-C₁-C₄-alkyl, C₁-C₄-Alkoxy-C₁-C₄-alkoxyC₁-C₄-alkyl, Hydroxy-C₁-C₄-alkoxy-C₁-C₄-alkyl, Carboxy, C₁-C₄-Alkoxycarbonyl, Carboxy-C₁-C₄-alkyl, C₁-C₄-Alkoxycarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Piperidino-C₁-C₄-alkyl, Hydroxypiperidino-C₁-C₄-alkyl, C₁-C₄-Alkoxypiperidino-C₁-C₄-alkyl, C₁-C₄-Alkoxy-C₁-C₄-alkoxypeddino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, Carboxy-C₁-C₄-alkyl, C₁-C₄-Alkoxycarbonyl-C₁-C₄-alkyl, unsubstituiertes oder durch C₁-C₄-Alkkyl, C₁-C₄-Alkoxy, Hydroxy, Nitro, Amino, C₁-C₄-Alkylamino, Di-C₁-C₄-alkylamino, Halogen und/oder Trifluormethyl substituiertes Phenyl-C₁-C₄-alkyl oder Pyridyl-C₁-C₄-alkyl steht,
einer der Reste X₁ und X₂ Carbonyl und der andere Methylen darstellt,
R₂ C₁-C₄-Alkyl ist,
R₃ Amino, C₁-C₄-Alkanoylamino, C₁-C₄-Alkylamino oder Di-C₁-C₄-alkylamino darstellt,
R₄ C₁-C₄-Alkyl oder Phenyl-C₁-C₄-alkyl bedeutet und
R₅ C₁-C₄-Alkyl, Cycloalkyl-C₁-C₄-alkyl, Hydroxy-C₁-C₄-alkyl, C₁-C₄-Alkoxy-C₁-C₄-alkyl, C₁-C₄-Alkanoyloxy-C₁-C₄-alkyl, Piperidino-C₁-C₄-alkyl, Hydroxypipeddino-C₁-C₄-alkyl, C₁-C₄-Alkoxypiperidino-C₁-C₄-alkyl, C₁-C₄-Alkoxy-C₁-C₄-alkoxyperidino-C₁-C₄-alkyl, C₁-C₄-Alkoxycarbonylpiperidino-C₁-C₄-alkyl, Pyrrolidino-C₁-C₄-alkyl, Hydroxypyrrolidino-C₁-C₄-alkyl, C₁-C₄-Alkoxypyrrolidino-C₁-C₄-alkyl, C₁-C₄-Alkoxy-C₁-C₄-alkoxypyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, N'-C₁-C₄-Alkoxycarbonylpiperazino-C₁-C₄-alkyl, N'-C₁-C₄-Alkoxy-C₁-C₄-alkylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, S-Oxythiomorpholino-C₁-C₄-alkyl, S,S-Dioxythiomorpholino-C₁-C₄-alkyl, Carboxy-C₁-C₄-alkyl, C₁-C₄-Alkoxycarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Piperidinocarbonyl-C₁-C₄-alkyl, Hydroxypiperidinocarbonyl-C₁-C₄-alkyl, C₁-C₄-Alkoxypiperidinocarbonyl-C₁-C₄-alkyl, C₁-C₄-Alkoxy-C₁-C₄-alkoxyperidinocarbonyl-C₁-C₄-alkyl, Pyrrolidinocarbonyl-C₁-C₄-alkyl, Hydroxypyrrolidinocarbonyl-C₁-C₄-alkyl, C₁-C₄-Alkoxypyrrolidinocarbonyl-C₁-C₄-alkyl, C₁-C₄-Alkoxy-C₁-C₄-alkoxypyrrolidinocarbonyl-C₁-C₄-alkyl, Piperazinocarbonyl-C₁-C₄-alkyl, N'-C₁-C₄-Alkylpiperazinocarbonyl-C₁-C₄-alkyl, N'-C₁-C₄-Alkanoylpiperazinocarbonyl-C₁-C₄-alkyl, N'-C₁-C₄-Alkoxycarbonylpiperazinocarbonyl, N'-C₁-C₄-Alkoxy-C₁-C₄-alkylpiperazinocarbonyl-C₁-C₄-alkyI, Morpholinocarbonyl-C₁-C₄-alkyl, C₁-C₄-Alkylmorpholinocarbonyl-C₁-C₄-alkyl, Thiomorpholinocarbonyl-C₁-C₄-alkyl, S-Oxythiornorpholinocarbonyl-C₁-C₄-alkyl, S,S-Dioxythiomorpholinocarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Cyano-C₁-C₄-alkyl, Dicarboxy-C₁-C₄-alkyl, C₁-C₄-Alkoxycarbonyl(carboxy)-C₁-C₄-alkyl, Di-C₁-C₄-alkoxycarbonyl-C₁-C₄-alkyl, Dicarbamoyl-C₁-C₄-alkyl, Carbamoyl(carboxy)-C₁-C₄-alkyl, Di(-C₁-C₄-alkylcarbamoyl)-C₁-C₄-alkyl, Di(di-C₁-C₄-alkylcarbamoyl)-C₁-C₄-alkyl, Carboxy(hydroxy)-C₁-C₄-alkyl, C₁-C₄-Alkoxycarbonyl(hydroxy)-C₁-C₄-alkyl, Carbamoyl(hydroxy)-C₁-C₄-alkyl, C₁-C₄-Alkylcarbamoyl(hydroxy)-C₁-C₄-alkyl oder Di-C₁-C₄-alkylcarbamoyl(hydroxy)-C₁-C₄-alkyl, Carboxycycloalkyl-C₁-C₄-alkyl, C₁-C₄-Alkoxycarbonylcydoalkyl-C₁-C₄-alkyl, Carbamoylcycloalkyl-C₁-C₄-alkyl, C₁-C₄-Alkylcarbamoylcydoalkyl-C₁-C₄-alkyl, Di-C₁-C₄-alkylcarbamoylcycloalkyl-C₁-C₄-alkyl, C₁-C₄-Alkansulfonyl-C₁-C₄-alkyl, Thiocarbamoyl-C₁-C₄-alkyl, N-C₁-C₄-Alkylthiocarbamoyl-C₁-C₄-alkyl oder N,N-Di-C₁-C₄-alkylthiocarbamoyl-C₁-C₄-alkyl, Sulfamoyl-C₁-C₄-alkyl, C₁-C₄-Alkylsulfamoyl-C₁-C₄-alkyl oder Di-C₁-C₄-alkylsulfamoyl-C₁-C₄-alkyl, gegebenenfalls oxosubstituiertes Pyrrolidinyl, Imidazolyl, Benzimidazolyl, Oxadiazolyl, Pyridyl, Oxopiperidinyl, Dioxopiperidinyl, Oxothiazolyl, Oxo-oxazolinyl oder Chinolinyl, gegebenenfalls oxosubstituiertes Pyrrolidinyl-C₁-C₄-alkyl, Imidazolyl-C₁-C₄-alkyl, Benzimidazolyl-C₁-C₄-alkyl, Oxadiazolyl-C₁-C₄-alkyl, Pyridyl-C₁-C₄-alkyl, Oxopiperidinyl-C₁-C₄-alkyl, Dioxopiperidinyl-C₁-C₄-alkyl, Oxothiazolyl-C₁-C₄-alkyl, Oxo-oxazolinyl-C₁-C₄-alkyl oder Chinolinyl-C₁-C₄-alkyl, Morpholinocarbonyl-C₁-C₄-alkyl oder gegebenenfalls N-C₁-C₄-alkanoyliertes Piperidyl-C₁-C₄-alkyl oder gegebenenfalls N-C₁-C₄-alkanoyliertes Piperidyl darstellt, und ihre Salze.

5. Verbindungen gemäß Anspruch 1 der Formel I, worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyddin-2-ylrest oder 1-R_{D}-Indol-3-ylrest bedeutet, wobei einer der Reste R_{A} und R_{B} C₁-C₄-Alkyl, C₁-C₄-Alkoxy-C₁-C₄-alkyl, Di-C₁-C₄-alkylamino-C₁-C₄-alkyl, Piperidino-C₁-C₄-alkyl, C₁-C₄-Alkanoylpiperidinyl-C₁-C₄-alkyl, C₁-C₄-Alkoxycarbonylpiperidino-C₁-C₄-alkyl, Pyrrolidino-C₁-C₄-alkyl, Piperazino-C₁-C₄-alkyl, N'-C₁-C₄-Alkylpiperazino-C₁-C₄-alkyl, N'-C₁-C₄-Alkanoylpiperazino-C₁-C₄-alkyl, Morpholino-C₁-C₄-alkyl, C₁-C₄-Alkylmorpholino-C₁-C₄-alkyl, Thiomorpholino-C₁-C₄-alkyl, Amino-C₁-C₇-alkoxy, C₁-C₄-Alkanoylamino-C₁-C₄-alkoxy, Di-C₁-C₄-alkylamino-C₁-C₄-alkoxy, Piperidino-C₁-C₄-alkoxy, Morpholino-C₁-C₄-alkoxy, Hydroxy, C₁-C₇-Alkoxy, C₁-C₄-Alkoxy-C₁-C₄-alkoxy, C₁-C₄-Alkoxy-C₁-C₄-alkoxy-C₁-C₄-alkoxy, C₁-C₄-Alkoxy-C₁-C₄-alkenyloxyy Amino-C₁-C₇-alkoxy, C₁-C₄-Alkanoylamino-C₁-C₄-alkoxy, Di-C₁-C₄-alkylamino-C₁-C₄-alkoxy, Piperidino-C₁-C₄-alkoxy, Morpholino-C₁-C₄-alkoxy, Carbamoyl oder Carbamoyl-C₁-C₄-alkoxy, und der andere Wasserstoff, C₁-C₄-Alkyl, Hydroxy, oder C₁-C₄-Alkoxy darstellt, Rc Wasserstoff, Hydroxy, C₁-C₄-Alkoxy, C₁-C₄-Alkoxy-C₁-C₄-alkoxy, Morpholino-C₁-C₄-alkylcarbamoyl-C₁-C₄-alkoxy, Di-C₁-C₄-alkylamino-C₁-C₄-alkyl, Piperidino-C₁-C₄-alkyl, C₁-C₄-Alkoxycarbonylpiperidino-C₁-C₄-alkyl, Pyrrolidino-C₁-C₄-alkyl, Piperazinocarbonyl-C₁-C₄-alkyl, N'-C₁-C₄-Alkylpiperazinocarbonyl-C₁-C₄-alkyl, N'-C₁-C₄-Alkanoylpiperazinocarbonyl-C₁-C₄-alkyl, Morpholino, Morpholino-C₁-C₄-alkyl, Thiomorpholino-C₁-C₄-alkyl, C₁-C₄-Alkoxy, Amino-C₁-C₄-alkoxy, C₁-C₄-Alkanoylamino-C₁-C₄-alkoxy, Di-C₁-C₄-alkylamino-C₁-C₄-alkoxy, Pipeddino-C₁-C₄-alkoxy, Morpholino-C₁-C₄-alkoxy, Morpholino-C₁-C₄-alkylcarbamoyl-C₁-C₄-alkoxy, Carboxy, Carbamoyl, C₁-C₄-Alkycarbamoyl, Carboxy-C₁-C₄-alkoxy, Carbamoyl-C₁-C₄-alkoxy, C₁-C₄-Alkylcarbamoyl-C₁-C₄-alkoxy, Di-C₁-C₄-alkylamino-C₁-C₄-alkoxy oder Tetrazolyl-C₁-C₄-alkoxyist und
R_{D} für C₁-C₄-Alkyl, C₁-C₄-Alkoxy-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Piperidino-C₁-C₄-alkyl oder C₁-C₄-Alkoxycarbonylpipeddino-C₁-C₄-alkyl steht,
einer der Reste X₁ und X₂ Carbonyl und der andere Methylen darstellt,
R₂ C₁-C₄-Alkyl ist,
R₃ Amino oder C₁-C₄-Alkanoylamino darstellt,
R₄ C₁-C₄-Alkyl bedeutet und
R₅ C₁-C₄-Alkyl, C₁-C₄-Alkoxy-C₁-C₄-alkyl, C₁-C₄-Alkoxycarbonylpiperidino-C₁-C₄-alkyl, Pyrrolidino-C₁-C₄-alkyl, N'-C₁-C₄-Alkylpiperazino-C₁-C₄-alkyl, N'-C₁-C₄-Alkoxycarbonylpiperazino-C₁-C₄-alkyl, oder N'-C₁-C₇-Alkanoylpiperazino-C₁-C₄-alkyl, Morpholino-C₁-C₄-alkyl, Thiomorpholino-C₁-C₄-alkyl, Morpholinocarbonyl-C₁-C₄-alkyl, Carbamoyl-C₁-C₄-alkyl, C₁-C₄-Alkylcarbamoyl-C₁-C₄-alkyl, Di-C₁-C₄-alkylcarbamoyl-C₁-C₄-alkyl, Piperidinocarbonyl-C₁-C₄-alkyl, Piperazinocarbonyl-C₁-C₄-alkyl, N'-C₁-C₄-Alkylpiperazinocarbonyl-C₁-C₄-alkyl, N'-C₁-C₄-Alkanoylpiperazinocarbonyl-C₁-C₄-alkyl, N'-C₁-C₄-Alkylpiperazinocarbonyl-C₁-C₄-alkyl oder Morpholinocarbonyl-C₁-C₄-alkyl, darstellt, und ihre Salze.

6. Verbindungen der Formel la 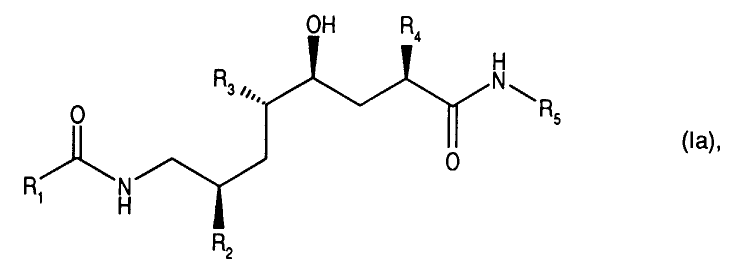 worin
R₁ einen 2-R_{A}-4-R_{C}-Phenylrest, 2-R_{A}-Pyridin-3-ylrest oder 3-R_{A}-Pyridin-2ylrest bedeutet, wobei
R_{A} Propyloxymethyl, 2-Morpholinoethyl, 3-Morpholinopropyl, N'-Acetylpiperazinomethyl, Propyloxy, 2-Methoxyethoxy, 3-Methoxypropyloxy, 4-Methoxybutyloxy, 5-Methoxypentyloxy, 4-Methoxy-but-2-enyloxy, 2-(Methoxymethoxy)ethoxy, 2-(2-Methoxyethoxy)ethoxy, 2-Aminoethoxy, 3-Aminopropyloxy, 3-Dimethylaminopropyloxy, Carbamoyl-C₁-C₄-alkoxy, wie 2-Carbamoylethoxy oder Carbamoyl bedeutet und
R_{C} Wasserstoff, Dimethylaminomethyl, Piperidinomethyl, Pyrrolidinomethyl, Morpholinomethyl, N'-Acetylpiperazinomethyl, N'-Methylpiperazinomethyl, 2-Morpholinoethoxy, 3-Morpholinopropyloxy, 2-Morpholinoethylcarbamoylmethoxy, 2-Piperidinoethoxy, Carboxy, Carbamoyl, Methylcarbamoyl, Carboxymethoxy, 3-Dimethylaminopropyloxy, Butylcarbamoylmethoxy oder Tetrazolylmethoxy darstellt,
X₁ für Carbonyl und X₂ für Methylen steht,
R₂ und R₄ unabhängig voneinander Methyl oder Isopropyl bedeuten,
R₃ für Amino steht und
R₅ Butyl, 2-Morpholinoethyl, 3-Morpholinopropyl, 2-Thiomorpholinoethyl, 2-Morpholinocarbonylethyl, 3-Carbamoylpropyl, 2-Carbamoyl-2-methyl-ethyl, 2-Methylcarbamoyl-2-methyl-ethyl, 2-Dimethylcarbamoylethyl, N'-Methylpiperazinomethyl, N'-Methoxycarbonylpiperazinomethyl oder N'-Acetylpiperazinomethyl bedeutet, und ihre Salze.

7. (2S,4S,5S,7R)-N-(4-Amino-7-butylcarbamoyl-5-hydroxy-2-isopropyl-octyl)-2-(3-methoxypropoxy)-benzamid;
(2S,4S,5S,7R)-N-(4-Amino-7-butylcarbamoyl-5-hydroxy-2-isopropyl-octyl)-3-methoxy-2-(3-methoxypropoxy)-benzamid;
(2S,4S,5S,7R)-N-(4-Amino-7-butylcarbamoyl-5-hydroxy-2-isopropyl-octyl)-4-methoxy-2-(3-methoxypropoxy)-benzamid;
(2S,4S,5S,7R)-N-(4-Amino-7-butylcarbamoyl-5-hydroxy-2-isopropyl-octyl)-3-(3-methoxypropoxy)-benzamid;
(2S,4S,5S,7R)-N-(7-Butylcarbamoyl-4-forrnylamino-5-hydroxy-2-isopropyl-octyl)-3-methoxy-2-(3-methoxypropoxy)-benzamid;
(2R,4S,5S,7R)-1-Benzyl-1H-indol-3-carbonsäure-N-(4-amino-7-butylcarbamoyl-5-hydroxy-2-isopropyl-octyl)-amid;
(2R,4S,5S,7R)-1-(2-Methoxyethyl)-1H-indol-3-carbonsäure-N-(4-amino-7-butylcarbamoyl-5-hydroxy-2-isopropyl-octyl)-amid;
(2R,4S,5S,7R)-1-Pyridin-2-yl-1H-indol-3-carbonsäure-N-(4-amino-7-butylcarbamoyl-5-hydroxy-2-isopropyl-octyl)-amid;
(2R,4S,5S,7R)-1-(2-Methoxybenzyl)-1H-indol-3-carbonsäure-N-(4-amino-7-butylcarbamoyl-5-hydroxy-2-isopropyl-octyl)-amid;
(2R,4S,5S,7R)-N-(4-Amino-7-butylcarbamoyl-5-hydroxy-2-isopropyl-octyl)-2-(3-methoxypropoxy)-benzamid;
(2R,4S,5S,7R)-N-(4-Amino-7-butylcarbamoyl-5-hydroxy-2-methyl-octyl)-2-(3-methoxypropoxy)-benzamid;
(2R,4S,5S,7R)-N-(4-Amino-7-butylcarbamoyl-5-hydroxy-2-methyl-octyl)-2-(3-methoxypropoxy)-benzamid;
(2S,4S,5S,7S)-N-(4-Amino-7-butylcarbamoyl-5-hydroxy-2-isopropyl-8-methyl-nonyl)-2-(3-methoxypropoxy)-benzamid;
(2S,4S,5S,7S)-N-(4-Amino-7-butylcarbamoyl-5-hydroxy-2-isopropyl-8-methyl-nonyl)-2-(4-methoxybutoxy)-benzamid;
(2S,4S,5S,7S)-N-(4-Amino-7-butylcarbamoyl-5-hydroxy-2-isopropyl-8-methyl-nonyl)-2-propoxy-benzamid;
(2S,4S,5S,7S)-N-(4-Amino-7-butylcarbamoyl-5-hydroxy-2-isopropyl-8-methyl-nonyl)-2-(2-methoxyethoxy)-benzamid;
(2S,4S,5S,7S)-N-(4-Amino-7-butylcarbamoyl-5-hydroxy-2-isopropyl-8-methyl-nonyl)-2-[2-(2-methoxyethoxy)-ethoxy]-benzamid;
(2S,4S,5S,7S)-N-(4-Amino-7-butylcarbamoyl-5-hydroxy-2-isopropyl-8-methyl-nonyl)-4-methoxy-2-(3-methoxypropoxy)-benzamid;
(2S,4S,5S,7S)-N-(4-Amino-7-butylcarbamoyl-5-hydroxy-2-isopropyl-8-methyl-nonyl)-4-methoxy-3-(3-methoxypropoxy)-benzamid;
(2S,4S,5S,7S)-N-(4-Amino-7-butylcarbamoyl-5-hydroxy-2-isopropyl-8-methyl-nonyl)-2-(propoxymethyl)-benzamid;
(2S,4S,5S,7S)-N-(4-Amino-7-butylcarbamoyl-5-hydroxy-2-isopropyl-8-methyl-nonyl)-2-acetamido-benzamid;
(2S,4S,5S,7S)-N-(4-Amino-7-butylcarbamoyl-5-hydroxy-2-isopropyl-8-methyl-nonyl)-2-[2-(acetamido)-ethoxy]-benzamid;
(2S,4S,5S,7S)-N-(4-Amino-7-butylcarbamoyl-5-hydroxy-2-isopropyl-8-methyl-nonyl)-2-(4-methoxybut-2-enoxy)-benzamid;
(2S,4S,5S,7S)-N-(4-Amino-7-butylcarbamoyl-5-hydroxy-2-isopropyl-8-methyl-nonyl)-2-(4-methoxybutoxy)-4-methyl-benzamid;
(2S,4S,5S,7S)-N-[4-Amino-7-butylcarbamoyl-5-hydroxy-2-isopropyl-8-methyl-nonyl]-2-(3-methoxypropoxy)-nicotinamid;
(2S,4S,5S,7S)-N-[4-Amino-7-butylcarbamoyl-5-hydroxy-2-isopropyl-8-methyl-nonyl]-3-(4-methoxybutoxy)-pyridin-2-carbonsäureamid;
(2S,4S,5S,7S)-N-(4-Amino-7-butylcarbamoyl-5-hydroxy-2-isopropyl-8-methyl-nonyl)-2-hydroxy-benzamid;
(2S,4S,5S,7S)-N-(4-Amino-7-butylcarbamoyl-5-hydroxy-2-isopropyl-8-methyl-nonyl)-2-[2-(methoxymethoxy)-ethoxy]-benzamid;
(2S,4S,5S, 7S)-N-[4-Amino-5-hydroxy-2-isopropyl-8-methyl-7-(2-morpholin-4-ylethylcarbamoyl)-nonyl]-2-(3-methoxypropoxy)-benzamid;
(2S,4S,5S,7S)-N-[4-Amino-5-hydroxy-2-isopropyl-8-methyl-7-(2-morpholin-4-ylethylcarbamoyl)-nonyl]-2-(4-methoxybutoxy)-benzamid;
(2S,4S,5S,7S)-N-[4-Amino-5-hydroxy-2-isopropyl-8-methyl-7-(2-morpholin-4-ylethylcarbamoyl)-nonyl]-2-(2-methoxyethoxy)-benzamid;
(2S,4S,5S,7S)-N-[4-Amino-5-hydroxy-2-isopropyl-8-methyl-7-(2-morpholin-4-yl-ethylcarbamoyl)-nonyl]-2-(3-methoxypropoxy)-nicotinamid;
(2S,4S,5S,7S)-N-[4-Amino-5-hydroxy-2-isopropyl-8-methyl-7-(2-morpholin-4-yl-ethylcarbamoyl)-nonyl]-3-(4-methoxybutoxy)-pyridin-2-carbonsäureamid;
(2S,4S,5S,7S)-N-[4-Amino-5-hydroxy-2-isopropyl-8-methyl-7-(2-morpholin-4-ylethylcarbamoyl)-nonyl]-2-(4-methoxybut-2-enoxy)-benzamid;
(2S,4S,5S,7S)-N-[4-Amino-5-hydroxy-2-isopropyl-8-methyl-7-(2-morpholin-4-ylethylcarbamoyl)-nonyl]-2-(4-methoxybutoxy)-4-methyl-benzamid;
(2S,4S,5S,7S)-N-[4-Amino-5-hydroxy-2-isopropyl-8-methyl-7-(2-morpholin-4-ylethylcarbamoyl)-methyl-nonyl]-2-(5-methoxypentoxy)-benzamid;
(2S,4S,5S,7S)-N-[4-Amino-5-hydroxy-2-isopropyl-8-methyl-7-(3-morpholin-4-ylpropylcarbamoyl)-nonyl]-2-(4-methoxybutoxy)-benzamid;
(2S,4S,5S,7S)-N-(4-Amino-7-butylcarbamoyl-5-hydroxy-2-isopropyl-8-methyl-nonyl)-2-(4-methoxybutoxy)-4-(morpholin-4-ylmethyl)-benzamid;
(2S,4S,5S,7S)-N-(4-Amino-7-butyl carbamoyl-5-hydroxy-2-isopropyl-8-methyl-nonyl)-2-(4-methoxybutoxy)-4-[2-(morpholin-4-yl)-ethoxy]-benzamid;
(2S,4S,5S,7S)-N-(4-Amino-7-butylcarbamoyl-5-hydroxy-2-isopropyl-8-methyl-nonyl)-4-[3-(dimethylamino)-propoxy]-2-(4-methoxybutoxy)-benzamid;
(2S,4S,5S,7S)-N-(4-Amino-7-butylcarbamoyl-5-hydroxy-2-isopropyl-8-methyl-nonyl)-2-(4-methoxybutoxy)-4-(piperidin-1-yl)methyl-benzamid;
(2S,4S,5S,7S)-N-(4-Amino-7-butylcarbamoyl-5-hydroxy-2-isopropyl-8-methyl-nonyl)-2-(4-methoxybutoxy)-4-(pyrrolidin-1-yl)methyl-benzamid;
(2S,4S,5S,7S)-N-(4-Amino-7-butylcarbamoyl-5-hydroxy-2-isopropyl-8-methyl-nonyl)-2-(4-methoxybutoxy)-4-(2-piperidin-1 -ylethoxy)-benzamid;
(2S,4S,5S,7S)-N-(4-Amino-7-butylcarbamoyl-5-hydroxy-2-isopropyl-8-methyl-nonyl)-4-dimethylaminomethyl-2-(4-methoxybutoxy)-benzamid;
(2S,4S,5S,7S)-N-(4-Amino-7-butylcarbamoyl-5-hydroxy-2-isopropyl-8-methyl-nonyl)-2-(4-methoxybutoxy)-4-(4-methylpiperazin-1-yl)methyl-benzamid-trihydrochlorid (2S,4S,5S,7S)-N-(4-Amino-7-butylcarbamoyl-5-hydroxy-2-isopropyl-8-methyl-nonyl)-4-(4-acetylpiperazin-1-yl)methyl-2-(4-methoxybutoxy)-benzamid;
(2S,4S,5S,7S)-N-(4-Amino-7-butylcarbamoyl-5-hydroxy-2-isopropyl-8-methyl-nonyl)-2-(3-aminopropoxy)-benzamid;
(2S,4S,5S,7S)-N-(4-Amino-7-butylcarbamoyl-5-hydroxy-2-isopropyl-8-methyl-nonyl)-2-(2-aminoethoxy)-benzamid;
4S,5S,7S)-N-(4-Amino-7-butylcarbamoyl-5-hydroxy-2-isopropyl-8-methyl-nonyl)-2-[2-(4-acetylpiperazin-1-yl)-ethoxy]-benzamid;
(2S,4S,5S,7S)-N-(4-Amino-7-butylcarbamoyl-5-hydroxy-2-isopropyl-8-methyl-nonyl)-2-[2-(morpholin-4-yl)-ethyl]-benzamid;
(2S,4S,5S,7S)-N-(4-Amino-7-butyl carbamoyl-5-hydroxy-2-isopropyl-8-methyl-nonyl)-2-(3-dimethylaminopropoxy)-benzamid;
(2S,4S,5S,7S)-N-(4-Amino-7-butylcarbamoyl-5-hydroxy-2-isopropyl-8-methyl-nonyl)-2-[3-(morpholin-4-yl)-propoxy]-benzamid;
(2S,4S,5S,7S)-N-(4-Amino-7-butylcarbamoyl-5-hydroxy-2-isopropyl-8-methyl-nonyl)-2-[2-(morpholin-4-yl)-ethoxy]-benzamid;
(2S,4S,5S,7S)-N-(4-Amino-7-butylcarbamoyl-5-hydroxy-2-isopropyl-8-methyl-nonyl)-2-[2-(4-methoxypiperidin-1-yl)-ethyl]-benzamid;
(2S,4S,5S,7S)-N-(4-Amino-7-butyl carbamoyl-5-hydroxy-2-isopropyl-8-methyl-nonyl)-2-[2-(4-acetylpiperazin-1-yl)-ethyl]-benzamid;
(2S,4S,5S,7S)-4-Amino-5-hydroxy-2,7-diisopropyl-octandisäure-8-butylamid-1-[2-(3-methoxypropoxy)-benzyl]amid;
(2S,4S,5S,7S)-4-Amino-5-hydroxy-2,7-diisopropyl-octandisäure-8-butylamid-1-[3-(3-methoxypropoxy)-benzyl]amid;
(2S,4S,5S,7S)-4-Amino-5-hydroxy-2,7-diisopropyl-octandisäure-8-butylamid-1-[2-(4-methoxybutoxy)-benzyl]amid;
(2S,4S,5S,7S)-4-Amino-5-hydroxy-2,7-diisopropyl-octandisäure-8-butylamid-1-[2-(5-methoxypentoxy)-benzyl]amid;
(2S,4S,5S,7S)-N1-(4-Amino-7-butylcarbamoyl-5-hydroxy-2-isopropyl-8-methyl-nonyl)-N4-methyl-2-(4-methoxybutoxy)-terephthaldiamid;
(2S,4S,5S,7S)-N1-(4-Amino-7-butylcarbamoyl-5-hydroxy-2-isopropyl-8-methyl-nonyl)-N4-[2-morpholin-4-yl)-ethyl]-2-(4-methoxybutoxy)-terephthaldiamid-dihydrochlorid,c)
(2S,4S,5S,7S)-N1-(4-Amino-7-butylcarbamoyl-5-hydroxy-2-isopropyl-8-methyl-nonyl)-2-(4-methoxybutoxy)-terephthalsäurediamid;
(2S,4S,5S,7S)-N4-(4-Amino-7-butylcarbamoyl-5-hydroxy-2-isopropyl-8-methyl-nonyl)-3-(4-methoxybutoxy)-terephthalamidsäu re;
(2S,4S,5S,7S)-N-(4-Amino-7-butylcarbamoyl-5-hydroxy-2-isopropyl-8-methyl-nonyl)-4-butylcarbamoylmethoxy-2-(4-methoxybutoxy)-benzamid;
(2S,4S,SS,7S)-[4-(4-Amino-7-butylcarbamoyl-5-hydroxy-2-isopropyl-8-methyl-nonyl-carbamoyl)-3-(4-methoxybutoxy)-phenoxy]-essigsäure;
(2S,4S,5S,7S)-N-{4-Amino-5-hydroxy-2-isopropyl-8-methyl-7-[2-(morpholin-4-yl)-ethylcarbamoyl]-nonyl}-2-(4-methoxybutoxy)-4-[2-(morpholin-4-yl)-ethylcarbamoylmethoxy]-benzamid;
(2S,4S,5S,7S)-N-(4-Amino-7-butylcarbamoyl-5-hydroxy-2-isopropyl-8-methyl-nonyl)-2-(4-methoxybutoxy)-4-(1 H-tetrazol-5-ylmethoxy)-benzamid;
(2S,4S,5S,7S,2R')-N-[4-Amino-7-(2'-methylcarbamoyl-propylcarbamoyl)-5-hydroxy-2-isopropyl-8-methyl-nonyl]-2-(4-methoxybutoxy)-benzamid;
(2S,4S,5S,7S)-N-(4-Amino-7-[2-(dimethylaminocarbamoyl)-ethylcarbamoyl]-5-hydroxy-2-isopropyl-8-methyl-nonyl)2-(4-methoxybutoxy)-benzamid;
(2S,4S,5S,7S)-N-[4-Amino-7-(3-carbamoylpropylcarbamoyl)-5-hydroxy-2-isopropyl-8-methyl-nonyl]-2-(4-methoxybutoxy)-benzamid;
(2S,4S,5S,7S)-N-[4-Amino-7-(2-carbamoyl-2-methylpropylcarbamoyl)-5-hydroxy-2-isopropyl-8-methyl-nonyl]-2-(4-methoxybutoxy)-benzamid;
(2S,4S,5S,7S)-N-{4-Amino-5-hydroxy-2-isopropyl-8-methyl-7-[3-(morpholin-4-yl)-3-oxo-propylcarbamoyl]-nonyl}-2-(4-methoxybutoxy)-benzamid;
(2S,4S,5S,7S)-N-{7-[2-(4-Acetylpiperidin-1-yl)-ethylcarbamoyl]-4-amino-5-hydroxy-2-isopropyl-8-methyl-nonyl}-2-(4-methoxybutoxy)-benzamid;
(2S,4S,5S,7S)-N-[4-Amino-5-hydroxy-2-isopropyl-8-methyl-7-(2-thiomorpholin-4-ylethylcarbamoyl)-methyl-nonyl]-2-(4-methoxybutoxy)-benzamid;
(2S,4S,5S,7S)-N-(4-Amino-7-(2-carbamoyl-2-methylpropylcarbamoyl)-5-hydroxy-2-isopropyl-8-methyl-nonyl)-2-(4-methoxybutoxy)-4-(2-morpholin-4-ylethoxy-benzamid;
(2S,4S,5S,7S)-N-(4-Amino-7-(2-carbamoyl-2-methylpropylcarbamoyl)-5-hydroxy-2-isopropyl-8-methyl-nonyl)-2-(4-methoxybutoxy)-4-(morpholin-4-ylmethyl)-benzamid;
(2S,4S,5S,7S)-N-[4-Amino-7-(2-carbamoyl-2-methylpropylcarbamoyl)-5-hydroxy-2-isopropyl-8-methyl-nonyl]-2-(2-morpholin-4-ylethoxy)-benzamid;
(2S,4S,SS,7S)-N-{4-Amino-5-hydroxy-2-isopropyl-7-[2-(4-methoxycarbonylpiperidin-1-yl)-ethylcarbamoyl]-8-methyl-nonyl}-2-(4-methoxybutoxy)-benzamid;
(2S,4S,5S,7R)-N-[4-Amino-5-hydroxy-2-methyl-7-[(2-morpholin-4-ylethyl)-carbamoyl]-octyl}-2-(3-methoxypropoxy)-benzamid oder
(2S,4S,5S,7S)-N-{4-Amino-5-hydroxy-2-isopropyl-8-methyl-7-[2-(morpholin-4-yl)-ethylcarbamoyl]-nonyl}-4-carbamoylmethoxy-2-(4-methoxybutoxy)-benzamid oder jeweils ein Salz davon.

8. Verbindungen gemäss einem der Ansprüche 1 bis 7 zur Anwendung in einem Verfahren zur therapeutischen Behandlung des menschlichen oder tierischen Körpers.

9. Pharmazeutische Präparate, enthaltend eine Verbindung gemäss einem der Ansprüche 1 bis 8 oder eine pharmazeutisch verwendbares Salz davon.

10. Verfahren zur Herstellung von Verbindungen der Formel I 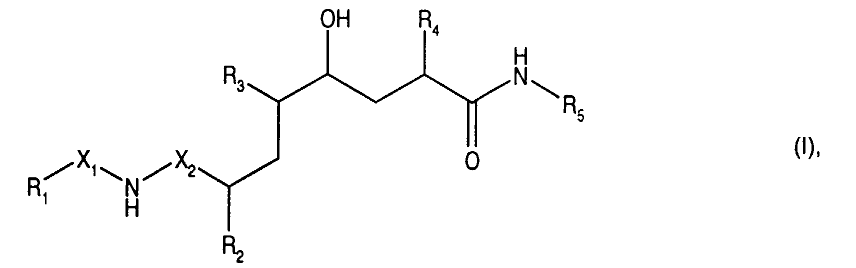 worin
R₁ einen 2-R_{A}-3-R_{B}-Phenylrest, 2-R_{A}-4-R_{C}-Phenylrest, 2-R_{A}-Pyridin-3-ylrest, 3-R_{A}-Pyddin-2-ylrest oder 1-R_{D}-Indol-3-ylrest bedeutet, wobei
einer der Reste R_{A} und R_{B} einen aliphatischen oder hetero cycloaliphatisch-aliphatischen Rest oder gegebenenfalls aliphatisch, araliphatisch oder heteroaraliphatisch verethertes Hydroxy und der andere Wasserstoff, einen aliphatischen Rest oder gegebenenfalls verestertes oder amidiertes Carboxy darstellt,
R_{C} Wasserstoff, einen aliphatischen Rest, gegebenenfalls aliphatisch, araliphatisch, heteroarylaliphatisch oder heterocycloaliphatisch-aliphatisch verethertes Hydroxy oder eine gegebenenfalls heteroaliphatisch substituierte Aminogruppe ist und
R_{D} für einen aliphatischen, araliphatischen oder heteroaliphatischen Rest steht, einer der Reste X₁ und X₂ Carbonyl und der andere Methylen darstellt,
R₂ ein aliphatischer Rest ist,
R₃ gegebenenfalls aliphatisch substituiertes Amino darstellt,
R₄ einen aliphatischen oder araliphatischen Rest bedeutet und
R₅ einen aliphatischen oder cycloaliphatisch-aliphatischen Rest oder einen gegebenenfalls hydrierten und/oder oxosubstituierten Heteroarylrest oder einen durch einen über ein C-Atom gebundenen und gegebenenfalls hydrierten und/oder oxosubstituierten Heteroaryl- oder Heteroaliphatylrest substituierten Niederalkylrest darstellt,und ihrer Salze, dadurch gekennzeichnet, daß man
a) eine Verbindung der Formel II 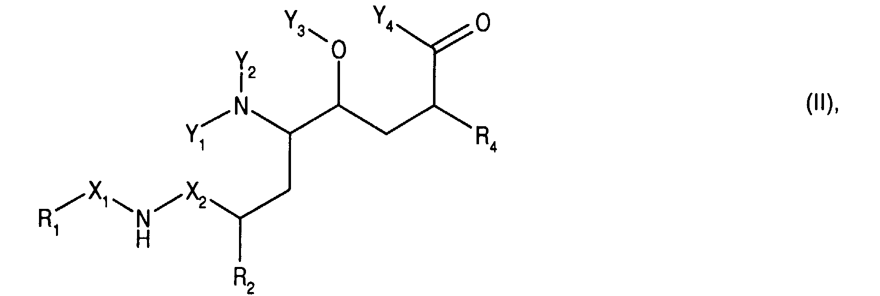 worin Y₁ Niederalkyl, Niederalkanoyl oder eine Aminoschutzgru ppe bedeutet, Y₂ Wasserstoff oder gemeinsam mit Y₃ eine bivalente Schutzgruppe darstellt, Y₃ Wasserstoff, eine Hydroxyschutzgruppe oder gemeinsam mit Y₂ eine bivalente Schutzgruppe darstellt bzw. gemeinsam mit Y₄ für eine direkte Bindung steht, Y₄ gegebenenfalls reaktionsfähig verethertes oder verestertes Hydroxy bedeutet oder gemeinsam mit Y₃ eine direkte Bindung darstellt und R₁, R₂, R₃, R₄, R₅, X₁ und X₂ die unter der Formel I angegebenen Bedeutungen haben, mit einem Amin der Formel III
H₂N-R₅ (III),
worin R₅ eine der unter der Formel I angegebenen Bedeutungen hat, unter Bildung einer Amidbindung umsetzt und vorhandene Schutzgruppen abspaltet, oder
b) Verbindungen der Formeln IV und V 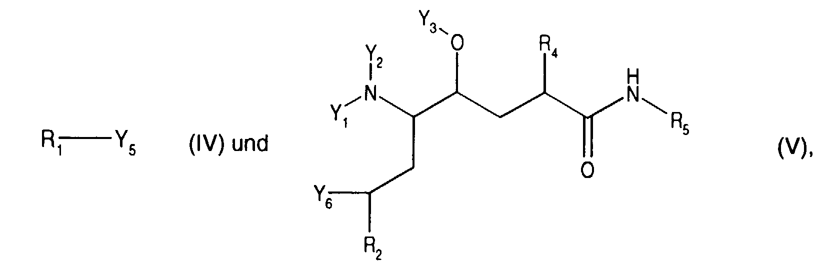 worin Y₁ Niederalkyl, Niederalkanoyl oder eine Aminoschutzgruppe bedeutet, Y₂ Wasserstoff oder gemeinsam mit Y₃ eine bivalente Schutzgruppe darstellt, Y₃ Wasserstoff, eine Hydroxyschutzgruppe oder gemeinsam mit Y₂ eine bivalente Schutzgruppe darstellt, einer der Reste Y₅ und Y₆ eine Aminomethylgruppe und der andere eine gegebenenfalls funktionell abgewandelte Carboxygruppe bedeutet und R₁, R₂, R₃, R₄ und R₅ die unter der Formel I angegebenen Bedeutungen haben, miteinander kondensiert und vorhandene Schutzgruppen abspaltet oder
c) zur Herstellung von Verbindungen der Formel I, worin R₃ Amino bedeutet, in einer Verbindung der Formel VI 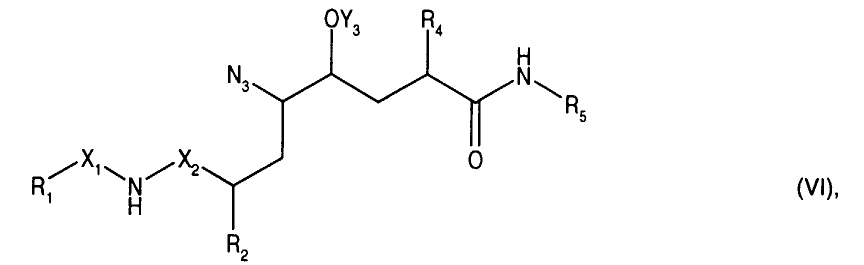 in der und R₁, R₂, R₄, R₅, X₁ und X₂ die unter der Formel I angegebenen Bedeutungen haben und Y₃ Wasserstoff oder eine Hydroxyschutzgruppe darstellt, die Azidogruppe zu Amino reduziert und vorhandene Schutzgruppen abspaltet und jeweils und gewünschtenfalls eine nach einem der vorstehend genannten Verfahren erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfinddungsgemäße Verbindung der Formel I in eine andere erfindungsgemäße Verbindung der Formel I umwandelt.

11. Verwendung einer Verbindung gemäss einem der Ansprüche 1 bis 8 zur Herstellung eines für die Behandlung von Bluthochdruck bestimmten Arzneimittels.
